# EUROPÄISCHE PATENTSCHRIFT

(11) **EP 1 966 218 B1**
(45) Veröffentlichungstag und Bekanntmachung des Hinweises auf die Patenterteilung: **14.07.2010**
(21) Anmeldenummer: 06840964.8
(22) Anmeldetag: 08.12.2006
(51) Int. Cl.: C07D 491/04, A61K 31/519, A61P 9/00

(54) **NEUE, ZYKLISCH SUBSTITUIERTE FUROPYRIMIDIN-DERIVATE UND IHRE VERWENDUNG ZUR BEHANDLUNG VON KARDIOVASKULÄREN ERKRANKUNGEN**
NOVEL, CYCLIC SUBSTITUTED FUROPYRIMIDINE DERIVATIVES AND USE THEREOF FOR TREATING CARDIOVASCULAR DISEASES
NOUVEAUX DERIVES DE FUROPYRIMIDINES A SUBSTITUTION CYCLIQUE ET LEUR UTILISATION POUR TRAITER DES MALADIES CARDIOVASCULAIRES

(30) Priorität: 21.12.2005 DE 102005061171
(43) Veröffentlichungstag der Anmeldung: 10.09.2008
(73) Patentinhaber: Bayer Schering Pharma Aktiengesellschaft, 13353 Berlin (DE)
(72) Erfinder: LAMPE, Thomas, 40545 Düsseldorf (DE); BECKER, Eva-Maria, 42117 Wuppertal (DE); KAST, Raimund, 42349 Wuppertal (DE); BECK, Hartmut, 50733 Köln (DE); JESKE, Mario, 42699 Solingen (DE); SCHUHMACHER, Joachim, 42113 Wuppertal (DE); STOLL, Friederike, 40215 Düsseldorf (DE); KLEIN, Martina, 40489 Düsseldorf (DE); AKBABA, Metin, 40880 Ratingen (DE); KNORR, Andreas, 40699 Erkrath (DE); STASCH, Johannes-Peter, 42651 Solingen (DE); BÄRFACKER, Lars, 46047 Oberhausen (DE); HILLISCH, Alexander, 42651 Solingen (DE); KARIG, Gunter, 65719 Hofheim am Taunus (DE); MEININGHAUS, Mark, 42327 Wuppertal (DE); SCHLEMMER, Karl-Heinz, 42113 Wuppertal (DE); SCHOHE-LOOP, Rudolf, 42327 Wuppertal (DE)
(86) Internationale Anmeldenummer: PCT/EP2006/011825
(87) Internationale Veröffentlichungsnummer: WO 2007/079861

(56) Entgegenhaltungen:
- WO-A-03/018589
- WO-A-2005/092896
- DE-A1- 10 148 883

## Beschreibung

Die vorliegende Anmeldung betrifft neue, cyclisch substituierte Furopyrimidin-Derivate, Verfahren zu ihrer Herstellung, sowie ihre Verwendung zur Herstellung von Arzneimitteln zur Behandlung und/oder Prophylaxe von Krankheiten, insbesondere zur Behandlung und/oder Prophylaxe von kardiovaskulären Erkrankungen.

Prostazyklin (PGI₂) gehört zur Familie der bioaktiven Prostaglandine, die Derivate der Arachidonsäure darstellen. PGI₂ ist das Hauptprodukt des Arachidonsäure-Stoffwechsels in Endothelzellen und hat potente gefäßerweiternde und anti-aggregatorische Eigenschaften. PGI₂ ist der physiolögische Gegenspieler von Thromboxan A₂ (TxA₂), einem starken Vasokonstriktor und Stimulator der Thrombozytenaggregation, und trägt somit zur Aufrechterhaltung der vaskulären Homeostase bei. Eine Reduktion der PGI₂-Spiegel ist vermutlich mitverantwortlich für die Entstehung verschiedener kardiovaskulärer Erkrankungen [Dusting, G.J. et al., Pharmac. Ther. 1990, 48: 323-344; Vane, J. et al., Eur. J. Vasc. Endovasc. Surg., 2003, 26: 571-578].

Nach Freisetzung der Arachidonsäure aus Phospholipiden über Phospholipasen A₂ wird PGI₂ durch Cyclooxygenasen und anschließend durch die PGI₂-Synthase synthetisiert. PGI₂ wird nicht gespeichert, sondern nach Synthese sofort freigesetzt, wodurch es lokal seine Wirkungen entfaltet. PGI₂ ist ein instabiles Molekül, welches schnell (Halbwertszeit ca. 3 Minuten) nicht-enzymatisch zu einem inaktiven Metaboliten, 6-Keto-Prostaglandin-F1alpha, umgelagert wird [Dusting, G.J. et al., Pharmac. Ther. 1990, 48: 323-344].

Die biologischen Effekte von PGI₂ kommen durch die Bindung an einen membranständigen Rezeptor, den sogenannten Prostacyclin- oder IP-Rezeptor [Narumiya, S. et al., Physiol. Rev. 1999, 79: 1193-1226], zustande. Der IP-Rezeptor gehört zu den G-Protein-gekoppelten Rezeptoren, die durch sieben Transmembrandomänen charakterisiert sind. Neben dem humanen IP-Rezeptor sind auch noch die Prostacyclin-Rezeptoren aus Ratte und Maus kloniert worden [Vane, J. et al., Eur. J. Vasc. Endovasc. Surg. 2003, 26: 571-578]. In den Glattmuskelzellen führt die Aktivierung des IP-Rezeptors zur Stimulation der Adenylatzyklase, die die Bildung von cAMP aus ATP katalysiert. Die Erhöhung der intrazellulären cAMP-Konzentration ist für die Prostacyclin-induzierte Vasodilatation sowie die Hemmung der Thrombozytenaggregation verantwortlich. Neben den vasoaktiven Eigenschaften wurden für PGI₂ noch anti-proliferative [Schroer, K. et al., Agents Actions Suppl. 1997, 48: 63-91; Kothapalli, D. et al., Mol. Pharmacol. 2003, 64: 249-258; Planchon, P. et al., Life Sci. 1995, 57: 1233-1240] und anti-arteriosklerotische Wirkungen beschrieben [Rudic, R.D. et al., Circ. Res. 2005, 96: 1240-1247; Egan K.M. et al., Science 2004, 114: 78.4-794]. Darüber hinaus wird die Metastasenbildung durch PGI₂ gehemmt [Schneider, M.R. et al., Cancer Metastasis Rev. 1994, 13: 349-64). Ob diese Effekte durch Stimulation der cAMP-Bildung oder durch eine IP-Rezeptor-vermittelte Aktivierung anderer Signaltransduktionswege in der jeweiligen Zielzelle [Wise, H. et al. TIPS 1996, 17: 17-21], wie z.B. der Phosphoinositidkaskade sowie von Kaliumkanälen, zustande kommen, ist unklar.

Obwohl die Wirkungen von PGI₂ insgesamt therapeutisch von Nutzen sind, ist ein klinische Verwendung von PGI₂ durch seine chemische und metabolische Instabilität stark eingeschränkt. Stabilere PGI₂-Analoga wie z.B. Iloprost [Badesch, D.B. et al., J. Am. Coll. Cardiol. 2004, 43: 56S-61 S] und Treprostinil [Chattaraj, S.C., Curr. Opion. Invest. Drugs 2002, 3: 582-586] konnten zwar zur Verfügung gestellt werden, allerdings ist die Wirkdauer dieser Verbindungen nach wie vor sehr kurz. Auch können die Substanzen nur über komplizierte Applikationswege dem Patienten verabreicht werden, wie z.B. durch Dauerinfusion, subkutan oder über mehrmalige Inhalationen. Diese Applikationswege können zudem zu zusätzlichen Nebenwirkungen, wie z.B. Infektionen oder Schmerzen an der Injektionsstelle, führen. Die Verwendung des bisher einzigen für den Patienten oral verfügbaren PGI₂-Derivates, Beraprost [Barst, R.J. et al., J. Am. Coll. Cardiol. 2003, 41: 2119-2125], ist wiederum durch seine kurze Wirkdauer limitiert.

Die in der vorliegenden Anmeldung beschriebenen Verbindungen sind im Vergleich zu PGI₂ chemisch und metabolisch stabile, nicht-prostanoide Aktivatoren des IP-Rezeptors, die die biologische Wirkung von PGI₂ nachahmen und somit zur Behandlung von Erkrankungen, insbesondere von kardiovaskulären Erkrankungen, eingesetzt werden können.

In DE 1 817 146, EP 1018 514, EP 1 132 093, WO 02/092603, WO 03/022852, WO 2005/092896, WO 2005/121149 und WO 2006/004658 werden verschiedene 4-Oxy-, 4-Thio- und/oder 4-Amino-furo[2,3-d]pyrimidin-Derivate und ihre Verwendung zur Behandlung von Erkrankungen beschrieben. In WO 03/018589 werden 4-Aminofuropyrimidine als Adenosinkinase-Inhibitoren zur Behandlung kardiovaskulärer Erkrankungen offenbart. Die Herstellung bestimmter 4-Aminofuropyrimidin-Derivate ist in Chemica Scripta 1986, 26 (2): 337-342, Yakugaku Zasshi 1969, 89 (10): 1434-1439 sowie Yakugaku Zasshi 1977, 97 (9): 1022-1033 publiziert. In WO 00/75145 werden Verbindungen mit einer bicyclischen Heteroaryl-Kernstruktur als Inhibitoren der Zelladhäsion beansprucht.

Die im Rahmen der vorliegenden Anmeldung beanspruchten Verbindungen zeichnen sich im Vergleich zu den Verbindungen aus dem Stand der Technik durch eine 5,6-Diphenylfuro[2,3-d]pyrimidin-Kernstruktur aus, die über die 4-Position in einem bestimmten räumlichen Abstand mit einer Carbonsäure- oder Carbonsäure-ähnlichen Funktionalität verknüpft ist.

Gegenstand der vorliegenden Erfindung sind Verbindungen der allgemeinen Formel (I) 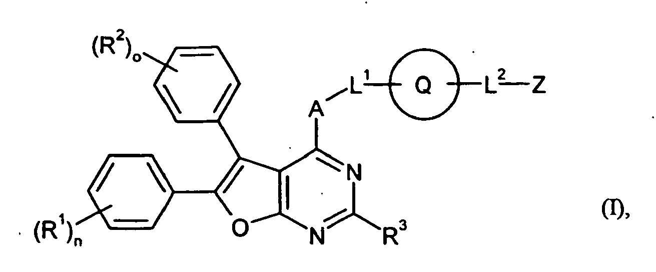 in welcher
- A: für O, S oder N-R⁴ steht, worin
R⁴ Wasserstoff, (C₁-C₆)-Alkyl, (C₃-C₇)-Cycloalkyl oder (C₄-C₇)-Cycloalkenyl bedeu- tet,
- L¹: für eine Bindung oder für (C₁-C₄)-Alkandiyl steht,
- der Ring Q: für (C₃-C₇)-Cycloalkyl, (C₄-C₇)Cycloalkenyl, einen 5- bis 7-gliedrigen Heterocyclus, Phenyl oder 5- oder 6-gliedriges Heteroaryl steht, welche jeweils bis zu zweifach, gleich oder verschieden, mit Fluor, Chlor, (C₁-C₄)-Alkyl, Trifluormethyl, Hydroxy, (C₁-C₄)- Alkoxy, Trifluormethoxy, Amino, Mono-(C₁-C₄)-alkylamino und/oder Di-(C₁-C₄)-alkyl- amino substituiert sein können,
wobei (C₁-C₄)-Alkyl seinerseits mit Hydroxy, (C₁-C₄)-Alkoxy, Amino, Mono- oder Di-(C₁- C₄)-alkylamino substituiert sein kann,
- L²: für (C₁-C₄)-Alkandiyl, welches ein- oder zweifach mit Fluor substituiert und in welchem eine Methylengruppe gegen O oder N-R⁵, worin
R⁵ Wasserstoff, (C₁-C₆)-Alkyl oder (C₃-C₇)-Cycloalkyl bedeutet,
ausgetauscht sein kann,
oder für (C₂-C₄)-Alkendiyl steht,
- Z: für eine Gruppe der Formel 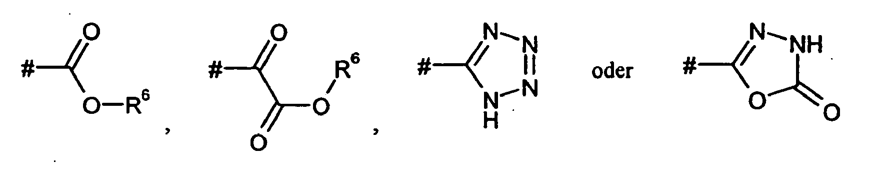 steht, worin
# die Verknüpfungsstelle mit der Gruppe L²
und
R⁶ Wasserstoff oder (C₁-C₄)-Alkyl bedeutet,
- R) und R²: unabhängig voneinander für einen Substituenten ausgewählt aus der Reihe Halogen, Cyano, Nitro, (C₁-C₆)Alkyl, (C₂-C₆)-Alkenyl, (C₂-C₄)-Alkinyl, (C₃-C₇)-Cycloalkyl, (C₄- C₇₎-Cycloalkenyl, (C₁-C₆)-Alkoxy, Trifluormethyl, Trifluormethoxy, (C₁-C₆)-Alkylthio, (C₁-C₆)-Acyl, Amino, Mono-(C₁-C₆)-alkylamino, Di-(C₁-C₆)-alkylamino und (C₁-C₆)-Acyl amino stehen,
wobei (C₁-C₆)-Alkyl und (C₁-C₆)-Alkoxy ihrerseits jeweils mit Cyano, Hydroxy, (C₁-C₄)- Alkoxy, (C₁-C₄)-Alkylthio, Amino, Mono- oder Di-(C₁-C₄)-alkylamino substituiert sein können,
oder
zwei an benachbarte Kohlenstoffatome des jeweiligen Phenylrings gebundene Reste R¹ und/oder R² zusammen eine Gruppe der Formel -O-CH₂-O-, -O-CHF-O-, -O-CF₂.O-, -O-CH₂-CH₂-O- oder -O-CF₂-CF₂-O- bilden,
- n und o: unabhängig voneinander für die Zahl 0, 1, 2 oder 3 stehen, wobei für den Fall, dass R¹ oder R² mehrfach auftreten, ihre Bedeutungen jeweils gleich oder verschieden sein können,
- und:
- R³: für Wasserstoff, (C₁-C₄)-Alkyl oder Cyclopropyl steht,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Trisethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Insbesondere umfasst die vorliegende Erfindung bei den Verbindungen der Formel (I), in welcher
- Z: für eine Gruppe der Formel 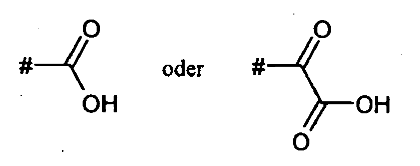 steht,
auch hydrolysierbare Ester-Derivate dieser Verbindungen. Hierunter werden Ester verstanden, die in physiologischen Medien, unter den Bedingungen der im weiteren beschriebenen biologischen Tests und insbesondere *in vivo* auf enzymatischem oder chemischem Wege zu den freien Carbonsäuren, als den biologisch hauptsächlich aktiven Verbindungen, hydrolysiert werden können. Als solche Ester werden (C₁-C₄)-Alkylester, in welchen die Alkylgruppe geradkettig oder verzweigt sein kann, bevorzugt. Besonders bevorzugt sind Methyl- oder Ethylester (siehe auch entsprechende Definitionen des Restes R⁶).

Im Rahmen der vorliegenden Erfindung haben die Substituenten, soweit nicht anders spezifiziert, die folgende Bedeutung:
(C₁-C₆)-Alkyl, (C₁-C₅)-Alkyl, (C₁-C₄)-Alkyl und (C₁-C₃)-Alkyl, stehen im Rahmen der Erfindung für einen geradkettigen oder verzweigten Alkylrest mit 1 bis 6, 1 bis 5, 1 bis 4 bzw. 1 bis 3 Kohlenstoffatomen. Bevorzugt ist ein geradkettiger oder verzweigter Alkylrest mit 1 bis 4, besonders bevorzugt mit 1 bis 3 Kohlenstoffatomen. Beispielhaft und vorzugsweise seien genannt: Methyl, Ethyl, n-Propyl, Isopropyl, n-Butyl, *iso*-Butyl, *sec*.-Butyl, *tert.-*Butyl, 1-Ethylpropyl, n-Pentyl und n-Hexyl..
(C₂-C₆)₋Alkenyl und (C₂-C₅)-Alkenyl stehen im Rahmen der Erfindung für einen geradkettigen oder verzweigten Alkenylrest mit 2 bis 6 bzw. 2 bis 5 Kohlenstoffatomen und einer oder zwei Doppelbindungen. Bevorzugt ist ein geradkettiger oder verzweigter Alkenylrest mit 2 bis 5 Kohlenstoffatomen und einer Doppelbindung. Beispielhaft und vorzugsweise seien genannt: Vinyl, Allyl, Isopropenyl und n-But-2-en-1-yl.
(C₂-C₄-Alkinyl steht im Rahmen der Erfindung für einen geradkettigen oder verzweigten Alkinylrest mit 2 bis 4 Kohlenstoffatomen und einer Dreifachbindung. Bevorzugt ist ein geradkettiger Alkinylrest mit 2 bis 4 Kohlenstoffatomen. Beispielhaft und vorzugsweise seien genannt: Ethinyl, n-Prop-1-in-1-yl, n-Prop-2-in-1-yl, n-But-2-in-1-yl und n-But-3-in-1-yl.
(C₁-C₄)-Alkandiyl und (C₁-C₃)-Alkandiyl stehen im Rahmen der Erfindung für einen geradkettigen oder verzweigten divalenten Alkylrest mit 1 bis 4 bzw. 1 bis 3 Kohlenstoffatomen. Bevorzugt ist jeweils ein geradkettiger Alkandiylrest mit 1 bis 4 bzw. 1 bis 3 Kohlenstoffatomen. Beispielhaft und vorzugsweise seien genannt: Methylen, 1,2-Ethylen, Ethan-1,1-diyl, 1,3-Propylen, Propan-1,1-diyl, Propan-1,2-diyl, Propan-2,2-diyl, 1,4-Butylen, Butan-1,2-diyl, Butan-1,3-diyl und Butan-2,3-diyl.
(C₂-C₄)-Alkendiyl und (C₂-C₃)-Alkendiyl stehen im Rahmen der Erfindung für einen geradkettigen oder verzweigten divalenten Alkenylrest mit 2 bis 4 bzw. 2 bis 3 Kohlenstoffatomen und bis zu 2 Doppelbindungen. Bevorzugt ist jeweils ein geradkettiger Alkendiylrest mit 2 bis 4 bzw. 2 bis 3 Kohlenstoffatomen und einer Doppelbindung. Beispielhaft und vorzugsweise seien genannt: Ethen-1,1-diyl, Ethen-1,2-diyl, Propen-1,1-diyl, Propen-1,2-diyl, Propen-1,3-diyl, But-1-en-1,4-diyl, But-1-en-1,3-diyl, But-2-en-1,4-diyl und Buta-1,3-dien-1,4-diyl.
(C₁-C₆)-Alkoxy und (C₁-C₄)-Alkoxy stehen im Rahmen der Erfindung für einen geradkettigen oder verzweigten Alkoxyrest mit 1 bis 6 bzw. 1 bis 4 Kohlenstoffatomen. Bevorzugt ist ein geradkettiger oder verzweigter Alkoxyrest mit 1 bis 4 Kohlenstoffatomen. Beispielhaft und vorzugsweise seien genannt: Methoxy, Ethoxy, n-Propoxy, Isopropoxy, n-Butoxy, *tert*.-Butoxy, n-Pentoxy und n-Hexoxy.
(C₁-C₆-Alkylthio und (C₁-C₄)-Alkylthio stehen im Rahmen der Erfindung für einen geradkettigen oder verzweigten Alkylthiorest mit 1 bis 6 bzw. 1 bis 4 Kohlenstoffatomen. Bevorzugt ist ein geradkettiger oder verzweigter Alkylthiorest mit 1 bis 4 Kohlenstoffatomen. Beispielhaft und vorzugsweise seien genannt: Methylthio, Ethylthio, n-Propylthio, Isopropylthio, n-Butylthio, *tert*.-Butylthio, n-Pentylthio und n-Hexylthio.
(C₁-C₆)-Acyl [(C₁-C₆)-Alkanoyl], (C₁-C₅)Acyl [(C₁-C₅)-Alkanoyl] und C₁-C₄-Acyl [(C₁-C₄)-Alkanoyl] stehen im Rahmen der Erfindung für einen geradkettigen oder verzweigten Alkylrest mit 1 bis 6, 1 bis 5 bzw. 1 bis 4 Kohlenstoffatomen, der in der 1-Position ein doppelt gebundenes Sauerstoffatom trägt und über die 1-Position verknüpft ist. Bevorzugt ist ein geradkettiger oder verzweigter Acylrest mit 1 bis 4 Kohlenstoffatomen. Beispielhaft und vorzugsweise seien genannt: Formyl, Acetyl, Propionyl, n-Butyryl, iso-Butyryl und Pivaloyl.
Mono-(C₁-C₆)-alkylamino und Mono-(C₁-C₄)-alkylamino stehen im Rahmen der Erfindung für eine Amino-Gruppe mit einem geradkettigen oder verzweigten Alkylsubstituenten, der 1 bis 6 bzw. 1 bis 4 Kohlenstoffatome aufweist. Bevorzugt ist ein geradkettiger oder verzweigter Monoalkylamino-Rest mit 1 bis 4 Kohlenstoffatomen. Beispielhaft und vorzugsweise seien genannt: Methylamino, Ethylamino, n-Propylamino, Isopropylamino und tert.-Butylamino.
Di-(C₁-C₆)-alkylamino und Di-(C₁-C₄)-alkylamino stehen im Rahmen der Erfindung für eine Amino-Gruppe mit zwei gleichen oder verschiedenen geradkettigen oder verzweigten Alkylsubstituenten, die jeweils 1 bis 6 bzw. 1 bis 4 Kohlenstoffatome aufweisen. Bevorzugt sind geradkettige oder verzweigte Dialkylamino-Reste mit jeweils 1 bis 4 Kohlenstoffatomen. Beispielhaft und vorzugsweise seien genannt: *N*,*N*-Dimethylamino, *N*,*N*-Diethylamino, *N*-Ethyl-*N*-methylamino, *N*-Methyl-*N*-n-propylamino, *N*-Isopropyl-*N*-n-propylamino, *N*-tert.-Butyl-*N*-methylamino, *N*-Ethyl-*N*-n-pentylamino und *N*-n-Hexyl-N-methylamino.
(C₁-C₆)-Acylamino und (C₁-C₄)-Acylamino stehen im Rahmen der Ereindung für eine Amino-Gruppe mit einem geradkettigen oder verzweigten Acyl-Substituenten, der 1 bis 6 bzw. 1 bis 4 Kohlenstoffatome aufweist und über die Carbonylgruppe verknüpft ist. Bevorzugt ist ein Acylamino-Rest mit 1 bis 4 Kohlenstoffatomen. Beispielhaft und vorzugsweise seien genannt: Formamido, Acetamido, Propionamido, n-Butyramido und Pivaloylamido.
(C₃-C₇)-Cycloalkyl und (C₃-C₆)-Cycloalkyl stehen im Rahmen der Erfindung für eine monocyclische, gesättigte Cycloalkylgruppe mit 3 bis 7 bzw. 3 bis 6 Kohlenstoffatomen. Bevorzugt ist ein Cycloalkylrest mit 3 bis 6 Kohlenstoffatomen. Beispielhaft und vorzugsweise seien genannt: Cyclopropyl, Cyclobutyl, Cyclopentyl, Cyclohexyl und Cycloheptyl.
(C₄-C₇)-Cycloalkenyl, (C₄-C₆)-Cycloalkenyl und (C₅-C₆)-Cycloalkenyl stehen im Rahmen der Erfindung für eine monocyclische Cycloalkylgruppe mit 4 bis 7, 4 bis 6 bzw. 5 oder 6 Kohlenstoffatomen und einer Doppelbindung. Bevorzugt ist ein Cycloalkenylrest mit 4 bis 6, besonders bevorzugt mit 5 oder 6 Kohlenstoffatomen. Beispielhaft und vorzugsweise seien genannt: cyclobutenyl, Cyclopentenyl, Cyclohexenyl und Cycloheptenyl.
Ein 5- bis 7-gliedriger Heterocyclus steht im Rahmen der Erfindung für einen gesättigten oder partiell ungesättigten Heterocyclus mit 5 bis 7 Ringatomen, der ein oder zwei Ring-Heteroatome aus der Reihe N und/oder O enthält und über Ring-Kohlenstoffatome und/oder gegebenenfalls Ring-Stickstoffatome verknüpft ist. Bevorzugt ist ein 5- oder 6-gliedriger gesättigter Heterocyclus mit ein oder zwei Ring-Heteroatomen aus der Reihe N und/oder O. Beispielhaft seien genannt: Pyrrolidinyl, Pyrrolinyl, Pyrazolidinyl, Tetrahydrofuranyl, Piperidinyl, Piperazinyl, Tetrahydropyranyl, Morpholinyl, Hexahydroazepinyl und Hexahydro-1,4-diazepinyl. Bevorzugt sind Pyrrolidinyl, Tetrahydrofuranyl, Piperidinyl, Piperazinyl und Tetrahydropyranyl.
5- oder 6-gliedriges Heteroaryl steht im Rahmen der Erfindung für einen aromatischen Heterocyclus (Heteroaromaten) mit 5 oder 6 Ringatomen, der ein oder zwei Ring-Heteroatome aus der Reihe N, O und/oder S enthält und über Ring-Kohlenstoffatome und/oder gegebenenfalls ein RingStickstoffatom verknüpft ist. Beispielhaft seien genannt: Furyl, Pyrrolyl, Thienyl, Pyrazolyl, Imidazolyl, Thiazolyl, Oxazolyl, Isoxazolyl, Isothiazolyl, Pyridyl, Pyrimidinyl, Pyridazinyl und Pyrazinyl. Bevorzugt sind 6-gliedrige Heteroaryl-Reste wie beispielsweise Pyridyl, Pyrimidinyl, Pyridazinyl und Pyrazinyl.
Halogen schließt im Rahmen der Erfindung Fluor, Chlor, Brom und Iod ein. Bevorzugt sind Chlor oder Fluor.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem Substituenten.

Bevorzugt im Rahmen der vorliegenden Erfindung sind Verbindungen der Formel (I), in welcher
- A: für O, S oder N-R⁴ steht, worin
R⁴ Wasserstoff, (C₁-C₆)-Alkyl, (C₃-C₇)-Cycloalkyl oder (C₄-C₇)-Cycloalkenyl bedeu- tet,
- L': für eine Bindung oder für (C₁-C₄)-Alkandiyl steht,
- der Ring Q: für (C₃-C₇)-Cycloalkyl, (C₄-C₇)-Cycloalkenyl, einen 5- bis 7-gliedrigen Heterocyclus, Phenyl oder 5- oder 6-gliedriges Heteroaryl steht, welche jeweils bis zu zweifach, gleich oder verschieden, mit Fluor, Chlor, (C₁-C₄)-Alkyl, Trifluormethyl, Hydroxy, (C₁-C₄)- Alkoxy, Trifluormethoxy, Amino, Mono-(C₁-C₄)-alkylamino und/oder Di-(C₁-C₄)-alkyl- amino substituiert sein können,
wobei (C₁-C₄)-Alkyl seinerseits mit Hydroxy, (C₁-C₄)-Alkoxy, Amino, Mono- oder Di-(C₁- C₄)-alkylamino substituiert sein kann,
- L²: für (C₁-C₄)Alkandiyl, welches ein- oder zweifach mit Fluor substituiert und in welchem eine Methylengruppe gegen O oder N-R⁵, worin
R⁵ Wasserstoff, (C₁-C₆)-Alkyl oder (C₃-C₇)-Cycloalkyl bedeutet,
ausgetauscht sein kann,
oder für (C₂-C₄)-Alkendiyl steht,
- Z: für eine Gruppe der Formel 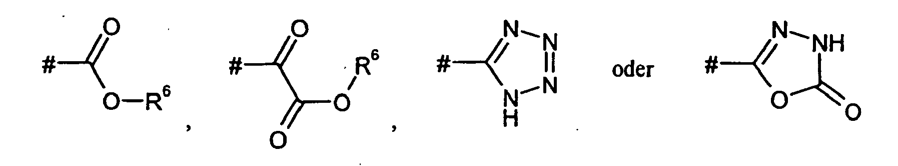 steht, worin
# die Verknüpfungsstelle mit der Gruppe L²
und
R⁶ Wasserstoff oder (C₁-C₄)-Alkyl bedeutet,
- R¹ und R²: unabhängig voneinander für einen Substituenten ausgewählt aus der Reihe Halogen, Cyano, Nitro, (C₁-C₆)-Alkyl, (C₂-C₆)-Alkenyl, (C₂-C₄)-Alkinyl, (C₃-C₇)-Cycloalkyl, (C₄- C₇)-Cycloalkenyl, (C₁-C₆)-Alkoxy, Trifluormethyl, Trifluormethoxy, (C₁-C₆)-Alkylthio, (C₁-C₆)-Acyl, Amino, Mono-(C₁-C₆)-alkylamino, Di-(C₁-C₆)-alkylamino und (C₁-C₆)-Acyl amino stehen,
wobei (C₁-C₆)-Alkyl und (C₁-C₆)-Alkoxy ihrerseits jeweils mit Hydroxy, (C₁-C₄)-Alkoxy, Amino, Mono- oder Di-(C₁-C₄)-alkylamino substituiert sein können,
oder
zwei an benachbarte Kohlenstoffatome des jeweiligen Phenylrings gebundene Reste R¹ und/oder R² zusammen eine Gruppe der Formel -O-CH₂-O-, -O-CHF-O-, -O-CF₂-O-, -O-CH₂-CH₂-O- oder -O-CF₂-CF₂-O- bilden,
- n und o: unabhängig voneinander für die Zahl 0, 1, 2 oder 3 stehen,
wobei für den Fall, dass R¹ oder R² mehrfach auftreten, ihre Bedeutungen jeweils gleich oder verschieden sein können,
- und:
- R³: für Wasserstoff, (C₁-C₄)-Alkyl oder Cyclopropyl steht,
sowie ihre Salze, Solvate und Solvate der Salze.

Besonders bevorzugt im Rahmen der vorliegenden Erfindung sind Verbindungen der Formel (I), in welcher
- A: für O oder N-R⁴ steht, worin
R⁴ Wasserstoff, (C₁-C₄)-Alkyl oder (C₃-C₆)-Cycloalkyl bedeutet,
- L¹: für eine Bindung oder für (C₁-C₃)-Alkandiyl steht,
- der Ring Q: für (C₃-C₆)-Cycloalkyl, (C₄-C₆)-Cycloalkenyl, einen 5- oder 6-gliedrigen Heterocyclus, Phenyl oder 5- oder 6-gliedriges Heteroaryl steht, welche jeweils bis zu zweifach, gleich oder verschieden, mit Fluor, Chlor, (C₁-C₃)-Alkyl, Trifluormethyl, Hydroxy, Methoxy, Ethoxy, Trifluormethoxy, Amino, Methylamino, Ethylamino, Dimethylamino und/oder Diethylamino substituiert sein können,
wobei (C₁-C₃)-Alkyl seinerseits mit Hydroxy, Methoxy, Ethoxy, Amino, Methylamin, Ethylamino, Dimethylamino oder Diethylamino substituiert sein kann,
- L²: für (C₁-C₃)-Alkandiyl, welches ein- oder zweifach mit Fluor substituiert sein kann, für (C₂- C₃)-Alkendiyl oder für eine Gruppe der Formel *-M-CR⁷R⁸-, *-M-CH₂-CR⁷R⁸- oder *-CH₂-M-CR⁷R⁸- steht, worin
* die Verknüpfungsstelle mit dem Ring Q,
M O oder N-R⁵, worin
R⁵ Wasserstoff, (C₁-C₃)-Alkyl oder Cyclopropyl darstellt,
und
R⁷ und R⁸ unabhängig voneinander Wasserstoff oder Fluor bedeuten,
- Z: für eine Gruppe der Formel 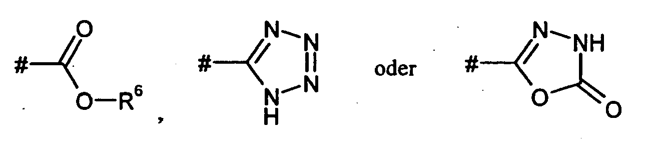 steht, worin
# die Verknüpfungsstelle mit der Gruppe L²
und
R⁶ Wasserstoff, Methyl oder Ethyl bedeutet,
- R¹ und R²: unabhängig voneinander für einen Substituenten ausgewählt aus der Reihe Fluor, Chlor, Cyano, (C₁-C₅)-Alkyl, (C₂-C₅)-Alkenyl, (C₃-C₆)-Cycloalkyl, (C₄-C₆)-Cycloalkenyl, (C₁-C₄)- Alkoxy, Trifluormethyl, Trifluormethoxy, (C₁-C₄)-Alkylthio, (C₁-C₅)-Acyl, Amino, Mono- (C₁-C₄)-alkylamino, Di-(C₁-C₄)-alkylamino und (C₁-C₄)-Acylamino stehen,
oder
zwei an benachbarte Kohlenstoffatome des jeweiligen Phenylrings gebundene Reste R¹ und/oder R² zusammen eine Gruppe der Formel -O-CH₂-O-, -O-CHF-O- oder -O-CF₂-O- bilden,
- n und o: unabhängig voneinander für die Zahl 0, 1, 2 oder 3 stehen,
wobei für den Fall, dass R¹ oder R² mehrfach auftreten, ihre Bedeutungen jeweils gleich oder verschieden sein können,
- und:
- R³: für Wasserstoff oder (C₁-C₃)-Alkyl steht,
sowie ihre Salze, Solvate und Solvate der Salze.

Ganz besonders bevorzugt im Rahmen der vorliegenden Erfindung sind Verbindungen der Formel (I), in welcher
- A: für O oder N-R⁴ steht, worin
R⁴ Wasserstoff oder (C₁-C₄)-Alkyl bedeutet,
- L¹: für eine Bindung oder für (C₁-C₃)-Alkandiyl steht,
- der Ring Q: für (C₄-C₆)-Cycloalkyl, (C₅-C₆)-Cycloalkenyl, einen 5- oder 6-gliedrigen Heterocyclus oder Phenyl steht, welche jeweils bis zu zweifach, gleich oder verschieden, mit Fluor, Chlor, (C₁-C₃)-Alkyl, Trifluormethyl, Hydroxy, Methoxy, Ethoxy, Trifluormethoxy, Amino, Methylamino, Ethylamino, Dimethylamino und/oder Diethylamino substituiert sein können,
- L²: für (C₁-C₃)-Alkandiyl, welches ein- oder zweifach mit Fluor substituiert sein kann, für (C₂- C₃)-Alkendiyl oder für eine Gruppe der Formel *-M-CR⁷R⁸-, *-M-CH₂-CR⁷R⁸- oder *-CH₂-M-CR⁷R⁸- steht, worin
* die Verknüpfungsstelle mit dem Ring Q,
M O oder N-R⁵, worin
R⁵ Wasserstoff oder (C₁-C₃)-Alkyl darstellt, und
R⁷ und R⁸ unabhängig voneinander Wasserstoff oder Fluor
bedeuten,
- Z: für eine Gruppe der Formel 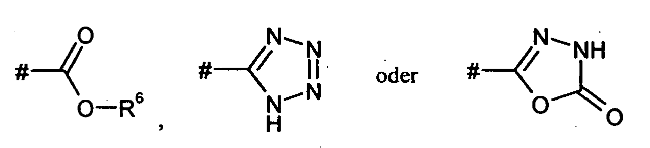 steht, worin
# die Verknüpfungsstelle mit der Gruppe L²
und
R⁶ Wasserstoff, Methyl oder Ethyl bedeutet,
- R¹ und R²: unabhängig voneinander für einen Substituenten ausgewählt aus der Reihe Fluor, Chlor, Cyano, (C₁-C₅)-Alkyl, (C₂-C₅)-Alkenyl, (C₃-C₆)-Cycloalkyl, (C₄-C₆)-Cycloalkenyl, (C₁-C₄)- Alkoxy, Trifluormethyl, Trifluormethoxy, (C₁-C₄)-Alkylthio, (C₁-C₅)-Acyl, Amino; Mono- (C₁-C₄)-alkylamino, Di-(C₁-C₄)-alkylamino und (C₁-C₄)-Acylamino stehen,
oder
zwei an benachbarte Kohlenstoffatome des jeweiligen Phenylrings gebundene Reste R¹ und/oder R² zusammen eine Gruppe der Formel -O-CH₂-O-, -O-CHF-O- oder -O-CF₂-O- bilden,
- n und o: unabhängig voneinander für die Zahl 0, 1 oder 2 stehen,
wobei für den Fall, dass R¹ oder R² zweifach auftreten, ihre Bedeutungen jeweils gleich oder verschieden sein können,
- und:
- R³: für Wasserstoff oder (C₁-C₃)-Alkyl steht,
sowie ihre Salze, Solvate und Solvate der Salze.

Von besonderer Bedeutung im Rahmen der vorliegenden Erfindung sind Verbindungen der Formel (I), in welcher
- A: für O oder NH steht,
- L¹: für eine Bindung, Methylen, Ethan-1,1-diyl oder Ethan-1,2-diyl steht,
- der Ring Q: für Cyclobutyl, Cyclopentyl, Cyclopentenyl, Cyclohexyl, Pyrrolidinyl, Piperidinyl, Tetrahydrofuranyl, Tetrahydropyranyl, Morpholinyl oder Phenyl steht, welche jeweils bis zu zweifach, gleich oder verschieden, mit Fluor, Methyl, Ethyl, Trifluormethyl, Hydroxy, Methoxy, Ethoxy, Amino, Methylamino und/oder Dimethylamino substituiert sein können,
- L²: für (C₁-C₃)-Alkandiyl, (C₂-C₃)-Alkendiyl oder eine Gruppe der Formel *-M-CH₂- oder *-M-CH₂-CH₂- steht, worin
* die Verknüpfungsstelle mit dem Ring Q
und
M O oder N-R⁵, worin
R⁵ Wasserstoff oder (C₁-C₃)-Alkyl darstellt, bedeutet,
- Z: für eine Gruppe der Formel 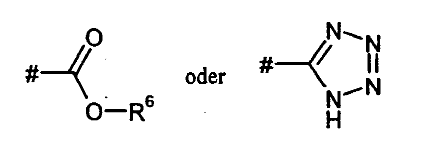 steht, worin
# die Verknüpfungsstelle mit der Gruppe L²
und
R⁶ Wasserstoff, Methyl oder Ethyl bedeutet,
- R¹ und R²: unabhängig voneinander für einen Substituenten ausgewählt aus der Reihe Fluor, Chlor, Cyano, (C₁-C₅)-Alkyl, (C₂-C₅)-Alkenyl, (C₃-C₆)-Cycloalkyl, (C₄-C₆)-Cycloalkenyl, (C₁-C₄). Alkoxy, Trifluormethyl, Trifluormethoxy, (C₁-C₄)-Alkylthio, (C₁-C₅)-Acyl, Amino, Mono- (C₁-C₄)-alkylamino, Di-(C₁-C₄)-alkylamino und (C₁-C₄)-Acylamino stehen,
oder
zwei an benachbarte Kohlenstoffatome des jeweiligen Phenylrings gebundene Reste R¹ und/oder R² zusammen eine Gruppe der Formel -O-CH₂-O-, -O-CHF-O- oder -O-CF₂-O- bilden,
- n und o: unabhängig voneinander für die Zahl 0, 1 oder 2 stehen, wobei für den Fall, dass R¹ oder R² zweifach auftreten, ihre Bedeutungen jeweils gleich oder verschieden sein können,
- und:
- R³: für Wasserstoff steht,
sowie ihre Salze, Solvate und Solvate der Salze.

Von ganz besonderer Bedeutung im Rahmen der vorliegenden Erfindung sind Verbindungen der Formel (I), in welcher
- A: für O oder NH steht,
- L¹ für: eine Bindung, Methylen oder Ethan-1,1-diyl steht,
- der Ring Q: für Cyclobutyl, Cyclopentyl, Cyclopentenyl, Cyclohexyl, Pyrrolidinyl, Piperidinyl oder Phenyl steht, welche jeweils bis zu zweifach, gleich oder verschieden, mit Fluor, Methyl, Hydroxy und/oder Methoxy substituiert sein können,
- L²: für (C₁-C₃)-Alkandiyl, (C₂-C₃)-Alkendiyl oder eine Gruppe der Formel *-M-CH₂- oder *-M-CH₂-CH₂- steht, worin
* die Verknüpfungsstelle mit dem Ring Q
und
M O oder NH bedeutet,
- Z: für eine Gruppe der Formel 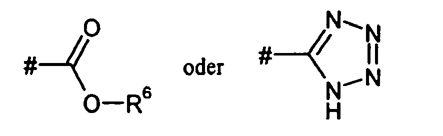 steht, worin
# die Verknüpfungsstelle mit der Gruppe L²
und
R⁶ Wasserstoff, Methyl oder Ethyl bedeutet,
- R¹: für einen Substituenten ausgewählt aus der Reihe Fluor, Chlor, Methyl, Ethyl, Vinyl, Tri- fluormethyl und Methoxy steht,
- R²: für einen Substituenten ausgewählt aus der Reihe Fluor, Chlor, Cyano, Methyl, Ethyl, n- Propyl, Vinyl, Trifluormethyl, Methoxy, Ethoxy, Trifluormethoxy, Methylthio, Ethylthio, Amino, Methylamino und Ethylamino steht,
- n und o: unabhängig voneinander für die Zahl 0, 1 oder 2 stehen,
wobei für den Fall, dass R¹ oder R² zweifach auftreten, ihre Bedeutungen jeweils gleich oder verschieden sein können,
- und:
- R³: für Wasserstoff steht,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I), in welcher Z für -COOH oder -C(=O)-COOH steht, dadurch gekennzeichnet, dass man entweder

### [A] Verbindungen der Formel (II)

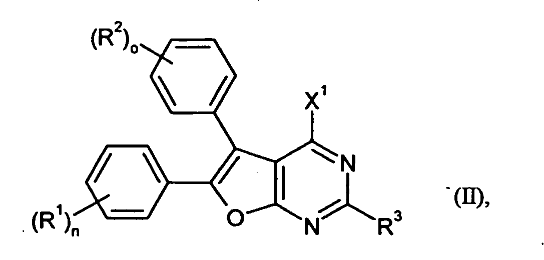

in welcher R¹, R², R³, n und o jeweils die oben angegebenen Bedeutungen haben und
- X¹: für eine Abgangsgruppe wie beispielsweise Halogen, insbesondere für Chlor steht,
in Gegenwart einer Base gegebenenfalls in einem inerten Lösungsmittel mit einer Verbin- dung der Formel (III) 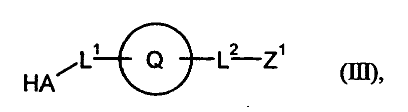 in welcher A, L¹, L²und Q jeweils die oben angegebenen Bedeutungen haben
und
- Z¹: für Cyano oder eine Gruppe der Formel -[C(O)]_{y}-COOR^{6A} steht, worin
y die Zahl 0 oder 1
und und
R^{6A} (C₁-C₄)-Alkyl bedeutet,
zu Verbindungen der Formel (IV) 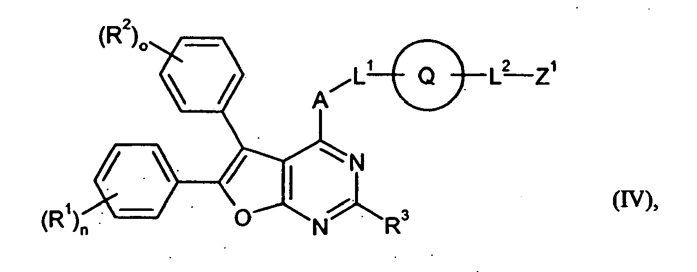 in welcher A, L¹, L², Q, Z¹, R¹, R², R³, n und o jeweils die oben angegebenen Bedeutungen haben,
umsetzt
oder

### [B] Verbindungen der Formel (V-1)

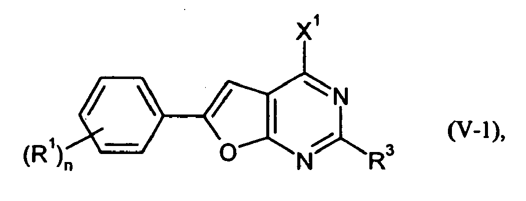

in welcher R¹, R³, X¹ und n jeweils die oben angegebenen Bedeutungen haben,
in Gegenwart einer Base gegebenenfalls in einem inerten Lösungsmittel mit einer Verbindung der Formel (III) zu Verbindungen der Formel (VI-1) 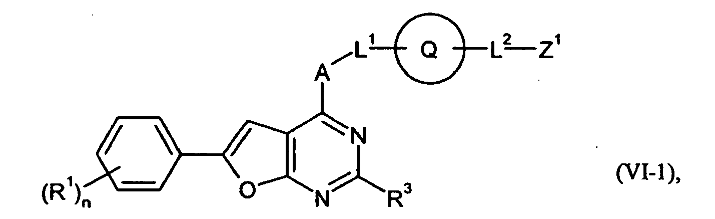 in welcher A, L¹, L², Q, Z¹, R¹, R³ und n jeweils die oben angegebenen Bedeutungen haben,
umsetzt, dann in einem inerten Lösungsmittel beispielsweise mit *N*-Bromsuccinimid zu Verbindungen der Formel (VII-1) 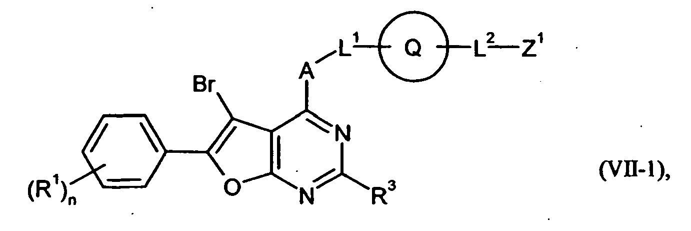 in welcher A, L¹, L², Q, Z¹, R¹, R³ und n jeweils die oben angegebenen Bedeutungen haben,
bromiert und diese anschließend in einem inerten Lösungsmittel in Gegenwart einer Base und eines geeigneten Palladium-Katalysators mit einer Phenylboronsäure der Formel (VIII-1) 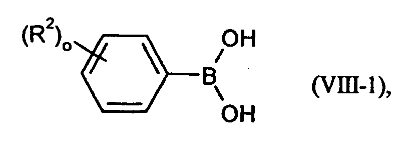 in welcher R² und o die oben angegebenen Bedeutungen haben,
zu Verbindungen der Formel (IV) kuppelt
oder

### [C] Verbindungen der Formel (V-2)

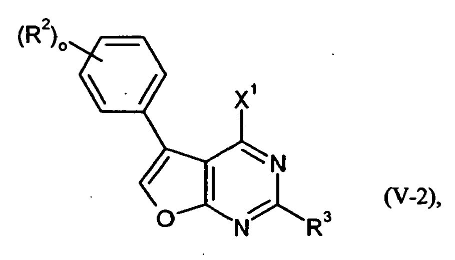

in welcher R², R³, X¹ und o jeweils die oben angegebenen Bedeutungen haben,
in Gegenwart einer Base gegebenenfalls in einem inerten Lösungsmittel mit einer Verbindung der Formel (III) zu Verbindungen der Formel (VI-2) 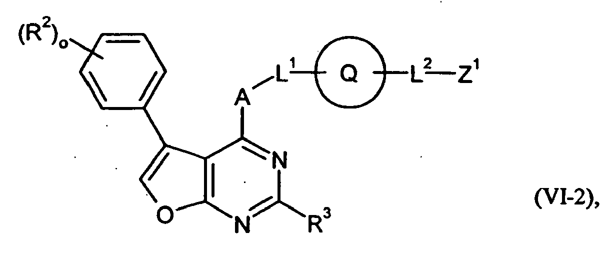 in welcher A, L¹, L², Q, Z¹, R², R³ und o jeweils die oben angegebenen Bedeutungen haben,
umsetzt, dann ein einem inerten Lösungsmittel beispielsweise mit *N*-Bromsuccinimid zu Verbindungen der Formel (VII-2). 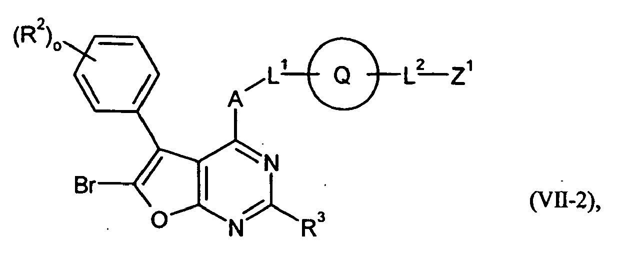 in welcher A, L¹, L², Q, Z¹, R², R³ und o jeweils die oben angegebenen Bedeutungen haben,
bromiert und diese anschließend in einem inerten Lösungsmittel in Gegenwart einer Base und eines geeigneten Palladium-Katalysators mit einer Phenylboronsäure der Formel (VIII-2) 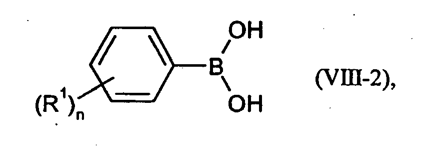 in welcher R¹ und n die oben angegebenen Bedeutungen haben,
zu Verbindungen der Formel (IV) kuppelt,
und die jeweils resultierenden Verbindungen der Formel (IV) dann durch Hydrolyse der Ester- bzw. Cyano-Gruppe Z¹ in die Carbonsäuren der Formel (I-A) 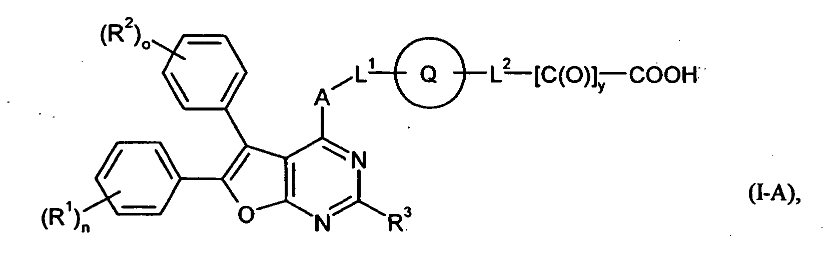 in welcher A, L¹, L², Q, R¹, R², R³, n, o und y jeweils die oben angegebenen Bedeutungen haben,
überführt und diese gegebenenfalls mit den entsprechenden (i) Lösungsmitteln und/oder (ii) Basen oder Säuren zu ihren Solvaten, Salzen und/oder Solvaten der Salze umsetzt.

Inerte Lösungsmittel für die Verfahrensschritte (II) + (III) → (IV), (V-1) + (III) → (VI-1) und (V-2) + (III) → (VI-2) sind-beispielsweise Ether wie Diethylether, Methyl-*tert*.-butylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethan, Tetrachlorethan, Trichlorethylen, Chlorbenzol oder Chlortoluol, oder andere Lösungsmittel wie Dimethylformamid (DMF), Dimethylsulfoxid (DMSO), *N*,*N*'-Dimethylpropylenharnstoff (DMPU), *N-*Methylpyrrolidon (NMP) oder Acetonitril. Ebenso ist es möglich, Gemische der genannten Lösungsmittel einzusetzen. Bevorzugt werden Tetrahydrofuran, Dimethylformamid, Dimethylsulfoxid oder Gemische aus diesen verwendet.

Gegebenenfalls können die Verfahrensschritte (II) + (III) → (IV), (V-1) + (III) → (VI-1) und (V-2) + (III) → (VI-2) jedoch auch ohne Lösungsmittel durchgeführt werden.

Als Basen für die Verfahrensschritte (II) + (III) → (IV), (V-1) + (III) → (VI-1) und (V-2) + (III) → (VI-2) eignen sich übliche anorganische oder organische Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium-, Calcium- oder Cäsiumcarbonat, Alkali-Alkoholate wie Natrium- oder Kalium-*tert*.-butylat, Alkalihydride wie Natrium- oder Kaliumhydrid, Amide wie Lithium- oder Kalium-bis(trimethylsilyl)amid oder Lithiumdiisopropylamid, metallorganische Verbindungen wie Butyllithium oder Phenyllithium, oder organische Amine wie Triethylamin, *N*-Methylmorpholin, *N*-Methylpiperidin, *N*,*N*-Diisopropylethylamin oder Pyridin.

Im Falle der Umsetzung mit Alkoholderivaten [A in (III) = O] sind auch Phosphazen-Basen (so genannte "Schwesinger-Basen") wie beispielsweise P2-t-Bu oder P4-t-Bu zweckmäßig [vgl. z.B. R. Schwesinger, H. Schlemper, Angew. Chem. Int. Ed. Engl. 26, 1167 (1987); T. Pietzonka, D. Seebach, Chem. Ber. 124. 1837 (1991)].

Bei der Umsetzung mit Aminderivaten [A in (III) = N] werden vorzugsweise tertiäre Amine; wie insbesondere *N*,*N*-Diisopropylethylamin, als Base verwendet. Gegebenenfalls können diese Umsetzungen aber auch - bei Verwendung eines Überschusses der Aminkomponente (III) - ohne Zusatz einer Hilfsbase erfolgen. Bei der Reaktion mit Alkoholderivaten [A in (III) = O] sind Kalium- oder Cäsiumcarbonat oder die Phosphazen-Basen P2-t-Bu und P4-t-Bu bevorzugt.

Die Verfahrensschritte (II) + (III) → (IV), (V-1) + (III) → (VI-1) und (V-2) + (III) → (VI-2) können gegebenenfalls vorteilhaft unter Zusatz eines Kronenethers durchgeführt werden.

In einer Verfahrensvariante können die Reaktionen (II) + (III) → (IV), (V-1) + (III) → (VI-1) und (V-2) + (III) → (VI-2) auch in einem Zwei-Phasen-Gemisch, bestehend aus einer wässrigen Alkalihydroxid-Lösung als Base und einem der oben genannten Kohlenwasserstoffe oder Halogenkohlenwasserstoffe als weiterem Lösungsmittel, unter Verwendung eines Phasentransfer-Katalysators wie Tetrabutylammoniumhydrogensulfat oder Tetrabutylammoniumbromid durchgeführt werden.

Die Verfahrensschritte (II) + (III) → (IV), (V-1) + (III) → (VI-1) und (V-2) + (III) → (VI-2) erfolgen bei der Umsetzung mit Aminderivaten [A in (III) = N] im Allgemeinen in einem Temperaturbereich von +50°C bis +150°C. Bei der Umsetzung mit Alkoholderivaten [A in (III) = O] werden die Reaktionen im Allgemeinen in einem Temperaturbereich von -20°C bis +120°C, bevorzugt bei 0°C bis +60°C durchgeführt.

Die Bromierung in den Verfahrensschritten (VI-1) → (VII-1) bzw. (VI-2) → (VII-2) wird vorzugsweise in einem Halogenkohlenwasserstoff als Lösungsmittel, insbesondere in Tetrachlormethan, in einem Temperaturbereich von +50°C bis +100°C durchgeführt. Als Bromierungsmittel eignen sich elementares Brom sowie insbesondere *N*-Bromsuccinimid (NBS), gegebenenfalls unter Zusatz von α,α'-Azobis(isobutyronitril) (AIBN) als Initiator.

Inerte Lösungsmittel für die Verfahrensschritte (VII-1) + (VIII-1) → (IV) und (VII-2) + (VIII-2) → (IV) sind beispielsweise Alkohole wie Methanol, Ethanol, n-Propanol, Isopropanol, n-Butanol oder *tert.-*Butanol, .Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid, Dimethylsulfoxid, *N*,*N*'-Dimethylpropylenharnstoff (DMPU), *N*-Methylpyrrolidon (NMP), Pyridin, Acetonitril oder auch Wasser. Ebenso ist es möglich, Gemische der genannten Lösungsmittel einzusetzen. Bevorzugt ist ein Gemisch aus Dimethylsulfoxid und Wasser.

Als Basen für die Verfahrensschritte (VII-1) + (VITI-1) → (IV) und (VII-2) + (VIII-2) → (IV) eignen sich übliche anorganische Basen. Hierzu gehören insbesondere Alkalihydroxide wie beispielsweise Lithium-, Natrium- oder Kaliumhydroxid, Alkalihydrogencarbonate wie Natrium- oder Kaliumhydrogencarbonat, Alkali- oder Erdalkalicarbonate wie Lithium-, Natrium-, Kalium-, Calcium- oder Cäsiumcarbonat, oder Alkalihydrogenphosphate wie Dinatrium- oder Dikaliumhydrogenphosphat. Bevorzugt wird Natrium- oder Kaliumcarbonat verwendet.

Als Palladium-Katalysator für die Verfahrensschritte (VII-1) + (VITI-1) → (IV) und (VII-2) + (VIII-2) → (IV) ["Suzuki-Kupplung"] sind beispielsweise Palladium auf Aktivkohle, Palladium-(II)-acetat, Tetrakis-(triphenylphosphin)-palladium(0), Bis-(triphenylphosphin)-palladium(II)-chlorid, Bis-(acetonitril)-palladium(II)-chlorid und [1,1'-Bis(diphenylphosphino)ferrocen]dichlor-palladium(II)-Dichlormethan-Komplex geeignet [vgl. z.B. J. Hassan et al., Chem. Rev. 102, 1359-1469 (2002)].

Die Reaktionen (VII-1) + (VIII-1) → (IV) und (VII-2) + (VII-2) → (IV) werden im Allgemeinen in einem Temperaturbereich von +20°C bis + 150°C, bevorzugt bei +50°C bis +100°C durchgeführt.

Die Hydrolyse der Ester- bzw. Nitril-Gruppe Z¹ im Verfahrensschritt. (IV) → (1-A) erfolgt nach üblichen Methoden, indem man die Ester bzw. Nitrile in inerten Lösungsmitteln mit Säuren oder Basen behandelt, wobei bei letzterem die zunächst entstehenden Salze durch Behandeln mit Säure in die freien Carbonsäuren überführt werden. Im Falle der *tert*.-Butylester erfolgt die Esterspaltung bevorzugt mit Säuren.

Als inerte Lösungsmittel eignen sich für diese Reaktionen Wasser oder die für eine Esterspaltung üblichen organischen Lösungsmittel. Hierzu gehören bevorzugt Alkohole wie Methanol, Ethanol, n-Propanol, Isopropanol, n-Butanol oder *tert*.-Butanol, oder Ether wie Diethylether, Tetrahydrofuran, Dioxan oder Glykoldimethylether, oder andere Lösungsmittel wie Aceton, Dichlormethan, Dimethylformamid oder Dimethylsulfoxid. Ebenso ist es möglich, Gemische der genannten Lösungsmittel einzusetzen. Im Falle einer basischen Ester-Hydrolyse werden bevorzugt Gemische von Wasser mit Dioxan, Tetrahydrofuran, Methanol und/oder Ethanol, bei der Nitril-Hydrolyse bevorzugt Wasser und/oder n-Propanol eingesetzt. Im Falle der Umsetzung mit Trifluoressigsäure wird bevorzugt Dichlormethan und im Falle der Umsetzung mit Chlorwasserstoff bevorzugt Tetrahydrofuran, Diethylether, Dioxan oder Wasser verwendet.

Als Basen sind die üblichen anorganischen Basen geeignet. Hierzu gehören bevorzugt Alkali- oder Erdalkalihydroxide wie beispielsweise Natrium-, Lithium-, Kalium- oder Bariumhydroxid, oder Alkali- oder Erdalkalicarbonate wie Natrium-, Kalium- oder Calciumcarbonat. Besonders bevorzugt sind Natrium- oder Lithiumhydroxid.

Als Säuren eignen sich für die Esterspaltung im Allgemeinen Schwefelsäure, Chlorwasserstoff/ Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische gegebenenfalls unter Zusatz von Wasser. Bevorzugt sind Chlorwasserstoff oder Trifluoressigsäure im Falle der *tert*.-Butylester und Salzsäure im Falle der Methylester.

Die Esterspaltung erfolgt im Allgemeinen in einem Temperaturbereich von 0°C bis +100°C, bevorzugt bei +0°C bis +50°C. Die Nitril-Hydrolyse wird im Allgemeinen in einem Temperaturbereich von +50°C bis +150°C, bevorzugt bei +80°C bis +120°C durchgeführt.

Die genannten Umsetzungen können bei normalem, erhöhtem oder bei erniedrigtem Druck durchgeführt werden (z.B. von 0.5 bis 5 bar). Im Allgemeinen arbeitet man jeweils bei Normaldruck.

Die erfindungsgemäßen Verbindungen der Formel (I), in welcher Z für eine Gruppe der Formel 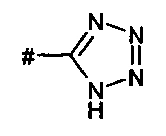 steht,
können hergestellt werden, indem man Verbindungen der Formel (IV), in welcher Z¹ für Cyano steht, in einem inerten Lösungsmittel mit einem Alkali-Azid in Gegenwart von Ammoniumchlorid oder mit Trimethylsilylazid gegebenenfalls in Gegenwart eines Katalysators umsetzt.

Inerte Lösungsmittel für diese Umsetzung sind beispielsweise Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, oder andere Lösungsmittel wie Dimethylsulfoxid, Dimethylformamid, *N,N*'-Dimethylpropylenharnstoff (DMPU) oder *N-*Methylpyrrolidon (NMP). Ebenso ist es möglich, Gemische der genannten Lösungsmittel einzusetzen. Bevorzugt wird Toluol verwendet.

Als Azid-Reagenz ist insbesondere Natriumazid in Gegenwart von Ammoniumchlorid oder Trimethylsilylazid geeignet. Letztere Reaktion kann vorteilhafterweise in Gegenwart eines Katalysators durchgeführt werden. Hierfür eignen sich insbesondere Verbindungen wie Di-n-butylzinnoxid, Trimethylaluminium oder Zinkbromid. Bevorzugt wird Trimethylsilylazid in Kombination mit Din-butylzinnoxid verwendet.

Die Reaktion wird im Allgemeinen in einem Temperaturbereich von +50°C bis +150°C, bevorzugt bei +60°C bis +110°C durchgeführt. Die Umsetzung kann bei normalem, erhöhtem oder erniedrigtem Druck durchgeführt werden (z.B. von 0.5 bis 5 bar). Im Allgemeinen arbeitet man bei Normaldruck.

Die erfindungsgemäßen Verbindungen der Formel (I), in welcher Z für eine Gruppe der Formel 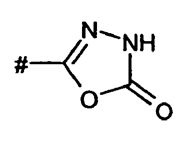 steht,
können hergestellt werden, indem man Verbindungen der Formel (IV), in welcher Z¹ für Methoxy- oder Ethoxycarbonyl steht, zunächst in einem inerten Lösungsmittel mit Hydrazin in Verbindungen der Formel (IX) 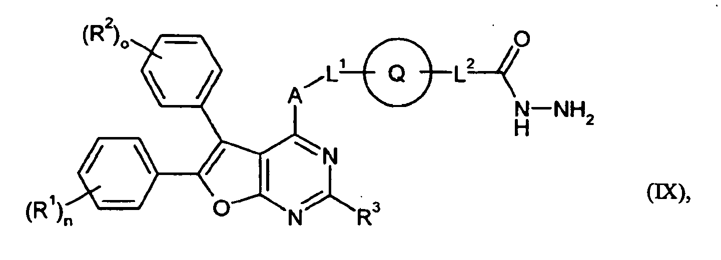 in welcher A, L¹, L², Q, R¹, R², R³, n und o jeweils die oben angegebenen Bedeutungen haben,
überführt und dann in einem inerten Lösungsmittel mit Phosgen oder einem Phosgen-Äquivalent, wie beispielsweise *N,N'*-Carbonyldiimidazol, umsetzt.

Als inerte Lösungsmittel sind für den ersten Schritt dieser Reaktionsfolge insbesondere Alkohole wie Methanol, Ethanol, n-Propanol, Isopropanol, n-Butanol oder *tert*.-Butanol, oder Ether wie Diethylether, Dioxan, Tetrahydrofuran, Glykoldimethylether oder Diethylenglykoldimethylether geeignet. Ebenso ist es möglich, Gemische dieser Lösungsmittel einzusetzen. Bevorzugt wird ein Gemisch aus Methanol und Tetrahydrofuran verwendet. Der zweite Reaktionsschritt wird vorzugsweise in einem Ether, insbesondere in Tetrahydrofuran durchgeführt. Die Umsetzungen erfolgen im Allgemeinen in einem Temperaturbereich von 0°C bis +70°C unter Normaldruck.

Die erfindungsgemäßen Verbindungen der Formel (I), in welcher L² für eine Gruppe der Formel *-M-CR⁷R⁸- oder *-M-CH₂-CR⁷R⁸- steht, worin M, R⁷ und R⁸ die oben angegebenen Bedeutungen haben, können alternativ auch dadurch hergestellt werden, dass man Verbindungen der Formel (X) 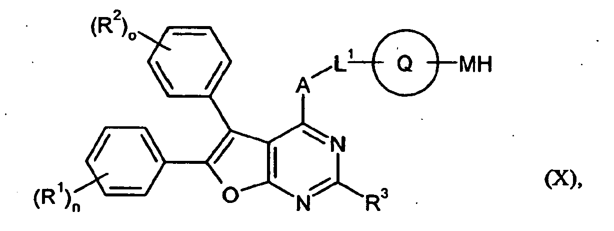 in welcher A, L¹, M, Q, R¹, R², R³, n und o jeweils die oben angegebenen Bedeutungen haben,
in Gegenwart einer Base gegebenenfalls in einem inerten Lösungsmittel mit einer Verbindung der Formel (XI) 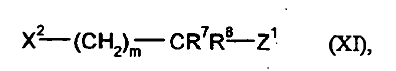 in welcher R⁷, R⁸ und Z¹ jeweils die oben angegebenen Bedeutungen haben,
- m: für die Zahl 0 oder 1
- und: und
- X²: für eine Abgangsgruppe, wie beispielsweise Halogen, Mesylat oder Tosylat, steht,
oder im Falle, dass L² für *-M-CH₂CH₂- steht, mit einer Verbindung der Formel (XII) 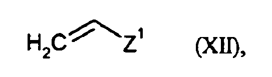 in welcher Z¹ die oben angegebene Bedeutung hat,
in Verbindungen der Formel (IV-A) 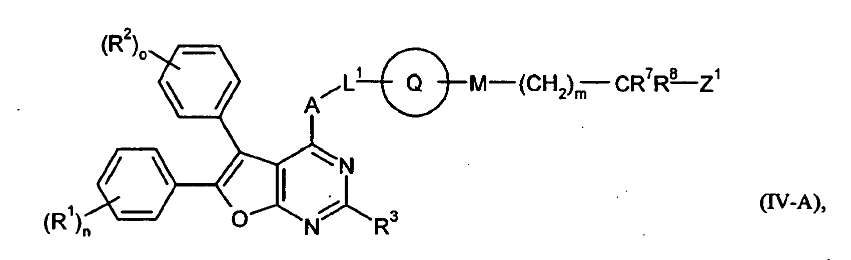 in welcher A, L¹, M, Q, Z¹, R¹, R², R³, R⁷, R⁸, m, n und o jeweils die oben angegebenen Bedeutungen haben,
überführt und diese dann entsprechend dem zuvor beschriebenen Verfahren weiter umsetzt.

Die Verbindungen der Formel (X) können ausgehend von einer Verbindung der Formel (II), (V-1) oder (V-2) durch basenkatalysierte Reaktion mit einer Verbindung der Formel (XIII) 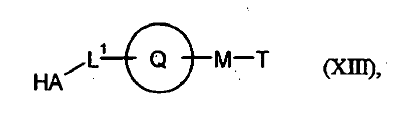 in welcher A, L¹, M und Q jeweils die oben angegebenen Bedeutungen haben
und
- T: für Wasserstoff oder eine temporäre O- bzw. N-Schutzgruppe steht,
sowie entsprechend weitere Umsetzung analog zu den zuvor beschriebenen Verfahrensvarianten [B] bzw. [C] erhalten werden, wobei im Falle der Reaktionssequenz (V-1) bzw. (V-2) → (IV-A) die Reihenfolge der einzelnen Verfahrensschritte, falls zweckmäßig, auch variiert werden kann (vgl. auch die nachfolgenden Reaktionsschemata 2-9).

Für die Verfahrensschritte (X) + (XI) bzw. (XII) → (IV-A) und (II) + (XIII) → (X) finden die zuvor für die Umsetzungen (II) + (III) → (IV), (V-1) + (III) → (VI-1) bzw. (V-2) + (III) → (VI-2) beschriebenen Reaktionsparameter wie Lösungsmittel, Basen und Reaktionstemperaturen in analoger Weise Anwendung.

Die Verbindungen der Formeln (II), (III), (V-1), (VIII-1), (V-2), (VIII-2), (XI), (XII) und (XIII sind kommerziell erhältlich, literaturbekannt oder können in Analogie zu literaturbekannten Verfahren hergestellt werden (vgl. z.B. WO 03/018589; siehe auch Reaktionsschema 1).

Die Herstellung der erfindungsgemäßen Verbindungen kann durch die folgenden Syntheseschemata veranschaulicht werden:

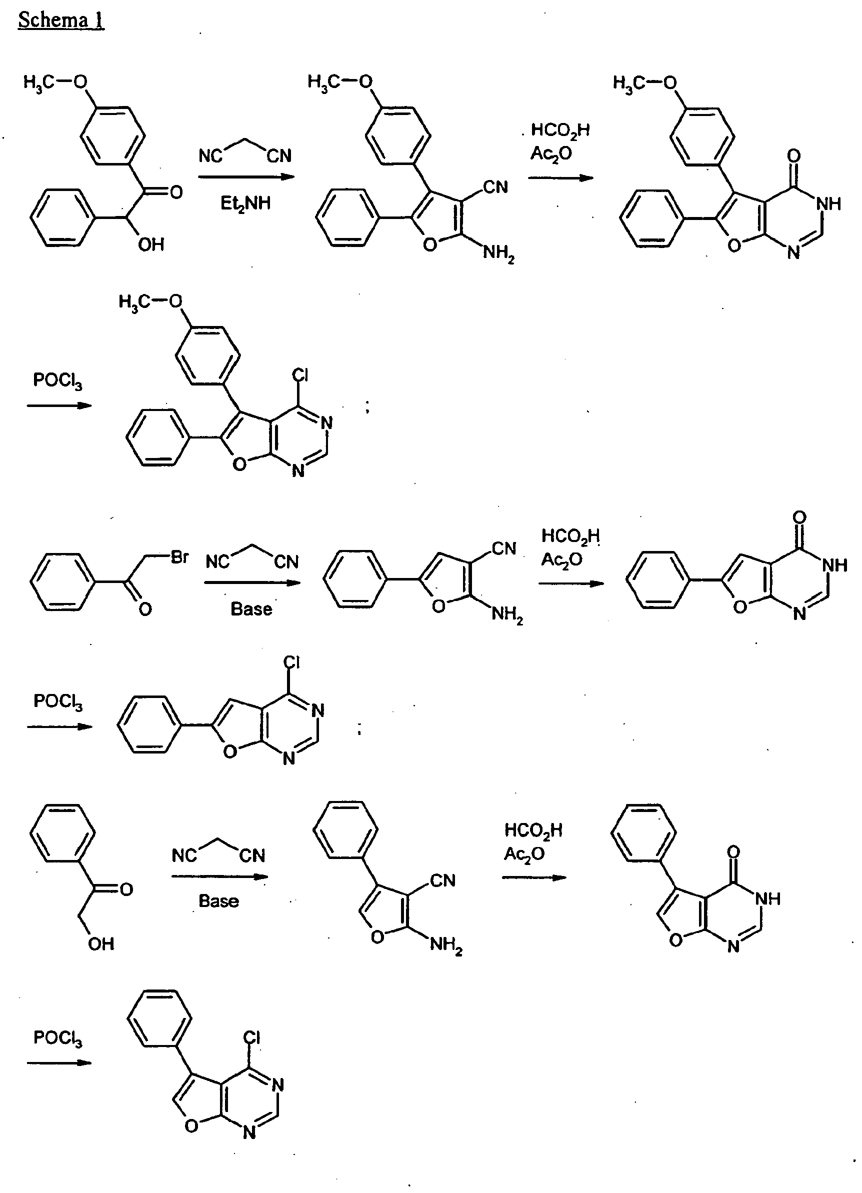

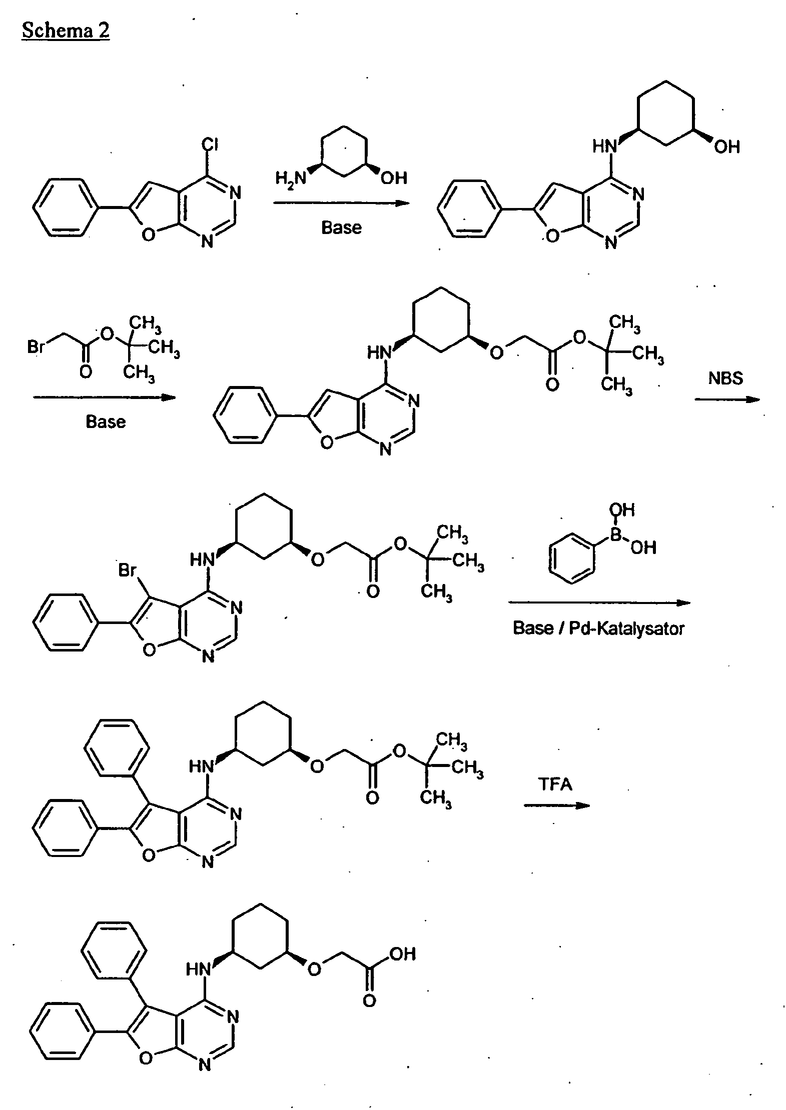

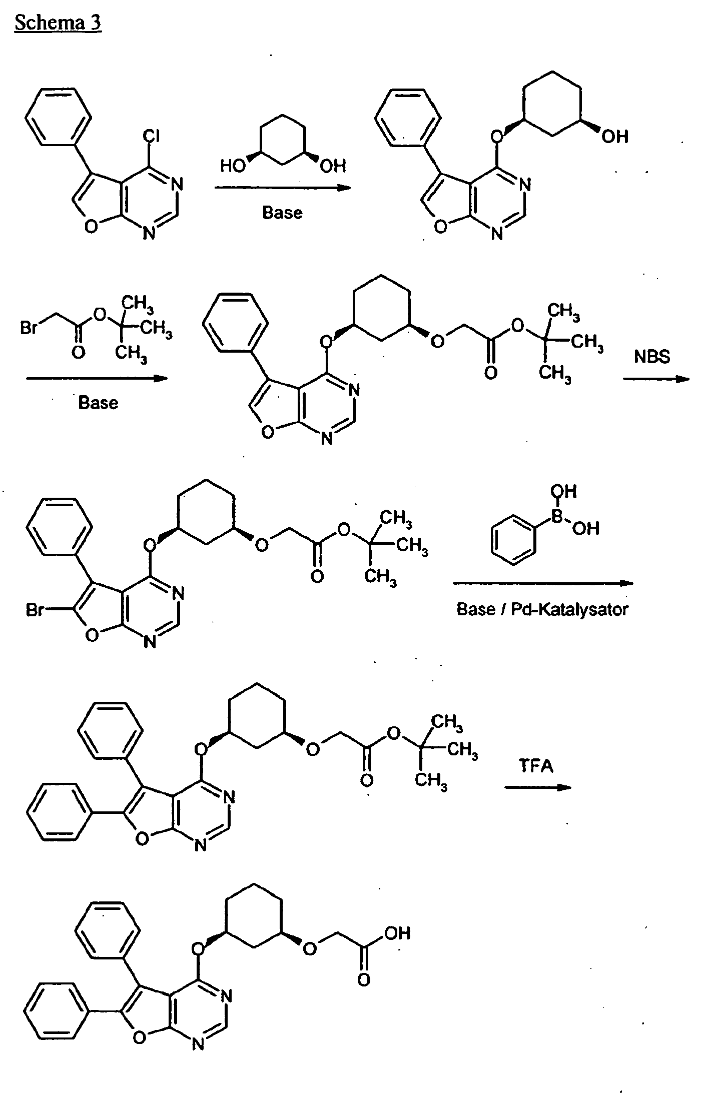

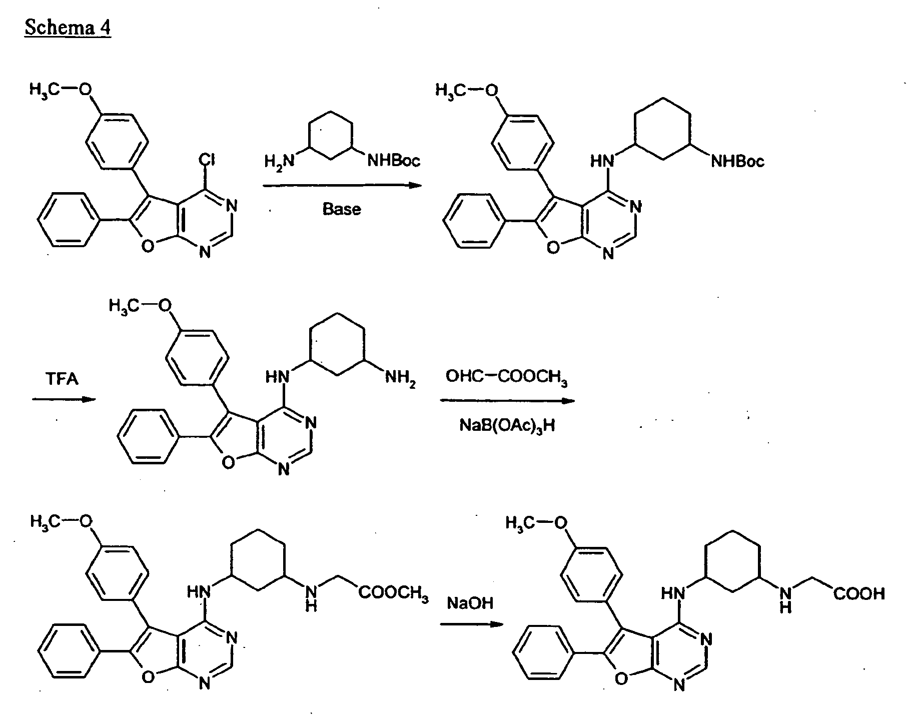

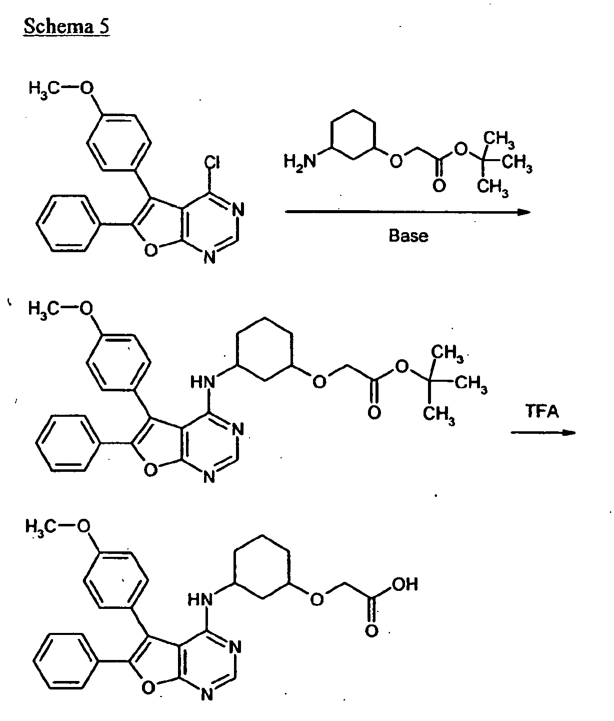

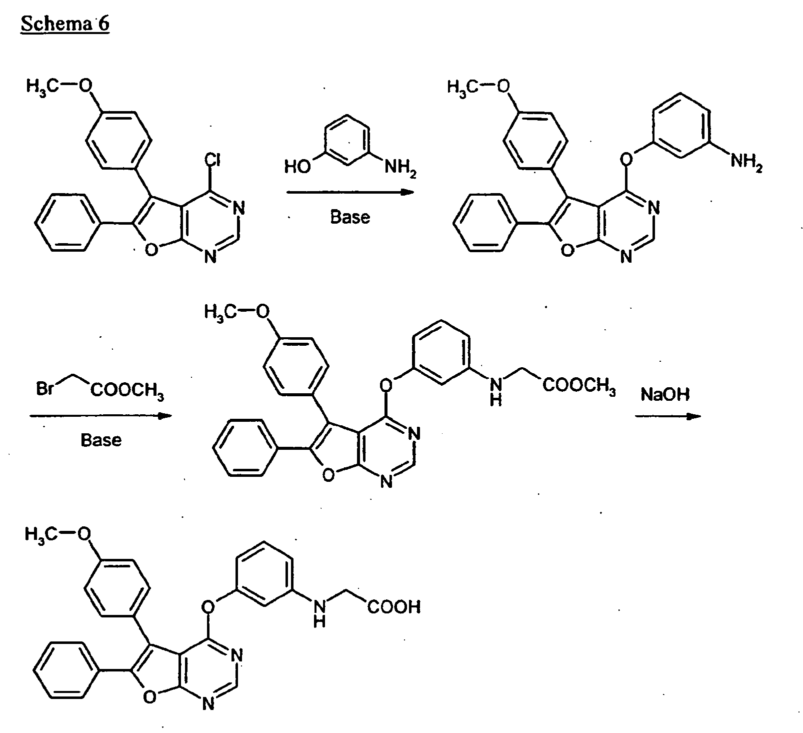

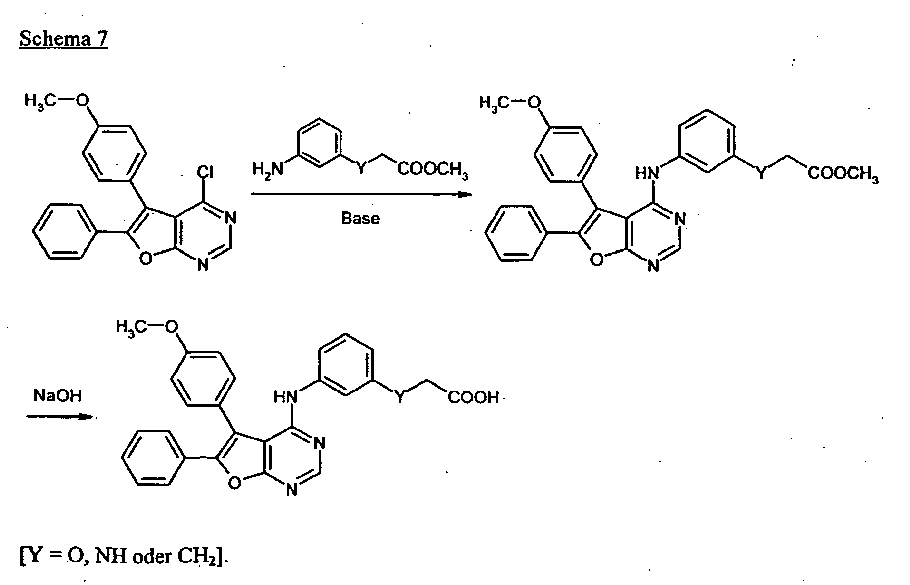

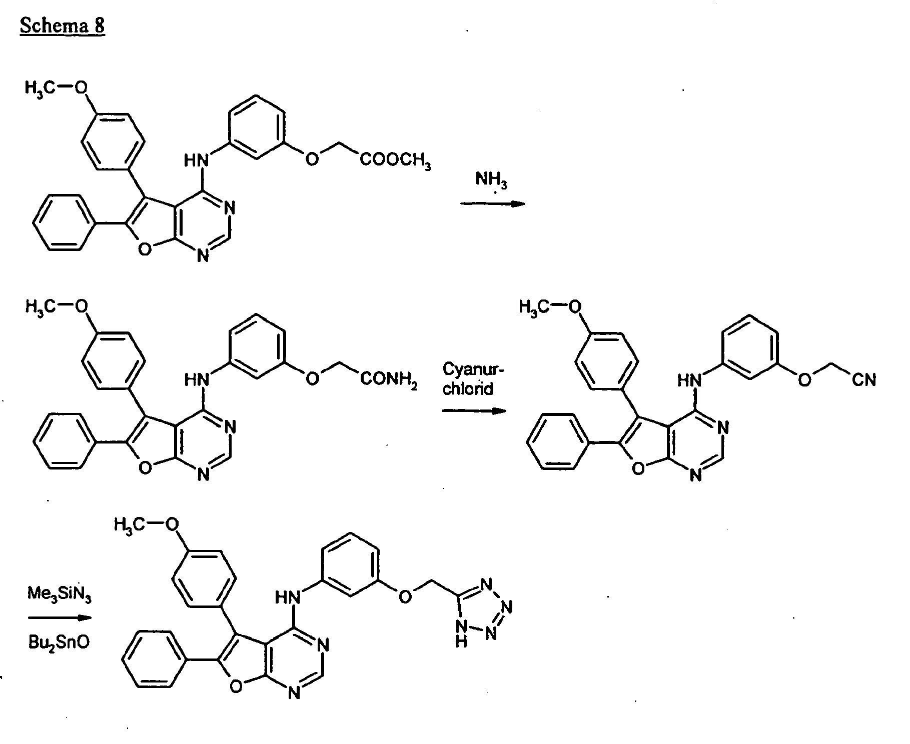

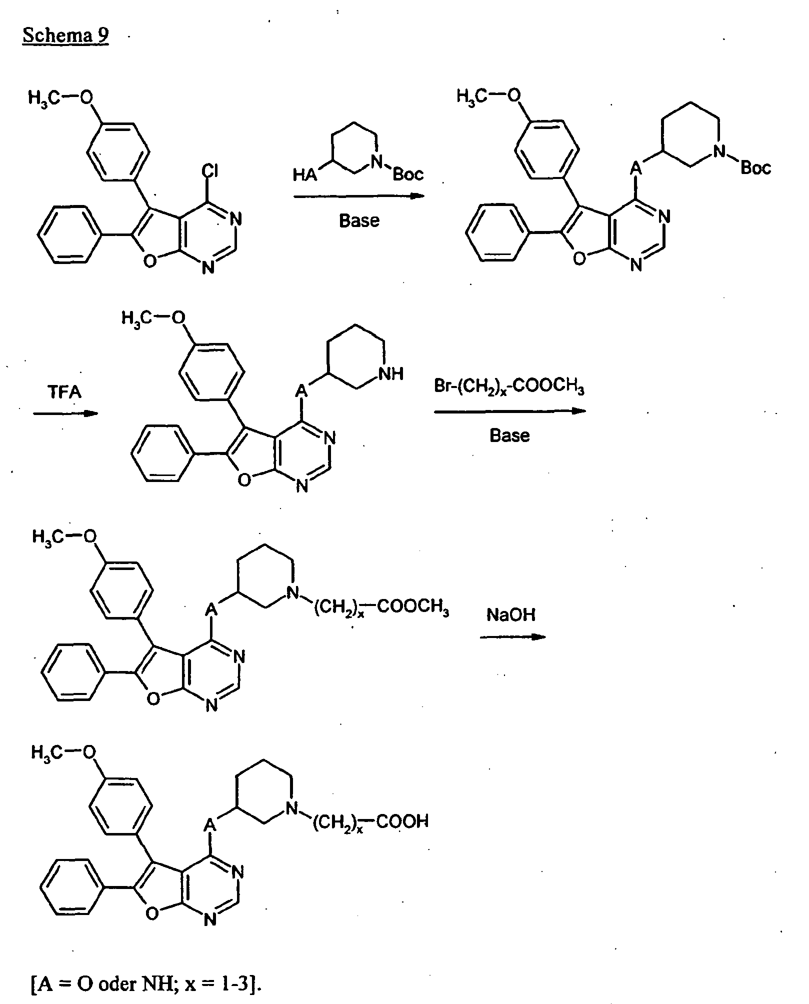

Die erfindungsgemäßen Verbindungen besitzen wertvolle pharmakologische Eigenschaften und können zur Vorbeugung und Behandlung von Erkrankungen bei Menschen und Tieren verwendet werden.

Sie eignen sich insbesondere zur Prophylaxe und/oder Behandlung von kardiovaskulären Erkrankungen wie beispielsweise der stabilen und instabilen Angina pectoris, von peripheren und kardialen Gefäßerkrankungen, des Bluthochdrucks und der Herzinsuffizienz, der pulmonalen Hypertonie, von peripheren Durchblutungsstörungen, zur Prophylaxe und/oder Behandlung von thromboembolischen Erkrankungen und Ischämien wie Myokardinfarkt, Hirnschlag, transistorischen und ischämischen Attacken sowie Subarachnoidalblutungen, und zur Verhinderung von Restenosen wie beispielsweise nach Thrombolysetherapien, percutan-transluminalen Angioplastien (PTA), Koronarangioplastien (PTCA) und Bypass.

Darüber hinaus können die erfindungsgemäßen Verbindungen zur Behandlung von Arteriosklerose, Hepatitis, asthmatischen Erkrankungen, chronisch-obstruktiven Atemwegserkrankungen (COPD), fibrosierenden Lungenerkrankungen wie idiopathische pulmonale Fibrose (IPF) und ARDS, entzündlichen vaskulären Erkrankungen wie Sklerodermie und Lupus erythematodes, Nierenversagen, Arthritis und Osteoporose verwendet werden.

Weiterhin können die erfindungsgemäßen Verbindungen zur Prophylaxe und/oder Behandlung von Krebserkrankungen, insbesondere von metastasierenden Tumoren eingesetzt werden.

Ferner können die erfindungsgemäßen Verbindungen auch als Zusatz zum Konservierungsmedium eines Organtransplantates wie z.B. Nieren, Lungen, Herz oder Inselzellen verwendet werden.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil und Tadalafil;
- NO-unabhängige, jedoch Häm-abhängige Stimulatoren der Guanylatcyclase, wie insbesondere die in WO 00/06568, WO 00/06569, WO 02/42301 und WO 03/095451 beschriebenen Verbindungen;
- NO- und Häm-unabhängige Aktivatoren der Guanylatcyclase, wie insbesondere die in WO 01/19355, WO 01/19776, WO 01/19778, WO 01/19780, WO 02/070462 und WO 02/070510 beschriebenen Verbindungen;
- Verbindungen, die die humane neutrophile Elastase inhibieren, wie beispielsweise Sivelestat oder DX-890 (Reltran);
- die Signaltransduktionskaskade inhibierende Verbindungen, wie beispielsweise Tyrosinkinase- und/oder Serin/Threoninkinase-Inhibitoren, insbesondere Imatinib, Gefitinib, Erlotinib, Sorafenib und Sunitinib;
- den Energiestoffwechsel des Herzens beeinflussende Verbindungen, wie beispielhaft und vorzugsweise Etomoxir, Dichloracetat, Ranolazine oder Trimetazidine;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin All-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten, Rho-Kinase-Inhibitoren sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise BAY 59-7939, DU-176b, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin All-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten, Rho-Kinase-Inhibitoren sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Rho-Kinase-Inhibitor, wie beispielhaft und vorzugsweise Fasudil, Y-27632, SLx-2119, BF-66851, BF-66852, BF-66853, KI-23095 oder BA-1049, verabreicht.

Bei einer bevorzugten Ausführungsform der Erfindung werden die erfindungsgemäßen Verbindungen in Kombination mit einem Diuretikum, wie beispielhaft und vorzugsweise Furosemid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), JJT-705 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen:

- abs.: absolut
- Ac: Acetyl
- Ac₂O: Essigsäureanhydrid
- Boc: *tert*.-Butoxycarbonyl
- Bu: Butyl
- c: Konzentration
- DCI: direkte chemische Ionisation (bei MS)
- DIEA: Diisopropylethylamin ("Hünig-Base")
- DMF: *N,N'*-Dimethylformamid
- DMSO: Dimethylsulfoxid
- d. Th.: der Theorie (bei Ausbeute)
- EI: Elektronenstoß-Ionisation (bei MS)
- eq: Äquivalent(e)
- ESI: Elektrospray-Ionisation (bei MS)
- Et: Ethyl
- Fp.: Schmelzpunkt
- GC-MS: Gaschromatographie-gekoppelte Massenspektrometrie
- ges.: gesättigt
- h: Stunde(n)
- HPLC: Hochdruckflüssigchromatographie
- konz.: konzentriert
- LC-MS: Flüssigchromatographie-gekoppelte Massenspektrometrie
- Me: Methyl
- min: Minute(n)
- Ms: Methansulfonyl (Mesyl)
- MS: Massenspektrometrie
- NBS: *N*-Bromsuccinimid
- NMR: Kernresonanzspektrometrie
- Pd/C: Palladium auf Aktivkohle
- rac.: racemisch
- RP: reverse phase (Umkehrphase, bei HPLC)
- RT: Raumtemperatur
- Rₜ: Retentionszeit (bei HPLC)
- TFA: Trifluoressigsäure
- THF: Tetrahydrofuran

### LC-MS-, GC-MS- und HPLC-Methoden:

### Methode 1 (HPLC):

Instrument: HP 1100 mit DAD-Detektion; Säule: Kromasil 100 RP-18, 60 mm x 2.1 mm, 3.5 µm; Eluent A: 5 ml HClO₄ (70%-ig) / 1 Wasser, Eluent B: Acetonitril; Gradient: 0 min 2% B → 0.5 min 2% B → 4.5 min 90% B → 9 min 90% B → 9.2 min 2% B → 10 min 2% B; Fluss: 0.75 ml/min; Säulentemperatur: 30°C; UV-Detektion: 210 nm.

### Methode 2 (HPLC):

Instrument: HP 1100 mit DAD-Detektion; Säule: Kromasil 100 RP-18, 60 mm x 2.1 mm, 3.5 µm; Eluent A: 5 ml HClO₄ (70%-ig) / 1 Wasser, Eluent B: Acetonitril; Gradient: 0 min 2% B → 0.5 min 2% B → 4.5 min 90% B → 6.5 min 90% B → 6.7 min 2% B → 7.5 min 2% B; Fluss: 0.75 ml/min; Säulentemperatur: 30°C; UV-Detektion: 210 nm.

### Methode 3 (LC-MS):

Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 4 (LC-MS):

Instrument: Micromass Plattform LCZ mit HPLC Agilent Serie 1100; Säule: Thermo Hypersil GOLD 3µ 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 100% A → 0.2 min 100% A → 2.9 min 30% A → 3.1 min 10% A → 5.5 min 10% A; Ofen: 50°C; Fluss: 0.8 ml/min; UV-Detektion: 210 nm.

### Methode 5 (LC-MS):

Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 6 (LC-MS):

Instrument: Micromass Quattro LCZ mit HPLC Agilent Serie 1100; Säule: Phenomenex Synergi 2µ Hydro-RP Mercury 20 mm x 4 mm; Eluent A: 1 1 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 208-400 nm.

### Methode 7 (LC-MS):

Gerätetyp MS: Micromass ZQ; Gerätetyp HPLC: Waters Alliance 2795; Säule: Merck Chromolith SpeedROD RP-18e 100 x 4.6 mm; Eluent A: Wasser + 500 µl 50%-ige Ameisensäure /1, Eluent B: Acetonitril + 500 µl 50%-ige Ameisensäure /1; Gradient: 0.0 min 10% B → 7.0 min 95% B → 9.0 min 95% B; Ofen: 35°C; Fluss: 0.0 min 1.0 ml/min → 7.0 min 2.0 ml/min → 9.0 min 2.0 ml/min; UV-Detektion: 210 nm.

### Methode 8 (LC-MS):

Gerätetyp MS: Micromass ZQ; Gerätetyp HPLC: HP 1100 Series; UV DAD; Säule: Phenomenex Gemini 3µ 30 mm x 3.00 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 9 (GC-MS):

Instrument: Micromass GCT, GC6890; Säule: Restek RTX-35, 15 m x 200 µm x 0.33 µm; konstanter Fluss mit Helium: 0.88 ml/min; Ofen: 70°C; Inlet: 250°C; Gradient: 70°C, 30°C/min → 310°C (3 min halten).

### Methode 10 (GC-MS):

Instrument: Micromass GCT, GC6890; Säule: Restek RTX-35MS, 30 m x 250 µm x 0.25 µm; konstanter Fluss mit Helium: 0.88 ml/min; Ofen: 60°C; Inlet: 250°C; Gradient: 60°C (0.30 min halten), 50°C/min → 120°C, 16°C/min → 250°C, 30°C/min → 300°C (1.7 min halten).

### Methode 11 (GC-MS):

Instrument: Micromass GCT, GC6890; Säule: Restek RT-35, 15 m x 200 µm x 0.33 µm; konstanter Fluss mit Helium: 0.88 ml/min; Ofen: 70°C; Inlet: 250°C; Gradient: 70°C, 30°C/min → 310°C (12 min halten).

### Methode 12 (LC-MS):

Instrument: Micromass Quattro LCZ mit HPLC Agilent Serie 1100; Säule: Phenomenex Gemini 3µ 30 mm x 3.00 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 208-400 nm.

### Methode 13 (LC-MS):

Instrument: Micromass Quattro LCZ mit HPLC Agilent Serie 1100; Säule: Phenomenex Onyx Monolithic C18, 100 mm x 3 mm; Eluent A: 1 1 Wasser + 0.5 ml 50%-ige Ameisensäure, Eluent B: 11 Acetonitril + 0.5 ml 50%-ige Ameisensäure; Gradient: 0.0 min 90% A → 2 min 65% A → 4.5 min 5% A → 6 min 5% A; Fluss: 2 ml/min; Ofen: 40°C; UV-Detektion: 208-400 nm.

### Ausgangsverbindungen und Intermediate:

### Beispiel 1A

### 3-Nitrophenoxy-essigsäuremethylester

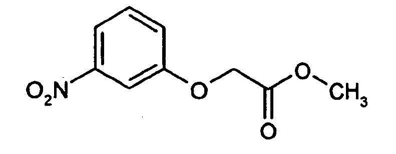

50 g (359.4 mmol) 3-Nitrophenol und 175.67 g (539 mmol) Cäsiumcarbonat werden in 1.0 Liter Aceton vorgelegt und mit 71.5 g (467.3 mmol) Bromessigsäuremethylester versetzt. Die Mischung wird 1 h bei 50°C gerührt und nach dem Abkühlen auf 7.5 Liter Wasser gegossen. Die Suspension wird 30 min gerührt, dann abgesaugt und der Filterrückstand mit Wasser gewaschen. Man trocknet den Feststoff im Trockenschrank bei 50°C und 100 mbar. Erhalten werden 64.3 g (84.7% d. Th.) der Zielverbindung.
HPLC (Methode 1): Rₜ = 4.07 min
MS (DCI): m/z = 229 (M+NH₄)⁺
¹H-NMR (300 MHz, CDCl₃): δ = 7.90 (dd, 1H), 7.43 (t, 1H), 7.48 (t, 1H), 7.28 (dd, 1H), 4.75 (s, 2H), 3.86 (s, 3H).

### Beispiel 2A

### 3-Aminophenoxy-essigsäuremethylester

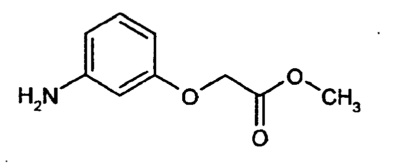

Zu 13 g (61.6 mmol) 3-Nitrophenoxy-essigsäuremethylester in 150 ml Methanol werden unter Argon 1.3 g Palladium auf Aktivkohle (10%) gegeben. Die Mischung wird 18 h bei RT unter Wasserstoffatmosphäre (Normaldruck) gerührt. Der Katalysator wird über Kieselgur abfiltriert und das Filtrat im Vakuum eingeengt. Man erhält nach Trocknen im Hochvakuum 10.7 g (95.9% d. Th.) der Zielverbindung.
HPLC (Methode 2): Rₜ = 2.81 min
MS (DCI): m/z = 199 (M+NH₄)⁺, 182 (M+H)⁺
¹H-NMR (400 MHz, CDCl₃): δ = 7.10-7.02 (m, 1H), 6.35-6.23 (m, 2H), 4.58 (s, 2H), 3.79 (s, 3H), 3.65 (br. s, 2H).

### Beispiel 3A

### 2-Amino-4,5-diphenyl-3-furonitril

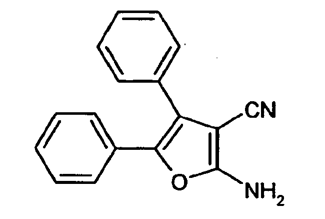

100 g (470 mmol) Benzoin, 62.25 g (940 mmol) Malonsäuredinitril und 47.68 g (470 mmol) Triethylamin werden über Nacht bei RT in 1345 ml DMF. gerührt. Weitere 41 g (620 mmol) Malonsäuredinitril werden hinzugefügt und die Mischung erneut für 24 h bei RT gerührt. Danach werden Ethylacetat und Wasser hinzugefügt und die wässrige Phase zweimal mit Ethylacetat extrahiert. Die vereinigten organischen Phasen werden über Magnesiumsulfat getrocknet und im Vakuum konzentriert. Nach Säulenchromatographie an Silicagel (Laufmittel: Dichlormethan → Dichlormethan/Methanol 98:2) werden 120 g (97.9% d. Th.) des Zielprodukts als gelblicher Feststoff erhalten.
HPLC (Methode 2): Rₜ = 4.68 min
MS (DCI): m/z = 278 (M+NH₄)⁺, 261 (M+H)⁺.

### Beispiel 4A

### 5,6-Diphenylfuro[2,3-d]pyrimidin-4(3H)-on

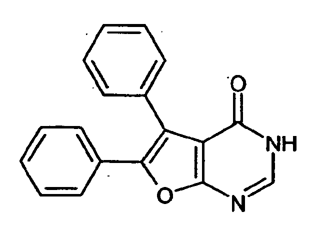

Zu 57 ml Essigsäureanhydrid werden bei 0°C 28.5 ml Ameisensäure getropft. Die Mischung wird 30 min bei 0°C gerührt und dann 10.0 g (40 mmol) 2-Amino-4,5-diphenyl-3-furonitril zugesetzt. Die Kühlung wird entfernt und die Mischung über Nacht unter Rückfluss erhitzt. Nach dem Abkühlen wird wenig Diethylether zugesetzt und der ausgefallene Feststoff abgesaugt. Man wäscht den Rückstand mit Diethylether und trocknet im Hochvakuum. Erhalten werden 6 g (52.2% d. Th.) des Zielprodukts.
HPLC (Methode 2): Rₜ = 4.40 min
MS (DCI): m/z = 306 (M+NH₄)⁺, 289 (M+H)⁺.

### Beispiel 5A

### 4-Chlor-5,6-diphenylfuro[2,3-d]pyrimidin

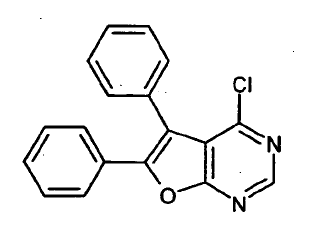

Zu 57 g (200 mmol) 5,6-Diphenylfuro[2,3-d]pyrimidin-4(3*H*)-on werden 570 ml Phosphoroxychlorid gegeben. Die Mischung wird 3 h unter Rückfluss gerührt, dann abgekühlt und im Vakuum konzentriert. Der Rückstand wird für 30 min mit Eiswasser verrührt und dann mit Dichlormethan versetzt. Die resultierende organische Phase wird dreimal mit Wasser gewaschen, über Natriumsulfat getrocknet und im Vakuum konzentriert. Erhalten werden 58 g (93.2% d. Th.) des Zielprodukts.
HPLC (Methode 1): Rₜ = 5.26 min
MS (DCI): m/z = 324 (M+NH₄)⁺, 307 (M+H)⁺
¹H-NMR (400 MHz, CDCl₃): δ = 8.78 (s, 1H), 7.62-7.58 (m, 2H), 7.55-7.42 (m, 5H), 7.38-7.30 (m, 3H).

### Beispiel 6A

### (4-Methoxyphenyl)[(trimethylsilyl)oxy]acetonitril

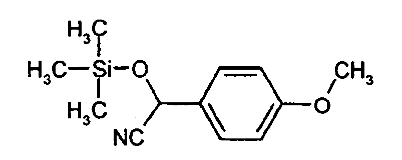

Analog Literaturvorschrift [J. Chem. Soc. Perkin Trans. I, 1992, 2409-2417] wird zu einer Mischung von 290.0 g (2130 mmol) 4-Methoxybenzaldehyd und 1.156 g (3.622 mmol) Zinkiodid in 37.5 Liter Benzol bei RT unter Kühlung innerhalb von ca. 5 min eine Lösung von 221.88 g (2236 mmol) Trimethylsilylcyanid in 25 Liter Benzol gegeben. Die Mischung wird 90 min bei RT gerührt und dann im Vakuum eingeengt. Der Rückstand wird durch Säulenfiltration an Kieselgel (Laufmittel: Cyclohexan/Ethylacetat 4:1) gereinigt. Es werden 442.4 g (88.3% d. Th.) der Zielverbindung erhalten.
HPLC (Methode 2): Rₜ = 3.76 min
MS (DCI): m/z = 253 (M+NH₄)⁺
¹H-NMR (400 MHz, CDCl₃): δ = 7.49 (d, 2H), 6.92 (d, 2H), 5.42 (s, 1H) 3.81 (s, 3H).

### Beispiel 7A

### 2-Hydroxy-1-(4-methoxyphenyl)-2-phenylethanon

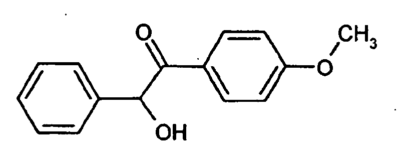

Gemäß Literaturvorschrift [J. Chem. Soc. Perkin Trans. I, 1992, 2409-2417] werden 292 ml (2.08 mol) Diisopropylamin in 3.6 Liter 1,2-Dimethoxyethan gelöst und auf -78°C abgekühlt. 826 ml n-Butyllithium-Lösung (2.5 M in n-Hexan, 2.066 mol) werden unterhalb von -60°C zudosiert. Die Mischung wird 15 min bei < -60°C nachgerührt und dann eine Lösung von 442 g (1.877 mol) (4-Methoxyphenyl)[(trimethylsilyl)oxy]acetonitril in 1.41 Liter 1,2-Dimethoxyethan bei < -60°C zugetropft. Nach weiterem Rühren für 30 min bei -60°C wird eine Lösung von 199.3 g (1.878 mol) Benzaldehyd in 1.4 Liter 1,2-Dimethoxyethan innerhalb von 20 min bei -60°C zugegeben. Anschließend wird das Reaktionsgemisch langsam über 4 h auf RT erwärmt. Man setzt 7 Liter gesättigte Ammoniumchlorid-Lösung hinzu und extrahiert mit Ethylacetat. Die organische Phase wird mit gesättigter Ammoniumchlorid-Lösung gewaschen, getrocknet und im Vakuum konzentriert. Der Rückstand wird in 7 Liter Dioxan und 5 Liter Methanol aufgenommen und mit 6 Liter 1 N Salzsäure versetzt. Die Mischung wird 3 h bei RT gerührt, dann werden 3 Liter gesättigte Natriumchlorid-Lösung zugesetzt und das Gemisch mit 6.5 Liter Ethylacetat extrahiert. Die organische Phase wird mit 1.0 Liter 1 N Natronlauge und mit gesättigter Natriumchlorid-Lösung gewaschen, getrocknet und im Vakuum konzentriert. Der Rückstand wird in 2 Liter Diisopropylether aufgenommen, vom Unlöslichen abdekantiert und mit Kristallen angeimpft. Die entstehende Suspension wird 2 h bei RT gerührt und die Kristalle dann abgesaugt. Man wäscht mit 300 ml Diisopropylether und Petrolether und trocknet im Vakuum. Es werden 236.8 g (47.8% d. Th.) der Zielverbindung erhalten.
HPLC (Methode 2): Rₜ = 4.23 min
MS (DCI): m/z = 260 (M+NH₄)⁺, 243 (M+H)⁺
¹H-NMR (400 MHz, CDCl₃): δ = 7.92 (d, 2H), 7.38-7.28 (m, 5H), 6.88 (d, 2H), 5.90 (d, 1H), 4.64 (d, 1H), 3.82 (s, 3H).

### Beispiel 8A

### 2-Amino-4-(4-methoxyphenyl)-5-phenyl-3-furonitril

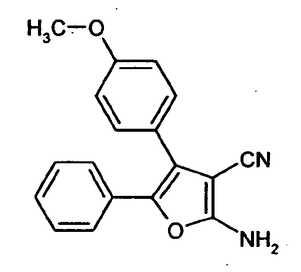

236 g (974 mmol) 2-Hydroxy-1-(4-methoxyphenyl)-2-phenylethanon und 83.66 g (1266 mmol) Malonsäüredinitril werden in 470 ml DMF gelöst und unter Eisbad-Kühlung mit 86.6 ml (836.7 mmol) Diethylamin versetzt. Nach 1 h wird die Mischung auf RT erwärmt und noch 4 h bei RT nachgerührt, bevor 2.5 Liter Wasser und einige Impfkristalle zugefügt werden. Nach 30 min wird überstehendes Wasser abdekantiert und durch 1.25 Liter frisches Wasser ersetzt. Die Suspension wird durchgemischt und überstehendes Wasser erneut abdekantiert. Der klebrig-kristalline Rückstand wird in Ethylacetat aufgenommen und dann im Vakuum beinahe vollständig eingeengt. Man verrührt den Rückstand mit 730 ml Diisopropylether und lässt die Suspension über Nacht bei RT stehen. Der Feststoff wird dann abgesaugt und im Vakuum getrocknet. Es werden 211.5 g (57.6% d. Th.) der Titelverbindung erhalten.
HPLC (Methode 2): Rₜ = 4.60 min
MS (DCI): m/z = 308 (M+NH₄)⁺, 291 (M+H)⁺
¹H-NMR (400 MHz, CDCl₃): δ = 7.39-7.33 (m, 5H), 7.28-7.18 (m, 3H), 6.93 (d, 2H), 5.02 (s, 2H), 3.85 (s, 3H).

### Beispiel 9A

### 5-(4-Methoxyphenyl)-6-phenylfuro[2,3-d]pyrimidin-4(3H)-on

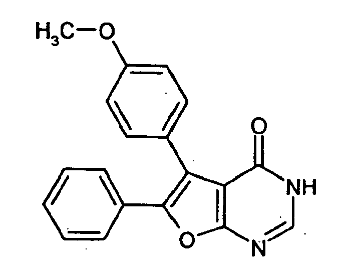

Zu 1600 ml (16.96 mol) Essigsäureanhydrid werden bei 0°C 800 ml (21.21 mol) Ameisensäure getropft. Die Mischung wird 30 min bei 0°C gerührt und dann 211 g (727 mmol) 2-Amino-4-(4-methoxyphenyl)-5-phenyl-3-furonitril zugesetzt. Die Kühlung wird entfernt und die Mischung erwärmt; bei ca. 80°C setzt Gasentwicklung ein, die nach ca. 3 h aufhört. Man rührt insgesamt 24 h unter Rückfluss (Badtemperatur ca. 130°C). Nach Abkühlen auf RT wird 2 h bei 10°C gerührt und der entstandene Feststoff abfiltriert. Man wäscht den Rückstand mit Diethylether und trocknet im Hochvakuum. Es werden 135.6 g (58.6% d. Th.) der Titelverbindung erhalten.
HPLC (Methode 2): Rₜ = 4.38 min
MS (DCI): m/z = 336 (M+NH₄)⁺, 319 (M+H)⁺
¹H-NMR (400 MHz, CDCl₃): δ = 10.3 (br. s, 1H), 7.95 (s, 1H), 7.58-7.53 (m, 2H), 7.47 (d, 2H), 7.33-7.27 (m, 3H), 6.95 (d, 2H), 3.86 (s, 3H).

### Beispiel 10A

### 4-Chlor-5-(4-methoxyphenyl)-6-phenylfuro[2,3-d]pyrimidin

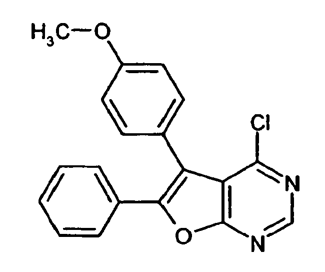

135 g (424 mmol) 5-(4-Methoxyphenyl)-6-phenylfuro[2,3-d]pyrimidin-4(3*H*)-on werden bei RT in 675 ml (7241 mmol) Phosphoroxychlorid suspendiert und die Mischung dann zum Sieden erhitzt (HCl-Entwicklung). Nach 1 h wird die dunkle Lösung auf RT abgekühlt und zu einer kräftig gerührten Mischung von 2.25 Liter Wasser und 4.05 Liter konz. Ammoniak-Lösung (25 Gew.-%) getropft (Erwärmung auf 55-75°C, pH >9). Nach Ende der Zugabe wird auf RT abgekühlt und die Mischung dreimal mit je 1.0 Liter Dichlormethan extrahiert. Die vereinigten organischen Phasen werden getrocknet und im Vakuum eingeengt. Der Rückstand wird mit Diethylether verrührt, abgesaugt und im Hochvakuum getrocknet. Es werden 134.4 g (94.1% d. Th.) der Titelverbindung erhalten.
HPLC (Methode 2): Rₜ = 4.96 min
MS (DCI): m/z = 354 (M+NH₄)⁺, 337 (M+H)⁺
¹H-NMR (400 MHz, CDCl₃): δ = 8.76 (s, 1H), 7.62 (d, 2H), 7.40-7.30 (m, 5H), 7.03 (d, 2H), 3.90 (s, 3H).

### Beispiel 11A

### 2-Amino-5-phenyl-3-furonitril

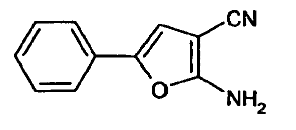

Zu einer Mischung von 60.0 g (301 mmol) Bromacetophenon und 25.89 g (391.86 mmol) Malonsäuredinitril in 130 ml DMF werden bei RT 68.6 ml (663 mmol) Diethylamin getropft (Kühlung erforderlich, um die Temperatur zu halten). Gegen Ende der Zugabe wird die Kühlung entfernt, die Mischung 1 h bei RT gerührt und dann auf 385 ml Wasser gegeben. Es wird mit weiteren 125 ml Wasser verdünnt und 20 min bei RT gerührt. Man saugt den ausgefallenen Feststoff ab, wäscht zweimal mit je 125 ml Wasser, saugt trocken und wäscht mit Petrolether. Der Rückstand wird im Hochvakuum getrocknet. Es werden 33.3 g (50.1% d. Th.) der Zielverbindung als gelb-braune Kristalle erhalten.
HPLC (Methode 2): Rₜ = 4.27 min
MS (DCI): m/z = 202 (M+NH₄)⁺, 185 (M+H)⁺
¹H-NMR (400 MHz, CDCl₃): δ = 7.51-7.45 (m, 2H), 7.39-7.32 (m, 3H), 6.54 (s, 1H), 4.89 (br. s, 1H).

### Beispiel 12A

### 6-Phenylfuro[2,3-d]pyrimidin-4(3H)-on

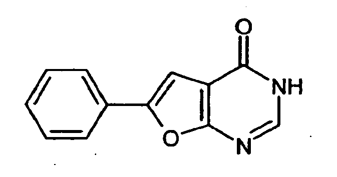

Zu 884.9 ml (9.378 mol) Essigsäureanhydrid werden bei 0°C 424.5 ml (11.25 mol) Ameisensäure getropft. Die Mischung wird 30 min bei 0°C gerührt und dann 69.1 g (0.375 mol) 2-Amino-5-phenyl-3-furonitril zugesetzt. Die Kühlung wird entfernt und die Mischung erwärmt; bei ca. 80°C setzt Gasentwicklung ein, die nach ca. 3 h aufhört. Man rührt insgesamt 24 h unter Rückfluss (Badtemperatur ca. 130°C). Nach Abkühlen der Suspension auf RT wird mit 750 ml Diisopropylether versetzt, auf 0°C gekühlt und abfiltriert. Man wäscht den Rückstand mit Diisopropylether und trocknet im Hochvakuum. Erhalten werden 50.83 g (58.7% d. Th.) der Zielverbindung als brauner Feststoff.
HPLC (Methode 2): Rₜ = 3.92 min
MS: m/z = 213 (M+H)⁺
¹H-NMR (400 MHz, DMSO-d₆): δ = 12.68 (br. s, 1H), 8.17 (s, 1H), 7.88 (d, 2H), 7.52-7.48 (m, 3H), 7.42-7.38 (m, 1H).

### Beispiel 13A

### 4-Chlor-6-phenylfuro[2,3-d]pyrimidin

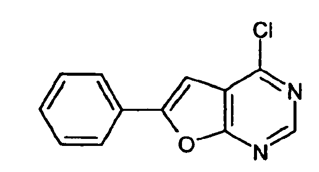

50 g (235.6 mmol) 6-Phenylfuro[2,3-d]pyrimidin-4(3*H*)-on werden bei RT in 375 ml (4023 mmol) Phosphoroxychlorid suspendiert und die Mischung zum Sieden erhitzt (HCl-Entwicklung). Nach 1 h wird die dunkle Lösung auf RT abgekühlt und zu einer kräftig gerührten Mischung von 1.25 Liter Wasser und 2.25 Liter konz. Ammoniak-Lösung (25 Gew.-%) getropft (Erwärmung auf 55-75°C, pH >9). Nach Ende der Zugabe wird auf RT abgekühlt und die Mischung dreimal mit je 1.6 Liter Dichlormethan extrahiert. Die vereinigten organischen Phasen werden getrocknet und im Vakuum eingeengt. Der Rückstand wird mit Diethylether verrührt, abgesaugt und im Hochvakuum getrocknet. Erhalten werden 47.3 g (87% d. Th.) der Zielverbindung.
HPLC (Methode 2): Rₜ = 4.67 min
MS: m/z = 231 (M+H)⁺
¹H-NMR (300 MHz, DMSO-d₆): δ = 8.84 (s, 1H), 8.05 (m, 2H), 7.77 (s, 1H), 7.61-7.50 (m, 3H).

### Beispiel 14A

### 2-Amino-4-phenyl-3-furonitril

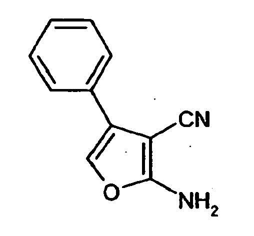

Zu einer Mischung von 10 g (73.4mmol) Hydroxyacetophenon und 4.852 g (73.4 mmol) Malonsäuredinitril in 24 ml DMF werden unter Kühlung bei RT 3.78 ml (36.7 mmol) Diethylamin getropft. Die dunkle Mischung wird 2 h bei RT gerührt und dann langsam unter Rühren und Kühlung auf Wasser (200 ml) gegeben. Der ausgefallene Niederschlag wird 30 min bei ca. 10°C nachgerührt, abgesaugt, zweimal mit Wasser wieder aufgeschlämmt und erneut abgesaugt. Der Rückstand wird im Hochvakuum bis zur Gewichtskonstanz getrocknet. Erhalten werden 10.99 g (81.2% d. Th.) der Zielverbindung als gelb-brauner Feststoff.
LC-MS (Methode 3): Rₜ = 1.81 min.; m/z = 185 (M+H)⁺
¹H-NMR (400 MHz, DMSO-d₆): δ = 7.54 (d, 2H), 7.50 (s, 2H), 7.45-7.32 (m, 4H).

### Beispiel 15A

### 5-Phenylfuro[2,3-d]pyrimidin-4(3H)-on

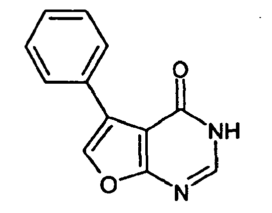

108.5 ml (1154 mmol) Essigsäureanhydrid werden auf 0°C gekühlt und unter Argon mit 52.2 ml (1384 mmol) Ameisensäure versetzt. Die Mischung wird ca. 45 min bei 0°C gerührt und dann 8.5 g (46.2 mmol) 2-Amino-4-phenyl-3-furonitril in Portionen zugegeben. Es entsteht eine dunkle Mischung, die nach 15 min bei 0°C eine violette Farbe annimmt. Die Kühlung wird entfernt und die nun blaue Suspension auf RT erwärmt. Nach 15 min wird die Mischung auf Rückfluss (Badtemperatur 125-130°C) erhitzt, worauf Gasentwicklung einsetzt. Die Mischung wird über Nacht unter Rückfluss gerührt. Nach dem Abkühlen wird die Mischung im Vakuum konzentriert und der Rückstand im Hockvakuum getrocknet. Aus dem Rohprodukt werden durch Säulenfiltration an Kieselgel (Laufmittelgradient: Dichlormethan → Dichlormethan/Methanol 50: 1) ca. 3 g eines tiefdunkelroten bis schwarzen Feststoffs erhalten. Dieser wird in ca. 8 ml Dichlormethan gelöst, mit Diisopropylether gefällt, abgesaugt und im Hochvakuum getrocknet. Erhalten werden 1.81 g (Reinheit ca. 84%, Ausbeute ca. 15% d. Th.) der Zielverbindung als dunkelroter Feststoff.
LC-MS (Methode 4): Rₜ = 3.2 min.; m/z = 211 (M-H)⁺
¹H-NMR (400 MHz, DMSO-d₆): δ = 12.7 (s, 1H), 8.26 (s, 1H), 8.19 (s, 1H), 7.98 (d, 2H), 7.50-7.30 (m, 3H).

### Beispiel 16A

### 4-Chlor-5-phenylfuro[2,3-d]pyrimidin

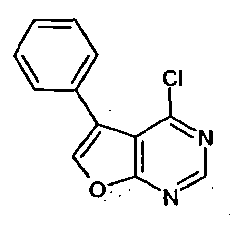

1.8 g (ca. 6.8 mmol) 5-Phenylfuro[2,3-d]pyrimidin-4(3*H*)-on werden bei RT mit 9.5 ml (101.8 mmol) Phosphoroxychlorid versetzt und die Mischung 1 h unter Rückfluss erhitzt. Die resultierende schwarze Mischung wird auf RT gekühlt und vorsichtig tropfenweise bei <10°C zu einer gut gerührten, auf 0°C gekühlten Lösung von 70 ml konz. Ammoniak-Lösung und 50 ml Wasser getropft (pH >9). Nach Ende der Zugabe wird die schwarze Suspension auf RT erwärmt und noch 15 min nachgerührt. Der schwarze Feststoff wird abgesaugt, dreimal mit Wasser wieder aufgeschlämmt, erneut abgesaugt und im Hochvakuum getrocknet. Der Feststoff wird in Dichlormethan gelöst und an Kieselgel säulenfiltriert (Laufmittel: Dichlormethan). Es werden 1371 mg (80.6% d. Th.) der Zielverbindung als gelber Feststoff erhalten.
LC-MS (Methode 5): Rₜ = 2.47 min.; m/z = 231 (M+H)⁺
¹H-NMR (400 MHz, DMSO-d₆): δ = 8.90 (s, 1H), 8.49 (s, 1H), 7.64-7.58 (m, 2H), 7.52-7.45 (m, 3H).

### Beispiel 17A

### 3-{[5-(4-Methoxyphenyl)-6-phenylfuro[2,3-d]pyrimidin-yl]oxy}phenol

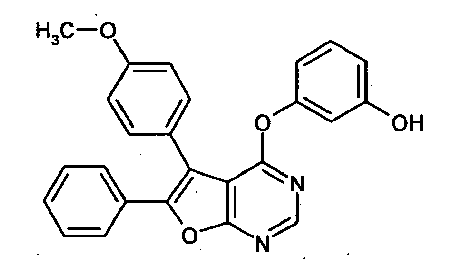

500 mg (1.49 mmol) 4-Chlor-5-(4-methoxyphenyl)-6-phenylfuro[2,3-d]pyrimidin, 654 mg (5.94 mmol) Resorcin und 726 mg (2.23 mmol) Cäsiumcarbonat in 10 ml DMF werden 2 h auf 120°C erhitzt. Nach dem Abkühlen wird abfiltriert und das Filtrat direkt mittels präparativer HPLC gereinigt. Das erhaltene Produkt wird mit Dichlormethan verrührt, abgesaugt, mit Dichlormethan nachgewaschen und im Vakuum getrocknet. Erhalten werden 171.4 mg (27% d. Th.) des Zielprodukts als beiger Feststoff.
LC-MS (Methode 5): Rₜ = 2.78 min.; m/z = 411 (M+H)⁺.

### Beispiel 18A

### 3-{[5-(4-Methoxyphenyl)-6-phenylfuro[2,3-d]pyrimidin-4-yl]oxy}anilin

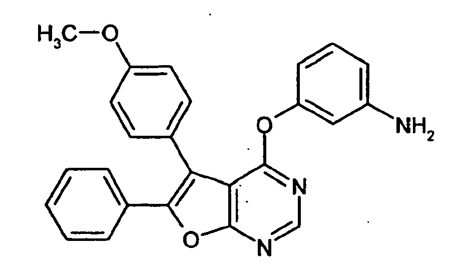

1000 mg (2.97 mmol) 4-Chlor-5-(4-methoxyphenyl)-6-phenylfuro[2,3-d]pyrimidin, 1296 mg (11.9 mmol) 3-Aminophenol und 615.6 mg (4.45 mmol) Kaliumcarbonat in 10 ml DMF werden 8 h bei 80°C gerührt. Nach dem Abkühlen wird im Vakuum eingeengt und der Rückstand in Wasser aufgenommen. Man filtriert vom ausgefallenen Feststoff ab, wäscht den Filterrückstand mehrfach mit Wasser und trocknet den Feststoff bei 50°C im Hochvakuum. Erhalten werden 1195 mg (98.3% d. Th.) des Zielprodukts als bräunlicher Feststoff.
LC-MS (Methode 3): Rₜ = 2.53 min.; m/z = 410 (M+H)⁺
¹H-NMR (300 MHz, DMSO-d₆): δ = 8.53 (s, 1H), 7.60-7.40 (m, 7H), 7.06-6.99 (m, 3H), 6.45 (dd, 1H), 6.34-6.27 (m, 2H), 6.25 (br. s, 2H), 3.80 (s, 3H).

### Beispiel 19A

### 2-(3-{[5-(4-Methoxyphenyl)-6-phenylfuro[2,3-d]pyrimidin-4-yl]amino}phenoxy)essigsäureamid

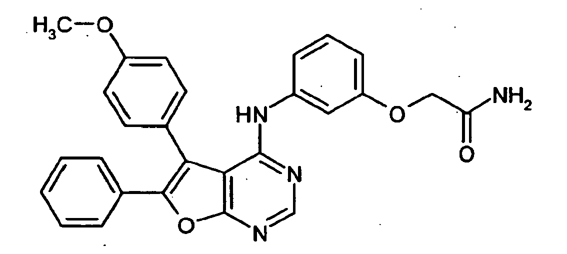

800 mg (1.66 mmol) 3-{[5-(4-Methoxyphenyl)-6-phenylfuro[2,3-d]pyrimidin-4-yl]amino}phenoxy-essigsäuremethylester (Beispiel 9) werden bei RT mit Ammoniak in Methanol (14.2 ml einer ca. 7 M Lösung) versetzt und über Nacht gerührt. Die Mischung wird im Vakuum eingeengt, mit wenig Methanol verrührt und abgesaugt. Der Filterrückstand wird mit Diisopropylether nachgewaschen und über Nacht bei 50°C im Hochvakuum getrocknet. Erhalten werden 663 mg (86.5% d. Th.) des Zielprodukts als nahezu weißer Feststoff.
LC-MS (Methode 3): Rₜ = 2.40 min.; m/z = 467 (M+H)⁺
¹H-NMR (300 MHz, DMSO-d₆): δ = 8.54 (s, 1H), 7.63-7.50 (m, 5H), 7.45-7.20 (m, 9H), 6.90 (s, 1H), 6.81 (dd, 1H), 6.64 (dd, 1H), 4.41 (s, 2H), 3.90 (s, 3H).

### Beispiel 20A

### (3-{[5-(4-Methoxyphenyl)-6-phenylfuro[2,3-d]pyrimidin4-yl]amino}phenoxy)acetonitril

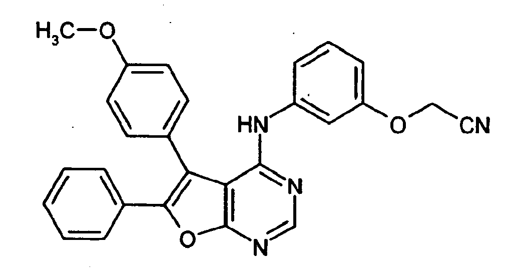

800 mg (1.72 mmol) 2-(3-{[5-(4-Methoxyphenyl)-6-phenylfuro[2,3-d]pyrimidin-4-yl]amino}phenoxy)-essigsäureamid werden in 5 ml DMF gelöst, auf 0°C gekühlt und mit 316 mg (1.72 mmol) Cyanurchlorid versetzt. Die Mischung wird 2 h bei 0°C gerührt. Dann werden Wasser und Ethylacetat zugegeben. Nach Phasentrennung wird die wässrige Phase zweimal mit Ethylacetat extrahiert. Die vereinigten organischen Phasen werden dreimal mit Pufferlösung (pH 7) gewaschen, über Magnesiumsulfat getrocknet und im Vakuum eingeengt. Das Rohprodukt wird durch Chromatographie an Silicagel gereinigt (Laufmittel: Dichlormethan/Ethylacetat 20:1). Es werden 179 mg (22.3% d. Th.) des Zielprodukts als weißer Feststoff erhalten.
LC-MS (Methode 3): Rₜ = 2.84 min.; m/z = 449 (M+H)⁺
¹H-NMR (300 MHz, DMSO-d₆): δ = 8.57 (s, 1H), 7.63-7.51 (m, 4H), 7.45-7.20 (m, 7H), 6.99 (s, 1H), 6.89 (dd, 1H), 6.28 (dd, 1H), 5.65 (s, 2H), 3.90 (s, 3H).

### Beispiel 21A

### 3-(3-Aminophenyl)-propansäuremethylester-Hydrochlorid

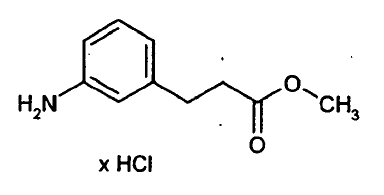

2000 mg (12.1 mmol) 3-(3-Aminophenyl)-propansäure werden in 30 ml Methanol vorgelegt, auf 0°C abgekühlt und tropfenweise mit 0.93 ml (12.7 mmol) Thionylchlorid versetzt. Die Mischung wird langsam auf RT erwärmt und über Nacht gerührt. Nach Einengen im Vakuum wird der Rückstand in wenig Methanol aufgenommen. Nach Zugabe von Diisopropylether wird der ausgefallene Feststoff abgesaugt. Erhalten werden 2450 mg (93.8% d. Th.) des Zielprodukts als weißer Feststoff.
LC-MS (Methode 4): Rₜ = 2.30 min.; m/z = 180 (M-Cl+H)⁺
¹H-NMR (300 MHz, DMSO-d₆): δ = 10.15 (br. s, ca. 3H), 7.42-7.38 (m, 1H), 7.28-7.17 (m, 3H), 3.60 (s, 3H), 2.38 (t, 2H), 2.68 (t, 2H).

Das freie Anilin wird durch Waschen einer Lösung (oder Suspension) des Hydrochlorids in Dichlormethan mit gesättigter Natriumhydrogencarbonat-Lösung und Einengen im Vakuum erhalten.

### Beispiel 22A

### 4-(4-Aminophenyl)-butansäuremethylester-Hydrochlorid

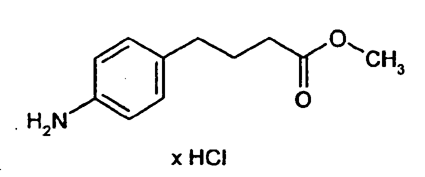

700 mg (3.91 mmol) 4-(4-Aminophenyl)-butansäure werden in 7 ml Methanol vorgelegt, auf 0°C abgekühlt und tropfenweise mit 0.3 ml (4.1 mmol) Thionylchlorid versetzt. Die Mischung wird langsam auf RT erwärmt und über Nacht gerührt. Nach Einengen im Vakuum wird der Rückstand in wenig Methanol verrührt und der anfallende Feststoff abgesaugt. Erhalten werden 800.6 mg (89.2% d. Th.) des Zielprodukts als weißer Feststoff.
LC-MS (Methode 5): Rₜ = 1.10 min.; m/z = 194 (M-Cl+H)⁺.

Das freie Anilin wird durch Waschen einer Lösung (oder Suspension) des Hydrochlorids in Dichlormethan mit gesättigter Natriumhydrogencarbonat-Lösung und Einengen im Vakuum erhalten.

### Beispiele 23A

### 4-(2-Nitrophenyl)-butansäuremethylester

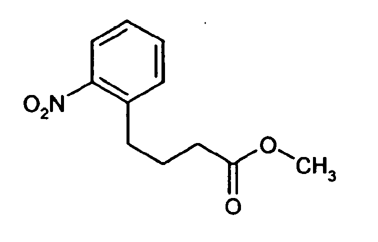

705 mg (3.37 mmol) 4-(2-Nitrophenyl)-butansäure werden in 7 ml Methanol vorgelegt, auf 0°C abgekühlt und tropfenweise mit 0.26 ml (3.54 mmol) Thionylchlorid versetzt. Die Mischung wird langsam auf RT erwärmt und über Nacht gerührt. Zur Vervollständigung der Reaktion werden ca. 20% Überschuss an Thionylchlorid nachgesetzt und die Mischung weitere 5 h bei RT gerührt. Nach Einengen im Vakuum wird der Rückstand in wenig Methanol verrührt und der anfallende Feststoff abgesaugt. Erhalten werden 700 mg (95.5% d. Th.) des Zielprodukts als weißer Feststoff.
LC-MS (Methode 5): Rₜ = 2.34 min.; m/z = 224 (M+H)⁺.

### Beispiel 24A

### 4-(2-Aminophenyl)-butansäuremethylester

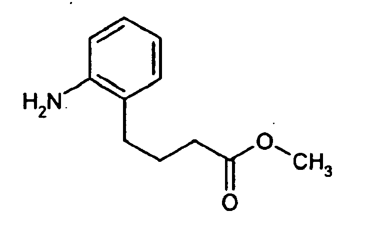

715 mg (3.2 mmol) 4-(2-Nitrophenyl)-butansäuremethylester werden in 1.5 ml Ethanol vorgelegt, unter Argon mit 34 mg Palladium auf Aktivkohle (10%) versetzt und unter Wasserstoffatmosphäre (Normaldruck) über Nacht bei RT gerührt. Um die Reaktion zu vervollständigen, wird erneut Palladium auf Aktivkohle zugesetzt und weitere 24 h bei RT unter Wasserstoffatmosphäre gerührt. Man filtriert vom Katalysator ab und konzentriert die Lösung im Vakuum. Erhalten werden 229 mg (37.1% d. Th.) des Zielprodukts.
LC-MS (Methode 5): Rₜ = 1.67 min.; m/z = 194 (M+H)⁺.

### Beispiel 25A

### 2-(3-{[5-(4-Methoxyphenyl)-6-phenylfuro[2,3-d]pyrimidin-4-yl]amino}phenyl)essigsäurehydrazid

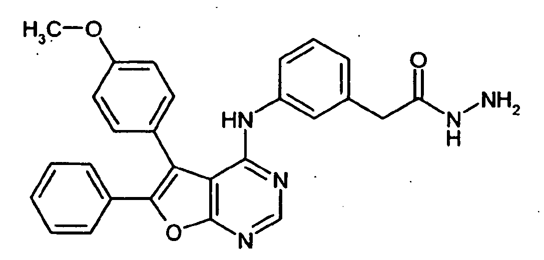

Zu 100 mg (0.215 mmol) 3-{[5-(4-Methoxyphenyl)-6-phenylfuro[2,3-d]pyrimidin-4-yl]amino}-phenyl-essigsäuremethylester (Beispiel 28), gelöst in 1.5 ml THF und 2 ml Methanol, werden bei RT 215 mg (4.3 mmol) Hydrazinhydrat gegeben. Die Mischung wird 1 h bei 65°C und über Nacht bei RT gerührt und dann im Vakuum eingeengt. Nach Verrühren mit Diisopropylether wird der Feststoff abgesaugt und im Vakuum getrocknet. Man erhält 89.3 mg (89.3% d. Th.) des Zielprodukts.
LC-MS (Methode 3): Rₜ = 2.16 min.; m/z = 466 (M+H)⁺
¹H-NMR (400 MHz, DMSO-d₆): δ = 9.20 (s, 1H), 8.51 (s, 1H), 7.62-7.51 (m, 4H), 7.43-7.36 (m, 3H), 7.30-7.20 (m, 5H), 6.95 (d, 1H), 6.89 (s, 1H), 4.24 (br. s, 2H), 3.89 (s, 3H).

### Allgemeine Vorschrift A: Umsetzung von Aminen mit 4-Chlorfuro[2.3-d]pyrimidin-Derivaten

Eine Mischung aus 1.0 eq. 4-Chlor-5-(4-methoxyphenyl)-6-phenylfuro[2,3-d]pyrimidin, 1.0 bis 1.4 eq. Amin-Komponente und 1.5 bis 3.0 eq. DIEA wird in DMF (Konzentration 0.5 bis 1.5 mol/l) 1-24 h lang bei 80-140°C gerührt. Nach dem Abkühlen wird das DMF im Vakuum entfernt und der Rückstand mit Wasser behandelt. Man extrahiert mit Dichlormethan, wäscht die organische Phase mit gesättigter Natriumhydrogencarbonat- und Natriumchlorid-Lösung, trocknet über Magnesiumsulfat und konzentriert im Vakuum. Aus dem Rohprodukt kann die Zielverbindung durch Kristallisation aus alkoholischen Lösungsmitteln (z.B. Methanol), durch Chromatographie an Silicagel (bevorzugte Laufmittelsysteme sind Dichlorinethan/Methanol und Cyclohexan/Ethylacetat), durch präparative RP-HPLC (Eluent: Wasser/Acetonitril) oder durch eine Kombination dieser Methoden isoliert und gereinigt werden.

### Allgemeine Vorschrift B: Umsetzung von Alkoholen mit 4-Chlorfuro[2,3-d]pyrimidin-Derivaten

Zu einer Mischung aus 1.0 eq. 4-Chlor-5-(4-methoxyphenyl)-6-phenylfuro[2,3-d]pyrimidin und 1.0 bis 1.5 eq. Alkohol-Komponente in THF oder DMF (oder Gemischen hieraus; Konzentration 0.2 bis 1.0 mol/l) wird bei 0°C bis RT eine Lösung von 1.0 bis 1.5 eq. Phosphazen-Base P2-t-Bu in THF (ca. 2 mol/l) [Fa. Fluka, Art.-Nr. 79416] oder Phosphazen-Base P4-t-Bu in Cyclohexan (ca. 1 mol/l) [Fa. Fluka, Art.-Nr. 79421] getropft. Die Mischung wird 30 min bis 6 h bei RT gerührt. Danach wird mit Dichlormethan oder Ethylacetat verdünnt und wässrig aufgearbeitet Die organische Phase wird mit 1 N Salzsäure, gesättigter Natriumhydrogencarbonat-Lösung und/oder Natriumchlorid-Lösung gewaschen, über Magnesiumsulfat getrocknet und im Vakuum eingeengt. Aus dem Rohprodukt kann die Zielverbindung durch Kristallisation aus alkoholischen Lösungsmitteln (z.B. Methanol), durch Chromatographie an Silicagel (bevorzugte Laufmittelsysteme sind Dichlormethan/Methanol und Cyclohexan/Ethylacetat), durch präparative RP-HPLC (Eluent: Wasser/Acetonitril) oder durch eine Kombination dieser Methoden isoliert und gereinigt werden.

Die folgenden Verbindungen werden gemäß der Allgemeinen Vorschrift A oder B ausgehend von 4-Chlor-5-(4-methoxyphenyl)-6-phenylfuro[2,3-d]pyrimidin und den entsprechenden Aminen bzw. Alkoholen hergestellt:

| **Beispiel** | **Struktur** | **Analytische Daten** |
|---|---|---|
| **26A** | 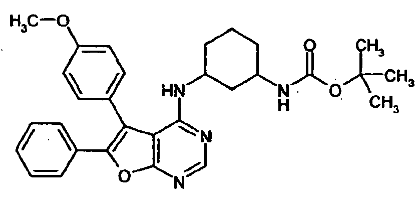 | LC-MS (Methode 6): Rₜ = 3.10 min.; m/z = 515 (M+H)⁺. |
| | (*+*/*-*)*-cis*/*trans* | |
| **27A** | 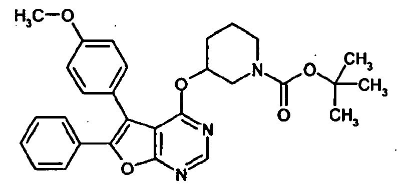 | LC-MS (Methode 6): Rₜ = 3.22 min.; m/z = 502 (M+H)⁺ ¹H-NMR (400 MHz, DMSO-d₆): δ = 8.61 (s, 1H), 7.52 (d, 2H), 7.45-7.31 (m, 5H), 7.0 (d, 2H), 5.20 (br. s, 1H), 4.34 (br. d, 1H), 3.90-3.80 (m, 4H), 3.14 (br. d, 1H), 2.28 (br. t, 1H), 1.85-1.68 (m, 2H), 1.45-1.25 (m, 5H), 0.97 (s, 9H). |
| | (rac.) | |
| **28A** | 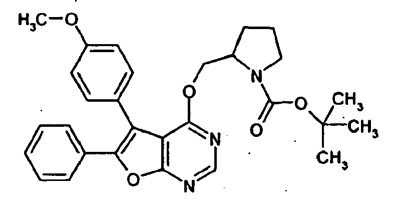 | LC-MS (Methode 6): Rₜ = 3.27 min.; m/z = 502 (M+H)⁺ ¹H=NMR (400 MHz, DMSO-d₆): δ = 8.58 (s, 1H), 7.52 (d, 2H), 7.42-7.37 (m, 5H), 7.03 (d, 2H), 4.58-4.40 (m, 2H), 3.90-3.81 (m, 4H), 3.22-3.13 (m, 1H), 2.95-2.80 (m, 1H), 1.88-1.75 (m, 1H), 1.64-1.45 (m, 3H), 1.35 (s, 9H). |
| | (rac.) | |
| **29A** | 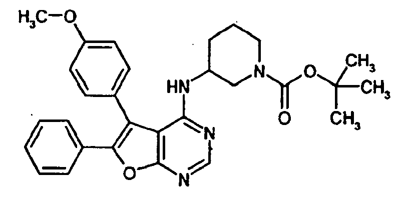 | LC-MS (Methode 6): Rₜ = 3.06 min.; m/z = 501 (M+H)⁺ ¹H-NMR (400 MHz, DMSO-d₆): δ = 8.39 (s, 1H), 7.49 (d, 2H), 7.47-7.33 (m, 5H), 7.15 (d, 2H), 4.92 (d, 1H), 4.05 (br. s, 1H), 3.86 (s, 3H), 3.60 (br. s, 1H), 3.17 (br. s, 1H), 2.89 (br. s, 1H), 1.75 (br. t, 1H), 1.45-1.0 (m, 12H). |
| | (rac.) | |
| **30A** | 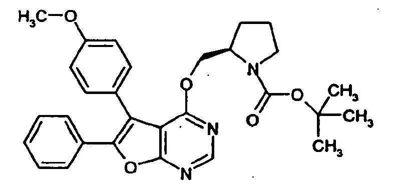 | LC-MS (Methode 6): Rₜ = 3.32 min.; m/z = 502 (M+H)⁺ ¹H-NMR (400 MHz, DMSO-d₆): δ = 8.58 (s, 1H), 7.52 (d, 2H), 7.42-7.37 (m, 5H), 7.03 (d, 2H), 4.59-4.40 (m, 2H), 3.87 (m, 1H), 3.81 (s, 3H), 3.22-3.13 (m, 1H), 2.95-2.80 (m, 1H), 1.88-1.75 (m, 1H), 1.64-1.45 (m, 3H), 1.35 (s, 9H). |
| | (R-Enantiomer) | |
| **31A** | 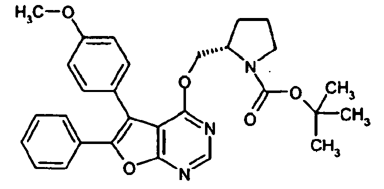 | LC-MS (Methode 3): Rₜ = 3.14 min.; m/z = 502 (M+H)⁺ ¹H-NMR (400 MHz, DMSO-d₆): δ = 8.58 (s, 1H), 7.52 (d, 2H), 7.42-7.37 (m, 5H), 7.03 (d, 2H), 4.59-4.40 (m, 2H), 3.87 (m, 1H), 3.81 (s, 3H), 3.22-3.13 (m, 1H), 2.95-2.80 (m, 1H), 1.88-1.75 (m, 1H), 1.64-1.45 (m, 3H), 1.35 (s, 9H). |
| | (*S*-Enantiomer) | |

### Beispiel 32A

### (+/-)-cis/trans-3-{[5-(4-Methoxyphenyl)-6-phenylfuro[2,3-d]pyrimidin-4-yl]oxy}cyclohexanol

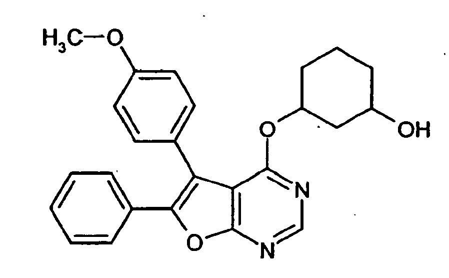

1.552 g (13.36 mmol) 1,3-Cyclohexandiol (*cis*/*trans*-Gemisch) werden in 12 ml absolutem THF gelöst, auf 0°C gekühlt und mit 13.36 ml Phosphazen-Base P2-t-Bu in THF (ca. 2 M Lösung) versetzt. Nach Ende der Zugabe wird ca. 10 min bei RT nachgerührt, dann erneut auf 0°C abgekühlt und 3.0 g (8.91 mmol) 4-Chlor-5-(4-methoxyphenyl)-6-phenylfuro[2,3-d]pyrimidin in Portionen hinzugefügt. Die Reaktionsmischung wird 1 h bei RT gerührt, auf Wasser gegeben und dreimal mit Ethylacetat extrahiert. Die vereinigten organischen Phasen werden mit gesättigter Natriumchlorid-Lösung gewaschen, über Magnesiumsulfat getrocknet und im Vakuum eingeengt. Der Rückstand wird durch Chromatographie an Silicagel gereinigt (Laufmittel: Cyclohexan/Ethylacetat 5:1 → 1:1). Erhalten werden 3.02 g (81% d. Th.) der Zielverbindung.
LC-MS (Methode 3):
Isomer 1 Rₜ = 2.52 min.; m/z = 417 (M+H)⁺,
Isomer 2 Rₜ = 2.55 min.; m/z = 417 (M+H)⁺.

Die Trennung des so erhaltenen *cis*/*trans*-Isomerengemisches erfolgt mittels HPLC [Eluent: Wasser/Acetonitril 1:1; Kromasil-Säule 100 C 18, 250 mm x 20 mm; Fluss: 25 ml/min; UV-Detektion: 210 nm; Temperatur: 30°C]. Aufgegeben werden 2.0 g Isomerengemisch, gelöst in 35 ml THF und ca. 15 ml Wasser, in mehreren Injektionen (Injektionsvolumen ca. 1 ml). Erhalten werden 750 mg (+/-)-*cis*-3-([5-(4-Methoxyphenyl)-6-phenylfuro[2,3-d]pyrimidin-4-yl]oxy)cyclohexanol (Beispiel 33A) sowie 640 mg (+/-)-*trans*-3-{[5-(4-Methoxyphenyl)-6-phenylfuro[2,3-d]pyrimidin-4-yl]oxy}-cyclohexanol (Beispiel 34A).

### Beispiel 33A

### (+/-)-cis-3-{[5-(4-Methoxyphenyl)-6-phenylfuro[2,3-d]pyrimidin4-yl]oxy}cyclohexanol

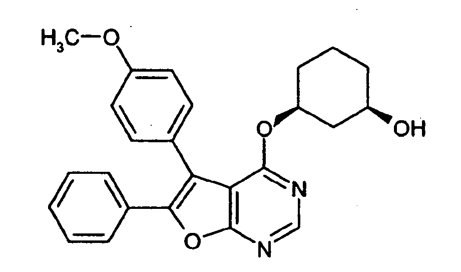

LC-MS (Methode 5): Rₜ = 2.86 min.; m/z = 417 (M+H)⁺
¹H-NMR (400 MHz, DMSO-d₆): δ = 8.57 (s, 1H), 7.53 (m, 2H), 7.42-7.37 (m, 5H), 7.0 (d, 2H), 5.11 (m, 1H), 4.69 (d, 1H), 3.83 (s, 3H), 2.27 (d, 1H), 2.01 (d, 1H), 1.79 (d, 1H), 1.62-1.68 (m, 1H), 1.30-1.05 (m, 4H).

### Beispiel 34A

### (+/-)-trans-3-{[5-(4-Methoxyphenyl)-6-phenylfuro[2,3-d]pyrimidin-4-yl]oxy}cyclohexanol

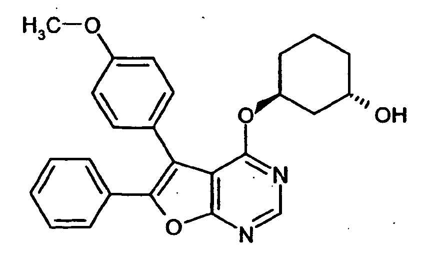

LC-MS (Methode 5): Rₜ = 2.88 min.; m/z = 417 (M+H)⁺
¹H-NMR (400 MHz, DMSO-d₆): δ = 8.58 (s, 1H), 7.54 (d, 2H), 7.42-7.39 (m, 5H), 7.04 (d, 2H), 5.59 (m, 1H), 4.42 (d, 1H), 3.82 (s, 3H), 3.48 (m, 1H), 1.90-1.82 (m, 1H), 1.62-1.45 (m, 5H), 1.25-1.15 (m, 2H).

### Beispiel 35A

### (+/-)-all-cis-5-{[5-(4-Methoxyphenyl)-6-phenylfuro[2,3-d]pyrimidin4-yl]oxy}cyclohexan-1,3-diol

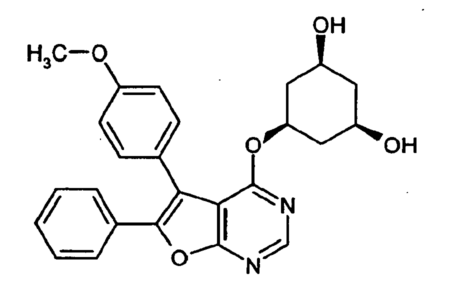

Dehydratisierung von *all*-*cis*-1,3,5-Cyclohexantriol-Dihydrat: *all*-*cis*-1,3,5-Cyclohexantriol-Dihydrat wird in DMF bei 70°C gelöst. Im Vakuum werden die flüchtigen Komponenten entfernt und der Rückstand im Hochvakuum getrocknet.

0.81 g (6.12 mmol) *all*-*cis*-1,3,5-Cyclohexantriol werden in 15 ml DMF gelöst, auf 0°C gekühlt und in Portionen mit 245 mg Natriumhydrid (ca. 60%-ige Dispersion in Öl, ca. 6.12 mmol) versetzt. Die Suspension wird 1 h bei RT und ca. 2.5 h bei 40-50°C gerührt. Nach Abkühlen auf RT werden 1.376 g (4.09 mmol) 4-Chlor-5-(4-methoxyphenyl)-phenylfuro[2,3-d]pyrimidin zugegeben und die Mischung über Nacht bei RT gerührt. Vorsichtig gibt man die Reaktionsmischung auf Wasser. Nach Sättigen mit Natriumchlorid wird dreimal mit Ethylacetat extrahiert. Die vereinigten organischen Phasen werden über Magnesiumsulfat getrocknet und im Vakuum eingeengt. Der Rückstand wird an Silicagel chromatographiert (Laufmittel: Dichlormethan/Methanol 30:1 → 4:1). Erhalten werden 1.38 g (77.8% d. Th.) der Zielverbindung.
LC-MS (Methode 3): Rₜ = 2.04 min.; m/z = 433 (M+H)⁺
¹H-NMR (400 MHz, DMSO-d₆): δ = 8.59 (s, 1H), 7.55 (m, 2H), 7.46-7.40 (m, 5H), 7.02 (d, 2H), 5.61 (m, 1H), 4.74 (d, 2H), 3.84 (s, 3H), 3.58-3.49 (m, 2H), 2.27-2.20 (m, 2H), 2.05 (d, 1H), 1.06 (q, 3H).

### Allgemeine Vorschrift C: Abspaltung von Boc-Schutzgruppen

Zu einer Lösung von Boc-geschütztem Amin in Dichlormethan (Konzentration 0.1 bis 1.5 mol/l, evtl: mit wenigen Tropfen Wasser) werden bei 0°C bis RT 0.5-1.0 Volumenteile TFA getropft (es resultiert ein Dichlormethan/TFA-Verhältnis von ca. 2:1 bis 1:1). Die Mischung wird für einen Zeitraum von 30 min bis 18 h bei RT gerührt. Nach Verdünnen mit Dichlormethan wird mit gesättigter Natriumcarbonat- oder Natriumhydrogencarbonat-Lösung gewaschen. Man trocknet die organische Phase über Magnesium- oder Natriumsulfat und konzentriert im Vakuum. Gegebenenfalls kann eine weitere Reinigung des Amins durch präparative HPLC oder Chromatographie an Silicagel (Laufmittel: Dichlormethan/Methanol) erfolgen.

Die folgenden Verbindungen werden gemäß der Allgemeiner Vorschrift C ausgehend von den Verbindungen 26A - 31A hergestellt:

| **Beispiel** | **Struktur** | **Analytische Daten** |
|---|---|---|
| **36A** | 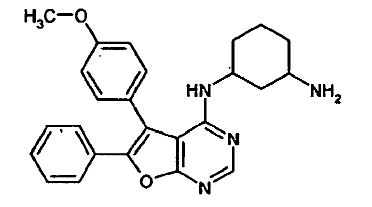 | LC-MS (Methode 3): Rₜ = 1.56 min.; m/z = 415 (M+H)⁺. |
| | (*+*/*-*)*-cis*/*trans* | |
| **37A** | 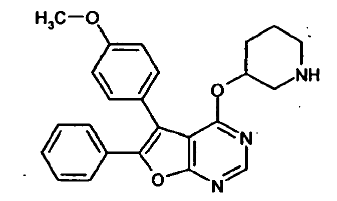 | LC-MS (Methode 5): Rₜ =1.81 min.; m/z = 402 (M+H)⁺ ¹H-NMR (400 MHz, DMSO-d₆): δ = 8.68 (s, 1H), 7.56 (d, 2H), 7.49-7.39 (m, 5H), 7.03 (d, 2H), 5.15 (m, 1H), 3.83 (s, 3H), 3.02 (dd, 1H), 2.72-2.55 (m, 3H), 1.97-1.90 (m, 1H), 1.56-1.32 (m, 3H). |
| | (rac.) | |
| **38A** | 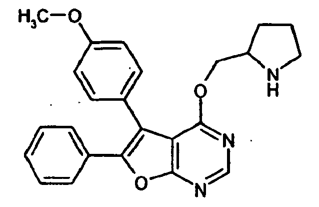 | LC-MS (Methode 3): Rₜ = 1.54 min.; m/z = 402 (M+H)⁺ ¹H-NMR (400 MHz, DMSO-d₆): δ = 8.59 (s, 1H), 7.55 (d, 2H), 7.46-7.39 (m, 5H), 7.03 (d, 2H), 4.31 (q, 2H), 3.82 (s, 3H), 3.5-3.30 (m, 2H), 2.80-2.72 (m, 1H), 2.20-2.12 (m, 1H), 1.78-1.70 (m, 1H), 1.60-1.55 (m, 2H), 1.40-1.30 (m, 1H). |
| | (rac.) | |
| **39A** | 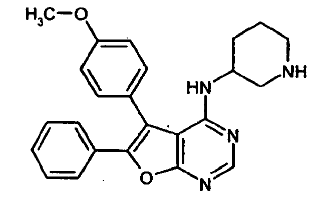 | LC-MS (Methode 3): **R**ₜ = 1.52 min.; m/z = 401 (M+H)⁺ ¹H-NMR (400 MHz, DMSO-d₆): δ = 8.35 (s, 1H), 7.50-7.47 (m, 4H), 7.40-7.31 (m, 4H), 7.15 (d, 2H), 5.29 (d, 1H), 4.19 (m, 1H), 3.86 (s, 3H), 2.95 (d, 1H), 2.75-2.69 (m, 1H), 2.62-2.58 (m, 1H), 2.50-2.47 (m, 2H), 1.73-1.68 (m, 1H), 1.47-1.32 (m, 3H). |
| | (rac.) | |
| **40A** | 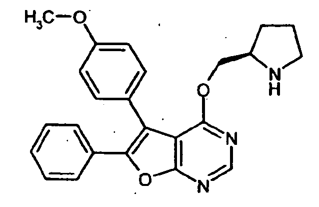 | LC-MS (Methode 6): Rₜ = 1.66 min.; m/z = 402 (M+H)⁺ ¹H-NMR (400 MHz, DMSO-d₆): δ = 8.58 (s, 1H), 7.55 (d, 2H), 7.46-7.39 (m, 5H), 7.03 (d, 2H), 4.30 (dd, 1H), 4.20 (dd, 1H), 3.82 (s, 3H), 3.20 (m, 1H), 2.70 (m, 2H), 1.69-1.52 (m, 3H), 1.30-1.20 (m, 2H). |
| | (R-Enantiomer) | |
| **41A** | 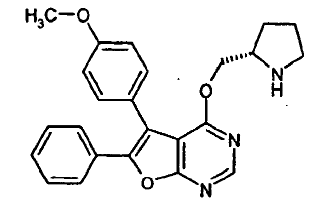 | LC-MS (Methode 6): Rₜ = 1.66 min.; m/z = 402 (M+H)⁺ ¹H-NMR (400 MHz, DMSO-d₆): δ = 8.58 (s, 1H), 7.55 (d, 2H), 7.46-7.39 (m, 5H), 7.03 (d, 2H), 4.30 (dd, 1H), 4.20 (dd, 1H), 3.82 (s, 3H), 3.20 (m, 1H), 2.70 (m, 2H), 1.69-1.52 (m, 3H), 1.30-1.20 (m, 2H). |
| | (*S*-Enantiomer) | |

### Beispiel 42A

### (+/-)-cis-{[3-Hydroxycyclohexyl]oxy}-essigsäure-tert.-butylester

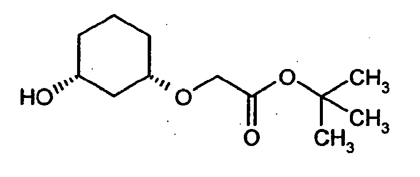

5.0 g (43 mmol) *cis*/*trans*-1,3-Cyclohexandiol (ca. 1.2:1 *cis*/*trans*-Gemisch) werden in 20 ml absolutem THF gelöst und bei RT tropfenweise mit 24.8 ml (ca. 49.5 mmol) Phosphazen-Base P2-t-Bu in THF (ca. 2 M Lösung) versetzt. Die Lösung wird 30 min bei RT nachgerührt und anschließend zu einer Mischung aus 9.5 ml (64.6 mmol) Bromessigsäure-*tert*.-butylester und 10 ml THF getropft. Man rührt über Nacht bei RT, verdünnt dann mit Dichlormethan, wäscht die organische Phase nacheinander mit 1 N Salzsäure, Pufferlösung (pH 7) sowie Natriumchlorid-Lösung, trocknet über Natriumsulfat und konzentriert im Vakuum. Das Rohprodukt wird mittels Chromatographie an Silicagel aufgetrennt (Laufmittel: Cyclohexan/Ethylacetat 5:1 → 1:1). Isoliert werden als Reinfraktion 2.73 g (27.6% d. Th.) der *cis*-konfigurierten Zielverbindung.
MS (DCI): m/z = 248 (M+NH₄)⁺
¹H-NMR (400 MHz, DMSO-d₆): δ = 4.59 (d, 1H), 3.95 (s, 2H), 3.38-3.21 (m, 2H), 2.20-2.12 (m, 1H), 1. 89 (d, 1H), 1.74 (d, 1H), 1.66-1.60 (m, 1H), 1.41 (s, 9H), 1.14-0.95 (m, 4H).

### Beispiel 43A

### (+/-)-trans-{[3-Aminocyclohexyl]oxy}-essigsäure-tert.-butylester

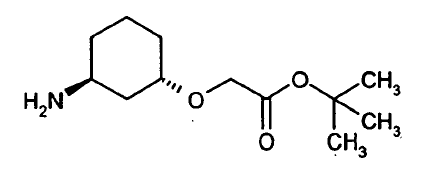

### Stufe a):

500 mg (2.17 mmol) (+/-)-*cis*-{[3-Hydroxycyclohexyl]oxy}-essigsäure-*tert*.-butylester und 0.907 ml (6.51 mmol) Triethylamin werden in 2 ml Dichlormethan gelöst und auf 0°C gekühlt. Tropfenweise werden 0.20 ml (2.61 mmol) Methansulfonsäurechlorid zugegeben. Die Mischung wird 1 h bei 0°C nachgerührt und dann auf Wasser gegeben. Die organische Phase wird abgetrennt und die wässrige mit Dichlormethan extrahiert. Die vereinigten organischen Phasen werden mit gesättigter Natriumchlorid-Lösung gewaschen, über Magnesiumsulfat getrocknet und im Vakuum konzentriert. Es werden 690 mg des Mesylats erhalten, welches direkt weiter umgesetzt wird.

### Stufe b):

690 mg des oben erhaltenen Mesylats werden bei RT in 2 ml DMF gelöst und mit 873 mg (13.4 mmol) Natriumazid versetzt. Die Suspension wird über Nacht bei 60°C kräftig gerührt und dann nach Abkühlen auf Wasser gegeben. Man extrahiert dreimal mit Ethylacetat, wäscht die vereinigten organischen Phasen mit gesättigter Natriumchlorid-Lösung, trocknet über Magnesiumsulfat und konzentriert im Vakuum. Es werden 416 mg des Azids als gelbliches Öl erhalten, welches direkt weiter umgesetzt wird.

### Stufe c):

418 mg des oben erhaltenen Azids werden in 1.8 ml Ethanol und 0.2 ml Wasser gelöst, mit Palladium auf Aktivkohle versetzt und 2 h bei RT unter Wasserstoffatmosphäre (Normaldruck) gerührt. Der Katalysator wird durch Filtration über Kieselgur entfernt, das Filtrat im Vakuum eingeengt und der Rückstand im Hochvakuum getrocknet. Es werden 456 mg der Titelverbindung erhalten, welche ohne weitere Aufreinigung eingesetzt wird.

### Beispiel 44A

### (+/-)-cis/trans-3-{[5-(4-Methoxyphenyl)-6-phenylfuro[2,3-d]pyrimidin-4-yl]amino}cyclohexanol

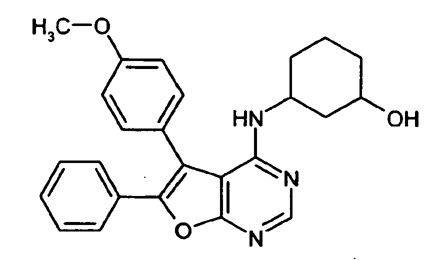

In 2.7 ml DMF werden 1.0 g (2.97 mmol) 4-Chlor-5-(4-methoxyphenyl)-6-phenylfuro[2,3-d]pyrimidin und 513 mg (ca. 4.5 mmol) (+/-)-*cis*/*trans*-3-Aminocyclohexanol vorgelegt (ca. 3:1 *cis*l*trans*-Gemisch; hergestellt gemäß J. Chem. Soc. Perkin Trans. 1, 1994, 537). Nach Zugabe von 1.03 ml (5.94 mmol) DIEA wird die Mischung für 2 h auf 120°C erhitzt. Nach Abkühlen wird auf Eiswasser gegeben. Der ausgefallene Feststoff wird abgesaugt, mit Wasser gewaschen und im Vakuum getrocknet. Das Rohprodukt wird durch Chromatographie an Silicagel gereinigt (Laufmittel: Cyclohexan/Ethylacetat 2:1 → 1:2). Erhalten werden 1.45 g (85.1% d. Th.) des Zielprodukts als *cis*/*trans*-Gemisch.
LC-MS (Methode 6): Rₜ = 2.53 min.; m/z = 416 (M+H)⁺.

### Beispiel 45A

### (+/-)-cis-3-[(6-Phenylfuro[2,3-d]pyrimidin-4-yl)amino]cyclohexanol

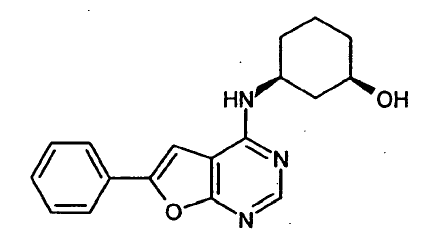

Eine Mischung aus 4.0 g (17.34 mmol) 4-Chlor-6-phenylfuro[2,3-d]pyrimidin, 4.5 ml (26 mmol) DIEA und 2.8 g (+/-)-*cis*/*trans*-3-Aminocyclohexanol (ca. 85%-ig, ca. 20.8 mmol; ca. 3:1 *cis*/*trans-*Gemisch; hergestellt gemäß J. Chem. Soc. Perkin Trans. I, 1994, 537) in 15 ml DMF wird über Nacht auf 120°C erhitzt. Nach dem Abkühlen wird die Reaktionsmischung auf Wasser gegeben und dreimal mit Ethylacetat extrahiert. Die vereinigten organischen Phasen werden mit gesättigter Natriumchlorid-Lösung gewaschen, über Magnesiumsulfat getrocknet und im Vakuum eingeengt. Durch wiederholtes Verrühren des Rohprodukts mit einem Gemisch aus Methyl-*tert*.-butylether und Dichlormethan wird das Produkt in der Mutterlauge angereichert. Nach Einengen der Mutterlauge wird das Produkt nach Kristallisation aus Dichlormethan/Methanol (10:1) abgesaugt und im Vakuum getrocknet. Erhalten werden 1.11 g (20.7% d. Th.) des Zielprodukts.
LC-MS (Methode 6): Rₜ = 1.95 min.; m/z = 310 (M+H)⁺
¹H-NMR (400 MHz, DMSO-d₆): δ = 8.25 (s, 1H), 7.88-7.72 (m, 3H), 7.55-7.47 (m, 2H), 7.46-7.38 (m, 2H), 4.71 (d, 1H), 4.11-4.01 (m, 1H), 3.59-3.47 (m, 1H), 2.19 (d, 1H), 1.96-1.19 (m, 3H)_{,} 1.36-1.05 (m, 4H).

### Beispiel 46A

### (+/-)-cis-({3-[(6-Phenylfuro[2,3-d]pyrimidin-4-yl)amino]cyclohexyl}oxy)-essigsäure-tert.-butylester

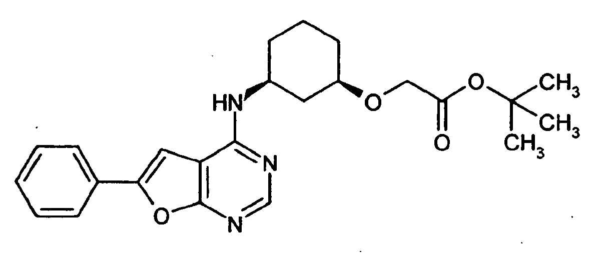

Zu einer Mischung von 517 mg 50%-iger Natronlauge (6.5 mmol) und 0.5 ml Toluol werden bei 40°C ca. 0.06 mmol Tetrabutylammoniumhydrogensulfat sowie eine Lösung von 200 mg (0.646 mmol) (+/-)-*cis*-3-[(6-Phenylfuro[2,3-d]pyrimidin-4-yl)amino]cyclohexanol in 0.5 ml Toluol und 0.1 ml THF gegeben. Die resultierende Mischung wird unter kräftigem Rühren mit 0.19 ml (1.29 mmol) Bromessigsäure-*tert*.-butylester versetzt und auf 70°C erhitzt. Nach 2 h wird die Mischung abgekühlt und auf Wasser gegeben. Man extrahiert dreimal mit Dichlormethan, wäscht die vereinigten organischen Phasen mit gesättigter Natriumchlorid-Lösung und konzentriert im Vakuum. Aus dem Rohprodukt werden nach präparativer RP-HPLC (Eluent: Acetonitril/Wasser) 152 mg (55.5% d. Th.) des Zielprodukts isoliert.
LC-MS (Methode 6): Rₜ = 2.87 min.; m/z = 424 (M+H)⁺
¹H-NMR (400 MHz, DMSO-d₆): δ = 8.26 (s, 1H), 7.89-7.78 (m, 3H), 7.51 (t, 2H), 7.45-7.39 (m, 2H), 4.17-4.05 (m, 1H), 4.01 (s, 2H), 3.49-3.40 (m, 1H), 2.33 (br. d, 1H), 2.01 (br. d, 1H), 1.91 (br. d, 1H), 1.81-1.75 (m, 1H), 1.42 (s, 9H), 1.34-1.10 (m, 4H).

### Beispiel 47A

### (+/-)-cis-({3-[(5-Brom-6-phenylfuro[2,3-d]pyrimidin-4-yl)amino]cyclohexyl}oxy)-essigsäure-tert-butylester

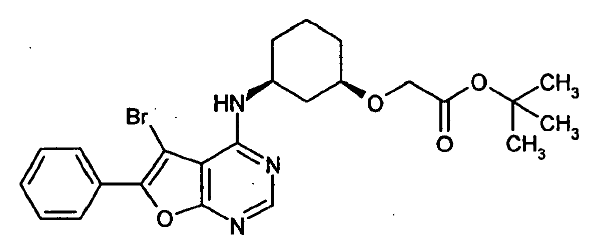

132 mg (0.312 mmol) (+/-)-*cis*-({3-[(6-Phenylfuro[2,3-d]pyrimidin-4-yl)amino]cyclohexyl}oxy)-essigsäure-*tert*.-butylester werden in 0.3 ml Tetrachlormethan suspendiert, mit 61 mg (0.343 mmol) NBS versetzt und auf Rückfluss erhitzt. Nach vollständigem Umsatz (ca. 1 h) wird die Reaktionsmischung abgekühlt und das Produkt direkt durch präparative RP-HPLC isoliert. Erhalten werden 104 mg (66.4% d. Th.) der Zielverbindung.
LC-MS (Methode 5): Rₜ = 3.36 min.; m/z = 502, 504 (M+H)⁺
¹H-NMR (400 MHz, DMSO-d₆): δ = 8.34 (s, 1H), 8.01 (d, 2H), 7.60-7.49 (m, 3H), 7.08 (br. d, 1H); 4.42-4.25 (m, 1H), 4.09 (s, 2H), 3.65-3.58 (m, 1H), 2.11 (br. d, 1H), 1.81-1.68 (m, 4H), 1.65-1.47 (m, 2H), 1.43 (s, 9H), 1.41-1.30 (m, 1H).

### Beispiel 48A

### (+/-)-cis-({3-[(5-Phenylfuro[2,3-d]pyrimidin-4-yl)oxy]cyclohexyl}oxy)-essigsäure-tert.-butylester

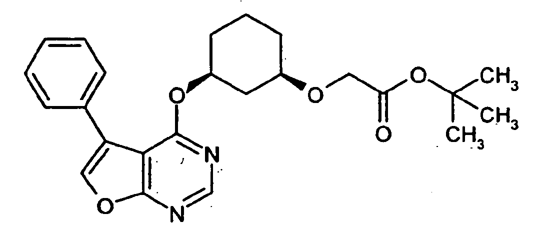

500 mg (2.17 mmol) (+/-)-*cis*-{[3-Hydroxycyclohexyl]oxy}-essigsäure-*tert*.-butylester werden in 2.0 ml trockenem THF gelöst, auf 0°C abgekühlt und mit 1.24 ml (ca. 2.5 mmol) einer 2 N Lösung von Phosphazen-Base P2-t-Bu in THF versetzt. Nach Entfernen der Kühlung wird die Mischung 30 min bei RT nachgerührt, bevor 500.7 mg (2.17 mmol) 4-Chlor-5-phenylfuro[2,3-d]pyrimidin bei RT zugegeben werden. Die Mischung wird über Nacht bei RT gerührt und dann im Vakuum eingeengt. Der Rückstand wird durch Chromatographie an Silicagel (Laufmittel: Cyclohexan/Ethylacetat 10:1 → 8:1) aufgereinigt. Das Zielprodukt wird nach weiterer Reinigung mittels RP-HPLC (Eluent: Acetonitril/Wasser) erhalten. Es werden 380 mg (41.2% d. Th.) isoliert.
LC-MS (Methode 3): Rₜ = 2.89 min.; m/z = 425 (M+H)⁺
¹H-NMR (400 MHz, DMSO-d₆): δ = 8.61 (s, 1H), 8.35 (s, 1H), 7.27 (d, 2H), 7.49-7.39 (m, 3H), 5.25 (m, 1H), 3.99 (s, 2H), 3.47 (m, 1H), 2.12 (br. s, 1H), 1.99 (br. d, 1H), 1.32-1.26 (m, 1H), 1.41 (s, 9H), 1.40-1.12 (m, 5H).

### Beispiel 49A

### (+/-)-cis-({3-[(6-Brom-5-phenylfuro[2,3-d]pyrimidin-4-yl)oxy]cyclohexyl}oxy)-essigsäure-tert.-butylester

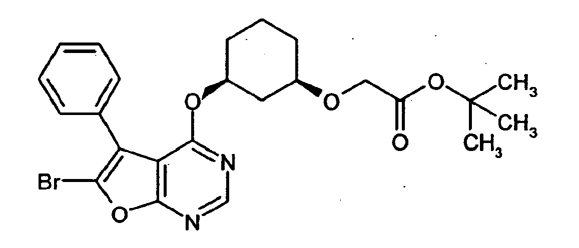

100 mg (0.236 mmol) (+/-)-*cis*-({3-[(5-Phenylfuro[2,3-d]pyrimidin-4-yl)oxy]cyclohexyl}oxy)-essigsäure-*tert*.-butylester werden in 0.2 ml Tetrachlormethan suspendiert und mit 46.1 mg (0.259 mmol) NBS versetzt. Die Reaktionsmischung wird insgesamt 2 h bei 60°C gerührt, wobei nach 1 h weitere 23 mg NBS zugegeben werden. Nach Abkühlen wird das Tetrachlormethan im Vakuum entfernt und der Rückstand durch präparative RP-HPLC aufgereinigt (Eluent: Acetonitril/Wasser). Erhalten werden 43.6 mg (36.8% d. Th.) des Zielprodukts.
LC-MS (Methode 5): Rₜ = 3.31 min.; m/z = 503, 505 (M+H)⁺
¹H-NMR (400 MHz, DMSO-d₆): δ = 8.59 (s, 1H), 7.62 (d, 2H), 7.53-7.43 (m, 3H), 5.15 (m, 1H), 3.98 (s, 2H), 3.41 (m, 1H), 2.45 (br. d, 1H), 2.04 (br. d, 1H), 1.95 (br. d, 1H), 1.41 (s, 9H), 1.30-1.06 (m, 5H).

### Beispiel 50A

### (4-Ethylphenyl)[(trimethylsilyl)oxy]acetonitril

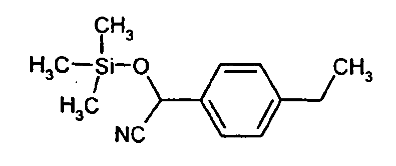

600 g (4.47 mol) 4-Ethylbenzaldehyd werden in 5.3 Litern Toluol mit 2.4 g (7.5 mmol) Zinkiodid vermischt. Bei RT werden unter gelinder Kühlung 587.4 ml (4.7 mol) Trimethylsilylcyanid, gelöst in 3.6 Litern Toluol, über ca. 5 min zugegeben. Die Mischung wird 90 min bei RT gerührt, bevor flüchtige Komponenten im Vakuum entfernt werden und der Rückstand an Kieselgel schnell chromatographiert wird (Eluent: Petrolether/Ethylacetat 9:1). Erhalten werden 990 g (94.9% d. Th.) der Titelverbindung als farbloses Öl.
¹H-NMR (400 MHz, CDCl₃): δ = 7.38 (d, 2H), 7.23 (d, 2H), 4.97 (s, 1H), 2.68 (q, 2H), 1.25 (t, 3H), 0.23 (s, 9H).

### Beispiel 51A

### 1-(4-Ethylphenyl)-2-hydroxy-2-phenylethanon

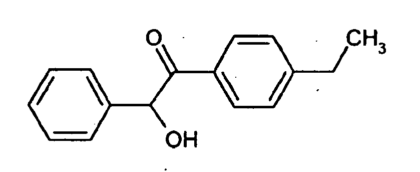

290 ml (2.069 mol) Diisopropylamin werden in 3.6 Litern DME gelöst und auf -78°C vorgekühlt. 820 ml (2.05 mol) n-Butyllithium (2.5 M Lösung in Hexan) werden innerhalb von ca. 20 min zugetropft (Temperatur < -60°C). Nach 15 min bei -60°C wird eine Lösung von 435 g (1.864 mol) (4-Ethylphenyl)[(trimethylsilyl)oxy]acetonitril in 1.4 Litern DME zugetropft (Temperatur < -60°C). Die Mischung wird 30 min bei -60°C nachgerührt, bevor eine Lösung von 189.5 ml (1.864 mol) Benzaldehyd in 1.4 Litern DME zugegeben wird (Dauer ca. 20 min, Temperatur -60°C). Die Mischung wird über 4 h auf RT erwärmt, bevor 7 Liter ges. Ammoniumchlorid-Lösung zugesetzt werden. Die Reaktionsmischung wird mit Ethylacetat extrahiert. Nach Phasentrennung wird die organische Phase mit ges. Ammoniumchlorid-Lösung gewaschen, getrocknet und im Vakuum eingeengt. Der Rückstand wird in 7 Litern Dioxan sowie 5 Litern Methanol gelöst und mit 6 Litern 1 N Salzsäure versetzt. Die Mischung wird über Nacht bei RT gerührt, bevor nach Zugabe von 11 Litern ges. Natriumchlorid-Lösung mit 6.5 Litern Ethylacetat extrahiert wird. Die organische Phase wird mit Wasser und mit ges. Natriumchlorid-Lösung gewaschen, getrocknet und im Vakuum eingeengt. Der Rückstand wird in 2 Litern Diisopropylether gelöst, mit Impfkristallen versetzt und 2 h gerührt. Der ausgefallene Feststoff wird abgesaugt, mit 300 ml Diisopropylether und Petrolether gewaschen und im Vakuum getrocknet. Die Mutterlauge wird aufkonzentriert, und nach Lagern über 2 Tage bei 4°C wird erneut vom ausgefallenen Feststoff abgesaugt, mit ca. 100 ml Diisopropylether und Petrolether gewaschen und im Vakuum getrocknet. Beide Feststoffe werden vereinigt, erhalten werden 154.9 g (34% d. Th.) des Zielprodukts.
HPLC (Methode 1): Rₜ = 4.55 min.
MS (DCI): m/z = 258 (M+NH₄)⁺
¹H-NMR (400 MHz, CDCl₃): δ = 7.85 (d, 2H), 7.48-7.35 (m, 5H), 7.21 (d, 2H), 5.92 (d, 1H), 4.59 (d, 1H), 2.65 (q, 2H), 1.20 (t, 3H).

### Beispiel 52A

### 2-Amino-4-(4-ethylphenyl)-5-phenyl-3-furonitril

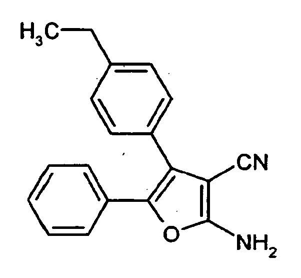

Eine Mischung aus 145 g (603 mmol) 1-(4-Ethylphenyl)-2-hydroxy-2-phenylethanon und 51.8 g (784.4 mmol) Malonsäuredinitril in 2.23 Litern DMF wird auf 0°C gekühlt und unter Kühlung mit 53.7 ml (518 mmol) Diethylamin versetzt. Nach 1 h wird die Reaktionsmischung auf RT erwärmt und weitere 4 h gerührt, bevor 1.5 Liter Wasser zugesetzt werden. Nach 30 min wird ein Großteil des Wassers abdekantiert und durch 750 ml frisches Wasser ersetzt. Die Mischung wird kräftig gerührt, bevor vom klebrigen organischen Rückstand abdekantiert wird. Man löst den Rückstand in Ethylacetat, trocknet und konzentriert im Vakuum, bis das Produkt auszukristallisieren beginnt. Es werden 450 ml Diisopropylether zugesetzt, verrührt und dann über Nacht stehengelassen. Der kristalline Niederschlag wird abgesaugt, zweimal mit 50 ml Diisopropylether gewaschen und im Vakuum getrocknet. Es werden 98.5 g (56.6% d. Th.) des Zielprodukts erhalten.
HPLC (Methode 1): Rₜ= 5.10 min.
MS (DCI): m/z = 306 (M+NH₄)⁺
¹H-NMR (400 MHz, CDCl₃): δ = 7.90-7.82 (m, 4H), 7.28-7.18 (m, 5H), 4.98 (s, 2H), 2.69 (q, 2H), 1.28 (t, 3H).

### Beispiel 53A

### 5-(4-Ethylphenyl)-6-phenylfuro[2,3-d]pyrimidin-4(3H)-on

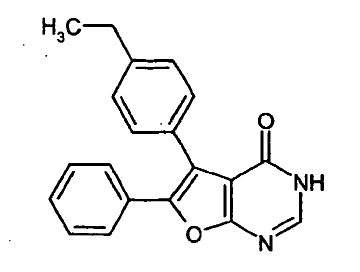

770 ml (8.16 mol) Essigsäureanhydrid werden auf 0°C gekühlt und unter Kühlung mit 372 ml · (10.4 mol) Ameisensäure versetzt. Die Mischung wird 30 min bei 0°C gerührt, bevor 98 g (340 mmol) 2-Amino-4-(4-ethylphenyl)-5-phenyl-3-furonitril eingetragen werden. Die Mischung wird auf Rückfluss erhitzt (dabei zunehmend starke Gasentwicklung) und 24 h bei Rückfluss gerührt. Nach Abkühlen wird etwa 2 h bei 10°C gerührt und dann der ausgefallene Feststoff abgesaugt, mit Diisopropylether gewaschen und im Hochvakuum getrocknet. Erhalten werden 69.3 g (64.5% d. Th.) des Zielprodukts.
HPLC (Methode 1): Rₜ = 4.77 min.
MS (DCI): m/z = 334 (M+NH₄)⁺, 317 (M+H)⁺
¹H-NMR (400 MHz, DMSO-d₆): δ = 12.63 (br. s, 1H), 8.19 (s, 1H), 7.43 (d, 2H), 7.40-7.30 (m, 5H), 7.25 (m, 2H), 3.35 (s, 2H), 2.68 (d, 2H), 1.25 (t, 3H).

### Beispiel 54A

### 4-Chlor-5-(4-ethylphenyl)-6-phenylfuro[2,3-d]pyrimidin

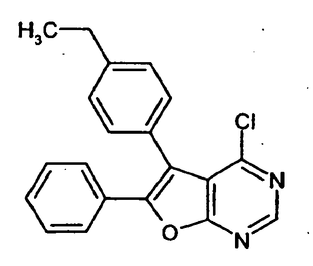

72 g (227.6 mmol) 5-(4-Ethylphenyl)-6-phenylfuro[2,3-d]pyrimidin-4(3H)-on werden in 360 ml (4.6 mol) Phosphoroxychlorid vorgelegt und auf Rückfluss erhitzt. Die Mischung wird ca. 1 h bei 120°C gerührt, bevor das Reaktionsgemisch nach Abkühlen auf RT dosier-kontrolliert unter kräftigem Rühren zu einer Mischung von 2.2 Litern 25%-iger Ammoniaklösung und 1.2 Litern Wasser getropft wird (pH >9, Temperatur 55-75°C). Die wässrige Mischung wird dreimal mit Dichlormethan extrahiert, und die vereinigten organischen Phasen werden über Natriumsulfat getrocknet und im Vakuum eingeengt. Der Rückstand wird mit wenig Diisopropylether gewaschen, und nach Abfiltrieren und Trocknen im Hochvakuum erhält man 66.1 g (85.2% d. Th.) des Zielprodukts.
HPLC (Methode 1): Rₜ = 5.68 min.
MS (DCI): m/z = 335 (M+H)⁺
¹H-NMR (400 MHz, CDCl₃): δ = 8.76 (s, 1H), 7.61 (d, 2H), 7.48-7.30 (m, 7H), 2.78 (q, 2H), 1.36 (t, 3H).

### Beispiel 55A

### 6-Phenylfuro[2,3-d]pyrimidin-4-amin

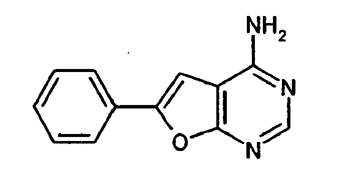

110 g (597 mmol) 2-Amino-5'-phenyl-3-furonitril werden in 355 ml (9 mol) Formamid suspendiert und 1.5 h lang erhitzt (Badtemperatur ca. 210°C). Die Mischung wird danach auf RT abgekühlt und in Wasser eingerührt. Der ausgefallene Feststoff wird abgesaugt und mit Wasser gewaschen. Das noch feuchte Produkt wird in Dichlormethan verrührt, erneut abgesaugt und im Vakuum getrocknet. Erhalten werden 106 g (80% d. Th.) der Zielverbindung.
LC-MS (Methode 4): Rₜ = 3.1 min.; m/z = 212 (M+H)⁺
HPLC (Methode 1): Rₜ = 3.63 min.
¹H-NMR (400 MHz, DMSO-d₆): δ = 8.20 (s, 1H), 7.8 (d, 2H), 7.55-7.32 (m, 6H).

### Beispiel 56A

### 5-Brom-6-phenylfuro[2,3-d]pyrimidin-4-amin

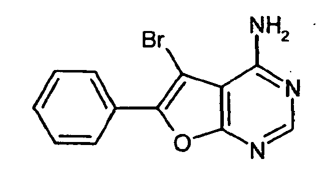

80 g (378.7 mmol) 6-Phenylfuro[2,3-d]pyrimidin-4-amin werden in 770 ml Tetrachlorkohlenstoff auf 60°C erhitzt. 84.3 g (473.4 mmol) *N*-Bromsuccinimid werden hinzugefügt, und die Mischung wird über Nacht unter Rückfluss gerührt. Nach dem Abkühlen wird abfiltriert, der Filterkuchen nacheinander mit Dichlormethan und Acetonitril verrührt und erneut abfiltriert. Der Filterkuchen wird dann im Vakuum getrocknet. Man erhält 86 g des Zielprodukts (78.2% d. Th.).
MS (DCI): m/z = 290/292 (M+H)⁺
¹H-NMR (400 MHz, DMSO-d₆): δ = 8.28 (s, 1H), 8.03 (d, 2H), 7.60-7.50 (m, 5H).

### Beispiel 57A

### 5-Brom-4-chlor-6-phenylfuro[2,3-d]pyrimidin

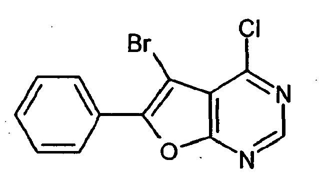

54 g (186 mmol) 5-Brom-6-phenylfuro[2,3-d]pyrimidin-4-amin werden in 135 ml Chloroform vorgelegt, mit 70 ml 4 N Chlorwasserstoff in Dioxan (280 mmol) versetzt und auf Rückfluss erhitzt. Tropfenweise werden unter Gasentwicklung 50 ml (372 mmol) Isoamylnitrit zugegeben. Nach Ende der Zugabe wird 3 h bei Rückfluss gerührt, bevor die abgekühlte Reaktionsmischung in Wasser gegeben und mit Dichlormethan extrahiert wird. Die organische Phase wird mit ges. Natriumhydrogencarbonat-Lösung gewaschen, über Natriumsulfat getrocknet und im Vakuum eingeengt. Das Rohprodukt wird durch Chromatographie an Silicagel (Eluent: Dichlormethan) gereinigt. Das Produkt wird zur weiteren Reinigung in Methanol verrührt, abgesaugt und im Hochvakuum getrocknet. Erhalten werden 32 g des Zielprodukts (55.5% d. Th.).
LC-MS (Methode 3): Rₜ = 2.54 min.; m/z = 309/310 (M+H)⁺
HPLC (Methode 1): Rₜ = 5.08 min.
¹H-NMR (400 MHz, CDCl₃): δ = 8.79 (s, 1H), 8.23-8.20 (m, 2H), 7.58-7.51 (m, 3H).

### Beispiel 58A und Beispiel 59A

### (+/-)-trans- und (+/-)-cis-3-{[5-(4-Methoxyphenyl)-6-phenylfuro[2,3-d]pyrimidin-4-yl]amino}-cyclohexanol

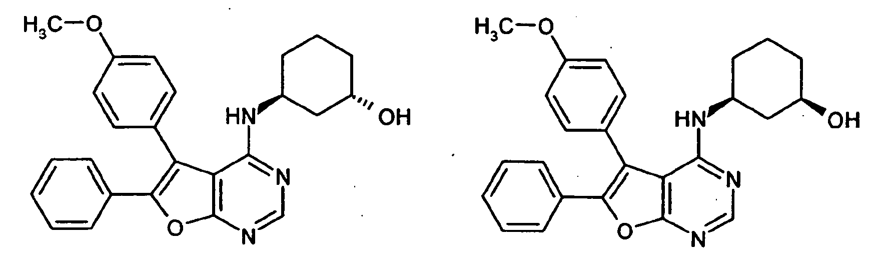

300 mg (0.72 mmol) (+/-)-3-{[5-(4-Methoxyphenyl)-6-phenylfuro[2,3-d]pyrimidin-4-yl]amino}-cyclohexanol (*cis*/*trans*-Gemisch) werden durch präparative HPLC in die reinen *cis-* und *trans-*Isomeren aufgetrennt [Säule: Phenomenex Gemini, C-18, 5 µm, 250 mm x 21.2 mm; Fluss: 20 ml/min; Temperatur: 25°C; Eluent: Wasser/THF 60:40]. Erhalten werden 43 mg (14.3% d. Th.) (+/-)-*trans*-3-{[5-(4-Methoxyphenyl)-6-phenylfuro[2,3-d]pyrimidin-4-yl]amino}cyclohexanol (*Beispiel 58A*) sowie 150 mg (50.0% d. Th.) (+/-)-*cis*-3-{[5-(4-Methoxyphenyl)-6-phenylfuro[2,3-d]-pyrimidin-4-yl]amino}cyclohexanol (*Beispiel 59A*)*.*

### Beispiel 58A:

LC-MS (Methode 6): Rₜ = 2.49 min.; m/z = 416 (M+H)⁺
¹H-NMR (400 MHz, DMSO-d₆): δ = 8.32 (s, 1H), 7.50 (d, 4H), 7.40-7.30 (m, 3H), 7.19 (d, 2H), 4.75 (d, 1H), 4.49 (s, 1H), 4.40-4.30 (m, 1H), 3.86 (s, 3H), 3.48 (s, 1H), 1.67-1.01 (m, 8H).

### Beispiel 59A:

LC-MS (Methode 6): Rₜ = 2.51 min.; m/z = 416 (M+H)⁺
¹H-NMR (400 MHz, DMSO-d₆): δ = 8.32 (s, 1H), 7.49-7.41 (m, 4H), 7.40-7.30 (m, 3H), 7.13 (d, 2H), 5.15 (s, 1H), 4.52 (s, 1H); 4.10-4.00 (m, 1H), 3.88 (s, 3H), 3.53-3.48 (m, 1H), 1.80-0.89 (m, 8H).

### Beispiel 60A

### (+/-)- 4-(3-{[5-(4-Methoxyphenyl)-6-phenylfuro[2,3-d]pyrimidin-4-yl]oxy}piperidin-1-yl)butannitril

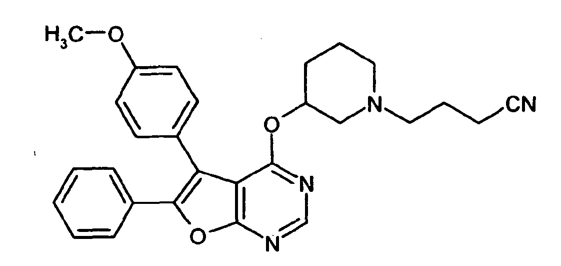

Zu einer Mischung von 200 mg (0.498 mmol) (+/-)-5-(4-Methoxyphenyl)-6-phenyl-4-(piperidin-3-yloxy)furo[2,3-d]pyrimidin, 0.25 ml (1.5 mmol) Diisopropylethylamin und 8.3 mg Kaliumiodid in 2 ml THF werden bei RT 147.5 mg (0.996 mmol) 4-Brombuttersäurenitril gegeben. Die Mischung wird 10 h unter Rückfluss gerührt. Nach Zusatz von weiteren 0.25 ml (1.5 mmol) Diisopropylethylamin und 147.5 mg (0.996 mmol) 4-Brombuttersäurenitril wird erneut über Nacht unter Rückfluss gerührt. Nach Abkühlen auf RT wird mit Dichlormethan verdünnt, mit ges. Natriumhydrogencarbonat-Lösung gewaschen; die organische Phase abgetrennt, über Natriumsulfat getrocknet und im Vakuum eingeengt. Nach Reinigung des Rückstands mittels präparativer RP-HPLC (Eluent: Acetonitri/Wasser-Gradient) werden 189 mg des Zielprodukts erhalten (81.1% d. Th.).
LC-MS (Methode 6): Rₜ = 1.80 min.; m/z = 469 (M+H)⁺
¹H-NMR (400 MHz, DMSO-d₆): δ = 8.59 (s, 1H), 7.55 (d, 2H), 7.45-7.35 (m, 5H), 7.00 (d, 2H), 5.31-5.23 (m, 1H), 3.81 (s, 3H), 2.822.76 (m, 1H), 2.40 (t, 3H), 2.31 (t, 2H), 2.29-2.12 (m, 2H), 1.93-1.85 (m, 1H), 1.67 (t, 3H), 1.50-1.30 (m, 2H).

### Beispiel 61A

### (+/-)-cis-3-{[3-{[5-(4-Methoxyphenyl)-6-phenylfuro[2,3-d]pyrimidin-4-y)]oxy}cyclohexyl]oxy}-propannitril

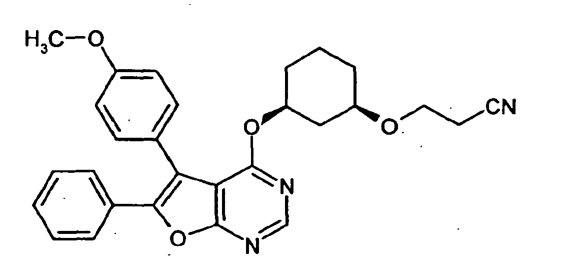

Zu einer Lösung von 150 mg (0.36 mmol) (+/-)-*cis*-3-{[5-(4-Methoxyphenyl)-6-phenylfuro[2,3-d]-pyrimidin-4-yl]oxy}cyclohexanol in 1 ml Acrylnitril wird eine Lösung von 10 mg Kalium-*tert.-*butylat in 0.5 ml THF getropft. Die Reaktionsmischung wird ca. 2 h bei RT unter Lichtausschluss gerührt. Nach Verdünnen mit Dichlormethan wird nacheinander mit 1 N Salzsäure, ges. Natriumhydrogencarbonat-Lösung und ges. Natriumchlorid-Lösung gewaschen und die organische Phase im Vakuum eingeengt. Nach Reinigung des Rückstands mittels präparativer RP-HPLC (Eluent: Acetonitril/Wasser-Gradient) werden 136.9 mg des Zielprodukts erhalten (81% d. Th.).
LC-MS (Methode 3): Rₜ = 2.88 min.; m/z = 470 (M+H)⁺
¹H-NMR (400 MHz, DMSO-d₆): δ = 8.60 (s, 1H), 7.55 (d, 2H), 7.43-7.34 (m, 5H), 7.00 (d, 2H), 5.20-5.10 (m, 1H), 3.81 (s, 3H), 3.63-3.55 (m, 2H), 3.50-3.40 (m, 1H), 2.70 (t, 2H), 2.48-1.40 (m, 1H), 2.19-1.90 (m, 2H), 1.80-1.70 (m, 1H), 1.38-1.02 (m, 4H).

### Beispiel 62A

### (+/-)-trans-3-{[3-{[5-(4-Methoxyphenyl)-6-phenylfuro[2,3-d]pyrimidin4-yl]amino}cyclohexyl]-oxy}propannitril

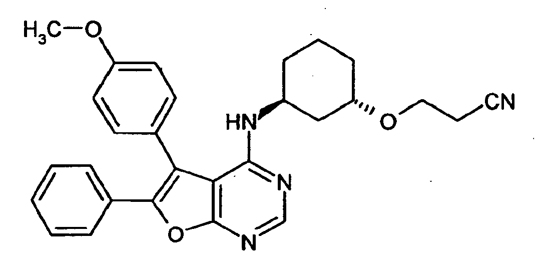

Zu einer Lösung von 34.4 mg (0.059 mmol) (+/-)-*trans*-3-{[5-(4-Methoxyphenyl)-6-phenylfuro-[2,3-d]pyrimidin4-yl]amino}cyclohexanol in 0.23 ml Acrylnitril wird eine Lösung von ca. 2 mg Kalium-*tert*.-butylat in 0.2 ml THF getropft. Die Reaktionsmischung wird ca. 2 h bei RT unter Lichtausschluss gerührt. Nach Verdünnen mit Dichlormethan wird nacheinander mit 1 N Salzsäure, ges. Natriumhydrogencarbonat-Lösung und ges. Natriumchlorid-Lösung gewaschen und die organische Phase im Vakuum eingeengt. Nach Reinigung des Rückstands mittels präparativer RP-HPLC (Eluent: Acetonitril/Wasser-Gradient) werden 33.6 mg des Zielprodukts erhalten (86.6% d. Th.).
LC-MS (Methode 6): Rₜ = 2.86 min.; m/z = 469 (M+H)⁺
¹H-NMR (400 MHz, DMS0-d₆): δ = 8.32 (s, 1H), 7.52-7.49 (m, 3H), 7.19 (d, 2H), 4.73 (d, 1H), 4.85-4.25 (m, 1H), 3.88 (s, 3H), 3.60-3.50 (m, 2H), 2.78-2.70 (m, 2H), 1.80-1.62 (m, 2H), 1.60-. 1.49 (m, 4H), 1.27-1.12 (m, 3H).

### Beispiel 63A

### (+/-)-4-[2-({[5-(4-Methoxyphenyl)-6-phenylfuro[2,3-d]pyrimidin-4-yl]oxy}methyl)pyrrolidin-1-yl]butannitril

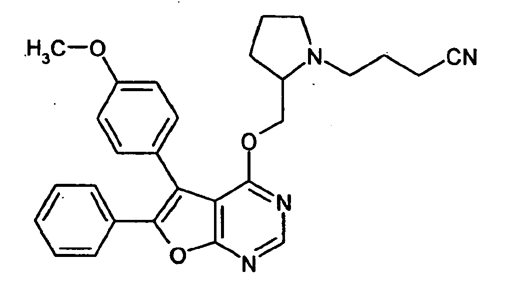

Zu einer Lösung von 300 mg (0.747 mmol) (+/-)-5-(4-Methoxyphenyl)-6-phenyl-4-(pyrrolidin-2-ylmethoxy)furo[2,3-d]pyrimidin, 0.37 ml (2.24 mmol) Diisopropylethylamin und 12.4 mg Kaliumiodid in 3 ml THF werden bei RT 221.2 mg (0.996 mmol) 4-Brombuttersäurenitril gegeben. Die Mischung wird 6 h unter Rückfluss gerührt. Nach Abkühlen auf RT wird mit Dichlormethan verdünnt, mit ges. Natriumhydrogencarbonat-Lösung gewaschen, die organische Phase abgetrennt, über Natriumsulfat getrocknet und im Vakuum eingeengt. Nach Reinigung des Rückstands mittels präparativer RP-HPLC (Eluent: Acetonitril/Wasser-Gradient) werden 178.1 mg des Zielprodukts erhalten (50.9% d. Th.).
LC-MS (Methode 3): Rₜ = 1.59 min.; m/z = 469 (M+H)⁺
¹H-NMR (400 MHz, DMSO-d₆): δ = 8.49 (s, 1H), 7.57-7.50 (m, 2H), 7.43-7.36 (m, 5H), 7.01 (d, 2H), 4.36 (dd, 1H), 4.24 (dd, 1H), 3.81 (s, 3H), 2.99-2.90 (m, 1H), 2.69-2.60 (m, 2H), 2.41-2.01 (m, 4H), 1.79-1.70 (m, 1H), 1.61-1.40 (m, 5H).

### Beispiel 64A

### (+/-)-3-(3-{[5-(4-Methoxyphenyl)-6-phenylfuro[2,3-d]pyrimidin-4-yl]oxy}piperidin-1-yl)propannitril

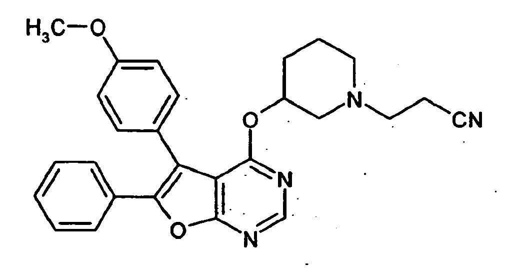

Eine Mischung von 900 mg (2.24 mmol) (+/-)-5-(4-Methoxyphenyl)-6-phenyl-4-(piperidin-3-yl-oxy)furo[2,3-d]pyrimidin und 1.5 ml (22.4 mmol) Acrylnitril wird 3 h unter Rückfluss gerührt. Nach Abkühlen auf RT wird im Vakuum eingeengt und der Rückstand im Hochvakuum getrocknet. Erhalten werden 1000 mg (98.1% d. Th.) der Zielverbindung.
LC-MS (Methode 3): Rₜ = 1.97 min.; m/z = 455 (M+H)⁴.
1.0 g (2.2 mmol) so erhaltenes (+/-)-3-(3-{[5-(4-Methoxyphenyl)-6-phenylfuro[2,3-d]pyrimidin-4-yl]oxy}-piperidin-1-yl)propannitril werden durch Chromatographie an chiraler Phase in die Enantiomere getrennt (siehe Beispiele 65A und 66A) [Säule: Daicel Chiralpak AS-H, 5 µm, 250 mm x 20 mm; Fluss: 15 ml/min; Temperatur: 30°C; Eluent: iso-Hexan/THF 50:50].

### Beispiel 65A

### (-)-3-(3-{[5-(4-Methoxyphenyl)-6-phenylfuro[2,3-d]pyrimidin-4-yl]oxy}piperidin-1-yl)propannitril (Enantiomer 1)

Ausbeute: 459 mg (45.1% d. Th.)
[α]_{D}²⁰ = -60.5°, c = 0.545, CHCl₃
LC-MS (Methode 6): Rₜ = 2.05 min.; m/z = 455 (M+H)⁺
¹H-NMR (400 MHz, DMSO-d₆): δ = 8.60 (s, 1H), 7.54 (d, 2H), 7.48-7.37 (m, 5H), 7.01 (d, 2H), 5.31-5.23 (m, 1H), 3.81 (s, 3H), 2.91-2.82 (m, 1H), 2.68-2.58 (m, 3H), 2.55 (s, 2H), 2.38-2.22 (m, 2H), 1.93-1.82 (m, 1H), 1.69-1.58 (m, 1H), 1.50-1.29 (m, 2H).

### Beispiel 66A

### (+)3-(3-{[5-(4-Methoxyphenyl)-6-phenylfuro[2,3-d]pyrimidin-4-yl]oxy}piperidin-1-yl)propannitril (Enantiomer 2)

Ausbeute: 479 mg (47.0% d. Th.)
[α]_{D}²⁰ = +59.1°, c = 0.545, CHCl₃
LC-MS (Methode 6): Rₜ = 2.05 min.; m/z = 455 (M+H)⁺
¹H-NMR (400 MHz, DMSO-d₆): δ = 8.60 (s, 1H), 7.54 (d, 2H), 7.48-7.37 (m, 5H), 7.01 (d, 2H), 5.31-5.23 (m, 1H), 3.81 (s, 3H), 2.91-2.82 (m, 1H), 2.68-2.58 (m, 3H), 2.55 (s, 2H), 2.38-2.22 (m, 2H), 1.93-1.82 (m, 1H), 1.69-1.58 (m, 1H), 1.50-1.29 (m, 2H).

### Beispiel 67A

### (2E)-3-{(2S,4R)-4-Hydroxy-1-[(1R)-1-phenylethyl]piperidin-2-yl}acrylsäuremethylester

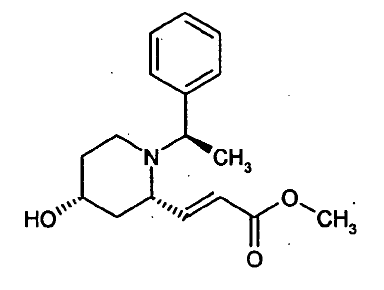

2.0 g (8.647 mmol) (1*S,*5*R*)2-[(1*R*)-1-Phenylethyl]-6-oxa-2-azabicyclo[3.2.1]octan-7-on [hergestellt aus *N*-[(1*R*)-1-Phenylethyl]but-3-en-1-amin gemäß Bioorg. Med. Chem. Lett. 6 (8), 964 (1996)] werden in 8 ml abs. THF gelöst, auf-78°C abgekühlt und mit 9.5 ml (9.5 mmol) einer 1 M Lösung von L-Selectrid in THF versetzt. Nach Ende der Zugabe wird 1 h bei -78°C nachgerührt, dann auf -20°C erwärmt und mit 2.1 ml (13 mmol) Phosphonoessigsäuretrimethylester versetzt. Die Reaktionsmischung wird anschließend auf 0°C erwärmt und 1 h gerührt. Danach wird Wasser zugesetzt und mit 1 N Salzsäure ein pH-Wert von ca. 7-8 eingestellt. Die Mischung wird dreimal mit Dichlormethan extrahiert, die organischen Phasen werden vereinigt, über Magnesiumsulfat getrocknet und im Vakuum eingeengt. Es werden 4.32 g an Rohprodukt erhalten, welches ohne weitere Reinigung in der Folgestufe eingesetzt wird.
LC-MS (Methode 4): Rₜ = 2.48 min.; m/z = 290 (M+H)⁺.

### Beispiel 68A

### tert.-Butyl-(2R,4R)-4-Hydroxy-2-(3-methoxy-3-oxopropyl)piperidin-1-carboxylat

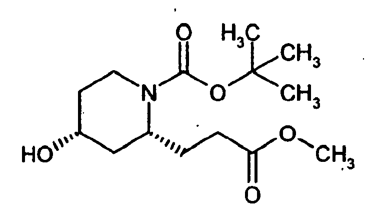

3.8 g (2*E*)-3-{(2*S*,4*R*)-4-Hydroxy-1-[(1*R*)-1-phenylethyl]piperidin-2-yl}acrylsäuremethylester (als Rohprodukt) werden in 50 ml Isopropanol gelöst und mit 4.3 g Di-*tert*.-butyldicarbonat sowie eine katalytischen Menge an 10%-igem Pd/C versetzt. Die Mischung wird über Nacht bei RT und Normaldruck unter einer Wasserstoffatmosphäre gerührt. Man filtriert über Kieselgur und engt das Filtrat im Vakuum ein. Aus dem Rückstand werden nach Reinigung durch Chromatographie an Silicagel (Laufmittel: Cyclohexan/Ethylacetat 5:1 → 1:1) 0.98 g des Zielprodukts erhalten.
LC-MS (Methode 8): Rₜ = 1.87 min.; m/z = 288 (M+H)⁺
¹H-NMR (400 MHz, DMSO-d₄): δ = 4.64 (d, 1H), 4.08-3.99 (m, 1H), 3.91 (d, 1H), 3.69 (d, 1H), 3.58 (s, 3H), 3.05 (dt, 1H), 2.36-2.12 (m, 3H), 1.82-1.71 (m, 1H), 1.60 (s, 2H), 1.55-1.41 (m, 2H), 1.38 (s, 9H).

### Beispiel 69A

### (+/-)-({3-[(6-Phenylfuro[2,3-d]pyrimidin-4-yl)amino]cyclohexyl}oxy)essigsäure-tert.-butylester

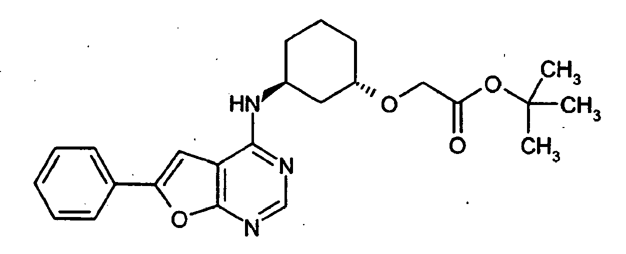

1.61 g (6.98 mmol) 4-Chlor-6-phenylfuro[2,3-d]pyridin und 1.60 g (6.98 mmol) (*+*/*-*)*-trans-*{[3-Aminocyclohexyl]oxy}essigsäure-*tert*.-butylester werden in 6.0 ml DMF vorgelegt und mit 1.8 ml (10.5 mmol) *N,N*-Diisopropylethylamin versetzt. Die Reaktionsmischung wird 3 h auf 120°C erhitzt, dann auf RT abgekühlt und auf Wasser gegeben. Man extrahiert dreimal mit Ethylacetat, vereinigt die organischen Phasen, wäscht mit ges. Natriumchlorid-Lösung und engt im Vakuum ein. Aus dem Rückstand werden nach Reinigung durch Chromatographie an Silicagel (Laufmittel: Cyclohexan/Ethylacetat 20:1 1 → 2:1) 1.67 g des Zielprodukts isoliert (56.5% d. Th.).
LC-MS (Methode 3): Rₜ = 2.62 min.; m/z = 424 (M+H)⁺
¹H-NMR (400 MHz, DMSO-d₆): δ = 8.27 (s, 1H), 7.88-7.68 (m, 3H), 7.50 (t, 2H), 7.47-7.39 (m, 1H), 4.40 (s, 1H), 4.03 (s, 2H), 3.80 (s, 1H), 2.15-1.50 (m, 8H), 1.50-1.30 (m, 9H).

### Beispiel 70A

### (+/-)-({3-[(5-Brom-6-phenylfuro[2,3-d]-pyrimidin-4-yl)amino]cyclohexyl}oxy)essigsäure-tert: butylester

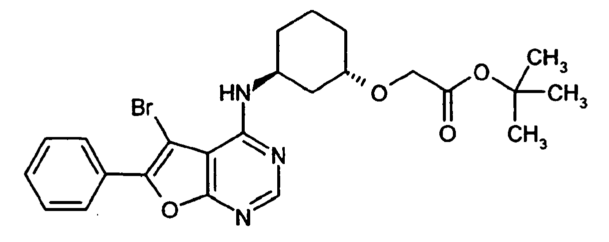

1.65 g (3.9 mmol) (+/-)-({3-[(6-Phenylfuro[2,3-d]pyrimidin-4-yl)amino]cyclohexyl}oxy)essigsäure-*tert*.-butylester werden in 4 ml Tetrachlorkohlenstoff suspendiert und mit 762 mg (4.3 mmol) *N*-Bromsuccinimid versetzt. Die Reaktionsmischung wird 1 h unter Rückfluss erhitzt. Nach Abkühlen auf RT werden weitere 350 mg *N*-Bromsuccinimid zugesetzt. Die Reaktionsmischung wird wiederum 1 h unter Rückfluss gerührt, dann abgekühlt und im Vakuum eingeengt. Aus dem Rückstand werden nach Reinigung durch Chromatographie an Silicagel (Laufmittel: Cyclohexan/ Ethylacetat 5:1) 0.99 g des Zielprodukts isoliert (50.6% d. Th.).
LC-MS (Methode 3): Rₜ = 3.09 min.; m/z = 502 (M+H)⁺
¹H-NMR (400 MHz, DMSO-d₆): δ = 8.38 (s, 1H), 8.00 (d, 2H), 7.61-7.49 (m, 3H), 6.39 (d, 1H), 4.53-4.42 (m, 1H), 4.03 (s, 2H), 3.78 (s, 1H), 2.09-1.42 (m, 8H), 1.40 (s, 9H).

### Beispiel 71A und Beispiel 72A

### (+)-cis-{[3-Hydroxycyclohexyl]oxy}-essigsäure-tert.-butylester (Enantiomer 1) und (-)cis-{[3-Hydroxycyclohexyl]oxy}-essigsäure-tert.-butylester (Enantiomer 2)

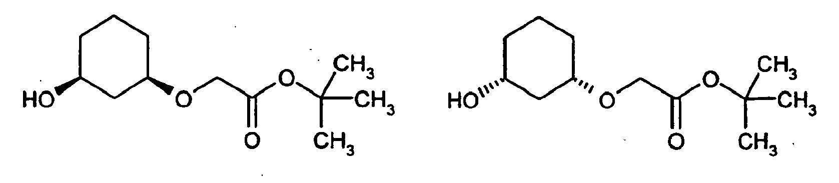

500 mg (2.17 mmol) (+/-)-*cis*-{[3-Hydroxycyclohexyl]oxy}-essigsäure-*tert*.-butylester werden durch Chromatographie an chiraler Phase in die Enantiomere getrennt [Säule: Daicel Chiralpak AS-H, 5 µm, 250 mm x 20 mm; Fluss: 15 ml/min; Temperatur: 30°C; Eluent: iso-Hexan/Ethanol 75:25].

### Enantiomer 1:

Ausbeute: 124 mg (24.8% d. Th.)
[α]_{D}²⁰ = +2.4°, c = 0.50, CHCl₃
¹H-NMR (400 MHz, DMSO-d₆): δ = 4.60 (d, 1H), 3.97 (s, 2H), 3.39-3.29 (m, 1H), 3.28-3.19 (m, 1H), 2.20-2.12 (m, 1H), 1.90 (d, 1H), 1.74 (d, 1M, 1.69-1.59 (m, 1H), 1.41 (s, 9H), 1.17-0.90 (m, 4H).

### Enantiomer 2:

Ausbeute: 121 mg (24.2% d. Th.)
[α]_{D}²⁰ = -3.4°, c = 0.50, CHCl₃
¹H-NMR (400 MHz, DMSO-d₆): δ =4.60 (d, 1H), 3.97 (s, 2H), 3.39-3.29 (m, 1H), 3.28-3.19 (m, 1H), 2.20-2.12 (m, 1H), 1.90 (d, 1H), 1.74 (d, 1H), 1.69-1.59 (m, 1H), 1.41 (s, 9H), 1.17-0.90 (m, 4H).

### Beispiel 73A

### (+/-)-3-(Benryloxy)piperidin-tert.-butylcarbamat

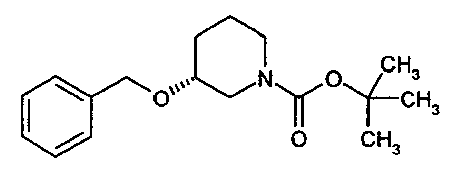

15 g (74.5 mmol) (+/-)-3-Hydroxypiperidin-*tert*.-butylcarbamat werden in der Hitze in 86.5 ml Toluol gelöst und nacheinander mit 11.9 ml 50%-iger Natronlauge (447 mmol), 2.53 g (7.5 mmol) Tetra-n-butylammoniumhydrogensulfat und 11.5 ml (96.9 mmol) Benzylbromid versetzt. Die zweiphasige Reaktionsmischung wird bei 70°C für 4 h kräftig gerührt. Nach Abkühlen wird Wasser zugegeben und mit konz. Salzsäure neutralisiert. Die organische Phase wird abgetrennt, über Natriumsulfat getrocknet und im Vakuum eingeengt. Das Rohprodukt wird durch Chromatographie an Silicagel (Laufmittel: Cyclohexan/Ethylacetat 30:1 → 10:1) gereinigt. Es werden 16.21 g des Zielprodukts erhalten (74.6% d. Th.).
LC-MS (Methode 3): Rₜ = 2.64 min.; m/z = 293 (M+H)⁺
¹H-NMR (400 MHz, DMSO-d₆): δ = 7.38-7.24 (m, 5H), 4.53 (dd, 2H), 3.43-3.35 (m, 2H), 3.30-3.18 (m, 2H), 1.84 (br. s, 1H), 1.71-1.48 (m, 4H), 1.36 (s, 9H).

### Beispiel 74A und Beispiel 75A

### (-)-(3R)-3-(Benzyloxy)-piperidin-tert.-butylcarbamat (Enantiomer 1) und (+)-(3S)-3-(Benzyloxy)piperidin-tert.-butylcarbamat (Enantiomer 2)

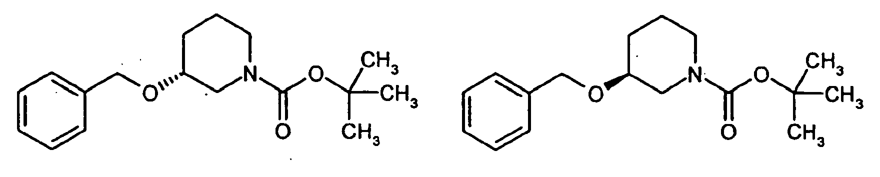

16.0 g (54.9 mmol) (+/-)-3-(Benzyloxy)piperidin-*tert*-butylcarbamat werden durch Chromatographie an chiraler Phase in die Enantiomere getrennt [Säule: Daicel Chiralpak AS-H, 5 µm, 250 mm x 20 mm; Fluss: 15 ml/min; Temperatur: 28°C; Eluent: iso-Hexan/2-Propanol 95:5].

### Enantiomer 1:

Ausbeute: 7.40 g (49.3% d. Th.)
[α]_{D}²⁰ = -5.8°, c = 0.635, CHCl₃
LC-MS (Methode 3): Rₜ = 2.65 min.; m/z = 292 (M+H)⁺
¹H-NMR (400 MHz, DMSO-d₆): δ = 7.49-7.23 (m, 5H), 4.60-4.45 (m, 2H), 3.42-3.38 (m, 2H), 1.82 (br. s, 1H), 1.70-1.48 (m, 2H), 1.34 (s, 9H), 1.40-1.26 (m, 4H).

### Enantiomer 2:

Ausbeute: 6.50 g (43.3% d. Th.)
[α]_{D}²⁰ = +6.0°, c = 1.045, CHCl₃
LC-MS (Methode 3): Rₜ = 2.65 min.; m/z = 292 (M+H)⁺
¹H-NMR (400 MHz, DMSO-d₆): δ = 7.49-7.23 (m, 5H), 4.60-4.45 (m, 2H), 3.42-3.38 (m, 2H), 1.82 (br. s, 1H), 1.70-1.48 (m, 2H), 1.34 (s, 9H), 1.40-1.26 (m, 4h).

### Beispiel 76A

### (+)-4-[(3R)-3-(Benzyloxy)piperidin-1-yl]butansäuremethylester

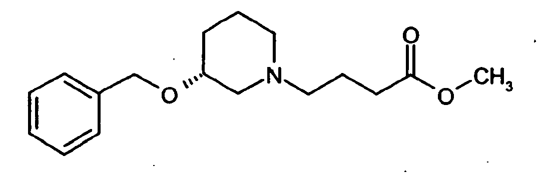

Zu einer Lösung von 3.025 g (10.38 mmol) (+)-(3S)-3-(Benzyloxy)piperidin-*tert*.-butylcarbamat in 14.4 ml Dichlormethan werden bei RT ein Tropfen Wasser und 7.7 ml Trifluoressigsäure gegeben. Die Mischung wird 1 h gerührt und anschließend mit Wasser und Dichlormethan verdünnt. Nach Phasentrennung wird die organische Phase mit ges. Natriumchlorid-Lösung und ges. Natriumhydrogencarbonat-Lösung gewaschen, über Natriumsulfat getrocknet und im Vakuum eingeengt. Man erhält 2.12 g Rohprodukt, das ohne weitere Reinigung in 37 ml THF gelöst und nacheinander mit 5.7 ml (32.9 mmol) *N,N*-Diisopropylethylamin, 182 mg (1.1 mmol) Kaliumiodid und 3.98 g (22 mmol) 4-Brombuttersäuremethylester versetzt wird. Die Mischung wird anschließend 3 h unter Rückfluss gerührt. Nach dem Abkühlen wird mit Dichlormethan verdünnt, nacheinander mit Wasser, ges. Ammoniumchlorid-Lösung und ges. Natriumchlorid-Lösung gewaschen, die organische Phase über Natriumsulfat getrocknet und im Vakuum eingeengt. Aus dem Rückstand werden nach Reinigung durch präparative RP-HPLC (Eluent: Acetonitril/Wasser) 2.0 g des Zielprodukts isoliert (62.6% d. Th. über beide Stufen).
[α]_{D}²⁰ = +1.3°, c = 0.51, CHCl₃
LC-MS (Methode 8): Rₜ = 0.89 min.; m/z = 292 (M+H)⁺
¹H-NMR (400 MHz, DMSO-d₆): δ = 7.38-7.23 (m, 5H), 4.51 (s, 2H), 3.59 (s, 2H), 3.45-3.31 (m, 1H), 3.00-2.90 (m, 1H), 2.69-2.56 (m, 1H), 2.37-2.19,(m, 5H), 1.99-1.80 (m, 3H), 1.70-1.60 (m, 3H), 1.44-1.30 (m, 1H), 1.22-1.10 (m, 1H).

### Beispiel 77A

### (-)-4-[(3R)-3-Hydroxypiperidin-1-yl]butansäuremethylester

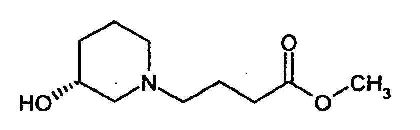

Zu einer Lösung von 2.0 g (6.86 mmol) (+)-4-(3*R*)-3-(Benzyloxy)piperidin-1-yl]butansäuremethylester in 15 ml Essigsäure werden bei RT ca. 200 mg 10%-iges Pd/C gegeben. Die Suspension wird bei RT über Nacht unter einer Wasserstoffatmosphäre (Normaldruck) kräftig gerührt. Die Reaktionsmischung wird danach über Celite filtriert, der Filterrückstand mit Dichlormethan nachgewaschen und das Filtrat im Vakuum eingeengt. Der Rückstand wird in Dichlormethan aufgenommen, mit ges. Natriumhydrogencarbonat-Lösung gewaschen, über Natriumsulfat getrocknet und im Vakuum eingeengt. Erhalten werden 833.9 mg (60.4% d. Th.) der Zielverbindung.
[α]_{D}²⁰ = +6.9°, c = 0.57, CHCl₃
GC-MS (Methode 9): Rₜ = 5.20 min.; m/z = 202 (M+H)⁺
¹H-NMR (400 MHz, DMSO-d₆): δ = 4.54 (br. s, 1H), 3.60 (s, 3H), 3.48-3.38 (m, 1H), 2.81-2.71 (m, 1H), 2.65-2.56 (m, 1H), 2.32-2.15 (m, 4H), 1.81-1.70 (m, 2H), 1.70-1.52 (m, 4H), 1.42-1.30 (m, 1H), 1.10-0.98 (m, 1H).

### Beispiel 78A

### rac-3-[(5-Brom-6-phenylfuro[2,3-d]pyrimidin-4-yl)oxy]piperidin-1-tert.-butylcarbamat

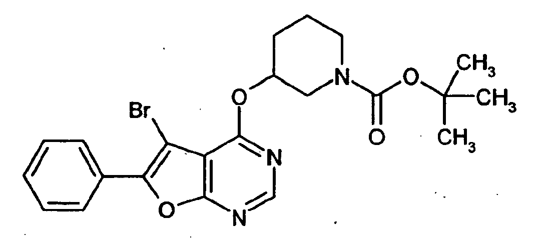

1.1 g (3.55 mmol) 5-Brom-4-chlor-6-phenylfuro[2,3-d]pyrimidin werden bei 70°C in 20 ml Toluol und 10 ml 1,2-Dimethoxyethan gelöst. 2.84 g 50%-ige Natronlauge (35.5 mmol), 120.6 mg (0.26 mmol) Tetra-n-butylammoniumhydrogensulfat und 1.79 g (8.88 mmol) 1-*tert*.-Butoxycarbonyl-3-hydroxypiperidin werden hinzugefügt und die Reaktionsmischung anschließend bei 70°C für 1 h kräftig gerührt. Nach Abkühlen wird die Mischung auf Wasser gegeben und mit konz. Salzsäure auf pH ca. 7 eingestellt. Man extrahiert die wässrige Phase dreimal mit Ethylacetat, wäscht die vereinigten organischen Phasen mit ges. Natriumchlorid-Lösung, trocknet über Magnesiumsulfat und engt im Vakuum ein. Aus dem Rückstand werden nach Reinigung durch Chromatographie an Silicagel (Laufmittel: Cyclohexan/Ethylacetat 5:1) 1.06 g der Zielverbindung isoliert (62.9% d. Th.).
LC-MS (Methode 3): Rₜ = 3.03 min.; m/z = 474 (M+H)⁺
¹H-NMR (400 MHz, DMSO-d₆): δ = 8.66 (s, 1H), 8.07 (d, 2H), 7.63-7.51 (m, 3H), 5.33 (br. s, 1H), 4.30 (br. d, 1H), 4.02-3.92 (m, 1H), 2.10-1.90 (m, 3H), 1.60-1.50 (m, 1H), 1.34 (s, 2H); 0.92 (s, 9H).

### Beispiel 79A

### rac-5-Brom-6-phenyl-4-(piperidin-3-yloxy)furo[2,3-d]pyrimidin

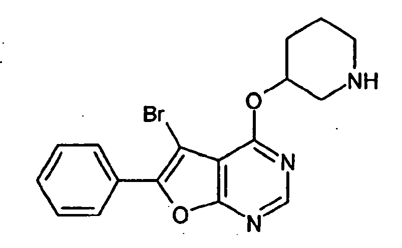

Eine Lösung von 1.05 g (2.21 mmol) *rac*-3-[(5-Brom-6-phenylfuro[2,3-d]pyrimidin-4-yl)oxy]-piperidin-1-*tert*.-butylcarbamat in 2 ml Dichlormethan wird bei RT in mehreren Portionen mit insgesamt 3.4 ml TFA versetzt und 2 h bei RT gerührt. Danach wird mit Dichlormethan verdünnt, die Lösung vorsichtig mit ges. Natriumhydrogencarbonat-Lösung versetzt und anschließend zweimal mit ges. Natriumhydrogencarbonat-Lösung gewaschen. Die organische Phase wird über Magnesiumsulfat getrocknet und im Vakuum eingeengt. Der ölige Rückstand wird mit Methanol verrührt, der ausgefallene Feststoff abgesaugt und mit Methanol gewaschen. Mutterlauge und Waschlösung werden vereinigt, im Vakuum eingeengt und erneut mit wenig Methanol verrührt. Die erhaltenen Kristalle werden abgesaugt, mit Methanol gewaschen und mit der ersten Kristallfraktion vereinigt. Erhalten werden insgesamt 550 mg (66.4% d. Th.) der Zielverbindung.
LC-MS (Methode 6): Rₜ = 1.61 min.; m/z = 374 (M+H)⁺
¹H-NMR (400 MHz, DMSO-d₆): δ = 8.60 (s, 1H), 8.09 (d, 2H), 7.64-7.51 (m, 3H), 5.29-5.20 (m, 1H), 3.15 (dd, 1H), 2.80-2.70 (m, 2H), 2.65-2.57 (m, 1H), 2.21-2.08 (m, 2H), 1.82-1.68 (m, 2H), 1.56-1.43 (m, 1H).

### Beispiel 80A

### (+/-)-4-{3-[(5-Brom-6-phenylfuro[2,3-d]pyrimidin-4-yl)oxy]piperidin-1-yl}butansäuremethylester

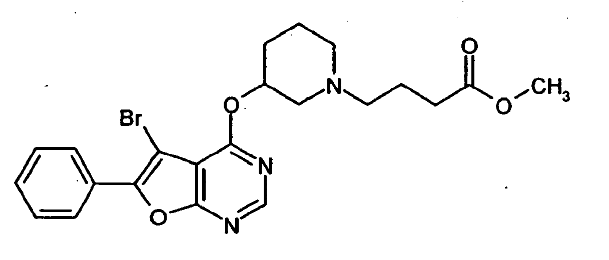

Eine Mischung von 550 mg (1.47 mmol) rac-5-Brom-6-phenyl-4-(piperidin-3-yloxy)furo[2,3-d]-pyrimidin, 532 mg (2.94 mmol) 4-Brombuttersäuremethylester, 24.4 mg (0.147 mmol) Kaliumiodid und 0.77 ml (4.41 mmol) *N,N*-Diisopropylethylamin wird in 1.5 ml THF) für 2 h unter Rückfluss erhitzt. Nach Abkühlen wird mit Dichlormethan verdünnt und auf Wasser gegeben. Nach Phasentrennung wird die wässrige Phase mit Dichlormethan extrahiert. Die organischen Phasen werden vereinigt, mit ges. Natriumhydrogencarbonat-Lösung gewaschen, über Magnesiumsulfat getrocknet und im Vakuum eingeengt. Aus dem Rückstand werden nach Reinigung durch präparative RP-HPLC (Eluent: Acetonitril/Wasser) 780 mg des Zielprodukts isoliert, welche ohne weitere Aufreinigung eingesetzt werden.
LC-MS (Methode 3): Rₜ = 1.62 min.; m/z = 474 (M+H)⁺
¹H-NMR (400 MHz, DMSO-d₆): δ = 8.61 (s, 1H), 8.09 (d, 2H), 7.62-7.51 (m, 3H), 5.39-5.30 (m, 1H), 3.52 (s, 3H), 2.90 (d, 1H), 2.60-2.52 (m, 1H), 2.47-2.39 (m, 1H), 2.38-2.28 (m, 4H), 2.26-2.19 (m, 1H), 2.19-2.00 (m, 1H), 1.90-1.80 (m, 1H), 1.70-1.51 (m, 4H).

### Beispiel 81A und Beispiel 82A

### (-)-4-{(3R)-3-[(5-Brom-6-phenylfuro[2,3-d]pyrimidin-4-yl)oxy]piperidin-1-yl}butansäuremethylester (Enantiomer 1) und (+)-{(3S)-3-[(5-Brom-6-phenylfuro[2,3-d]pyrimidin-4-yl)oxy]piperidin-1-yl}butansäuremethylester (Enantiomer 2)

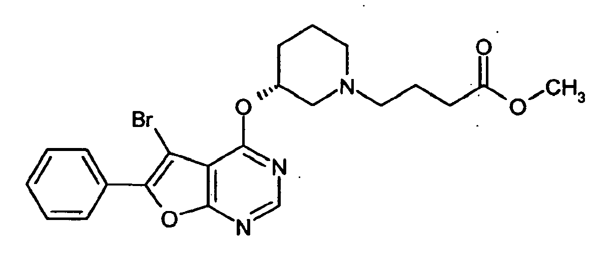

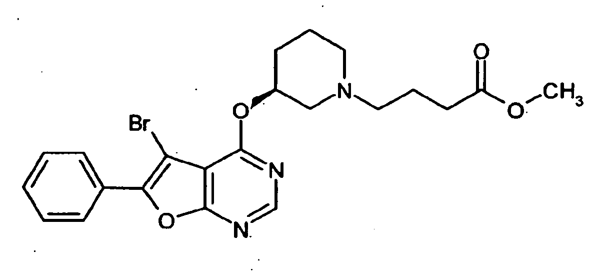

780 mg (1.64 mmol) (+/-)-3-[(5-Brom-6-phenylfuro[2,3-d]pyrimidin-4-yl)oxy]piperidin-1-yl}-butansäuremethylester werden durch Chromatographie an chiraler Phase in die Enantiomere getrennt [Säule: Daicel Chiralpak AS-H, 5 µm, 250 mm x 20 mm; Fluss: 15 ml/min; Temperatur: 28°C; Eluent: iso-Hexan/2-Propanol (+ 0.2% Diethylamin) 80:20].

### Enantiomer 1:

Ausbeute: 350 mg (44.8% d. Th.)
[α]_{D}²⁰ = -43.1°, c = 0.505, CHCl₃
LC-MS (Methode 3): Rₜ = 1.59 min.; m/z = 475 (M+H)⁺
¹H-NMR (400 MHz, DMSO-d₆): δ = 8.62 (s, 1H), 8.08 (d, 2H), 7.64-7.51 (m, 3H), 5.38-5.3.0 (m, 1H), 3.53 (s, 3H), 2.94-2.88 (m, 1H), 2.47-2.40 (m, 1H), 2.38-2.29 (m, 4H), 2.25-2.19 (m, 1H), 2.10-2.00 (m, 1H), 1.90-1.80 (m, 1H), 1.70-1.52 (m, 4H), 0.90-0.79 (m, 1H).

### Enantiomer 2:

Ausbeute: 320 mg (41.0% d. Th.)
[α]_{D}²⁰ = +42.2°, c = 0.53, CHCl₃
LC-MS (Methode 3): Rₜ = 1.59 min.; m/z = 475 (M+H)⁺
¹H-NMR (400 MHz, DMSO-d₆): δ = 8.62 (s, 1H), 8.08 (d, 2H), 7.64-7.51 (m, 3H), 5.38-5.30 (m, 1H), 3.53 (s, 3H), 2.94-2.88 (m, 1H), 2.47-2.40 (m, 1H), 2.38-2.29 (m, 4H), 2.25-2.19 (m, 1H), 2.10-2.00 (m, 1H), 1.90-1.80 (m, 1H); 1.70-1.52 (m, 4H), 0.90-0.79 (m, 1H).

### Beispiel 83A

### cis/trans-3-[(5-Brom-6-phenylfuro[2,3-d]pyrimidin-4-yl)oxy]cyclohexanol

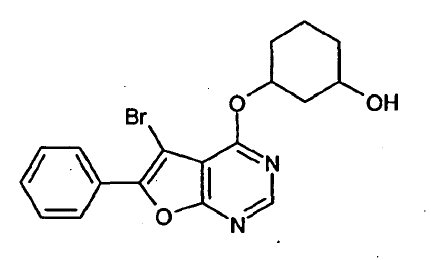

1.1 g (8.88 mmol) *cis*/*trans*-Cyclohexandiol werden bei 70°C in 10 ml Toluol und 5 ml 1,2-Dimethoxyethan gelöst und mit 2.84 g 50%-iger Natronlauge (35.5 mmol) versetzt. Es wird Wasser hinzugegeben, bis eine zweiphasige Reaktionsmischung entsteht. 120.6 mg (3.55 mmol) Tetra-n-butylammoniumhydrogensulfat und 1.10 g (3.55 mmol) 5-Brom-4-chlor-6-phenylfuro[2,3-d]pyrimidin werden hinzugefügt und die Mischung 1 h bei 70°C kräftig gerührt. Nach Abkühlen wird die Reaktionsmischung auf Wasser gegeben und mit konz. Salzsäure neutralisiert. Die wässrige Phase wird dreimal mit Ethylacetat extrahiert. Man vereinigt die organischen Phasen, wäscht mit ges. Natriumhydrogensulfat-Lösung, trocknet über Magnesiumsulfat und engt im Vakuum ein. Der Rückstand wird durch Chromatographie an Silicagel gereinigt (Laufmittel: Cyclohexan/Ethylacetat 5:1 1 → 3:1). Es werden 0.63 g der Zielverbindung erhalten (45.6% d. Th.).
LC-MS (Methode 3): Rₜ = 2.47 min.; m/z = 389 (M+H)⁺
¹H-NMR (400 MHz, DMSO-d₆): δ = 8.61 (s, 1H), 8.03 (d, 2H), 7.63-7.51 (m, 3H), 5.30-5.20 (m, 1H), 4.79 (d, 1H), 3.62-3.52 (m, 1H), 2.46-2.38 (m, 1H), 2.20-2.10 (m, 1H), 1.90-1.66 (m, 3H), 1.47-1.29 (m, 2H), 1.20-1.09 (m, 1H).

### Beispiel 84A

### cis/trans-({3-[(5-Brom-6-phenylfuro[2,3-d]pyrimidin-4-yl)oxy]cyclohexyl}oxy)essigsäure-tert.-butylester

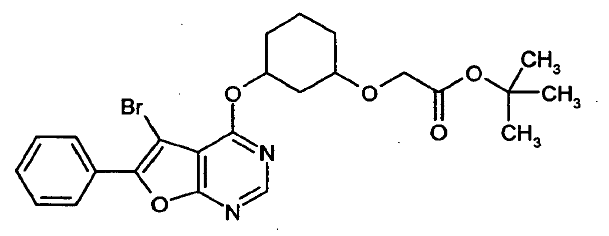

Zu einer Mischung von 2 ml Toluol und 1.28 g 50%-iger Natronlauge (16.05 mmol) wird eine Lösung von 625 mg (1.606 mmol) *cis*/*trans*-3-[(5-Brom-6-phenylfuro[2,3-d]pyrimidin-4-yl)oxy]-cyclohexanol in 3 ml Toluol gegeben. Zu der zweiphasigen Mischung werden anschließend 54.5 mg (0.16 mmol) Tetra-n-butylammoniumhydrogensulfat und 626 mg (3.21 mmol) Bromessigsäure-*tert*.-butylester hinzugefügt und das Reaktionsgemisch 3 h bei 60°C kräftig gerührt. Danach wird auf Wasser gegeben und mit konz. Salzsäure neutralisiert. Man extrahiert die wässrige Phase dreimal mit Ethylacetat, vereinigt die organischen Phasen und trocknet über Magnesiumsulfat. Der Rückstand wird durch Chromatographie an Silicagel gereinigt (Laufmittel: Cyclohexan/Ethylacetat 10:1 → 8:1). Es werden 592 mg der Zielverbindung erhalten (73.2% d. Th.).
LC-MS (Methode 8): Rₜ = 3.36 min.; m/z = 503 (M+H)⁺
¹H-NMR (400 MHz, DMSO-d₆): δ = 8.61 (s, 1H), 8.10-8.04 (m, 2H), 7.63-7.51 (m, 3H), 5.30-5.20 (m, 1H), 4.00 (s, 2H), 3.52-3.42 (m, 1H), 2.20-2.10 (m, 1H), 2.06-1.98 (m, 1H), 1.88-1.78 (m, 1H), 1.50-1.12 (m, 14H).

### Beispiel 85A und Beispiel 86A

### (+)-({3-[(5-Brom-6-phenylfuro[2,3-d]pyrimidin-4-yl)oxy]cyclohexyl}oxy)essigsäure-tert.-butylester (Enantiomer 1) und (-)-({3-[(5-Brom-6-phenylfuro[2,3-d]pyrimidin-4-yl)oxy]cyclohexyl}oxy)essigsäure-tert.-butylester (Enantiomer 2)

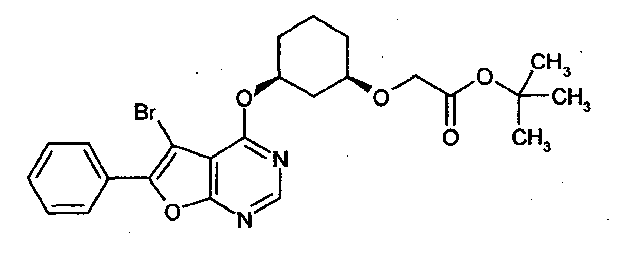

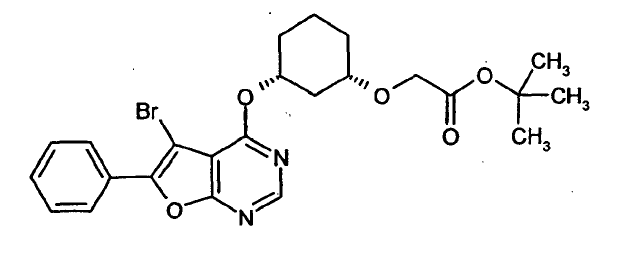

780 mg (1.64 mmol) (+/-)-({3-[(5-Brom-6-phenylfuro[2,3-d]pyrimidin-4-yl)oxy]cyclohexyl}oxy)-essigsäure-*tert*.-butylester werden durch Chromatographie an chiraler Phase in die Enantiomere getrennt [Säule: Daicel Chiralpak AS-H, 5 µm, 250 mm x 20 mm; Fluss: 15 ml/min; Temperatur: 28°C; Eluent: iso-Hexan/2-Propanol (+ 0.2% Diethylamin) 80:20].

### Enantiomer 1:

Ausbeute: 159 mg (20.4% d. Th.)
[α]_{D}²⁰ = +64.5°, c = 0.495, CHCl₃
LC-MS (Methode 8): Rₜ = 3.37 min.; m/z = 503 (M+H)⁺
¹H-NMR (400 MHz, DMSO-d₆): δ = 8.61 (s, 1H), 8.10 (d, 2H), 7.65-7.50 (m, 3H), 5.30-5.20 (m, 1H), 4.01 (s, 2H), 3.53-3.42 (m, 1H), 2.20-2.10 (m, 1H), 2.06-1.98 (m, 1H), 1.88-1.80 (m, 1H), 1.41 (s, 9H), 1.50-1.23 (m, 5H).

### Enantiomer 2:

Ausbeute: 320 mg (41.0% d. Th.)
[α]_{D}²⁰ = -68.9°, c = 0.54, CHCl₃
LC-MS (Methode 8): Rₜ = 3.37 min.; m/z = 503 (M+H)⁺
¹H-NMR (400 MHz, DMSO-d₆): δ = 8.61 (s, 1H), 8.10 (d, 2H), 7.65-7.50 (m, 3H), 5.30-5.20 (m, 1H), 4.01 (s, 2H), 3.53-3.42 (m, 1H), 2.20-2.10 (m, 1H), 2.06-1.98 (m, 1H), 1.88-1.80 (m, 1H), 1.41 (s, 9H), 1.50-1.23 (m, 5H).

### Beispiel 87A

### (+/-)-cis/trans-3-{[5-(4-Methoxyphenyly)-6-phenylfuro[2,3-d]pyrimidin-4-yl]oxy)cyclopentanol

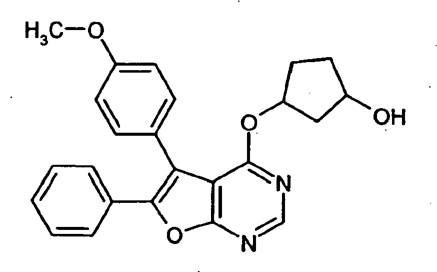

10 g (29.7 mmol) 4-Chlor-5-(4-methoxyphenyl)-6-phenylfuro[2,3-d]pyrimidin werden in 150 ml 70°C heissem Toluol mit 1,2-Dimethoxyethan versetzt, bis eine homogene Lösung entsteht. Anschließend werden 23.75 g (296.9 mmol) 50%-ige Natronlauge und 2.5 ml Wasser sowie, unter kräftigem Rühren, 1.0 g (2.97 mmol) Tetra-n-butylammoniumhydrogensulfat und 4.55 g (44.54 mmol) (+/-)-*cis*/*trans*-1,3-Cyclopentandiol hinzugefügt und die Reaktionsmischung für 4 h bei 70°C gerührt. Nach Abkühlen wird die Mischung auf Wasser gegeben und mit konz. Salzsäure neutralisiert. Man extrahiert dreimal mit Dichlormethan, wäscht die vereinigten organischen Phasen mit ges. Natriumchlorid-Lösung, trocknet über Magnesiumsulfat und engt im Vakuum ein. Aus dem Rückstand wird durch Chromatographie an Silicagel (Laufmittel: Cyclohexan/Ethylacetat 4:1 → 2:1) verunreinigtes Produkt erhalten. Durch Verrühren mit Methanol wird dieses Rohprodukt kristallisiert. Die erhaltenen Kristalle werden abfiltriert und getrocknet (Ausbeute: 690 mg). Die Mutterlauge wird im Vakuum konzentriert und durch präparative RP-HPLC weiter aufgereinigt. Auf diese Weise werden weitere 1230 mg des Zielprodukts gewonnen. Insgesamt werden 1920 mg der Zielverbindung erhalten (16.1% d. Th.).
LC-MS (Methode 8): Rₜ = 2.64 min.; m/z = 403 (M+H)⁺.
¹H-NMR (400 MHz, DMSO-d₆): δ = 8.59 (s, 1H), 7.57 (d, 2H), 7.42-7.33 (m, 5H), 7.00 (d, 2H), 5.64-5.59 (m, 1H), 4.60 (d, 1H), 4.15-4.09 (m, 1H), 3.82 (s, 3H), 2.15-2.03 (m, 1H), 1.92-1.84 (m, 1H), 1.83-1.74 (m, 1H), 1.73-1.62 (m, 1H), 1.61-1.51 (m, 1H), 1.50-0.91 (m, 1H).

### Beispiel 88A

### (+/-)-cis/trans-[(3-Hydroxycyclopentyl)oxy]essigsäure-tert.-butylester

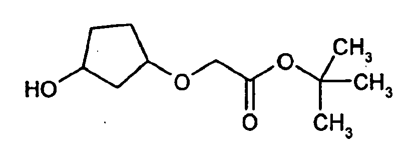

2.5 g (24.5 mmol) *cis*/*trans*-Cyclopentandiol werden in 5 ml THF gelöst und bei 0°C mit 16.3 ml (16.3 mmol) Phosphazen-Base P4-t-Bu (ca. 1 M Lösung in Hexan) versetzt. Nach 10 min wird die resultierende Lösung zu einer eisgekühlten Lösung von 4.77 g (24.5 mmol) Bromessigsäure-*tert*.-butylester getropft. Nach Ende der Zugabe wird auf RT erwärmt und die Mischung über Nacht gerührt. Man entfernt Teile des THF im Vakuum; verdünnt mit Ethylacetat und wäscht nacheinander mit 1 N Salzsäure, pH 7-Pufferlösung und ges. Natriumchlorid-Lösung, trocknet über Natriumsulfat und engt im Vakuum ein. Aus dem Rückstand wird das Produkt durch Chromatographie an Silicagel (Laufmittel: Cyclohexan/Ethylacetat 10:1 → 2:1) isoliert. Erhalten werden 631.4 mg (10.7% d. Th.) der Zielverbindung als *cis*/*trans*-Gemisch.
GC-MS (Methode 10): Rₜ = 7.35 min (*cis*), 7.21 min (*trans*); m/z = 217 (M+H)⁺
¹H-NMR (400 MHz, DMSO-d₆): δ = 4.52 (d, 0.25H, *trans*)*,* 4.48 (d, 0.75H, *cis*), 4.20-4.13 (m, 1H), 4.07-3.98 (m, 1H), 3.90 (d, 2H), 2.14-1.49 (m, 5H), 1.41 (s, 9H).

### Beispiel 89A

### (+/-)-cis/trans-[(3-Aminocyclopentyl)oxy]essigsäure-tert.-butylester

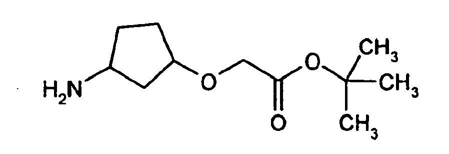

### Stufe a):

620 mg (2.87 mmol) (+/-)-*cis*/*trans*-[(3-Hydroxycyclopentyl)oxy]essigsäure-*tert*.-butylester und 1.2 ml (8.6 mmol) Triethylamin werden in 6.5 ml Dichlormethan gelöst und auf 0°C gekühlt. Tropfenweise werden 0.28 ml (3.58 mmol) Methansulfonsäurechlorid zugegeben. Die Mischung wird über 2 h auf RT erwärmt und dann mit Dichlormethan verdünnt. Die organische Phase wird nacheinander mit Wasser, 1 N Salzsäure, ges. Natriumhydrogencarbonat-Lösung und ges. Natriumchlorid-Lösung gewaschen, über Magnesiumsulfat getrocknet und im Vakuum konzentriert. Es werden 849 mg des Mesylats erhalten, welches direkt weiter umgesetzt wird.

### Stufe b):

849 mg des oben erhaltenen Mesylats werden bei RT in 10 ml DMF gelöst und mit 1125 mg (17.3 mmol) Natriumazid versetzt. Die Suspension wird über Nacht bei 70°C kräftig gerührt und dann nach Abkühlen auf Wasser gegeben. Man extrahiert dreimal mit Ethylacetat, wäscht die vereinigten organischen Phasen mit gesättigter Natriumchlorid-Lösung, trocknet über Magnesiumsulfat und konzentriert im Vakuum. Es werden 695 mg des Azids erhalten, welches direkt weiter umgesetzt wird.

### Stufe c):

695 mg des oben erhaltenen Azids werden in 3 ml Ethanol und 0.3 ml Wasser gelöst, mit 70 mg Palladium auf Aktivkohle versetzt und 4 h bei RT unter Wasserstoffatmosphäre (Normaldruck) gerührt. Der Katalysator wird durch Filtration über Kieselgur entfernt, das Filtrat im Vakuum eingeengt und der Rückstand im Hochvakuum getrocknet. Es werden 560 mg der Titelverbindung erhalten, welche ohne weitere Aufreinigung eingesetzt wird.

### Beispiel 90A

### cis-(+/-)-{[(4-Hydroxycyclopent-2-en-1-yl]oxy}essigsäure-tert.-butylester

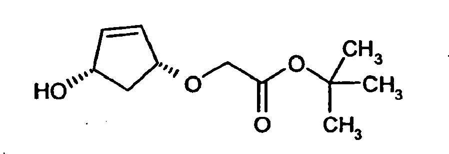

2.0 g (20 mmol) *cis*-4-Cyclopenten-1,3-diol werden in 1.5 ml DMF und 15 ml THF gelöst und bei 0°C portionsweise mit 799 mg (60%-ig, ca. 20 mmol) Natriumhydrid versetzt. Nach Ende der Zugabe wird die Mischung auf RT erwärmt und 1 h bei RT nachgerührt, bevor 2.7 ml (18.2 mmol) Bromessigsäure-*tert*.-butylester zugesetzt werden. Die Mischung wird anschließend über Nacht bei RT gerührt. Man gibt danach Wasser hinzu, extrahiert mit Dichlormethan, wäscht die organische Phase mit ges. Natriumchlorid-Lösung, engt im Vakuum ein und trocknet den Rückstand im Hochvakuum. Durch Chromatographie an Silicagel (Laufmittel: Cyclohexan/Ethylacetat 5:1 → 2:1) wird das Produkt isoliert. Es werden 1.10 g (25.6% d. Th.) der Zielverbindung erhalten.
GC-MS (Methode 11): Rₜ = 7.19 min.; m/z = 233 (M+H)⁺
¹H-NMR (400 MHz, DMSO-d₆): δ = 5.90 (dt, 2H), 5.00 (d, 1H), 4.44 (q, 1H), 4.35 (t, 1H), 3.99 (s, 2H), 2.60 (dd, 1H), 1.62 (s, 9H), 1.37 (dd, 1H).

### Beispiel 91A

### cis-(-)-{[(1R,4S)-4-Acetoxycyclopent-2-en-1-yl]oxy}essigsäure-tert.-butylester

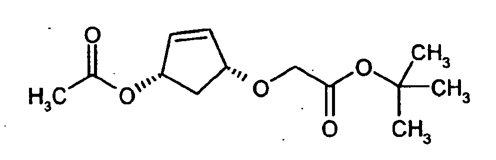

1.0 g (7.04 mmol) (1*R*,4*S*)-*cis*-4-Acetoxy-2-cyclopenten-1-ol werden in 5 ml Dichlormethan vorgelegt und unter Argon mit 155 mg (0.25 mmol) Rhodium(II)acetat (als Dimer) versetzt. Zur der kräftig gerührten Suspension werden dann bei RT 1.56 g (90%-ig, ca. 9.84 mmol) *tert*.-Butyldiazoessigester getropft. Nach 30 min werden weitere 0.3 eq. *tert*.-Butyldiazoessigester hinzugetropft und die Reaktionsmischung für weitere 30 min bei RT gerührt. Danach wird mit Dichlormethan verdünnt und dreimal mit Wasser und mit ges. Natriumchlorid-Lösung gewaschen. Man trocknet über Magnesiumsulfat und engt im Vakuum ein. Aus dem Rückstand wird das Produkt durch Chromatographie an Silicagel isoliert (Laufmittel: Cyclohexan/Ethylacetat 8:1). Es werden 1.33 g der Zielverbindung erhalten (73.7% d. Th.).
[α]_{D}²⁰ = -23°, c = 0.55, CHCl₃
GC-MS (Methode 9): Rₜ = 5.49 min.; m/z = 257 (M+H)⁺
¹H-NMR (400 MHz, DMSO-d₆): δ = 6.18 (dd, 1H), 5.97 (dd, 1H), 5.41-5.36 (m, 1H), 4.48-4.41 (m, 1H), 4.01 (s, 2H), 2.72 (dt, 1H), 2.10-1.98 (m, 3H), 1.55 (td, 1H), 1.43 (s, 9H)_{.}

### Beispiel 92A

### cis-(+)-{[(4-Hydroxycyclopent-2-en-1-yl]oxy}essigsäure-tert.-butylester

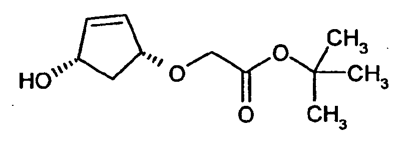

1.30 g (5.07 mmol) *cis*-(-)-{[(1*R*,4*S*)-4-Acetoxycyclopent-2-en-1-yl]oxy}essigsäure-*tert*.-butylester werden in 3 ml THF und 2 ml Methanol gelöst und bei RT tropfenweise mit 5.6 ml 1 N Natronlauge versetzt. Nach 10 min wird die Mischung mit Wasser verdünnt und dreimal mit Dichlormethan extrahiert. Die organischen Phasen werden vereinigt, mit ges. Natriumhydrogencarbonat-Lösung und ges. Natriumchlorid-Lösung gewaschen, über Magnesiumsulfat getrocknet und im Vakuum eingeengt. Erhalten werden 780 mg der Zielverbindung (71.8% d. Th.).
[α]_{D}²⁰ = +6.5°, c = 0.515, CHCl₃
GC-MS (Methode 9): Rₜ = 4.93 min.; m/z= 232 (M+NH₄)⁺
¹H-NMR (400 MHz, DMSO-d₆): δ = 5.93-5.89 (m, 2H), 5.00 (d, 1H), 4.45 (q, 1H), 4.36 (t, 1H), 3.99 (s, 2H), 2.65-2.56 (m, 1H), 1.42 (s, 9H), 1.40-1.31 (m, 1H).

### Beispiel 93A

### cis-(+)-{[(1S,3R)-3-Hydroxycyclopentyl]oxy}essigsäure-tert.-butylester

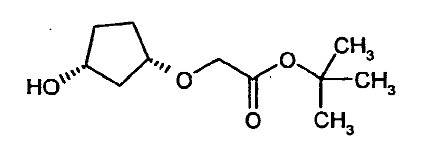

350 mg (1.63 mmol) *cis*-(+)-{[(4-Hydroxycyclopent-2-en-1-yl]oxy}essigsäure-*tert*.-butylester werden in 7 ml Ethanol gelöst und mit 35 mg (0.163 mmol) Platin(IV)oxid versetzt. Die Suspension wird bei RT 4 h lang kräftig unter einer Wasserstoffatmosphäre (Normaldruck) gerührt. Danach wird die Reaktionsmischung über Kieselgur filtriert. Man wäscht mit Ethanol nach, vereinigt alle Filtrate und engt im Vakuum ein. Erhalten werden 278.8 mg der Zielverbindung (81.5% d. Th.).
[α]_{D}²⁰ = +6.8°, c = 0.51, CHCl₃
GC-MS (Methode 9): Rₜ = 4.93 min.; m/z = 234 (M+NH₄)⁺
¹H-NMR (400 MHz, DMSO-d₆): δ = 4.50 (d, 1H), 4.01-3.96 (m, 1H), 3.90 (s, 2H), 3.88-3.81 (m, 1H), 2.14-2.05 (m, 2H), 1.71-0.96 (m, 4H), 1.40 (s, 9H).

### Beispiel 94A

### cis-(+)-{[(1S,4R)-4-Acetoxycyclopent-2-en-1-yl]oxy}essigsäure-tert.-butylester

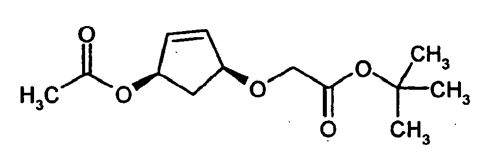

Zu einer Lösung von 1.6 g (11.26 mmol) (1*R*,3*S*)-(+)-*cis*-4-Cyclopenten-1,3-diol-1-acetat in 9.6 ml Dichlormethan werden 248.7 mg (0.56 mmol) Rhodium(11)-acetat (als Dimer) gegeben. Die resultierende Suspension wird kräftig gerührt und tropfenweise mit 2.49 g (90%-ig, ca. 15.8 mmol) *tert*.-Butyldiazoessigester versetzt. Nach Ende der Zugabe wird 2 h bei RT gerührt. Danach werden weitere 0.5 eq. *tert*.- Butyldiazoessigester nachgesetzt und die Mischung für eine weitere Stunde bei RT gerührt. Nach Einengen im Vakuum wird der Rückstand durch Chromatographie an Silicagel gereinigt (Laufmittel: Cyclohexan/Ethylacetat 10:1). Es werden 1.96 g der Zielverbindung erhalten (68% d. Th.).
[α]_{D}²⁰ = +27.2°, c = 0.59, CHCl₃
LC-MS (Methode 12): Rₜ = 2.06 min.; m/z= 257 (M+H)⁺
¹H-NMR (400 MHz, DMSO-d₆): δ = 6.20-6.15 (m, 1H), 6.00-5.94 (m, 1H), 5.41-5.37 (m, 1H), 4.47-4.41 (m, 1H), 4.00 (s, 2H), 2.79-2.70 (m, 1H), 2.00 (s, 3H), 1.58-1.50 (td, 1H), 1.42 (s, 9H).

### Beispiel 95A

### cis-(-)-{[(1S,4R)-4-Hydroxycyclopent-2-en-1-yl]oxy}essigsäure-tert.-butylester

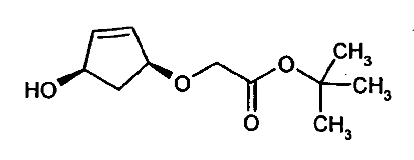

1000 mg (3.9 mmol) *cis*-(+)-{[(1*S*,4*R*)-4-Acetoxycyclopent-2-en-1-yl]oxy}essigsäure-*tert*.-butylester werden in 3 ml THF und 2 ml Methanol gelöst und bei RT tropfenweise mit 4.7 ml 1 N Natronlauge versetzt. Nach 10 min wird die Mischung mit Wasser verdünnt und dreimal mit Dichlormethan extrahiert. Die organischen Phasen werden vereinigt, mit ges. Natriumhydrogencarbonat-Lösung und ges. Natriumchlorid-Lösung gewaschen, über Magnesiumsulfat getrocknet und im Vakuum eingeengt. Erhalten werden 570 mg der Zielverbindung (68.2% d. Th.).
[α]_{D}²⁰ = -9.3°, c = 0.515, CHCl₃
GC-MS (Methode 9): Rₜ = 4.93 min.; m/z = 232 (M+NH₄)⁺
¹H-NMR (400 MHz, DMSO-d₆): δ = 6.39-5.92 (m, 2H), 5.00 (d, 1H), 4.45 (q, 1H), 4.35 (t, 1H), 3.99 (s, 2H), 2.64-2.56 (m, 1H), 1.42 (s, 9H), 1.40-1.31 (m, 1H).

### Beispiel 96A

### cis-(-)-{[(1R,3S)-3-Hydroxycyclopentyl]oxy}essigsäure-tert.-butylester

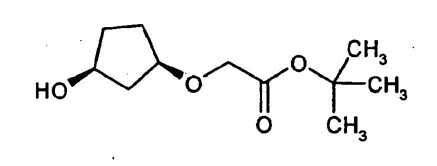

1170 mg (5.46 mmol) *cis*-(-)-{[(1*S*,4*R*)-4-Hydroxycyclopent-2-en-1-yl]oxy}essigsäure-*tert*.-butylester werden in 10 ml Ethanol gelöst und mit 25 mg Platin(IV)oxid versetzt. Die Suspension wird bei RT 4 h lang kräftig unter einer Wasserstoffatmosphäre (Normaldruck) gerührt. Danach wird die Reaktionsmischung über Kieselgur filtriert. Man wäscht zweimal mit einem Gemisch aus Ethanol und Wasser nach, vereinigt alle Filtrate und engt im Vakuum ein. Erhalten werden 940 mg der Zielverbindung (79.9% d. Th.).
[α]_{D}²⁰ = -7_{.}4°, c = 0.475, CHCl₃
GC-MS (Methode 9): Rₜ = 3.88 min.; m/z = 234 (M+NH₄)⁺
¹H-NMR (400 MHz, DMSO-d₆): δ = 4.50 (d, 1H), 4.00-3.91 (m, 2H), 3.90 (s, 2H), 3.89-3.80 (m, 2H), 1.71-1.45 (m, 6H), 1.40 (s, 9H).

### Beispiel 97A

### trans-(-)-4-(2-tert.-Butoxy-2-oxoethoxy)cyclopent-2-en-1-yl-4-nitrobenzoat

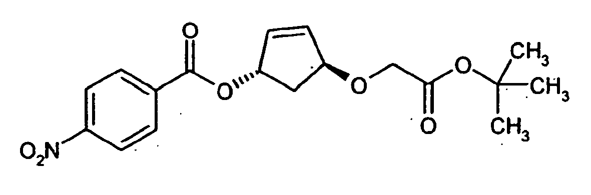

Zu einer Lösung von 400 mg (1.87 mmol) *cis*-(-)-{[(1*R*,3*S*)-3-Hydroxycyclopentyl]oxy}essigsäure-*tert*.-butylester in 1.8 ml THF werden unter Argon bei RT nacheinander 881.4 mg (3.36 mmol) Triphenylphosphin, 561.6 mg (3.36 mmol) 4-Nitrobenzoesäure und tropfenweise 1.46 g (ca. 3.36 mmol) einer 40%-igen Lösung von Diethylazodicarboxylat in Toluol gegeben. Nach 3 h werden weitere 0.5 eq. Triphenylphosphin und 0.5 eq. Diethylazodicarboxylat in Toluol hinzugefügt und die Reaktionsmischung für weitere 2 h bei RT gerührt. Man gibt dann die Reaktionsmischung auf Wasser und extrahiert dreimal mit Dichlormethan. Die vereinigten organischen Phasen werden über Magnesiumsulfat getrocknet und im Vakuum eingeengt. Aus dem Rückstand wird das Produkt durch Chromatographie an Silicagel isoliert (Laufmittel: Cyclohexan/Ethylacetat 8:1 → 3:1). Es werden 555 mg (81.8% d. Th.) der Zielverbindung erhalten.
[α]_{D}²⁰ = -169.8°, c = 0.48, CHCl₃
LC-MS (Methode 12): Rₜ = 2.71 min.; m/z = 381 (M+NH₄)⁺
¹H-NMR (400 MHz, DMSO-d₆): δ = 8.35 (d, 2H), 8.19 (d, 2H), 6.39-6.34 (m, 1H), 6.20-6.17 (m, 1H), 6.00-5.95 (m, 1H), 4.88-4.81 (m, 1H), 4.04 (s, 2H), 2.32-2.19 (m, 2H), 1.43 (s, 9H).

### Beispiel 98A

### trans-(-)-{[(4-Hydroxycyclopent-2-en-1-yl]oxy}essigsäure-tert.-butylester

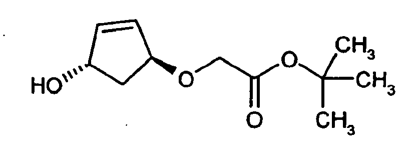

300 mg (0.83 mmol) *trans*-(-)-4-(2-*tert*.-Butoxy-2-oxoethoxy)cyclopent-2-en-1-yl-4-nitrobenzoat werden in 2.4 ml THF und 0.6 ml Methanol gelöst und bei RT mit 0.9 ml 1 N Natronlauge versetzt. Nach 30 min wird die Reaktionsmischung auf Wasser gegeben und dreimal mit Dichlormethan extrahiert. Man vereinigt die organischen Phasen und wäscht nacheinander mit ges. Natriumcarbonat-, Natriumhydrogencarbonat- und Natriumchlorid-Lösung. Es wird über Magnesiumsulfat getrocknet und im Vakuum eingeengt. Erhalten werden 137.3 mg (77.6% d. Th.) der Zielverbindung.
[α]_{D}²⁰ = -83.1 °, c = 0.525, CHCl₃
GC-MS (Methode 9): Rₜ = 5.03 min.; m/z = 157 (M-C₄H₉)⁺
¹H-NMR (400 MHz, DMSO-d₆): δ = 5.97 (s, 2H), 4.81 (d, 1H), 4.79-4.71 (m, 1H), 4.68-4.62 (m, 1H), 3.93 (s, 2H), 2.00-1.93 (m, 1H), 1.80-1.73 (m, 1H), 1.40 (s, 9H).

### Beispiel 99A

### trans-{[4-(1,3-Dioxo-1,3-dihydro-2H-isoindol-2-yl)cyclopent-2-en-1-yl]oxy}essigsäure-tert: butylester

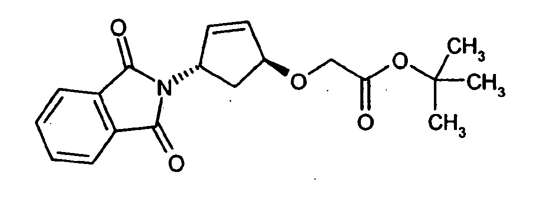

250 mg (1.17 mmol) *cis*-(-)-{[(4-Hydroxycyclopent-2-en-1-yl]oxy}essigsäure-*tert*.-butylester werden in 1.25 ml THF gelöst und unter Argon nacheinander mit 309 mg (2.1 mmol) Phthalimid, 550.9 mg (2.1 mmol) Triphenylphosphin und tropfenweise mit 914 mg (ca. 2.1 mmol) einer 40%-igen Lösung von Diethylazodicarboxylat in Toluol versetzt. Nach 3 h werden weitere 0.5 eq. Triphenylphosphin und 0.5 eq. Diethylazodicarboxylat in Toluol hinzugefügt und die Reaktionsmischung für weitere 2 h bei RT gerührt. Man gibt die Mischung dann auf Wasser und extrahiert dreimal mit Dichlormethan. Die vereinigten organischen Phasen werden über Magnesiumsulfat getrocknet und im Vakuum eingeengt. Aus dem Rückstand wird das Produkt durch Chromatographie an Silicagel isoliert (Laufmittel: Cyclohexan/Ethylacetat 8:1 → 3:1). Erhalten werden 255 mg der Zielverbindung (63.7% d. Th.).
[α]_{D}²⁰ = -256.6°, c = 0.545, CHCl₃
LC-MS (Methode 3): Rₜ = 2.29 min.; m/z = 361 (M+NH₄)⁺
¹H-NMR (400 MHz, DMSO-d₆): δ = 7.83 (s, 4H), 6.20-6.15 (m, 1H), 5.97-5.90 (m, 1H), 5.38-5.30 (m, 1H), 4.92-4.88 (m, 1H), 4.03 (s, 2H), 2.40-2.30 (m, 1H), 2.19-2.19 (m, 1H), 1.46 (s, 9H).

### Beispiel 100A

### trans-{[(4-Aminocyclopent-2-en-1-yl]oxy}essigsäure-tert.-butylester

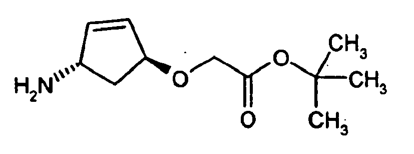

120 mg (0.349 mmol) *trans*-{[4-(1,3-Dioxo-1,3-dihydro-2*H*-isoindol-2-yl)cyclopent-2-en-1-yl]-oxy}essigsäure-*tert*.-butylester werden in 0.3 ml Ethanol mit 22 µl (0.545 mmol) Hydräzinmonohydrat versetzt. Die Mischung wird 15 min unter Rückfluss erhitzt. Es entsteht ein voluminöser Niederschlag, der nach Abkühlen durch Filtration entfernt und mit wenig Ethanol nachgewaschen wird. Das Filtrat wird im Vakuum eingeengt und das erhaltene Produkt (74 mg) ohne weitere Reinigung eingesetzt.
GC-MS (Methode 9): Rₜ = 4.93 min.; m/z = 214 (M+H)⁺
¹H-NMR (400 MHz, DMSO-d_{b}): δ = 5.96-5.90 (m, 1H), 5.89-5.83 (m, 1H), 4.68-4.60 (m, 1H), 4.04-3.85 (m, 4H), 2.07-1.98 (m, 1H), 1.97-1.79 (m, 1H), 1.68-1.57 (m, 1H), 1.41 (s, 9H).

### Beispiel 101A

### 2-(2-Fluorphenyl)-2-hydroxy-1-(4-methoxyphenyl)ethanon

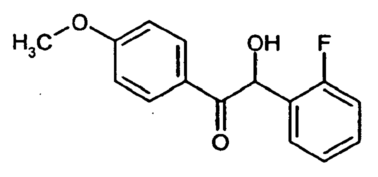

441 ml (1.10 mol) einer 2.5 M n-Butyllithium-Lösung in n-Hexan werden bei -78°C zu einer Lösung von 156 ml (1.11 mol) *N*,*N*-Diisopropylamin in 1937 ml 1,2-Dimethoxyethan in dem Maße zugetropft, dass die Temperatur -60°C nicht überschreitet. Nach 1 min Rühren bei dieser Temperatur wird innerhalb von 30 min eine Lösung von 236 g (1.00 mol) (4-Methoxyphenyl)[(trimethylsilyl)oxy]acetonitril [N. Kurono, J. Org. Chem. 2005, 16, 6530-6532] in 753 ml 1,2-Dimethoxyethan zugetropft. Anschließend wird nach 30 min Rühren bei dieser Temperatur eine Lösung von 128 g (1.00 mol) 2-Fluorbenzaldehyd in 753 ml 1,2-Dimethoxyethan innerhalb von 20 min zugetropft. Das Reaktionsgemisch lässt man innerhalb von 4 h auf Raumtemperatur erwärmen. Nach Zugabe von 3800 ml ges. wässriger Ammoniumchlorid-Lösung wird mit Essigsäureethylester extrahiert. Die organische Phase wird mit ges. Ammoniumchlorid-Lösung gewaschen, über Natriumsulfat getrocknet, filtriert und das Filtrat im Vakuum eingeengt. Der Rückstand wird mit 3800 ml Dioxan, 2700 ml Methanol sowie 3120 ml 1 M Salzsäure versetzt und 16 h bei Raumtemperatur gerührt. Nach Zugabe von 8000 ml ges. wässriger Natriumchlorid-Lösung wird mit 4000 ml Essigsäureethylester extrahiert. Die wässrige Phase wird mit 2000 ml Essigsäureethylester nachextrahiert. Die vereinigten organischen Phasen werden mit 2000 ml Wasser und 2000 ml ges. Natriumchlorid-Lösung gewaschen, über Natriumsulfat getrocknet, filtriert und das Filtrat im Vakuum eingeengt. Der Rückstand wird in 600 ml Diisopropylether verrührt und filtriert. Die Mutterlauge wird im Vakuum eingeengt. Der Rückstand wird in Dichlormethan aufgenommen und mittels Flash-Chromatographie an Kieselgel (Laufmittel: Cyclohexan/Essigsäureethylester 4:1) gereinigt. Die so erhaltene Produktfraktion wird in Diisopropylether/Petrolether (1:1) verrührt, filtriert und im Vakuum getrocknet. Es werden 94 g (80% Reinheit, 29% d. Th.) der Titelverbindung erhalten.
LC-MS (Methode 7): Rₜ = 4.59 min.; m/z = 261 (M+H)⁺
¹H-NMR (400 MHz, CDCl₃): δ = 7.93-7.91 (m, 2H), 7.28-7.18 (m, 2H), 7.10-7.04 (m, 2H), 6.89-6.86 (m, 2H), 6.19 (d, 1H), 4.69 (s, 1H), 3.82 (s, 3H).

### Beispiel 102A

### 2-Amino-5-(2-fluorphenyl)-4-(4-methoxyphenyl)-3-furonitril

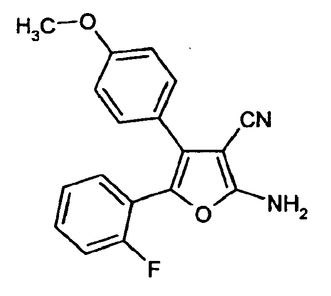

84 g (0.32 mol) 2-(2-Fluorphenyl)-2-hydroxy-1-(4-methoxyphenyl)ethanon und 32 g (0.48 mol) Malonsäuredinitril werden in 153 ml THF vorgelegt. Nach fünf Minuten Rühren werden unter Eiskühlung 49 ml (36 g, 0.36 mol) Triethylamin zugesetzt. Das Reaktionsgemisch wird 1 h unter Eiskühlung gerührt. Dann lässt man das Reaktionsgemisch auf Raumtemperatur erwärmen und 4 h bei dieser Temperatur rühren. Nach Zugabe von 1000 ml Essigsäureethylester wird die organische Phase fünfmal mit 300 ml Wasser gewaschen, über Natriumsulfat getrocknet und filtriert. Das Filtrat wird im Vakuum eingeengt. Der Rückstand wird in Dichlormethan aufgenommen und mittels Flash-Chromatographie an Kieselgel (Laufmittel: Dichlormethan/Methanol 70:1, dann Cyclohexan/Essigsäureethylester 2:1) gereinigt. 37 g (0.11 mol) an dabei zurückgewonnenem 2-(2-Fluorphenyl)-2-hydroxy-1-(4-methoxyphenyl)ethanon werden nach obiger Vorschrift erneut mit 14 g (0.03 mol) Malonsäuredinitril und 21 ml (15 g, 0.15 mol) Triethylamin in 67 ml THF umgesetzt. Es werden insgesamt 70 g (52% Reinheit, 36% d. Th.) der Zielverbindung erhalten.
¹H-NMR (400 MHz, CDCl₃): δ = 7.23-7.1 (m, 4H), 7.03-6.95 (m, 2H), 6.82-6.79 (m, 2H), 4.86 (s, NH₂), 3.74 (s, 3H).

### Beispiel 103A

### 6-(2-Fluorphenyl)-5-(4-methoxyphenyl)furo[2,3-d]pyrimidin-4(3H)-on

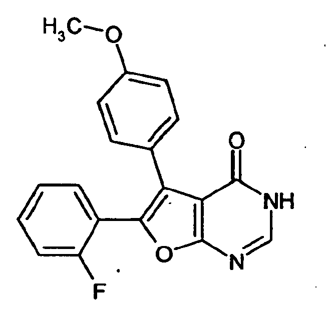

436 ml Essigsäureanhydrid werden bei 0°C tropfenweise mit 268 ml Ameisensäure versetzt und 30 min bei dieser Temperatur gerührt. Dann wird eine Lösung von 70 g (0.12 mol) 2-Amino-5-(2-fluorphenyl)-4-(4-methoxyphenyl)-3-furonitril in 100 ml Essigsäureanhydrid hinzugefügt und das Gemisch 24 h bei 130°C gerührt. Nach Abkühlen auf Raumtemperatur wird der Ansatz im Ölpumpenvakuum bei 50°C eingeengt. Der Rückstand wird in 250 ml Diisopropylether unter Eiskühlung 30 min verrührt, filtriert, mit 70 ml Diisopropylether gewaschen und im Vakuum getrocknet. Es werden 23.7 g (60% d. Th.) der Titelverbindung erhalten.
HPLC (Methode 1): Rₜ = 4.27 min.
MS (DCI): m/z = 354 (M+NH₄)⁺
¹H-NMR (400 MHz, CDCl₃): δ = 12.68 (br. s, NH), 8.19 (d, 1H), 7.53-7.45 (m, 2H), 7.34-7.25 (m, 4H), 6.91-6.88 (m, 2H), 3.76 (s, 3H).

### Beispiel 104A

### 4-Chlor-6-(2-fluorphenyl)-5-(4-methoxyphenyl)furo[2,3-d]pyrimidin

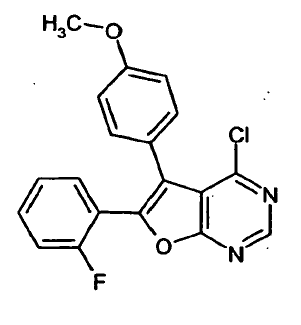

Ein Gemisch aus 20 g (0.06 mol) 6-(2-Fluorphenyl)-5-(4-methoxyphenyl)furo[2,3-d]pyrimidin-4(3*H*)-on in 78 ml Sulfolan und 11 ml (18 g, 0.12 mol) Phosphorylchlorid wird 1 h bei 120°C gerührt. Nach Abkühlen auf Raumtemperatur wird die Reaktionslösung unter kräftigem Rühren und Eiskühlung in ein Gemisch aus 1000 ml Wasser und 100 ml 25%-ige wässrige Ammoniak-Lösung getropft. Der bei 10°C ausfallende Feststoff wird abfiltriert und mehrmals mit Wasser gewaschen. Der Feststoff wird erneut in 700 ml Essigsäureethylester gelöst und die Lösung zweimal mit je 500 ml Wasser gewaschen. Die organische Phase wird über Natriumsulfat getrocknet, filtriert und das Filtrat im Vakuum eingeengt. Der Rückstand wird in 60 ml Diisopropylether verrührt, filtriert und im Vakuum getrocknet. Es werden 18 g (81% d. Th.) der Titelverbindung erhalten.
HPLC (Methode 1): Rₜ = 5.03 min.
¹H-NMR (400 MHz, DMSO-d₆): δ = 8.90 (s, 1H), 7.58-7.50 (m, 2H), 7.36-7.27 (m, 4H), 7.01-6.97 (m, 2H), 3.79 (s, 3H).

### Beispiel 105A

### 1-(4-Ethylphenyl)-2-(2-fluorphenyl)-2-hydroxyethanon

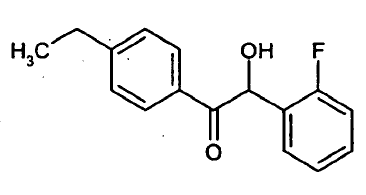

217 ml (0.54 mol) einer 2.5 M n-Butyllithium-Lösung in Hexan werden bei -78°C zu einer Lösung von 77 ml (56 g, 0.55 mol) *N*,*N*-Diisopropylamin in 960 ml 1,2-Dimethoxyethan so zugetropft, dass die Temperatur -60°C nicht überschreitet. Nach 15 min Rühren bei dieser Temperatur wird innerhalb von 30 min eine Lösung von 116 g (0.50 mol) (4-Ethylphenyl)[(trimethylsilyl)oxy]acetonitril [D.S. Dhanoa, J. Med. Chem. 1993, 36 (23), 3738-3742] in 373 ml 1,2-Dimethoxyethan zugetropft. Anschließend wird nach 30 min Rühren bei dieser Temperatur eine Lösung von 64 g (0.50 mol) 2-Fluorbenzaldehyd in 373 ml 1,2-Dimethoxyethan innerhalb von 20 min zugetropft. Das Reaktionsgemisch lässt man innerhalb von 4 h auf Raumtemperatur erwärmen. Nach Zugabe von 1900 ml ges. wässriger Ammoniumchlorid-Lösung wird mit Essigsäureethylester extrahiert. Die organische Phase wird mit ges. Ammoniumchlorid-Lösung gewaschen, über Natriumsulfat getrocknet und filtriert. Das Filtrat wird im Vakuum eingeengt. Der Rückstand wird mit 1900 ml Dioxan, 1350 ml Methanol sowie 1560 ml 1 M Salzsäure versetzt und 16 h bei Raumtemperatur gerührt. Nach Zugabe von 4000 ml ges. wässriger Natriumchlorid-Lösung wird mit 2000 ml Essigsäureethylester extrahiert. Die organische Phase wird mit 1000 ml Wasser und 1000 ml ges. Natriumchlorid-Lösung gewaschen, über Natriumsulfat getrocknet und filtriert. Das Filtrat wird im Vakuum eingeengt. Der Rückstand wird mittels Flash-Chromatographie an Kieselgel (Laufmittel: Cyclohexan/Essigsäureethylester 5:1) gereinigt. Die so erhaltene Produktfraktion wird in 80 ml Diisopropylether und 240 ml Petrolether verrührt, filtriert, mit Petrolether gewaschen und im Vakuum getrocknet. Es werden 50 g (85% Reinheit, 33% d. Th.) der Titelverbindung erhalten.
HPLC (Methode 1): Rₜ = 4.50 min.
MS (DCI): m/z = 276 (M+NH₄)⁺
¹H-NMR (400 MHz, CDCl₃): δ = 7.87-7.85 (m, 2H), 7.28-7.19 (m, 4H), 7.11-7.04 (m, 2H), 6.22 (d, 1H), 4.64 (d, 1H), 2.65 (q, 2H), 1.21 (t, 3H).

### Beispiel 106A

### 2-Amino-4-(4-ethylphenyl)-5-(2-fluorphenyl)-3-furonitril

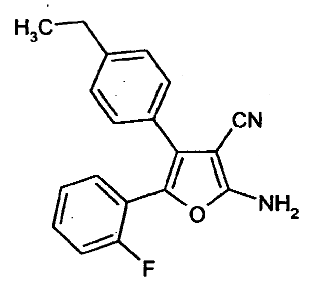

50 g (0.19 mol) 1-(4-Ethylphenyl)-2-(2-fluorphenyl)-2-hydroxyethanon und 17 g (0.25 mol) Malonsäuredinitril werden in 93 ml DMF vorgelegt. Nach fünf Minuten Rühren werden unter Eiskühlung 17 ml (12 g, 0.12 mol) Diethylamin hinzugefügt. Das Reaktionsgemisch wird 1 h unter Eiskühlung gerührt. Dann lässt man auf Raumtemperatur erwärmen und 4 h bei dieser Temperatur rühren. Nach Zugabe von 500 ml Wasser wird nach 30 min Rühren die wässrige Phase abdekantiert. Durch erneute Zugabe von 500 ml Wasser und erneutes Dekantieren erhält man einen öligen Rückstand, der in Essigsäureethylester gelöst, über Natriumsulfat getrocknet und filtriert wird. Das Filtrat wird im Vakuum eingeengt. Der Rückstand enthält laut DC-Analyse (Laufmittel: Cyclohexan/Essigsäureethylester 4:1) noch 1-(4-Ethylphenyl)-2-(2-fluorphenyl)-2-hydroxyethanon. Der Rückstand wird daher nach obiger Vorschrift in 90 ml DMF erneut mit 5.5 g (0.08 mol) Malonsäuredinitril und 10 ml (7 g, 0.10 mol) Diethylamin umgesetzt. Das Reaktionsgemisch wird in 500 ml Essigsäureethylester gegeben und dreimal mit je 300 ml Wasser und einmal mit 300 ml ges. Natriumchlorid-Lösung gewaschen. Die organische Phase wird über Natriumsulfat getrocknet und filtriert. Das Filtrat wird im Vakuum eingeengt. Der Rückstand wird mittels Flash-Chromatographie an Kieselgel (Laufmittel: Cyclohexan/Essigsäureethylester 3:1) gereinigt. Es werden 36 g (61% d. Th.) der Titelverbindung erhalten, welche ohne weitere Charakterisierung umgesetzt werden.

### Beispiel 107A

### 5-(4-Ethylphenyl)-6-(2-fluorphenyl)furo[2,3-d]pyrimidin-4(3H)-on

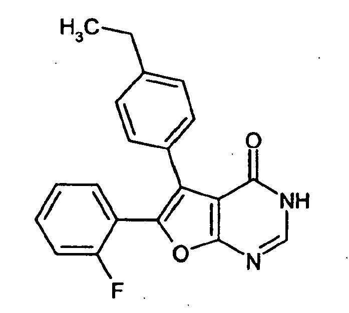

280 ml (2.97 mol) Essigsäureanhydrid werden bei 0°C tropfenweise mit 140 ml (3.71 mol) Ameisensäure versetzt und 30 min bei dieser Temperatur gerührt. Dann werden 36.0 g (0.12 mol) 2-Amino-4-(4-ethylphenyl)-5-(2-fluorphenyl)-3-furonitril hinzugefügt und das Gemisch 24 h bei 130°C gerührt. Nach Abkühlen auf Raumtemperatur wird der Ansatz im Ölpumpenvakuum bei 50°C eingeengt. Der Rückstand wird in 150 ml Diisopropylether bei -10°C 30 min lang verrührt, abfiltriert, mit 50 ml eisgekühltem Diisopropylether gewaschen und im Vakuum getrocknet. Es werden 20.6 g (86% Reinheit, 45% d. Th.) der Titelverbindung erhalten.
HPLC (Methode 1): Rₜ = 4.65 min.
MS (ESIpos): m/z= 335 (M+H)⁺
¹H-NMR (400 MHz, DMSO-d₆): δ = 12.68 (br. s, NH), 8.20 (s, 1H), 7.53-7.45 (m, 2H), 7.36-7.25 (m, 4H), 7.21-7.16 (m, 2H), 2.61 (q, 2H), 1.19 (t, 3H).

### Beispiel 108A

### 4-Chlor-5-(4-ethylphenyl)-6-(2-fluorphenyl)furo[2,3-d]pyrimidin

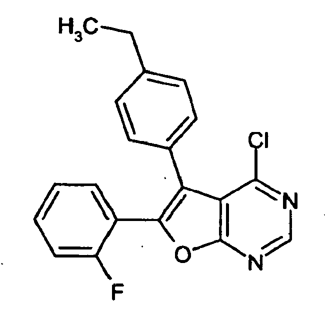

Eine Suspension von 20.0 g (0.06 mol) 5-(4-Ethylphenyl)-6-(2-fluorphenyl)furo[2,3-d]pyrimidin-4(3*H*)-on in 100 ml (165 g, 1.07 mol) Phosphorylchlorid wird 1 h bei 120°C gerührt. Nach Abkühlen auf Raumtemperatur wird die Reaktionslösung unter kräftigem Rühren in ein Gemisch aus 330 ml Wasser und 610 ml 25%-iger wässriger Ammoniak-Lösung getropft, wobei ein Temperaturanstieg auf 55-65°C beobachtet wird. Man lässt das Reaktionsgemisch auf Raumtemperatur abkühlen. Nach zweimaliger Extraktion mit je 500 ml Dichlormethan wird die organische Phase mit ges. wässriger Natriumchlorid-Lösung gewaschen, über Natriumsulfat getrocknet und filtriert. Das Filtrat wird im Vakuum eingeengt. Der Rückstand wird in 150 ml Petrolether verrührt, abfiltriert, mit eisgekühltem Petrolether gewaschen und im Vakuum getrocknet. Es werden 18.7 g (90% Reinheit, 80% d. Th.) der Titelverbindung erhalten.
LC-MS (Methode 6): Rₜ = 3.14 min.; m/z = 353 (M+H)⁺
¹H-NMR (400 MHz, DMSO-d₆): δ = 8.91 (s, 1H), 7.58-7.49 (m, 2H), 7.36-7.24 (m, 6H), 2.66 (q, 2H), 1.21 (t, 3H).

### Beispiel 109A

### 1-[(Z)-2-Chlor-2-nitrovinyl]-4-ethylbenzol

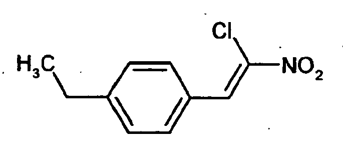

Analog Literaturvorschrift [D. Dauzonne, Synthesis, 1990, 66-70] wird eine Mischung von 10.0 g (74.5 mmol) 4-Ethylbenzaldehyd, 6.8 ml (13.7 g, 97.6 mmol) Bromnitromethan, 54.7 g (670.7 mmol) Dimethylammoniumchlorid und 0.6 g (11.2 mmol) Kaliumfluorid in 150 ml Xylol am Wasserabscheider bei 160°C für 15 Stunden und anschließend bei 175°C für sieben Stunden gerührt. Nach Zugabe von 25 ml Wasser und 100 ml Dichlormethan wird die organische Phase abgetrennt und die wässrige Phase dreimal mit je 100 ml Dichlormethan extrahiert. Die vereinigten organischen Extrakte werden über Natriumsulfat getrocknet, filtriert und im Vakuum eingeengt. Der Rückstand wird an Kieselgel (Laufmittel: Cyclohexan/Dichlormethan 1:1) chromatographiert. Es werden 11.9 g (85% Reinheit, 64% d. Th.) der Zielverbindung erhalten.
LC-MS (Methode 8): Rₜ = 2.84 min.
¹H-NMR (400 MHz, DMSO-d₆): δ = 8.60 (s, 1H), 7.94 (d, 2H), 7.42 (d, 2H), 2.68 (q, 2H), 1.21 (t, 3H).

### Beispiel 110A

### 5-(4-Ethylphenyl)furo[2,3-d]pyrimidin-4(3H)-on

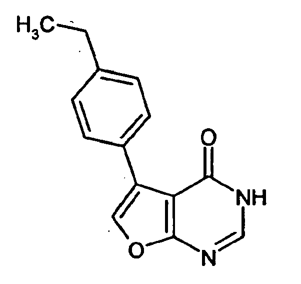

Analog Literaturvorschrift [D. Dauzonne, Tetrahedron, 1992, 3069-3080] wird eine Suspension von 11.9 g (85% Reinheit, 47.6 mmol) 1-[(*Z*)-2-Chlor-2-nitrovinyl]-4-ethylbenzol und 5.9 g (52.3 mmol) 4,6-Dihydroxypyrimidin in 200 ml Ethanol 30 Minuten bei 60-70°C gerührt. Anschließend werden 14.4 ml (14.6 g, 96.1 mmol) 1,8-Diazabicyclo[5.4.0]undec-7-en langsam zugegeben. Die resultierende Reaktionslösung wird sechs Stunden unter Rückfluss und danach 15 Stunden bei 60°C gerührt. Nach Einengen im Vakuum wird der Rückstand in Dichlormethan aufgenommen und an Kieselgel (Laufmittel: Cyclohexan/Essigsäureethylester 1:1 → 1:5) chromatographiert. Der erhaltene Feststoff wird in Diethylether verrührt und filtriert. Es werden 5.0 g (44% d. Th.) der Zielverbindung erhalten.
LC-MS (Methode 8): Rₜ = 2.14 min.; m/z = 241 (M+H)⁺
¹H-NMR (400 MHz, DMSO-d₆): δ = 12.68 (br. s, NH), 8.18 (d, 2H), 7.87 (d, 2H), 7.26 (d, 2H), 2.63 (q, 2H), 1.20 (t, 3H).

### Beispiel 111A

### 5-(4-Ethylphenyl)-6-iodfuro[2,3-d]pyrimidin-4(3H)-on

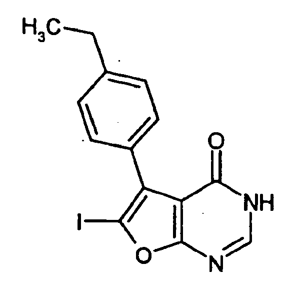

Eine Lösung von 5.0 g (20.9 mmol) 5-(4-Ethylphenyl)furo[2,3-d]pyrimidin-4(3*H*)-on in 250 ml Acetonitril/Tetrachlormethan (1:1) wird mit 7.0 g (31.3 mmol) *N-*Iodsuccinimid versetzt. Die resultierende Suspension wird zwei Stunden unter Rückfluss gerührt. Nach Abkühlen auf Raumtemperatur wird das Reaktionsgemisch im Vakuum eingeengt. Der Rückstand wird in Essigsäureethylester verrührt und filtriert. Das Filtrat wird mit Wasser versetzt. Nach Abtrennung der organischen Phase wird die wässrige Phase mehrmals mit Essigsäureethylester extrahiert. Die vereinigten organischen Phasen werden im Vakuum eingeengt. Der Rückstand wird in Essigsäureethylester aufgenommen und an Kieselgel (Laufmittel: Cyclohexan/Essigsäureethylester 1:1 → 1:2) chromatographiert. Der erhaltene Feststoff wird in Diethylether/n-Pentan verrührt und filtriert. Es werden 1.4 g (85% Reinheit, 16% d. Th.) der Zielverbindung erhalten.
LC-MS (Methode 8): Rₜ = 2.34 min.; m/z = 367 (M+H)⁺
¹H-NMR (400 MHz, DMSO-d₆): δ = 12.68 (br. s, NH), 8.10 (s, 1H), 7.48 (d, 2H), 7.29 (d, 2H), 2.66 (q, 2H), 1.23 (t, 3H).

### Beispiel 112A

### 4-Chlor-5-(4-ethylphenyl)-6-iodfuro[2,3-d]pyrimidin

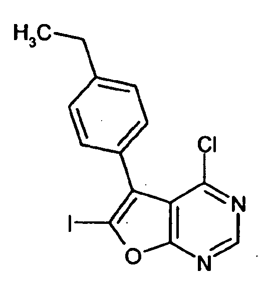

Eine Suspension von 1.4 g (85% Reinheit, 3.3 mmol) 5-(4-Ethylphenyl)-6-iodfuro[2,3-d]pyrimidin-4(3H)-on in 20 ml (32.9 g, 214.6 mmol) Phosphorylchlorid wird eine Stunde unter Rückfluss gerührt. Nach Einengen im Vakuum wird der Rückstand mit eiskaltem Wasser und Dichlormethan versetzt. Die organische Phase wird über Natriumsulfat getrocknet und filtriert. Das Filtrat wird im Vakuum eingeengt und getrocknet. Es wird 1.0 g (70% Reinheit, 57% d. Th.) der Zielverbindung erhalten.
LC-MS (Methode 6): Rₜ = 2.97 min.; m/z = 385 (M+H)⁺
¹H-NMR (400 MHz, DMSO-d₆): δ = 8.80 (s, 1H), 7.43-7.36 (m, 4H), 2.70 (q, 2H), 1.26 (t, 3H).

### Beispiel 113A

### 4-{[(3R)-1-Benzylpiperidin-3-yl]oxy}-5-(4-ethylphenyl)-6-iodfuro[2,3-d]pyrimidin

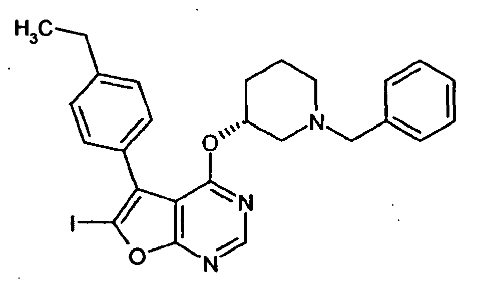

Eine Lösung von 483 mg (2.53 mmol) (3*R*)-1-Benzylpiperidin-3-ol [H. Tomori, Bull. Chem. Soc. Jpn. 69 1, 207-216 (1996)] in 5 ml THF wird mit 106 mg (2.65 mmol) Natriumhydrid (60%-ige Dispersion in Mineralöl) versetzt. Nach zehn Minuten Rühren wird eine Lösung von 1020 mg (70% Reinheit, 1.86 mmol) 4-Chlor-5-(4-ethylphenyl)-6-iodfuro[2,3-d]pyrimidin in 5 ml THF sowie 47 mg (0.13 mmol) Tetra-n-butylammoniumiodid zugegeben. Das Reaktionsgemisch wird fünf Stunden bei Raumtemperatur gerührt. Nach Zugabe von Wasser und Essigsäureethylester wird die abgetrennte organische Phase mit 1 N Salzsäure und ges. Natriumchlorid-Lösung gewaschen und anschließend im Vakuum eingeengt. Der Rückstand wird in Acetonitril aufgenommen und mittels präparativer RP-HPLC (Gradient: Wasser/Acetonitril) gereinigt. Es werden 266 mg (20% d. Th.) des gewünschten Produkts erhalten.
LC-MS (Methode 8): Rₜ = 1.93 min.; m/z = 540 (M+H)⁺
¹H-NMR (400 MHz, DMSO-d₆): δ = 8.46 (s, 1H), 7.58-7.56 (m, 2H), 7.32-7.29 (m, 2H), 7.24-7.17 (m, 5H), 5.34-5.29 (m, 1H), 3.45 (d, 2H), 2.71-2.63 (m, 3H), 2.39-2.33 (m, 2H), 2.30-2.24 (m, 1H), 1.89-1.84 (m, 1H), 1.66-1.60 (m, 1H), 1.47-1.39 (m, 2H), 1.21 (t, 3H).

### Beispiel 114A

### 4-{[(3R)-1-Benzylpiperidin-3-yl]oxy}-5-(4-ethylphenyl)-6-(2-fluorphenyl)furo[2,3-d]pyrimidin

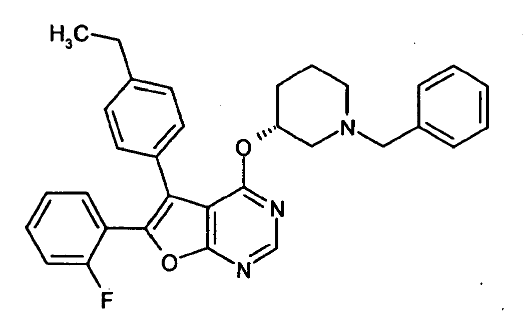

Ein Gemisch aus 365 mg (0.68 mmol) 4-{(3*R*)-1-Benzylpiperidin-3-yl]oxy}-5-(4-ethylphenyl)-6-iodfuro[2,3-d]pyrimidin und 24 mg (0.03 mmol) Bis(triphenylphosphin)palladium(II)chlorid in 15 ml DMSO wird mit 0.68 ml einer 2 M wässrigen Natriumcarbonat-Lösung versetzt. Anschließend werden 118 mg (0.85 mmol) (2-Fluorphenyl)boronsäure zugegeben und der Ansatz 15 Stunden bei 80°C gerührt. Das Reaktionsgemisch wird danach filtriert und direkt mittels präparativer RP-HPLC (Gradient: Wasser/Acetonitril) gereinigt. Es werden 291 mg (84% d. Th.) der Zielverbindung erhalten.
LC-MS (Methode 12): Rₜ = 2.18 min.; m/z = 508 (M+H)⁺
¹H-NMR (400 MHz, DMSO-d₆): δ = 8.57 (s, 1H), 7.57-7.53 (m, 2H), 7.39-7.29 (m, 4H), 7.24-7.18 (m, 7H), 5.40-5.34 (m, 1H), 3.49-3.46 (m, 2H), 2.74-2.69 (m, 1H), 2.62 (q, 2H), 2.43-2.35 (m, 2H), 2.32-2.25 (m, 1H), 1.94-1.87 (m, 1H), 1.71-1.64 (m, 1H), 1.50-1.44 (m, 2H), 1.17 (t, 3H).

### Beispiel 115A

### (3R)-3-{[5-(4-Ethylphenyl)-6-(2-fluorphenyl)furo[2,3-d]pyrimidin-4-yl]oxy}piperidiniumformiat

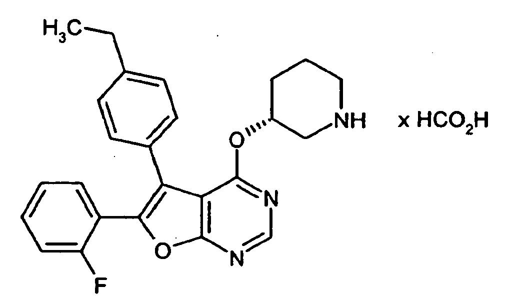

Eine mit Argon überschichtete Lösung von 275 mg (0.54 mmol) 4-{[(3*R*)-1-Benzylpiperidin-3-yl]oxy}-5-(4-ethylphenyl)-6-(2-fluorphenyl)furo[2,3-d]pyrimidin in 5 ml Methanol/Ethanol (1:2) wird mit 30 mg 10%-igem Palladium auf Aktivkohle versetzt und drei Stunden unter einer Wasserstoffatmosphäre (Normaldruck) bei Raumtemperatur gerührt. Nach Zugabe von weiteren 70 mg 10%-igem Palladium auf Aktivkohle wird das Reaktionsgemisch erneut für 19 Stunden unter einer Wasserstoffatmosphäre (Normaldruck) bei Raumtemperatur gerührt. Weitere 175 mg 10%-iges Palladium auf Aktivkohle sowie 0.2 ml Ameisensäure werden hinzugefügt und das Reaktionsgemisch abermals für 15 Stunden unter einer Wasserstoffatmosphäre (Normaldruck) bei Raumtemperatur gerührt. Nach dem Abfiltrieren des Katalysators wird der Katalysator-Rückstand mit Methanol/Wasser gewaschen. Das Filtrat wird im Vakuum eingeengt, der Rückstand in Acetonitril/DMSO aufgenommen und mittels präparativer RP-HPLC (Gradient: Wasser/Acetonitril/Ameisensäure) gereinigt. Es werden 137 mg (54% d. Th.) des gewünschten Produkts erhalten.
LC-MS (Methode 8): Rₜ = 1.74 min.; m/z = 418 (M-HCO₂H+H)⁺
¹H-NMR (400 MHz, DMSO-d₆): δ = 8.57 (s, 1H), 7.57-7.53 (m, 2H), 7.39-7.29 (m, 4H), 7.24-7.18 (m, 7H), 5.40-5.34 (m, 1H), 3.49-3.46 (m, 2H), 2.74-2.69 (m, 1H), 2.62 (q, 2H), 2.43-2.35 (m, 2H), 2.32-2.25 (m, 1H), 1.94-1.87 (m, 1H), 1.71-1.64 (m, 1H), 1.50-1.44 (m, 2H), 1.17 (t, 3H).

### Beispiel 116A

### 3-{[5-(4-Ethylphenyl)-6-(2-fluorphenyl)furo[2,3-d]pyrimidin-4-yl]oxy}cyclohexanol

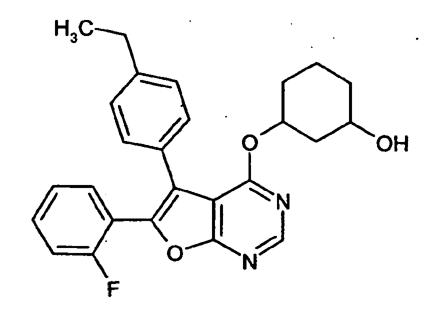

Ein Gemisch aus 1.65 g (14.17 mmol) Cyclohexan-1,3-diol in 45 ml Toluol, 15 ml 1,2-Dimethoxyethan und 15 ml Wasser wird bei 70°C mit 4.5 ml einer 12.5 N Natronlauge versetzt. Nach Zugabe von 0.19 g (0.57 mmol) Tetra-n-butylammoniumhydrogensulfat und 2.0 g (5.67 mmol) 4-Chlor-5-(4-ethylphenyl)-6-(2-fluorphenyl)furo[2,3-d]pyrimidin wird das Reaktionsgemisch 17 Stunden bei 70°C gerührt. Nach Abkühlen auf Raumtemperatur wird mit konz. Salzsäure auf pH 7 eingestellt. Es wird mit Dichlormethan extrahiert. Die organische Phase wird mit ges. Natriumchlorid-Lösung gewaschen, über Natriumsulfat getrocknet, filtriert und im Vakuum eingeengt. Der Rückstand wird an Kieselgel (Laufinittel: Cyclohexan/Essigsäureethylester 2:1) chromatographiert. Es werden 0.60 g (24% d. Th.) des gewünschten Produkts als racemisches Diastereomerengemisch erhalten.
LC-MS (Methode 8): Rₜ = 2.96 min.; m/z = 433 (M+H)⁺
¹H-NMR (400 MHz, DMSO-d₆): [Minder-Stereoisomer in Klammern) δ = 8.62 (s, 1H), [8.61, s, 1H], 7.57-7.52 (m, 2H), 7.34-7.28 (m, 4H), 7.20-7.18 (m, 2H), [5.68-5.64, m, 1H], 5.21-5.14 (m, 1H), 4.75 (d, OH), [4.45, d, OH], 3.57-3.48 (m, 1H), 2.63 (q, 2H), 2.37-2.31 (m, 1H), 2.08-2.03 (m, 1H), 1.82-1.77 (m, 1H), 1.74-1.69 (m, 1H), 1.34-1.02 (m, 4H), 1.20 (t, 3H).

### Beispiel 117A

### 4-{[(3R)-1-Benzylpiperidin-3-yl]oxy}-6-(2-fluorphenyl)-5-(4-methoxyphenyl)furo[2,3-d]pyrimidin

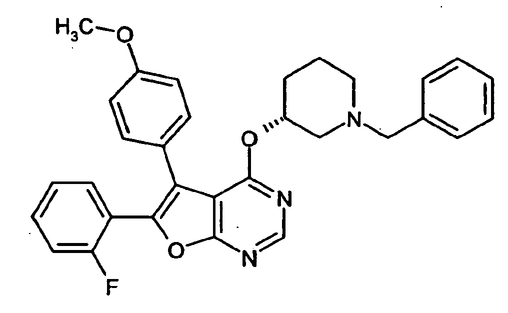

1037 mg (5.37 mmol) (3*R*)-1-Benzylpiperidin-3-ol [H. Tomori, Bull. Chem. Soc. Jpn. 69 1, 207-216 (1996)] werden in 10 ml THF gelöst und mit 268 mg (6.71 mmol) Natriumhydrid (60%-ig in Mineralöl) versetzt. Nach 10 Minuten wird eine Lösung von 2000 mg (5.64 mmol) 4-Chlor-6-(2-fluorphenyl)-5-(4-methoxyphenyl)furo[2,3-d]pyrimidin in 10 ml THF sowie 99 mg (0.27 mmol) Tetra-n-butylammoniumiodid zugesetzt. Das Reaktionsgemisch wird 16 Stunden unter Rückfluss erhitzt. Anschließend werden 100 ml Wasser und 100 ml Essigsäureethylester zugegeben. Die organische Phase wird abgetrennt und mit 50 ml 1 N Salzsäure sowie 100 ml ges. Natriumchlorid-Lösung gewaschen. Die wässrige Phase wird mit 50 ml Essigsäureethylester rückextrahiert, die vereinigten organischen Extrakte werden über Natriumsulfat getrocknet, filtriert und im Vakuum eingeengt. Es werden 2645 mg (89% d. Th., 92% Reinheit) des gewünschten Produkts erhalten.
LC-MS (Methode 8): Rₜ = 1.92 min.; m/z = 510 (M+H)⁺
¹H-NMR (400 MHz, DMSO-d₆): δ = 8.63 (s, 1H), 7.58-7.51 (m, 2H), 7.51-7.43 (m, 510, 7.34-7.25 (m, 4H), 6.93-6.88 (m, 2H), 5.62-5.58 (m, 1H), 3.77 (s, 3H), 3.68-3.66 (m, 1H), 2.89-2.83 (m, 1H), 2.36-2.27 (m, 2H), 1.91-1.81 (m, 3H), 1.51-1.45 (m, 1H).

### Beispiel 118A

### 6-(2-Fluorphenyl)-5-(4-methoxyphenyl)-4-[(3R)-piperidin-3-yloxy]furo[2,3-d]pyrimidin-Formiat

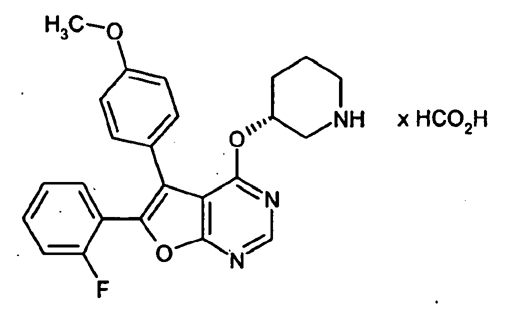

Eine mit Argon überschichtete Lösung von 510 mg (1.00 mmol) 4-{[(3*R*)-1-Benzylpiperidin-3-yl]-oxy}-6-(2-fluorphenyl)-5-(4-rnethoxyphenyl)furo[2,3-d]pyrimidin in 5 ml einer 4.4%-igen Lösung von Ameisensäure in Methanol wird mit 400 mg Palladium black versetzt und zwei Tage bei Raumtemperatur gerührt. Nach dem Abfiltrieren des Katalysators wird der Katalysator-Rückstand mit Methanol/Wasser gewaschen. Das Filtrat wird im Vakuum eingeengt und der Rückstand mittels präparativer RP-HPLC (Eluent: Wasser/Acetonitril-Gradient mit 0.1% Ameisensäure) gereinigt. Es werden 70 mg (12% d. Th., 80% Reinheit) des gewünschten Produkts erhalten.
LC-MS (Methode 8): Rₜ =1.66 min.; m/z = 420 (M-HCO₂H+H)⁺.

### Beispiele 119A

### 6-(2-Fluorphenyl)-5-(4-methoxyphenyl)-4-[(3R)-piperidin-3-yloxy]furo[2,3-d]pyrimidin

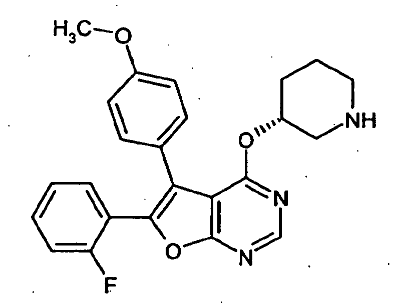

Eine mit Argon überschichtete Lösung von 2600 mg (4.69 mmol) 4-{[(3*R*)-1-Benzylpiperidin-3-yl]oxy}-6-(2-fluorphenyl)-5-(4-methoxyphenyl)furo[2,3-d]pyrimidin in 25 ml einer 4.4%-igen Lösung von Ameisensäure in Methanol wird mit 300 mg Palladium black versetzt und fünf Stunden bei Raumtemperatur gerührt. Danach werden erneut 300 mg Palladium black sowie 0.9 ml Ameisensäure zugesetzt und weitere 16 Stunden bei Raumtemperatur gerührt. Nach dem Abfiltrieren des Katalysators wird der Katalysator-Rückstand mit Methanol/Wasser gewaschen. Das Filtrat wird im Vakuum eingeengt, der Rückstand in Acetonitril verrührt, abfiltriert und im Vakuum getrocknet. Es werden 1376 mg (68% d. Th.) des gewünschten Produkts erhalten.
LC-MS (Methode 8): Rₜ = 1.62 min.; m/z = 420 (M+H)⁺
¹H-NMR (400 MHz, DMSO-d₆): δ = 8.66 (s, 1H), 7.58-7.52 (m, 2H), 7.42 (d, 2H), 7.35-7.30 (m, 2H), 6.93 (d, 2H), 5.45-5.40 (m, 1H), 3.77 (s, 3H), 3.47-3.30 (m, 2H), 3.10-3.04 (m, 2H), 2.07-2.02 1H), 1.78-1.71 (m, 3H).

### Beispiel 120A

### 3-[(2R,4R)-1-(tert.-Butoxycarbonyl)-4-{[5-(4-methoxyphenyl)-6-phenylfuro[2,3-d]pyrimidin-4-yl]-oxy}piperidin-2-yl]propansäuremethylester

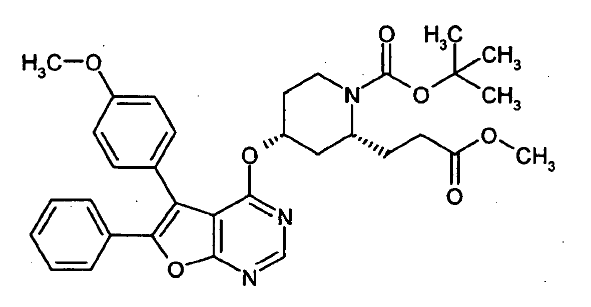

631.7 mg (1.87 mmol) 4-Chlor-5-(4-methoxyphenyl)-6-phenylfuro[2,3-d]pyrimidin und 490 mg (1.71 mmol) *tert*.-Butyl-(2*R*,4*R*)-4-hydroxy-2-(3-methoxy-3-oxopropyl)piperidin-1-carboxylat werden in 1 ml DMF gelöst, auf -10°C gekühlt und mit 1.02 ml (2.05 mmol) Phosphazen-Base P2-t-Bu (ca. 2 M Lösung in THF) versetzt. Die Reaktionsmischung wird 1h bei 0°C gerührt und dann auf Wasser gegeben. Man extrahiert dreimal mit Dichlormethan, vereinigt die organischen Phasen, wäscht mit ges. Natriumchlorid-Lösung und trocknet über Magnesiumsulfat. Nach dem Einengen im Vakuum wird das Produkt durch Chromatographie an Silicagel gereinigt (Laufmittel: Cyclohexan/Ethylacetat 20:1 → 3:1). Erhalten werden 420 mg (38.1% d. Th.) der Zielverbindung.
LC-MS (Methode 8): Rₜ = 3.26 min.; m/z = 588 (M+H)⁺
¹H-NMR (400 MHz, DMSO-d₆): δ = 8.60 (s, 1H), 7.55 (d, 2H), 7.43-7.32 (m, 5H), 7.02 (d, 2H), 5.58 (s, 1H), 4.02-3.93 (m, 1H), 3.82 (s, 3H), 3.70-3.60 (m, 1H), 3.51 (s, 3H), 1.91-1.60 (m, 6H), 1.47-1.39 (m, 1H), 1.36 (s, 9H), 1.16-1.05 (m, 2H).

### Beispiel 121A

### 3-[(2R,4R)-1-(tert.-Butoxycarbonyl)-4-{[5-(4-methoxyphenyl)-6-phenylfuro[2,3-d]pyrimidin-4-yl]-oxy}piperidin-2-yl]propansäure

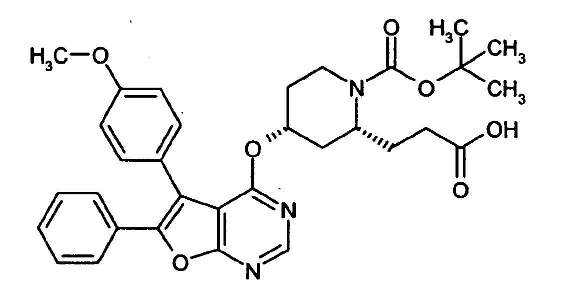

35 mg (0.06 mmol) 3-[(2*R*,4*R*)-1-(*tert*.-Butoxycarbonyl)-4-{[5-(4-methoxyphenyl)-6-phenylfuro-[2,3-d]pyrimidin-4-yl]oxy}piperidin-2-yl]propansäuremethylester werden in 0.1 ml Methanol gelöst, auf 0°C gekühlt und mit ca. 240 mg 10%-iger Natronlauge versetzt. Die Mischung wird bei ca. 40°C für mehrere Stunden, anschließend bei RT über Nacht gerührt. Die Reaktionsmischung wird dann mit 1 N Salzsäure leicht sauer gestellt (pH ca. 3) und mehrfach mit Dichlormethan extrahiert. Die vereinigten organischen Phasen werden mit ges. Natriumchlorid-Lösung gewaschen, über Magnesiumsulfat getrocknet und im Vakuum eingeengt. Die Zielverbindung wird in quantitativer Ausbeute (34 mg) erhalten und nicht weiter gereinigt.

### Beispiel 122A

### [1-({[5-(4-Methoxyphenyl)-6-phenylfuro[2,3-d]pyrimidin-4-yl]oxy}methyl)cyclobutyl]methanol

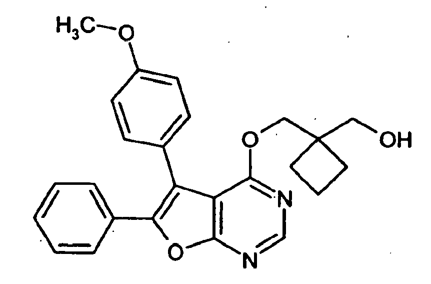

Eine Lösung von 1.72 g (14.85 mmol) Cyclobutan-1,1-diyl-dimethanol [F.X. Tavares, J. Med. Chem. 2004, 47 (21), 5057-5068] in 20 ml Toluol, 8 ml 1,2-Dimethoxyethan und 8 ml Wasser wird bei 70°C mit 2.6 ml einer 11.25 N Natronlauge versetzt. Nach Zugabe von 0.10 g (0.30 mmol) Tetra-n-butylammoniumhydrogensulfat und 1.00 g (2.97 mmol) 4-Chlor-5-(4-methoxyphenyl)-6-phenylfuro[2,3-d]pyrimidin wird das Reaktionsgemisch 17 h bei 70°C gerührt. Nach Abkühlen auf Raumtemperatur wird mit konzentrierter Salzsäure auf pH 7 eingestellt. Es wird dreimal mit je 50 ml Dichlormethan extrahiert. Die vereinigten organischen Extrakte werden mit ges. Natriumchlorid-Lösung gewaschen, über Natriumsulfat getrocknet und filtriert. Das Filtrat wird im Vakuum eingeengt. Der Rückstand wird in Acetonitril verrührt, filtriert und das Filtrat mittels präparativer RP-HPLC (Gradient: Wasser/Acetonitril) gereinigt. Es werden 0.30 g (24% d. Th.) des gewünschten Produkts erhalten.
LC-MS (Methode 3): Rₜ = 2.67 min.; m/z = 417 (M+H)⁺
¹H-NMR (400 MHz, DMSO-d₆): δ = 8.58 (s, 1H), 7.56-7.54 (m, 2H), 7.42-7.37 (m, 5H), 7.04-6.99 (m, 2H), 4.56 (t, 1H), 4.30 (s, 2H), 3.81 (s, 3H), 3.21 (d, 2H), 1.77-1.58 (m, 6H).

### Ausführungsbeispiele:

### Beispiel 1

### 3-{[6-(4-Bromphenyl)-5-phenylfuro[2,3-d]pyrimidin-4-yl]amino}phenoxy-essigsäuremethylester

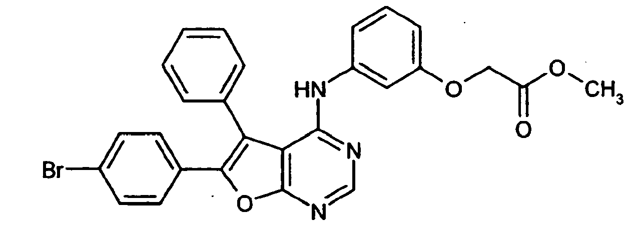

400 mg (1.04 mmol) 6-(4-Bromphenyl)-4-chlor-5-phenylfuro[2,3-d]pyrimidin (Herstellung gemäß WO 03/018589) und 225.5 mg (1.25 mmol) 3-Aminophenoxy-essigsäuremethylester werden in einem Ölbad für 1.5 h auf 150°C erhitzt. Nach Abkühlen wird der Rückstand in DMSO aufgenommen und über Kieselgel filtriert (Laufmittel: Cyclohexan/Ethylacetat 2:1). Erhalten werden 140 mg (25.5% d. Th.) der Zielverbindung als gelblicher Feststoff.
LC-MS (Methode 5): Rₜ = 3.30 min.; m/z= 530, 532 (M+H)⁺
¹H-NMR (400 MHz, DMSO-d₆): δ = 8.57 (s, 1H), 7.19 (s, 5H), 7.61 (d, 2H), 7.44 (d, 2H), 7.22-7.18 (m, 2H), 6.82 (s, 1H), 6.86 (dd, 1H), 6.61 (dd, 1H), 4.77 (s, 2H), 3.71 (s, 3H).

### Beispiel 2

### 3-[(5,6-Diphenylfuro[2,3-d]pyrimidin-4-yl)amino]phenoxy-essigsäuremethylester

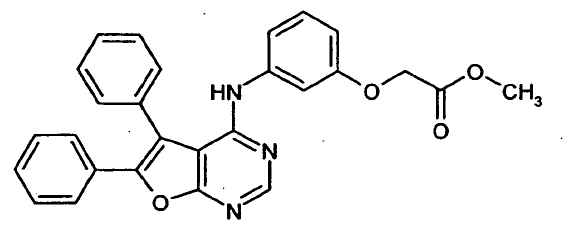

200 mg (0.377 mmol) 3-{[6-(4-Bromphenyl)-5-phenylfuro[2,3-d]pyrimidin-4-yl]amino}phenoxy-essigsäuremethylester werden in 5 ml Dichlormethan und 2 ml THF gelöst und unter Argon mit 40 mg 10%-igem Palladium auf Aktivkohle versetzt. Die Mischung wird unter einer Wasserstoffatmosphäre von 3 bar Überdruck für 3 h bei RT gerührt, bevor der Katalysator abfiltriert wird. Man wäscht den Katalysator-Rückstand mit Dichlormethan und Methanol, konzentriert die vereinigten Filtrate im Vakuum und chromatographiert den Rückstand an Silicagel (Laufmittel: Dichlormethan/Ethylacetat 10:1). Erhalten werden 79.1 mg (45.5% d. Th.) der Zielverbindung als farbloser Feststoff.
LC-MS (Methode 3): Rₜ = 2.87 min.; m/z = 452 (M+H)⁺
¹H-NMR (300 MHz, DMSO-d₆): δ = 8.58 (s, 1H), 7.70 (s, 5H), 7.56-7.51 (m, 2H), 7.44-7.38 (m, 3H), 7.25-7.18 (m, 2H), 6.80 (s, 1H), 6.78 (dd, 1H), 6.61 (dd, 1H), 4.78 (s, 2H), 3.72 (s, 3H).

### Beispiel 3

### 3-[(5,6-Diphenylfuro[2,3-d]pyrimidin-4-yl)amino]phenoxy-essigsäure

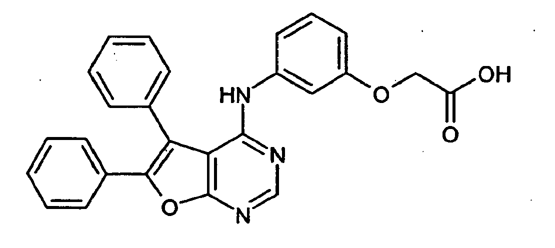

50 mg (0.111 mmol) 3-[(5,6-Diphenylfuro[2,3-d]pyrimidin-4-yl)amino]phenoxy-essigsäuremethylester werden in 2 ml THF gelöst, bei RT mit 0.33 ml 1 N Natronlauge versetzt und 1 h bei 50°C gerührt. Es wird auf RT abgekühlt und das THF im Vakuum entfernt. Der Rückstand wird mit Wasser und dann unter Eiskühlung mit 1 N Salzsäure versetzt. Der ausgefallene Feststoff wird abfiltriert, mehrmals mit Wasser gewaschen und im Vakuum getrocknet. Erhalten werden 39.4 mg (81.3% d. Th.) der Zielverbindung als weißer Feststoff.
LC-MS (Methode 3): Rₜ = 2.52 min.; m/z = 438 (M+H)⁺
¹H-NMR (300 MHz, DMSO-d₆): δ = 13.04 (br. s, 1H), 8.58 (s, 1H), 7.19 (s, 5H), 7.55-7.50 (m, 2H), 7.44-7.36 (m, 3H), 7.24-7.14 (m, 2H), 6.82-6.77 (m, 2H); 6.60 (dd, 1H), 4.62 (s, 2H).

### Beispiel 4

### 3-{[6-(4-Bromphenyl)-5-(4-fluorphenyl)furo[2,3-d]pyrimidin-4-yl]amino}phenoxy-essigsäuremethylester

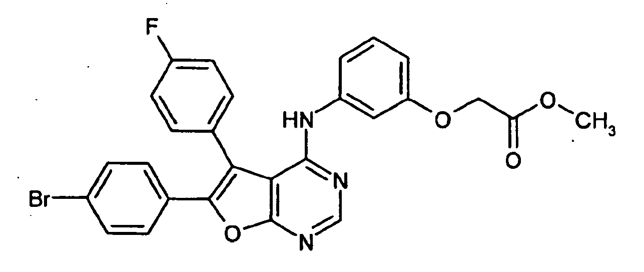

400 mg (0.991 mmol) 6-(4-Bromphenyl)-4-chlor-5-(4-fluorphenyl)furo[2,3-d]pyrimidin (Herstellung siehe WO 03/018589) und 215.5 mg (1.19 mmol) 3-Aminophenoxy-essigsäuremethylester werden in einem Ölbad für 1.5 h auf 150°C erhitzt. Nach Abkühlen wird der Rückstand in DMSO aufgenommen und über Kieselgel filtriert (Laufmittel: Dichlormethan/Ethylacetat 10:1). Isoliert werden 242 mg eines Gemischs, das durch präparative HPLC weiter aufgereinigt wird. Erhalten werden 120 mg (15% d. Th.) der Zielverbindung als farbloser Feststoff.
LC-MS (Methode 6): Rₜ = 3.2 min.; m/z = 548, 550 (M+H)⁺.

### Beispiel 5

### 3-{[5-(4-Fluorphenyl)-6-phenylfuro[2,3-d]pyrimidin-4-yl]amino} phenoxy-essigsäuremethylester

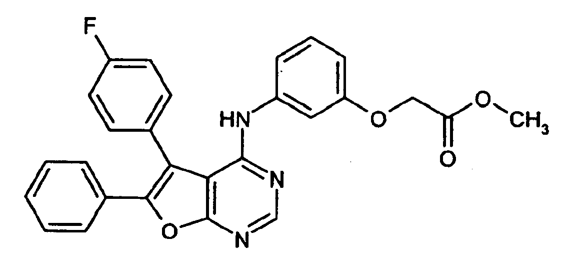

115 mg (0.21 mmol) 3-{[6-(4-Bromphenyl)-5-(4-fluorphenyl)furo[2,3-d]pyrimidin-4-yl]amino}-phenoxy-essigsäuremethylester werden in 5 ml Dichlormethan und 5 ml Ethylacetat gelöst und unter Argon mit 22 mg 10%-igem Palladium auf Aktivkohle versetzt. Die Mischung wird unter einer Wasserstoffatmosphäre von 3 bar Überdruck bei RT gerührt, bis das Startmaterial komplett umgesetzt ist. Der Katalysator wird abfiltriert, das erhaltene Filtrat im Vakuum konzentriert und der Rückstand an Silicagel chromatographiert (Laufmittel: Dichlormethan/Ethylacetat 10:1). Erhalten werden 27.9 mg (28.3% d. Th.) der Zielverbindung als farbloser Feststoff.
LC-MS (Methode 6): Rₜ = 3.02 min.; m/z = 470 (M+H)⁺
¹H-NMR (300 MHz, DMSO-d₆): δ = 8.55 (s, 1H), 7.74-7.68(m, 2H), 7.55-7.39 (m, 6H), 7.26-7.20 (m, 2H), 7.02 (s, 1H), 6.86 (d, 1H), 6.63 (dd, 1H), 4.78 (s, 2H), 3.71 (s, 3H).

### Beispiel 6

### 3-{[5-(4-Fluorphenyl)-6-phenylfuro[2,3-d]pyrimidin-4-yl]amino}phenoxy-essigsäure

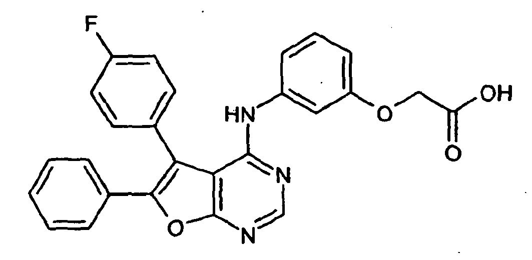

21.1 mg (0.045 mmol) 3-{[5-(4-Fluorphenyl)-6-phenylfuro[2,3-d]pyrimidin-4-yl]amino}phenoxy-essigsäuremethylester werden in 1 ml THF gelöst, bei RT mit 0.135 ml 1 N Natronlauge versetzt und 1 h bei 50°C gerührt. Es wird auf RT abgekühlt und das THF im Vakuum entfernt. Der Rückstand wird mit Wasser und dann unter Eiskühlung mit 1 N Sahsäure versetzt. Der ausgefallene Feststoff wird abfiltriert, mehrmals mit Wasser gewaschen und bei 40°C im Vakuum getrocknet. Erhalten werden 11.5 mg (56.2% d. Th.) der Zielverbindung als weißer Feststoff.
LC-MS (Methode 3): Rₜ = 2.51 min.; m/z = 456 (M+H)⁺
¹H-NMR (300 MHz, DMSO-d₆):δ = 13.03 (br. s, 1H), 8.55 (s, 1H), 7.75-7.69 (m, 2H), 7.55-7.38 (m, 7H), 7.8-7.19 (m, 2H), 6.98 (s, 1H), 6.83 (dd, 1H), 6.61 (dd, 1H), 4.65 (s, 3 H).

### Beispiel 7

### 3-{[5,6-Bis(4-methoxyphenyl)-furo[2,3-d]pyrimidin-4-yl]amino}phenoxy-essigsäuremethylester

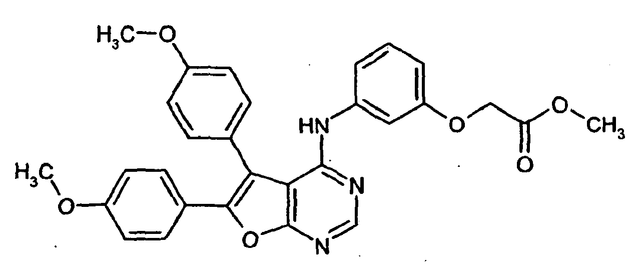

400 mg (1.091 mmol) 4-Chlor-5,6-bis(4-methoxyphenyl)furo[2,3-d]pyrimidin (Herstellung siehe WO 03/018589) und 237.1 mg (1.309 mmol) 3-Aminophenoxy-essigsäuremethylester werden in einem Ölbad für 1.5h auf 150°C erhitzt. Nach dem Abkühlen wird der Rückstand mit Dichlormethan versetzt und über Kieselgel gereinigt (Laufmittel: Dichlormethan/Ethylacetat 10:1). Erhalten werden 139.3 mg (25% d. Th.) der Zielverbindung als hellgelblicher Feststoff.
LC-MS (Methode 6): Rₜ = 3.02 min.; m/z = 512 (M+H)⁺
¹H-NMR (400 MHz, DMSO-d₆): δ = 8.51 (s, 1H), 7.58 (d, 2H), 7.48 (d, 2H), 7.28-7.18 (m, 4H), 6.99 (d, 2H), 6.87 (s, 1H), 6.80 (d, 1H), 6.61 (dd, 1H), 4.78 (s, 2H), 3.89 (s, 3H), 3.28 (s, 3H), 3.21 (s, 3H).

### Beispiel 8

### 3-{[5,6-Bis(4-methoxyphenyl)-furo[2,3-d]pyrimidin-4-yl]amino}phenoxy-essigsäure

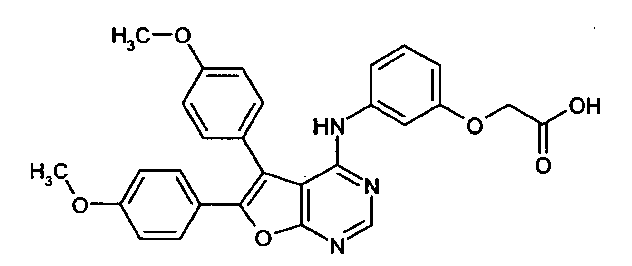

107 mg (0.209 mmol) 3-{[5,6-Bis(4-methoxyphenyl)furo[2,3-d]pyrimidin-4-yl]amino}phenoxy-essigsäuremethylester werden in 2 ml THF gelöst, bei RT mit 0.628 ml 1 N Natronlauge versetzt und 1h bei 50°C gerührt. Es wird auf RT abgekühlt und das THF im Vakuum entfernt. Der Rückstand wird mit Wasser und dann unter Eiskühlung mit 1 N Salzsäure versetzt. Der ausgefallene Feststoff wird abfiltriert, mehrmals mit Wasser gewaschen und bei 40°C im Vakuum getrocknet. Erhalten werden 92.5 mg (88.9% d. Th.) der Zielverbindung als weißer Feststoff.
LC-MS (Methode 5): Rₜ = 2.74 min.; m/z= 498 (M+H)⁺.
¹H-NMR (300 MHz, DMSO-d₆).-_{.} δ = 13.04 (br. s, 1H), 8.51 (s, 1H), 7.61 (d, 2H), 7.46 (d, 2H), 7.28-7.18 (m, 4H), 6.98 (d, 2H), 6.85 (s, 1H), 6.77 (d, 1H), 6.59 (dd, 1H), 4.65 (s, 2H), 3.90 (s, 3H), 3.78 (s, 3H).

### Beispiel 9

### 3-{[5-(4-Methoxyphenyl)-6-phenylfuro[2,3-d]pyrimidin-4-yl]amino}phenoxy-essigsäuremethylester

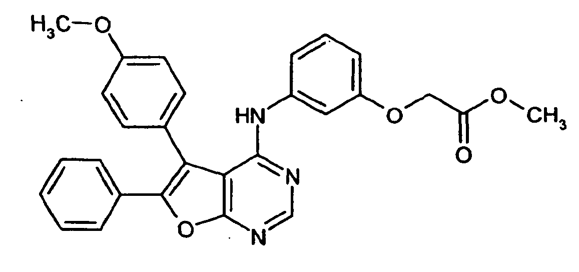

4.7 g (14 mmol) 4-Chlor-5-(4-methoxyphenyl)-6-phenylfuro[2,3-d]pyrimidin und 3.03 g (16.7 mmol) 3-Aminophenoxy-essigsäuremethylester werden in einem Ölbad für 1.5 h auf 150°C erhitzt. Nach dem Abkühlen wird der Rückstand mit Dichlormethan versetzt und über Kieselgel gereinigt (Laufmittel: Cyclohexan/Ethylacetat 2:1). Erhalten werden 2.29 g (34.1% d. Th.) der Zielverbindung als hellgelblicher Feststoff.
LC-MS (Methode 5): Rₜ = 3.08 min.; m/z = 482 (M+H)⁺
¹H-NMR (300 MHz, DMSO-d₆): δ = 8.56 (s, 1H), 7.60 (d, 2H), 7.56-7.50 (m, 2H), 7.44-7.35 (m, 3H), 7.28-7.20 (m, 4H), 6.91 (s, 1H), 6.81 (dd, 1H), 6.64 (dd, 1H), 4.78 (s, 2H), 3.90 (s, 3H), 3.71 (s, 3H).

### Beispiel 10

### 3-{[5-(4-Methoxyphenyl)-6-phenylfuro[2,3-d]pyrimidin-4-yl]amino}phenoxy-essigsäure

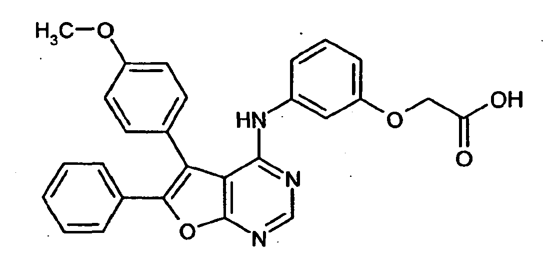

1000 mg (2.08 mmol) 3-{[5-(4-Methoxyphenyl)-6-phenylfuro[2,3-d]pyrimidin-4-yl]amino}phenoxy-essigsäuremethylester werden in 10 ml THF gelöst, bei RT mit 4.2 ml 1 N Natronlauge versetzt und 1 h bei 50°C gerührt. Es wird auf RT abgekühlt und das THF im Vakuum entfernt. Der Rückstand wird mit Wasser und dann unter Eiskühlung mit 1 N Salzsäure versetzt. Der ausgefallene Feststoff wird abfiltriert, mehrmals mit Wasser gewaschen und bei 40°C im Vakuum getrocknet. Erhalten werden 913.7 mg (92.5% d. Th.) der Zielverbindung als weißer Feststoff.
LC-MS (Methode 5): Rₜ = 2.75 min.; m/z.= 468 (M+H)⁺
¹H-NMR (300 MHz, DMSO-d₆): δ = 12.90 (br. s, 1H), 8.54 (s, 1H), 7.59 (d, 2H), 7.57-7.51 (m, 2H), 7.44-7.35 (m, 3H), 7.26-7.18 (m, 4H), 6.89 (s, 1H), 6.78 (d, 1H), 6.59 (dd, 1H), 4.60 (s, 2H), 3.91 (s, 3H).

### Beispiel 11

### 3-{[5-(4-Methoxyphenyl)-6-phenylfuro[2,3-d]pyrimidin-4-yl]amino}phenoxy-essigsäure Trisethanolamin-Salz

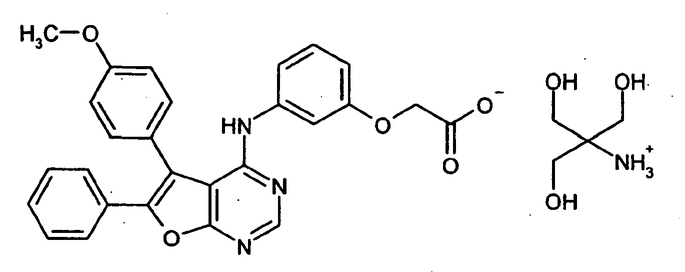

50 mg (0.107 mmol) 3-{[5-(4-Methoxyphenyl)-6-phenylfuro[2,3-d]pyrimidin-4-yl]amino}phenoxy-essigsäure werden in 2 ml einer 1:1-Mischung von Methanol und Dichlormethan vorgelegt, mit 13 mg (0.107 mmol) 2-Amino-2-hydroxymethyl-1,3-propandiol (Trisethanolamin) versetzt und über Nacht bei RT gerührt. Danach wird die Mischung im Vakuum konzentriert. Erhalten werden 60.3 mg der Zielverbindung als farbloses Glas.
LC-MS (Methode 5): Rₜ = 2.53 min.; m/z = 468 (C₂₇H₂₁N₃O₅)⁺
¹H-NMR (300 MHz, CD₃OD): δ = 8.45 (s, 1H), 7.60-7.51 (m, 4H), 7.36-7.11 (m, 7H), 6.81 (dd, 1H), 6.63 (dd, 1H), 4.38 (s, 2H), 3.94 (s, 3H), 3.65 (s, 6H).

### Beispiel 12

### 3-{[5-(4-Methoxyphenyl)-6-phenylfuro[2,3-d]pyrimidin-4-yl]amino}phenoxy-essigsäure Natriumsalz

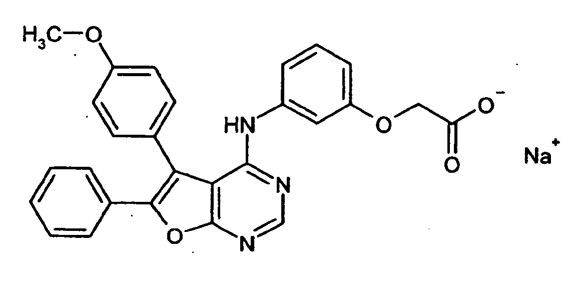

2.52 g (5.39 mmol) 3-{[5-(4-Methoxyphenyl)-6-phenylfuro[2,3-d]pyrimidin-4-yl]amino}phenoxy-essigsäure werden in einer Mischung aus 10 ml THF, 10 ml Methanol und 1 ml Wasser bei RT suspendiert. 5.39 ml 1N Natronlauge werden hinzugetropft und die resultierende Lösung 10 min lang bei RT gerührt, bevor über eine feine Fritte abgesaugt wird (Entfernung von Schwebeteilchen). Die Lösung wird im Vakuum eingeengt und der Rückstand mit Ethanol behandelt. Der unlösliche Feststoff wird abgesaugt, mit wenig Ethanol gewaschen und im Vakuum, danach im Hochvakuum getrocknet. Das Filtrat wird eingeengt, der Rückstand erneut mit wenig Ethanol verrührt und so nach Absaugen eine zweite Produktcharge erhalten. Nach Vereinigung der beiden Fraktionen werden insgesamt 2.32 g (87.9% d. Th.) der Zielverbindung als farbloser Feststoff erhalten.
LC-MS (Methode 5): Rₜ = 2.83 min.; m/z = 467 (M-Na+2H)⁺
¹H-NMR (400 MHz, DMSO-d₆): δ = 8.54 (s, 1H), 7.62-7.51 (m, 4H), 7.43-7.36 (m, 3H), 7.27 (d, 2H), 7.11 (t, 1H), 7.01 (s, 1H), 6.82-6.79 (m, 2H), 6.50 (d, 1H), 4.03 (s, 2H), 3.91 (s, 3H).

### Beispiel 13

### 3-{[5-(4-Methoxyphenyl)-6-phenylfuro[2,3-d]pyrimidin-4-yl]oxy}phenoxy}-essigsäureethylester

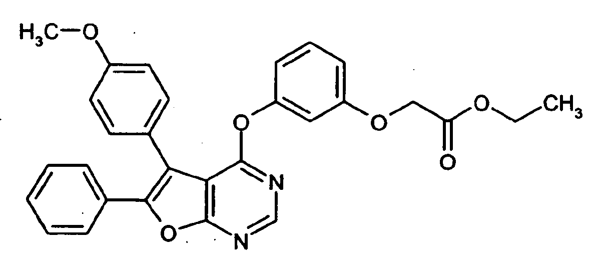

300 mg (0.731 mmol) 3-{[5-(4-Methoxyphenyl)-6-phenylfuro[2,3-d]pyrimidin-4-yl]oxy}phenol, 159 mg (0.95 mmol) Bromessigsäureethylester und 357 mg (1.1 mmol) Cäsiumcarbonat werden 1.5 h unter Rückfluss gerührt. Nach dem Abkühlen wird eingeengt, der Rückstand in Ethylacetat aufgenommen und die Mischung mehrfach mit Wasser gewaschen. Die organische Phase wird über Magnesiumsulfat getrocknet und im Vakuum konzentriert. Erhalten werden 331.4 mg (91.3% d. Th.) der Zielverbindung als gelblicher Feststoff.
LC-MS (Methode 3): Rₜ = 2.92 min.; m/z = 497 (M+H)⁺
¹H-NMR (300 MHz, DMSO-d₆): δ = 8.51 (s, 1H), 7.60-7.51 (m, 4H), 7.46-7.41 (m, 3H), 7.33 (t, 1H), 7.02 (d, 2H), 6.88-6.81 (m, 3H), 4.78 (s, 2H), 4.17 (q, 2H), 3.80 (s, 3H), 1.19 (t, 3H).

### Beispiel 14

### 3-{[5-(4-Methoxyphenyl)-6-phenylfuro[2,3-d]pyrimidin-4-yl]oxy}phenoxy)-essigsäuremethylester

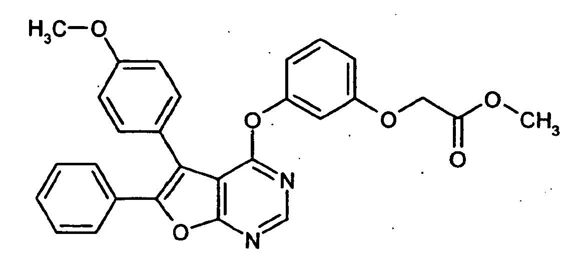

500 mg (01.22 mmol) 3-{[5-(4-Methoxyphenyl)-6-phenylfuro[2,3-d]pyrimidin-4-yl]oxy}phenol, 242 mg (1.58 mmol) Bromessigsäuremethylester und 595 mg (1.83 mmol) Cäsiumcarbonat werden 45 min unter Rückfluss gerührt. Nach dem Abkühlen wird eingeengt und das Rohprodukt ohne weitere Aufreinigung in der Folgereaktion umgesetzt.

### Beispiel 15

### 3-{[5-(4-Methoxyphenyl)-6-phenylfuro[2,3-d]pyrimidin-4-yl]oxy}phenoxy)-essigsäure

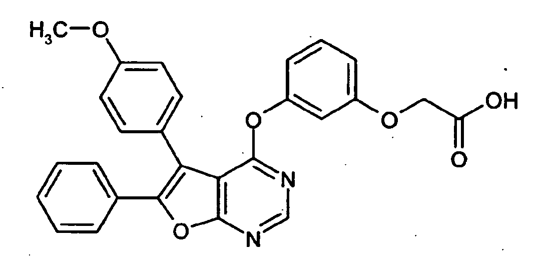

587 mg (ca. 1.217 mmol) 3-{[5-(4-Methoxyphenyl)-6-phenylfuro[2,3-d]pyrimidin-4-yl]oxy}phenoxy)-essigsäuremethylester (Rohprodukt aus Beispiel 14), 2.43 ml 1 N Natronlauge und 4 ml Dioxan werden über Nacht bei 50°C gerührt. Nach dem Abkühlen wird mit 1N Salzsäure angesäuert und der ausgefallene Feststoff abgesaugt, mit Wasser gewaschen und bei 40°C im Vakuum getrocknet. Der Feststoff wird in Dichlormethan und THF aufgenommen und die Lösung erneut zur Trockne eingeengt. Erhalten werden 477.1 mg (81.2% d. Th.) der Zielverbindung als gelblicher Feststoff.
LC-MS (Methode 3): Rₜ = 2.49 min.; m/z = 469 (M+H)⁺
¹H-NMR (400 MHz, DMSO-d₆): δ = 13.00 (br. s, 1H), 8.54 (s, 1H), 7.61-7.5.1 (m, 4H), 7.47-7.40 (m, 3H), 7:32 (t, 1H), 7.03 (d, 2H), 6.88-6.78 (m, 3H), 4.68 (s, 2H), 3.80 (s, 3H).

### Beispiel 16

### N-(3-{[5-(4-Methoxyphenyl)-6-phenylfuro[2,3-d]pyrimidin-4-yl]oxy}phenyl)-glycinmethylester

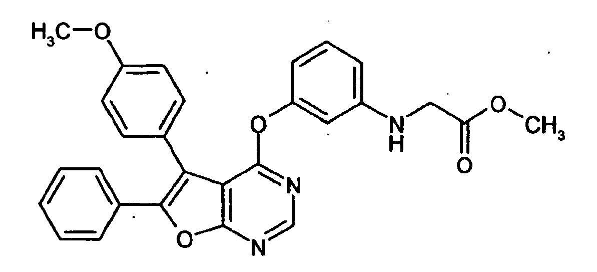

450 mg (1.1 mmol) 3-{[5-(4-Methoxyphenyl)-6-phenylfuro[2,3-d]pyrimidin-4-yl]oxy}anilin, 202 mg (1.32 mmol) Bromessigsäuremethylester und 465.5 mg (1.43 mmol) Cäsiumcarbonat in 10 ml Aceton werden über Nacht unter Rückfluss gerührt. Das Aceton wird im Vakuum entfernt und der Rückstand in Wasser aufgenommen und mehrfach mit Dichlormethan/Ethylacetat (1:1) extrahiert. Die vereinigten organischen Phasen werden über Magnesiumsulfat getrocknet und eingeengt. Der Rückstand wird zweimal an Silicagel chromatographiert (Laufmittel: Dichlormethan/Ethylacetat 1:1). Erhalten werden 75.4 mg (13.8% d. Th.) der Zielverbindung als weißer Schaum.
LC-MS (Methode 6): Rₜ = 2.92 min.; m/z = 482 (M+H)⁺
¹H-NMR (400 MHz, DMSO-d₆): δ = 8.53 (s, 1H), 7.59-7.40 (m, 7H), 7.09 (t, 1H), 7.02 (d, 2H), 6.45-6.39 (m, 3H), 6.20 (t, 1H), 3.91 (d, 2H), 3.70 (s, 3H), 3.65 (s, 3H).

### Beispiele 17

### N-(3-{[5-(4-Methoxyphenyl)-6-phenylfuro[2,3-d]pyrimidin-4-yl]oxy}phenyl)-glycin

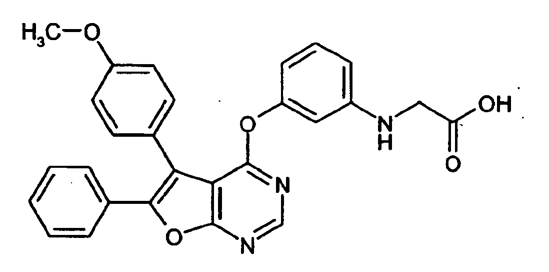

65 mg (0.135 mmol) *N*-(3-{[5-(4-Methoxyphenyl)-6-phenylfuro[2,3-d]pyrimidin-4-yl]oxy}-phenyl)-glycinmethylester, 0.27 ml 1N Natronlauge und 2 ml Dioxan werden über Nacht bei RT gerührt. Die Mischung wird mit 1 N Salzsäure sauer gestellt und mehrfach mit Ethylacetat/Dichlormethan (1:1) extrahiert. Die vereinigten organischen Phasen werden über Magnesiumsulfat getrocknet und eingeengt. Der Rückstand wird an Silicagel chromatographiert (Laufmittel: Dichlormethan/Methanol 100:1 → 50:1). Erhalten werden 16.8 mg (13.8% d. Th.) der Zielverbindung als gelbliches hartes Öl.
LC-MS (Methode 6): Rₜ = 2.63 min.; m/z = 468 (M+H)⁺
¹H-NMR (300 MHz, DMSO-d₆): δ = 12.30 (br. s, 1H), 8.52 (s, 1H), 7.59-7.40 (m, 8H), 7.09 (t, 1H), 7.04-7.00 (m, 2H), 6.93 (d, 1H), 6.40-6.35 (m, 2H), 3.80 (s, 3H), 3.78 (s, 2H).

### Beispiele 18

### 5-(4-Methoxyphenyl)-6-phenyl-N-[3-(1H-tetrazol-5-ylmethoxy)phenyl]furo[2,3-d]pyrimidin-4-amin

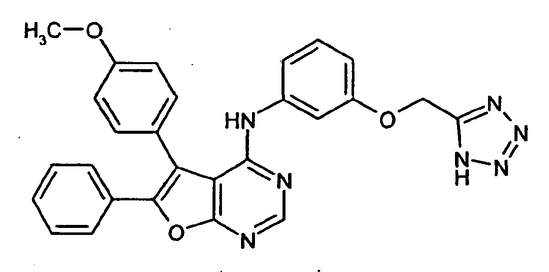

Eine Mischung aus 100 mg (0.223 mmol) (3-{[5-(4-Methoxyphenyl)-6-phenylfuro[2,3-d]pyrimidin-4-yl]amino}phenoxy)acetonitril, 383 mg (3.345 mmol) Trimethylsilylazid und 83.32 mg (0.334 mmol) Di-n-butylzinnoxid in 5 ml Toluol wird über Nacht bei 80°C gerührt. Nach dem Abkühlen wird der ausgefallene Feststoff abgesaugt, mit Toluol gewaschen und über Nacht bei 50°C im Hochvakuum getrocknet. Erhalten werden 80.1 mg (73.1% d. Th.) der Zielverbindung als weißlicher Feststoff:
LC-MS (Methode 6): Rₜ = 2.70 min.; m/z = 492 (M+H)⁺
¹H-NMR (300 MHz, DMSO-d₆): δ = 8.55 (s, 1H), 7.62-7.50 (m, 4H), 7.45-7.35 (m, 4H), 7.29-7.21 (m, 3H), 6.93 (s, 1H), 6.81-6.73 (m, 2H).

### Beispiel 19

### (3-{[5-(4-Hydroxyphenyl)-6-phenylfuro[2,3-d]pyrimidin-4-yl]amino}phenoxy)-essigsäure

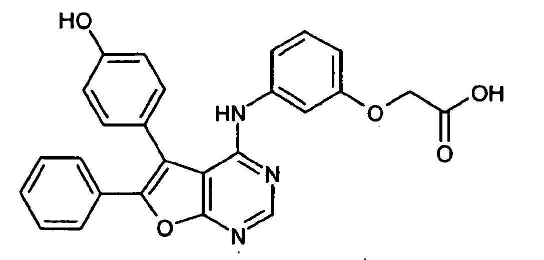

Zu einer Mischung von 170 mg (0.364 mmol) 3-{[5-(4-Methoxyphenyl)-6-phenylfuro[2,3-d]pyrimidin-4-yl]amino}phenoxy-essigsäure und 4.5 ml Dichlormethan werden bei RT 109 mg (0.435 mmol) Bortribromid gegeben. Die Mischung wird über Nacht bei RT gerührt und dann mit 1 N Salzsäure hydrolysiert. Nach Zugabe von Dichlormethan wird der unlösliche Feststoff abfiltriert und über Nacht im Hochvakuum getrocknet. Man erhält 128.6 mg der Zielverbindung, die durch Umkristallisation aus Isopropanol weiter aufgereinigt werden kann.
LC-MS (Methode 6): Rₜ = 2.41 min.; m/z = 454 (M+H)⁺.

### Beispiel 20

### (2E)-3-(3-{[5-(4-Methoxyphenyl)-6-phenylfuro[2,3-d]pyrimidin-4-yl]amino}phenyl)-acrylsäureethylester

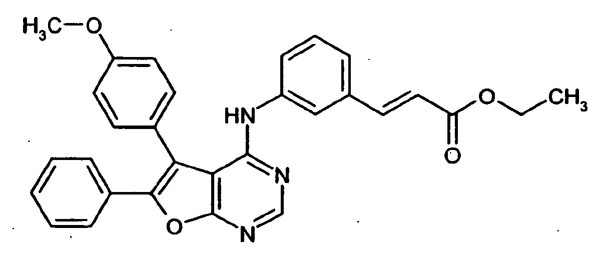

500 mg (1.85 mmol) 4-Chlor-5-(4-methoxyphenyl)-6-phenylfuro[2,3-d]pyrimidin und 369 mg (1.93 mmol) 3-Amino-*trans*-zimtsäureethylester werden für 1.5 h auf 150°C erhitzt. Nach dem Abkühlen wird Dichlormethan hinzugefügt und das Rohprodukt durch Chromatographie an Kieselgel gereinigt (Laufmittel: Dichlormethan/Ethylacelat 20:1). Erhalten werden 539.5 mg (73.9% d. Th.) des Zielprodukts als gelblicher Feststoff.
LC-MS (Methode 6): Rₜ =3.34 min.; m/z = 492 (M+H)⁺
¹H-NMR (300 MHz, DMSO-d₆): δ = 8.53 (s, 1H), 7.71 (s, 1H), 7.62-7.51 (m, 5H), 7.46-7.35 (m, 6H), 7.23 (d, 2H), 7.05 (s, 1H), 6.54 (d, 1H), 4.21 (q, 2H), 3.90 (s, 3H), 1.27 (t, 3H).

### Beispiel 21

### (2E)-3-(3-{[5-(4-Methoxyphenyl)-6-phenylfuro[2,3-d]pyrimidin-4-yl]amino}phenyl)-acrylsäure

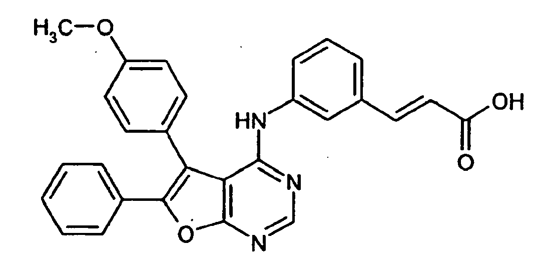

Zu einer Mischung von 120 mg (0.244 mmol) (2*E*)-3-(3-{[5-(4-Methoxyphenyl)-6-phenylfuro[2,3-d]pyrimidin-4-yl]amino}phenyl)-acrylsäureethylester in 2 ml THF werden 0.73 ml 1 N Natronlauge getropft. Die Mischung wird über Nacht bei 50°C gerührt, dann abgekühlt und mit 1N Salzsäure angesäuert. Der ausgefallene Feststoff wird abgesaugt, mehrfach mit Wasser gewaschen und über Nacht bei 50°C im Hochvakuum getrocknet. Erhalten werden 106 mg (93.7% d. Th.) der Zielverbindung als farbloser Feststoff.
LC-MS (Methode 7): Rₜ = 5.09 min.; m/z = 464 (M+H)⁺
¹H-NMR (400 MHz, DMSO-d₆): δ = 12.47 (br. s, 1H), 8.55 (s, 1H), 7.71 (s, 1H), 7.62-7.50 (m, 5H), 7.45-7.31 (m, 6H), 7.23 (d, 2H), 7.04 (s, 1H), 6.44 (d, 1H), 3.39 (s, 3H).

### Beispiel 22

### (2E)-3-(3-{[5-(4-Methoxyphenyl)-6-phenylfuro[2,3-d]pyrimidin-4-yl]amino}phenyl)-acrylsäure Natriumsalz

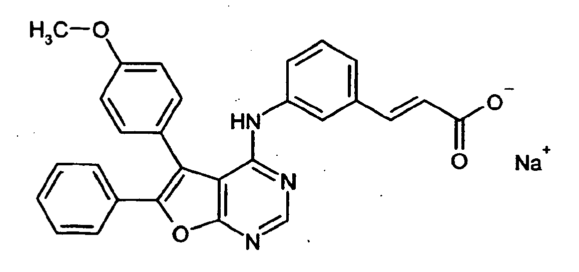

76 mg (0.164 mmol) (2*E*)-3-(3-{[5-(4-Methoxyphenyl)-6-phenylfuro[2,3-d]pyrimidin-4-yl]amino}-phenyl)-acrylsäure werden in 1.5 ml einer 1:1-Mischung aus Methanol und THF vorgelegt und bei RT mit 0.164 ml 1N Natronlauge versetzt. Die Mischung wird 2 h bei RT gerührt, dann im Vakuum konzentriert und der Rückstand über Nacht im Hochvakuum getrocknet. Erhalten werden 79.6 mg (99.9% d. Th.) der Zielverbindung als gelblicher Feststoff.
LC-MS (Methode 5): Rₜ = 3.01 min.; m/z = 464 (M-Na+2H)⁺
¹H-NMR (400 MHz, DMSO-d₆): δ = 8.54 (s, 1H), 7.65-7.48 (m, 5H), 7.43-7.34 (m, 3H), 7.31-7.21 (m, 4H), 7.15 (br. s, 1H), 6.97 (d, 1H), 6.92 (s, 1H), 6.71 (d, 1H), 3.91 (s, 3H).

### Beispiel 23

### 3-(4-{[5-(4-Methoxyphenyl)-6-phenylfuro[2,3-d]pyrimidin-4-yl]oxy}phenyl)-propansäuremethylester

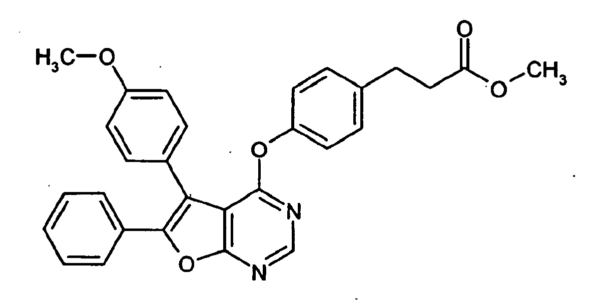

300 mg (0.891 mmol) 4-Chlor-5-(4-methoxyphenyl)-6-phenylfuro[2,3-d]pyrimidin, 642 mg (3.65 mmol) 3-(4-Hydroxyphenyl)-propansäuremethylester und 435.4 mg (1.34 mmol) Cäsiumcarbonat werden für 2 h auf 120°C erhitzt. Nach dem Abkühlen wird Wasser hinzugefügt und das ausgefallene Rohprodukt abfiltriert. Der Feststoff wird in Ethylacetat gelöst und die Lösung zweimal mit Pufferlösung (pH 7) gewaschen, über Magnesiumsulfat getrocknet und wieder eingeengt. Nach Behandeln des Rückstands mit Methanol fällt ein Feststoff aus, der abfiltriert, mit wenig Methanol gewaschen und im Vakuum getrocknet wird. Es werden 160 mg (36.3% d. Th.) der Zielverbindung als farbloser Feststofferhalten.
LC-MS (Methode 3): Rₜ = 2.93 min.; m/z = 481 (M+H)⁺
¹H-NMR (300 MHz, DMSO-d₆): δ = 8.52 (s, 1H), 7.60-7.50 (m, 4H), 7.45-7.38 (m, 3H), 7.28 (d, 2H), 7.12 (d, 2H), 7.02 (d, 2H), 3.80 (s, 3H), 3.60 (s, 3H), 2.87 (t, 2H), 2.67 (t, 2H).

### Beispiel 24

### 3-(4-{[5-(4-Methoxyphenyl)-6-phenylfuro[2,3-d]pyrimidin-4-yl]oxy}phenyl)-propansäure

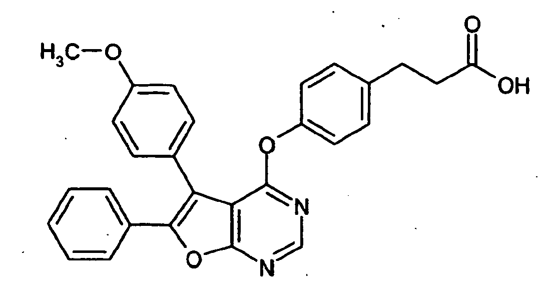

137 mg (0.285 mmol) 3-(4-{[5-(4-Methoxyphenyl)-6-phenylfuro[2,3-d]pyrimidin-4-yl]oxy}-phenyl)-propansäuremethylester werden in 4.5 ml THF vorgelegt und bei RT mit 0.855 ml 1N Natronlauge versetzt. Die Mischung wird 1 h bei 50°C gerührt. Nach Abkühlen wird mit 1N Salzsäure angesäuert und der ausgefallene Feststoff abgesaugt, mit Wasser gewaschen und im Hochvakuum getrocknet. Es werden 125.9 mg (94.7% d. Th.) der Zielverbindung erhalten.
LC-MS (Methode 6): Rₜ = 2.76 min.; m/z = 467 (M+H)⁺
¹H-NMR (400 MHz, DMSO-d₆): δ = 12.16 (s, 1H), 8.52 (s, 1H), 7.61-7.51 (m, 4H), 7.48-7.40 (m, 3H), 7.29 (d, 2H), 7.12 (d, 2H), 7.02 (d, 2H), 3.79 (s, 3H), 2.85 (t, 2H), 2.56 (t, 2H).

### Beispiel 25

### 3-(3-{[5-(4-Methoxyphenyl)-6-phenylfuro[2,3-d]pyrimidin-4-yl]amino}phenyl)-propansäuremethylester

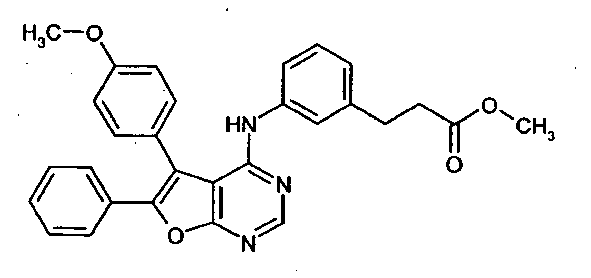

2100 mg (6.34 mmol) 4-Chlor-5-(4-methoxyphenyl)-6-phenylfuro[2,3-d]pyrimidin und 1453 mg (8.11 mmol) 3-(3-Aminophenyl)-propansäuremethylester werden für 1.5 h auf 150°C erhitzt. Nach dem Abkühlen wird Dichlormethan hinzugefügt und das Rohprodukt durch Chromatographie an Kieselgel aufgereinigt (Laufmittel: Dichlormethan/Ethylacetat 20:1). Das so erhaltene Produkt wird mit Diisopropylether verrührt und der anfallende Feststoff abgesaugt und mit wenig Diisopropylether gewaschen. Erhalten werden 1367 mg (45.7% d. Th.) des Zielprodukts als farbloser Feststoff.
LC-MS (Methode 5): Rₜ = 3.19 min.; m/z = 480 (M+H)⁺.

### Beispiel 26

### 3-(3-{[5-(4-Methoxyphenyl)-6-phenylfuro[2,3-d]pyrimidin-4-yl]amino}phenyl)-propansäure

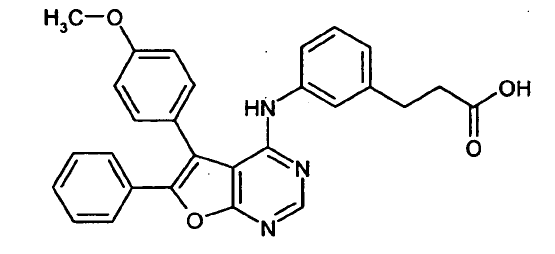

1000 mg (2.085 mmol) 3-(3-{[5-(4-Methoxyphenyl)-6-phenylfuro[2,3-d]pyrimidin-4-yl]amino}-phenyl)-propansäuremethylester werden in 30 ml THF vorgelegt und bei RT mit 6.3 ml 1 N Natronlauge versetzt. Die Mischung wird 1 h bei 50°C gerührt und dann nach Abkühlen mit 1 N Salzsäure schwach angesäuert. Der ausgefallene Feststoff wird abgesaugt, mehrfach mit Wasser gewaschen und über Nacht bei 40°C im Hochvakuum getrocknet. Erhalten werden 934.5 mg (96.3% d. Th.) des Zielprodukts als farbloser Feststoff.
LC-MS (Methode 3): Rₜ = 2.62 min.; m/z = 465 (M+H)⁺
¹H-NMR (300 MHz, DMSO-d₆): δ = 12.15 (br. s, 1H), 8.52 (s, 1H), 7.62-7.51 (m, 4H), 7.45-7.36 (m, 3H), 7.27-7.20 (m, 5H), 6.95-6.90 (m, 1H), 6.88 (s, 1H), 3.89 (s, 3H), 2.78 (t, 2H), 2.51 (t, 2H).

### Beispiel 27

### 3-(3-{[5-(4-Methoxyphenyl)-6-phenylfuro[2,3-d]pyrimidin-4-yl]amino}phenyl)-propänsäure Natriumsalz

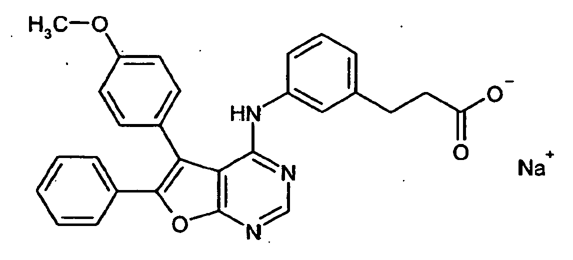

150 mg (0.322 mmol) 3-(3-{[5-(4-Methoxyphenyl)-6-phenylfuro[2,3-d]pyrimidin-4-yl]amino}-phenyl)-propansäure werden in 5 ml THF gelöst und mit 0.322 ml 1N Natronlauge versetzt- Die Mischung wird 1 h bei RT gerührt, dann im Vakuum eingeengt und der Rückstand über Nacht im Hochvakuum getrocknet. Erhalten werden 155.7 mg (99.1% d. Th.) des Zielprodukts als farbloser Feststoff.
LC-MS (Methode 5): Rₜ = 2.95 min.; m/z = 466 (M-Na+2H)⁺
¹H-NMR (400 MHz, DMSO-d₆): δ = 8.52 (s, 2H), 7.63-7.51 (m, 4H), 7.45-7.33 (m, 3H), 7.39-7.12 (m, 5H), 6.89 (d, 1H), 6.80 (s, 1H), 3.90 (s, 3H), 2.68 (br. s, 2H), 2.08 (br. s, 2H).

### Beispiel 28

### 3-{[5-(4-Methoxyphenyl)-6-phenylfuro[2,3-d]pyrimidin-4-yl]amino}phenyl-essigsäuremethylester

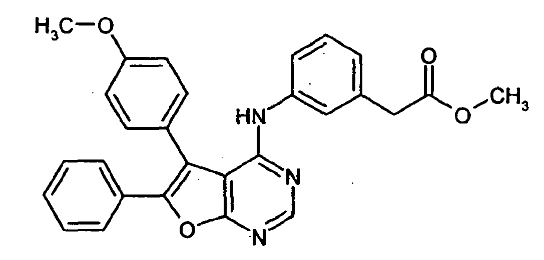

300 mg (0.89 mmol) 4-Chlor-5-(4-methoxyphenyl)-6-phenylfuro[2,3-d]pyrimidin und 191.3 mg (1.16 mmol) 3-Aminophenylessigsäuremethylester werden für 1.5 h auf 150°C erhitzt. Nach dem Abkühlen wird Dichlormethan hinzugefügt und der resultierende Feststoff abgesaugt, mit Dichlormethan nachgewaschen und über Nacht bei 50°C im Hochvakuum getrocknet. Das Rohprodukt wird durch Chromatographie an Silicagel gereinigt (Laufmittel: Dichlormethan/Ethylacetat 10:1). Erhalten werden 273.2 mg (65.9% d. Th.) des Zielprodukts als gelblicher Feststoff.
LC-MS (Methode 3): Rₜ = 2.88 min.; m/z = 466 (M+H)⁺
¹H-NMR (400 MHz, DMSO-d₆): δ = 8.52 (s, 1H), 7.59 (d, 2H), 7.53 (d, 2H), 7.44-7.37 (m, 3H), 7.32-7.21 (m, 5H), 6.95 (d, 1H), 6.90 (s, 1H), 3.90 (s, 3H), 3.65 (s, 2H), 3.61 (s, 3H).

### Beispiel 29

### 3-{[5-(4-Methoxyphenyl)-6-phenylfuro[2,3-d]pyrimidin-4-yl]amino}phenyl-essigsäure

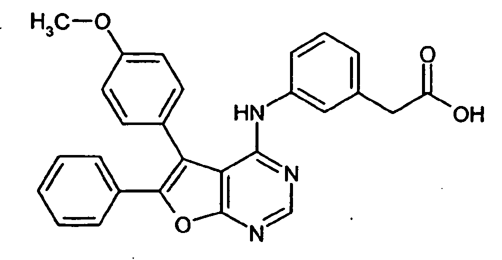

50 mg (0.107 mmol) 3-{[5-(4-Methoxyphenyl)-6-phenylfuro[2,3-d]pyrimidin-4-yl]amino}phenylessigsäuremethylester werden in 2 ml THF gelöst, bei RT mit 0.322 ml 1 N Natronlauge versetzt und 4 h bei 50°C gerührt. Es wird auf RT abgekühlt und das THF im Vakuum entfernt. Der Rückstand wird mit Wasser und dann mit 1 N Salzsäure versetzt. Der ausgefallene Feststoff wird abfiltriert, mit Wasser gewaschen und bei 50°C im Vakuum getrocknet. Erhalten werden 41.7 mg (86% d. Th.) der Zielverbindung als weißer Feststoff.
LC-MS (Methode 5): Rₜ = 2.85 min.; m/z = 452 (M+H)⁺
¹H-NMR (400 MHz, DMSO-d₆): δ = 8.51 (s, 1H), 7.60 (d, 2H), 7.53 (d, 2H), 7.44-7.37 (m, 3H), 7.32-7.20 (m, 5H), 6.96 (d, 1H), 6.91 (s, 1H), 3.91 (s, 3H), 3.53 (s, 2H).

### Beispiel 30

### 4-(4-{[5-(4-Methoxyphenyl)-6-phenylfuro[2,3-d]pyrimidin-4-yl]amino}phenyl)-butansäuremethylester

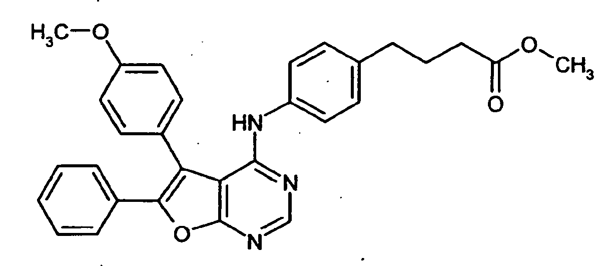

200.8 mg (0.596 mmol) 4-Chlor-5-(4-methoxyphenyl)-6-phenylfuro[2,3-d]pyrimidin und 149.8 mg (0.775 mmol) 4-(4-Aminophenyl)-butansäuremethylester werden für 1.5 h auf 150°C erhitzt. Nach dem Abkühlen wird im Vakuum konzentriert, Dichlormethan zum Rückstand hinzugefügt und das Rohprodukt durch Chromatographie an Kieselgel aufgereinigt (Laufmittel: Dichlormethan/Ethylacetat 20:1). Das so erhaltene Produkt wird mit Diisopropylether verrührt und der anfallende Feststoff abgesaugt und mit wenig Diisopropylether gewaschen. Erhalten werden 236.7 mg (80.4% d. Th.) des Zielprodukts als leicht rosa Feststoff.
LC-MS (Methode 3): Rₜ = 3.08 min.; m/z = 494 (M+H)⁺
¹H-NMR (400 MHz, DMSO-d₆): δ = 8.49 (s, 1H), 7.60 (d, 2H), 7.53 (d, 2H), 7.43-7.37 (m, 3H), 7.32 (d, 2H), 7.23 (d, 2H), 7.13 (d, 2H), 6.84 (s, 1H), 3.88 (s, 3H), 3.59 (s, 3H), 2.58-2.50 (m, 2H), 2.29 (t, 2H), 1.84-1.77 (m, 2H).

### Beispiel 31

### 4-(4-{[5-(4-Methoxyphenyl)-6-phenylfuro[2,3-d]pyrimidin-4-yl]amino}phenyl)-butansäure

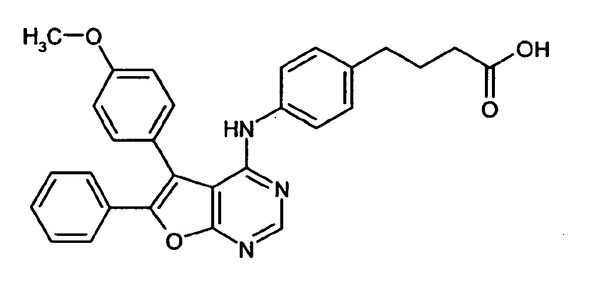

175.6 mg (0.356 mmol) 4-(4-{[5-(4-Methoxyphenyl)-6-phenylfuro[2,3-d]pyrimidin-4-yl]amino}-phenyl)-butansäuremethylester werden in 3.5 ml THF gelöst, bei RT mit 1.07 ml 1 N Natronlauge versetzt und 1 h bei 50°C gerührt. Es wird auf RT abgekühlt und das THF im Vakuum entfernt. Der Rückstand wird mit Wasser und dann mit 1 N Salzsäure versetzt. Der ausgefallene Feststoff wird abfiltriert, mehrfach mit Wasser gewaschen und über Nacht bei 40°C im Vakuum getrocknet. Erhalten werden 130 mg (76.2% d. Th.) der Zielverbindung als weißer Feststoff.
LC-MS (Methode 5): Rₜ = 3.03 min.; m/z = 480 (M+H)⁺
¹H-NMR (400 MHz, CDCl₃): δ = 8.54 (s, 1H), 7.59 (d, 2H), 7.49 (d, 2H), 7.38-7.25 (m, 5H), 7.18-7.10 (m, 4H), 6.60 (s, 1H), 2.68-2.61 (m, 2H), 2.40-2.33 (m, 2H), 1.98-1.90 (m, 2H).

### Beispiel 32

### 4-(2-{[5-(4-Methoxyphenyl)-6-phenylfuro[2,3-d]pyrimidin-4-yl]amino}phenyl)-butansäuremethylester

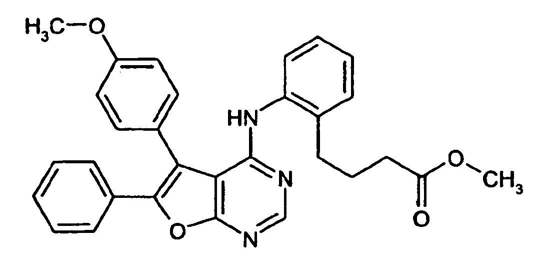

259.8 mg (0.771 mmol) 4-Chlor-5-(4-methoxyphenyl)-6-phenylfuro[2,3-d]pyrimidin und 228 mg 4-(2-Aminophenyl)-butabsäuremethylester (85%-ig, ca. 1.0 mmol) werden über Nacht auf 150°C erhitzt. Nach dem Abkühlen wird im Vakuum konzentriert, Dichlormethan zum Rückstand hinzugefügt und das Rohprodukt durch Chromatographie an Kieselgel aufgereinigt (Laufmittel: Dichlormethan/Ethylacetat 50:1 → 10:1). Das so erhaltene Produkt wird durch präparative RP-HPLC weiter gereinigt. Erhalten werden 33.1 mg (8.7% d. Th.) des Zielprodukts.
LC-MS (Methode 3): Rₜ = 3.08 min.; m/z = 494 (M+H)⁺
¹H-NMR (400 MHz, DMSO-d₆): δ = 8.48 (s, 1H), 7.61 (d, 2H), 7.53 (d, 2H), 7.42-7.12 (m, 9H), 6.83 (s, 1H), 6.39 (s, 3H), 3.58 (s, 3H), 2.59-2.50 (m, 2H), 2.33-2.28 (m, 2H), 1.83-1.75 (m, 2H).

### Beispiel 33

### 4-(2-{[5-(4-Methoxyphenyl)-6-phenylfuro[2,3-d]pyrimidin-4-yl]amino}phenyl)-butansäure

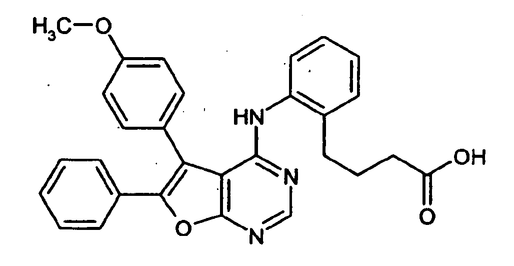

28.9 mg (0.059 mmol) 4-(2-{[5-(4-Methoxyphenyl)-6-phenylfuro[2,3-d]pyrimidin-4-yl]amino}-phenyl)-butansäuremethylester werden in 1 ml THF gelöst, bei RT mit 0.176 ml 1 N Natronlauge versetzt und 1 h bei 50°C gerührt. Es wird auf RT abgekühlt und das THF im Vakuum entfernt. Der Rückstand wird mit Wasser und dann mit 1 N Salzsäure versetzt. Der ausgefallene Feststoff wird abfiltriert, mehrfach mit Wasser gewaschen und über Nacht bei 40°C im Vakuum getrocknet. Erhalten werden 16.9 mg (60.2% d. Th.) der Zielverbindung als weißer Feststoff.
LC-MS (Methode 6): Rₜ = 2.89 min.; m/z = 480 (M+H)⁺
¹H-NMR (400 MHz, DMSO-d₆): δ = 12.02 (br. s, 1H), 8.50 (s, 1H), 7.60 (d, 2H), 7.52 (d, 2H), 7.43-7.12 (m, 9H), 6.84 (s, 1H), 3.89 (s, 3H), 2.58-2.49 (m, 2H), 2.20 (t, 2H), 1.27 (t, 2H).

### Beispiel 34

### 5-(3-{[5-(4-Methoxyphenyl)-6-phenylfuro[2,3-d]pyrimidin-4-yl]amino}benzyl)-1,3,4-oxadiazol-2(3H)-on

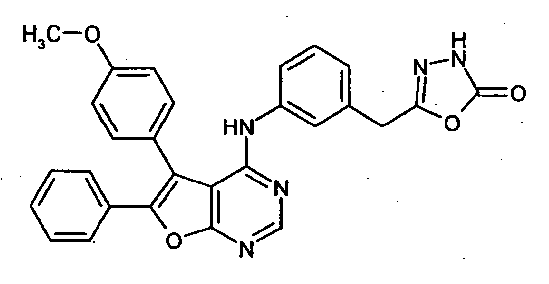

0.85 mg (0.183 mmol) 2-(3-{[5-(4-Methoxyphenyl)-6-phenylfuro[2,3-d]pyrimidin-4-yl]amino}-phenyl)-essigsäurehydrazid und 35.5 mg (0.219 mmol) *N,N'*-Carbonyldiimidazol werden in 3 ml THF 2 h lang unter Rückfluss erhitzt. Nach Abkühlen auf RT wird auf Wasser gegeben und mehrfach mit Dichlormethan extrahiert. Die vereinigten organischen Phasen werden über Magnesiumsulfat getrocknet und im Vakuum konzentriert. Man erhält 79.9 mg (89% d. Th.) des Zielprodukts als beigen Feststoff.
LC-MS (Methode 3): Rₜ = 2.55 min.; m/z = 492 (M+H)⁺
¹H-NMR (400 MHz, DMSO-d₆): δ = 8.51 (s, 1H), 7.68 (s, 1H), 7.58 (d, 2H), 7.54 (d, 2H), 7.44-7.38 (m, 3H), 7.30-7.27 (m, 2H), 7.21 (d, 2H), 7.06-6.97 (m, 3H), 3.90 (s, 2H), 3.88 (s, 3H).

### Beispiel 35 und Beispiel 36

### (+/-)-cis-N-(3-{[5-(4-Methoxyphenyl)-6-phenylfuro[2,3-d]pyrimidin-4-yl]amino}cyclohexyl)-glycinmethylester und (+/-)-trans-N-(3-{[5-(4-Methoxyphenyl)-6-phenylfuro[2,3-d]pyrimidin-4-yl]amino}cyclohexyl)-glycinmethylester

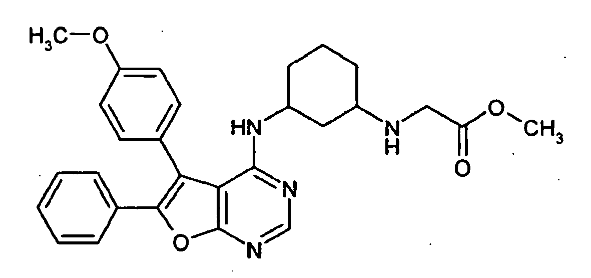

132 mg (0.318 mmol) (+/-)*cis*/*trans*-*N*-[5-(4-Methoxyphenyl)-6-phenylfuro[2,3-d]pyrimidin-4-yl]-cyclohexan-1,3-diamin (Beispiel 36A) werden in 1.5 ml Dichlormethan gelöst und bei RT mit 18.7 µl Essigsäure versetzt. 28 mg (0.318 mmol) Oxoessigsäuremethylester werden hinzugefügt und nach 5 min 101 mg (0.478 mmol) Natriumtriacetoxyborhydrid. Die Mischung wird 2 h bei RT gerührt und dann mit Wasser und Dichlormethan verdünnt. Die organische Phase wird mit gesättigter Natriumcarbonat-Lösung gewaschen, über Natriumsulfat getrocknet und im Vakuum eingeengt. Das Rohprodukt wird durch präparative RP-HPLC (Eluent: Acetonitril/Wasser-Gradient) gereinigt und dabei die *cis*/*trans*-Isomeren getrennt:

### (+/-)-cis-N-(3-{[5-(4-Methoxyphenyl)-6-phenylfuro[2,3-d]pyrimidin-4-yl]amino}cyclohexyl)-glycinmethylester (Beispiel 35)

Ausbeute: 26.5 mg (17.1% d. Th.)
LC-MS (Methode 3): Rₜ = 1.70 min.; m/z = 487 (M+H)⁺;

### (+/-)-trans-N-(3-{[5-(4-Methoxyphenyl)-6-phenylfuro[2,3-d]pyrimidin-4-yl]amino}cyclohexyl)-glycinmethylester (Beispiel 36)

Ausbeute: 22.1 mg (14.3% d. Th.)
LC-MS (Methode 3): Rₜ = 1.61 min.; m/z = 487 (M+H)⁺.

### Beispiel 37

### (+/-)-cis-[(3={[5-(4-Methoxyphenyl)-6-phenylfuro[2,3-d]pyrimidin-4-yl]oxy}cyclohexyl)oxy]-essigsäure-tert.-butylester

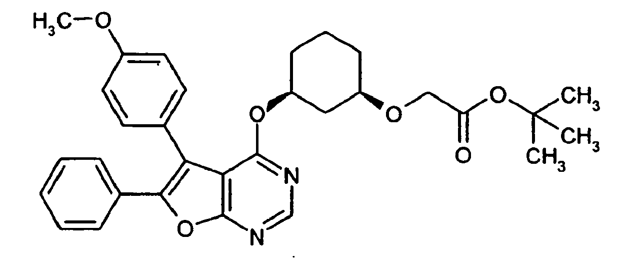

350 mg (0.84 mmol) (+/-)-*cis*-3-{[5-(4-Methoxyphenyl)-6-phenylfuro[2,3-d]pyrimidin-4-yl]oxy}-cyclohexanol werden in 1 ml absolutem THF gelöst, auf 0°C gekühlt und mit 0.48 ml (ca. 0.97 mmol) Phosphazen-Base P2-t-Bu (2 M Lösung in THF) versetzt. Die Kühlung wird entfernt und die Lösung 10 min bei RT gerührt und dann bei RT zu einer Lösung von 295 mg (1.51 mmol) Bromessigsäure-*tert*.-butylester in 2 ml THF) getropft. Nach 2 h bei RT wird die Reaktionsmischung im Vakuum konzentriert und direkt durch Chromatographie an Silicagel (Biotage, Laufmittel: Cyclohexan/Ethylacetat 10:1 → 1:1) gereinigt. Erhalten werden neben 180 mg Ausgangsmaterial 207 mg (46.4% d. Th.) des Zielprodukts.
LC-MS (Methode 6): Rₜ = 3.38 min.; m/z = 531 (M+H)⁺
¹H-NMR (400 MHz, DMSO-d₆): δ = 8.58 (s, 1H), 7.54 (dd, 2H), 7.43-7.39 (m, 5H), 7.0 (d, 2H), 5.13 (m, 1H), 3.98 (s, 2H), 3.82 (s, 3H), 3.42 (m, 1H), 2.41 (br. d, 1H), 2.05-1.93 (m, 2H), 1.78-1.70 (m, 1H), 1.40 (s, 9H), 1.30-1.05 (m, 4H).

### Trennung der Enantiomere:

0.2 g (+/-)-*cis*-[(3-{[5-4-Methoxyphenyl)-6-phenylfuro[2,3-d]pyrimidin-4-yl]oxy}cyclohexyl)-oxy]-essigsäure-*tert*.-butylester werden in 4 ml Ethanol und 16 ml iso-Hexan gelöst. Das Racemat wird durch präparative HPLC an chiraler Phase in die Enantiomere getrennt (siehe Beispiel 38 und 39) [Säule: Daicel Chiralcel OJ-H, 5 µm, 250 mm x 20 mm; Fluss: 15 ml/min; Detektion: 220 nm; Injektionsvolumen 0.5 ml; Temperatur: 45°C; Eluent: t = 0 min 90% iso-Hexan / 10% Ethanol → t = 7 min 90% iso-Hexan / 10% Ethanol].

### Beispiel 38

### cis-[(3-{[5-(4-Methoxyphenyl)-6-phenylfuro[2,3-d]pyrimidin-4-yl]oxy}cyclohexyl)oxy]-essigsäure-tert.-butylester (Enantiomer 1)

LC-MS (Methode 3): Rₜ = 3.22 min.; m/z = 531 (M+H)⁺
[α]_{D}²⁰ = -59°, c = 0.525, CHCl₃.

### Beispiel 39

### cis-[(3{[5-(4-Methoxyphenyl)-6-phenylfuro[2,3-d]pyrimidin-4-yl]oxy}cyclohexyl)oxy]-essigsäure-tert.-butylester (Enantiomer 2)

LC-MS (Methode 3): Rₜ = 3.22 min.; m/z= 531 (M+H)⁺
[α]_{D}²⁰ = +55.5°, c = 0.51, CHCl₃.

### Beispiel 40

### (+/-)-trans-[(3-([5-(4-Methoxyphenyl)-6-phenylfuro[2,3-d]pyrimidin-4-yl]oxy)cyclohexyl)oxy]-essigsäure-tert.-butylester

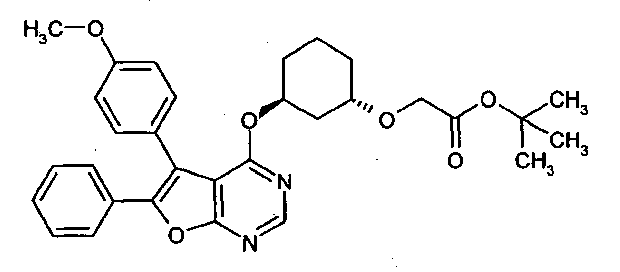

920 mg (11.53 mmol) 50%-ige Natronlauge und ca. 5 ml Toluol werden auf 40°C erwärmt und mit 65.2 mg (0.192 mmol) Tetrabutylammoniumhydrogensulfat sowie 800 mg (1.91 mmol) (*+*/*-*)*-trans-*3-{[5-(4-Methoxyphenyl)-6-phenylfuro[2,3-d]pyrimidin-4-yl]oxy}cyclohexanol versetzt. Die Suspension wird mit wenig THF verdünnt, wobei sich der Feststoff anlöst. Nach Zugabe von 749 mg (3.84 mmol) Bromessigsäure-*tert*.-butylester wird die Suspension unter sehr kräftigem Rühren auf 60°C erhitzt. Nach insgesamt 3 h bei 60°C, wobei zwischenzeitlich weitere 920 mg 50%-ige Natronlauge und ca. 1500 mg Bromessigsäure-*tert*.-butylester zugegeben werden, wird die Reaktionsmischung abgekühlt und auf Wasser gegeben. Es wird dreimal mit Dichlormethan extrahiert. Die vereinigten organischen Phasen werden über Magnesiumsulfat getrocknet und im Vakuum eingeengt. Das Rohprodukt wird durch Chromatographie an Silicagel (Laufmittel: Cyclohexan/Ethylacetat 10:1 → 1:1) getrennt. Isoliert werden neben 473 mg Ausgangsmaterial 286 mg (28.1% d. Th.) der Zielverbindung.
LC-MS (Methode 6): Rₜ = 3.36 min.; m/z = 531 (M+H)⁺
¹H-NMR (400 MHz, DMSO-d₆): δ = 8.58 (s, 1H), 7.54 (d, 2H), 7.43-7.39 (m, 5H), 7.05 (d, 2H), 5.58 (m, 1H), 3.88 (d; 2H), 3.82 (s, 3H), 3.21 (m, 1H), 1.99-1.92 (m, 1H), 1.78-1.70 (m, 1H), 1.61-1.53 (m, 3H), 1.49-1.40 (m, 1H), 1.38 (s, 9H), 1.27-1.17 (m, 2H).

### Trennung der Enantiomere:

0.3 g (+/-)-*trans*-[(3-{[5-(4-Methoxyphenyl)-6-phenylfuro[2,3-d]pyrimidin-4-yl]oxy}cyclohexyl)-oxy]-essigsäure-*tert*.-butylester werden in 2 ml Ethanol und 8 ml iso-Hexan gelöst. Das Racemat wird durch präparative HPLC an chiraler Phase in die Enantiomere getrennt (siehe Beispiel 41 und 42) [Säule: Daicel Chiralcel OJ-H, 5 µm, 250 mm x 20 mm; Fluss: 15 ml/min; Detektion: 220 nm; Injektionsvolumen 0.5 ml; Temperatur: 40°C; Eluent: t = 0 min 90% iso-Hexan / 10% Ethanol → t = 10 min 90% iso-Hexan / 10% Ethanol].

### Beispiel 41

### (+)-trans-[(3-{[5-(4-Methoxyphenyl)-6-phenylfuro[2,3-d]pyrimidin-4-yl]oxy}cyclohexyl)oxy]-essigsäure-tert.-butylester (Enantiomer 1)

[α]_{D}²⁰ = +60.6°, c = 0.50, CHCl₃
¹H-NMR (400 MHz, DMSO-d₆): δ = 8.58 (s, 1H), 7.54 (d, 2H), 7.43-7.39 (m, 5H), 7.05 (d, 2H), 5.58 (br. s, 1H), 3.88 (d, 2H), 3.82 (s, 3H), 3.21 (m, 1H), 1.99-1.92 (m, 1H), 1.78-1.70 (m, 1H), 1.61-1.53 (m, 3H), 1.49-1.40 (m, 1H), 1.38 (s, 9H), 1.27-1.17 (m, 2H).

### Beispiel 42

### (-)-trans-[(3-{[5-(4-Methoxyphenyl)-6-phenylfuro[2,3-d]pyrimidin-4-yl]oxy}cyclohexyl)oxy]-essigsäure-tert.-butylester (Enantiomer 2)

_{[a]}D²⁰ = -70.4°, c = 0.525, CHCl₃
¹H-NMR (400 MHz, DMSO-d₆): δ = 8.58 (s, 1H), 7.54 (d, 2H), 7.43-7.39 (m, 5H), 7.05 (d, 2H), 5.58 (br. s, 1H), 3.88 (d, 2H), 3.82 (s, 3H), 3.21 (m, 1H), 1.99-1.92 (m, 1H), 1.78-1.70 (m, 1H), 1.61-1.53 (m, 3H), 1.49-1.40 (m, 1H), 1.38 (s, 9H), 1.27-1.17 (m, 2H).

### Beispiel 43

### (+/-)-all-cis-[(3-Hydroxy-5-{[5-(4-methoxyphenyl)-6-phenylfuro[2,3-d]pyrimidin-4-yl]oxy}cyclohexyl)oxy]-essigsäure-tert.-butylester

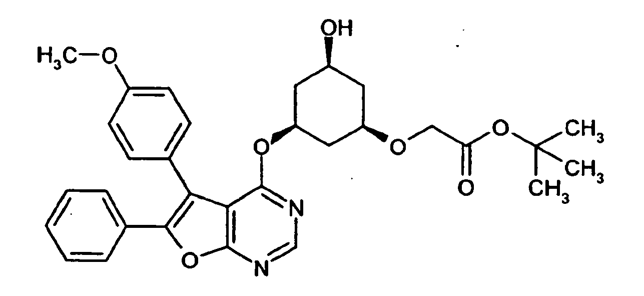

Eine auf 0°C gekühlte Lösung von 600 mg (1.39 mmol) (+/-)-*all*-*cis*-5-{[5-(4-Methoxyphenyl)-6-phenylfuro[2,3-d]pyrimidin-4-yl]oxylcyclohexan-1,3-diol in 1.2 ml DMF wird mit 0.69 ml (ca. 1.39 mmol) Phosphazen-Base P2-t-Bu (ca. 2 M Lösung in THF) versetzt. Die resultierende Lösung wird 5 min bei 0°C gerührt und dann mit 0.25 ml (1.67 mmol) Bromessigsäure-*tert*.-butylester versetzt. Die Kühlung wird entfernt und die Mischung 15 min bei RT gerührt. Danach wird auf Wasser gegeben und dreimal mit Ethylacetat extrahiert. Die vereinigten organischen Phasen werden über Magnesiumsulfat getrocknet und im Vakuum eingeengt. Nach Aufreinigung durch präparative RP-HPLC (Eluent: Acetonitril/Wasser-Gradient) werden 207 mg (27.3% d. Th.) des Zielprodukts erhalten.
LC-MS (Methode 6): Rₜ = 2.90 min.; m/z = 547 (M+H)⁺
¹H-NMR (400 MHz, DMSO-d₆): δ = 8.60 (s, 1H), 7.56 (dd, 2H), 7.43-7.38 (m, 5H), 7.02 (d, 2H), 5.13 (m, 1H), 4.85 (d, 1H), 3.98 (s, 2H), 3.83 (s, 3H), 3.59-3.49 (m, 2H), 2.96-2.90 (m, 1H), 2.30-2.18 (m, 2H), 1.42 (s, 9H), 1.13-1.02 (m, 3H).

### Beispiel 44

### (+/-)-3-(3-{[5-(4-Methoxyphenyl)-6-phenylfuro[2,3-d]pyrimidin-4-yl]oxy}piperidin-1-yl)-propansäuremethylester

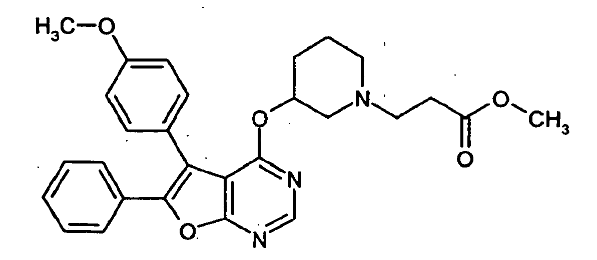

200 mg (0.5 mmol) (+/-)-S-(4-Methoxyphenyl)-6-phenyl-4-(piperidin-3-yloxy)furo[2,3-djpyrimidin (Beispiel 37A) werden in 1 ml THF gelöst und mit 69 µl (0.5 mmol) Triethylamin versetzt. Nach Zugabe von 54 µl (0.5 mmol) 3-Brompropionsäuremethylester wird die Mischung ca. 8 h lang bei 20-40°C gerührt. Zwischenzeitlich werden noch zweimal Triethylamin (insgesamt ca. 1.2 mmol) und 3-Brompropionsäuremethylester (insgesamt ca. 1.2 mmol) nachgegeben. Nach Verdünnen mit Dichlormethan wird die Mischung mit gesättigter Natriumhydrogencarbonat-Lösung gewaschen und im Vakuum eingeengt. Das Rohprodukt wird mittels präparativer RP-HPLC (Eluent: Acetonitrit/Wasser-Gradient) gereinigt. Erhalten werden 118 mg (45.7% d. Th.) des Zielprodukts.
LC-MS (Methode 5): Rₜ = 1.91 min.; m/z = 488 (M+H)⁺
¹H-NMR (400 MHz, DMSO-d₆): δ = 8.59 (s, 1H), 7.58-7.52 (m, 2H), 7.43-7.39 (m, 5H), 7.01 (d, 2H), 5.26 (m, 1H), 3.82 (s, 3H), 3.50 (s, 3H), 2.80-2.75 (m, 1H), 2.60-2.50 (m, 2H), 2.46-2.22 (m, 5H), 1.89-1.81 (m, 1H), 1.68-1.59 (m, 1H), 1.45-1.32 (m, 2H).

### Beispiel 45

### (+/-)-4-(3-{[5-(4-Methoxyphenyl)-6-phenylfuro[2,3-d]pyrimidin-4-yl]oxy}piperidin-1-yl)-butansäuremethylester

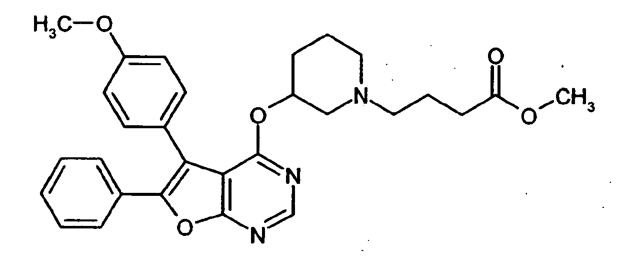

500 mg (1.25 mmol) (+/-)-5-(4-Methoxyphenyl)-6-phenyl-4-(piperidin-3-yloxy)furo[2,3-d]pyrimidin (Beispiel 37A) in 1 ml THF werden nacheinander mit 0.65 ml (3.74 mmol) DIEA, 20.6 mg (0.125 mmol) Kaliumiodid sowie 450 mg (2.5 mmol) 4-Brombuttersäuremethylester versetzt. Die Mischung wird 4 h unter Rückfluss erhitzt, dann abgekühlt, mit Dichlormethan verdünnt, mit gesättigter Natriumhydrogencarbonat-Lösung gewaschen und im Vakuum konzentriert. Das Rohprodukt wird durch Chromatographie an Silicagel (Laufmittel: Cyclohexan/Ethylacetat 4:1 → 1:1) gereinigt. Erhalten werden 662 mg (100% d. Th.) der Zielverbindung.
LC-MS (Methode 5): Rₜ = 1.89 min.; m/z = 502 (M+H)⁺
¹H-NMR (400 MHz, DMSO-d₆): δ = 8.58 (s, 1H), 7.54 (d, 2H), 7.43-7.39 (m, 5H), 7.00 (d, 2H), 5.24 (m, 1H), 3.81 (s, 3H), 3.52 (s, 3H), 2.79-2.74 (m, 1H), 2.48-2.41 (m, 1H), 2.28-2.21 (m, 5H), 2.14 (m, 1H), 1.92-1.85 (m, 1H), 1.68-1.58 (m, 3H), 1.47-1.30 (m, 2H).

### Trennung der Enantiomere:

Racemischer (+/-)-4-(3-{[5-(4-Methoxyphenyl)-6-phenylfuro[2,3-d]pyrimidin-4-yl]oxy}piperidin-1-yl)-butansäuremethylester wird in einem 1:4-Gemisch aus Ethanol und iso-Hexan gelöst und durch präparative HPLC an chiraler Phase in die Enantiomere getrennt (siehe Beispiel 46 und 47) [Säule: Daicel Chiralpak AS-H, 5 µm, 250 mm x 20 mm; Fluss: 15 mm/min; Detektion: 220 nm; Injektionsvolumen 0.5 ml; Temperatur: 28°C; Eluent: t = 0 min 90% iso-Hexan / 10% Ethanol → t = 8 min 90% iso-Hexan / 10% Ethanol].

### Beispiel 46

### 4-(3-{[5-(4-Methoxyphenyl)-6-phenylfuro[2,3-d]pyrimidin-4-yl]oxy}piperidin-1-yl)-butansäuremethylester (Enantiomer 1)

LC-MS (Methode 6): Rₜ =1.84 min.; m/z = 502 (M+H)⁺
Rₜ = 7.26 min [Säule: Daicel Chiralpak AS-H, 5 µm, 250 mm x 4.6 mm; Fluss: 1.0 ml/min; Detektion: 230 nm; Temperatur: 25°C; Eluent: 90% iso-Hexan / 10% Ethanol mit 0.2% Diethylamin];
¹H-NMR (400 MHz, DMSO-d₆): δ = 8.58 (s, 1H), 7.54 (d, 2H), 7.43-7.39 (m, 5H), 7.00 (d, 2H), 5.24 (m, 1H), 3.81 (s, 3H), 3.52 (s, 3H), 2.79-2.74 (m, 1H), 2.48-2.41 (m, 1H), 2.28-2.21 (m, 5H), 2.18-2.12 (m, 1H), 1.92-1.85 (m, 1H), 1.68-1.58 (m, 3H), 1.47-1.30 (m, 2H).

### Beispiel 47

### 4-(3-{[5-(4-Methoxyphenyl)-6-phenylfuro[2,3-d]pyrimidin-4-yl]oxy}piperidin1-yl)-butansäuremethylester (Enantiomer 2)

LC-MS (Methode 3): Rₜ = 1.67 min.; m/z = 502 (M+H)⁺
Rₜ = 7.63 min [Säule: Daicel Chiralpak AS-H, 5 µm, 250 mm x 4.6 mm; Fluss: 1.0 ml/min; Detektion: 230 nm; Temperatur: 25°C; Eluent: 90% iso-Hexan / 10% Ethanol mit 0.2% Diethylamin];
¹H-NMR (400 MHz, DMSO-d₆): δ = 8.58 (s, 1H), 7.54 (d, 2H), 7.43-7.39 (m, 5H), 7.00 (d, 2H), 5.24 (m, 1H), 3.81 (s, 3H), 3.52 (s, 3H), 2.79-2.74 (m, 1H), 2.48-2.41 (m, 1H), 2.28-2.21 (m, 5H), 2.19-2.12 (m, 1H), 1.92-1.85 (m, 1H), 1.68-1.58 (m, 3H), 1.47-1.30 (m, 2H).

### Beispiel 48

### (+/-)-3-[2-({[5-(4-Methoxyphenyl)-6-phenylfuro[2,3-d]pyrimidin-4-yl]oxy}methyl)pyrrolidin-1-yl]-propansäuremethylester

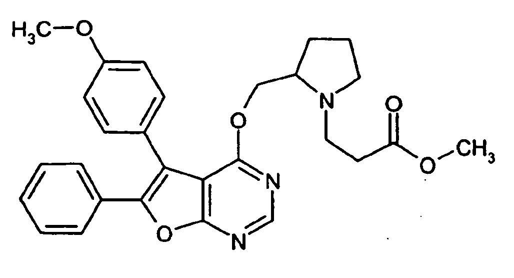

160 mg (0.4 mmol) (+/-)-5-(4-Methoxyphenyl)-6-phenyl-4-(pyrrolidin-2-ylmethoxy)furo[2,3-d]-pyrimidin (Beispiel 38A) werden in 0.8 ml THF gelöst und mit 110 µl (0.8 mmol) Triethylamin sowie 87 µl (0.8 mmol) 3-Brompropionsäuremethylester versetzt. Die Mischung wird über Nacht bei 20-40°C gerührt, dann mit Dichlormethan verdünnt und mit gesättigter Natriumhydrogencarbonat-Lösung gewaschen. Die Lösung wird im Vakuum eingeengt und das erhaltene Öl durch präparative RP-HPLC (Eluent: Acetonitril/Wasser-Gradient) gereinigt. Erhalten werden 90 mg (44% d. Th.) des Zielprodukts.
LC-MS (Methode 6): Rₜ = 1.79 min.; m/z = 488 (M+H)⁺
¹H-NMR (400 MHz, DMSO-d₆): δ = 8.58 (s, 1H), 7.53 (d, 2H), 7.42-7.36 (m, 5H), 7.01 (d, 2H), 4.39 (dd, 1H), 4.18 (dd, 1H), 3.81 (s, 3H), 3.51 (s, 3H), 2.96-2.89 (m, 2H), 2.64 (m, 1H), 2.41-2.30 (m, 3H), 2.12 (q, 1H), 1.75-1.35 (m, 4H).

Die folgenden enantiomerenreinen Verbindungen werden auf analoge Weise (4 h Reaktionszeit bei ca. 40°C, insgesamt größere Überschüsse an DIEA und 3-Brompropionsäuremethylester) ausgehend von den enantiomerenreinen Pyrrolidin-Derivaten Beispiel 40A bzw. 41A hergestellt:

| **Beispiel** | **Struktur** | **Analytische Daten** |
|---|---|---|
| **49** | 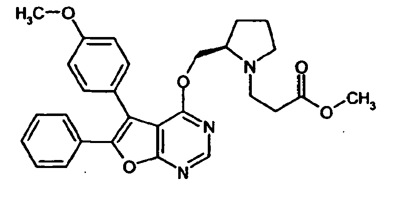 | LC-MS (Methode 5): Rₜ = 1.91 min.; m/z = 488 (M+H)⁺ ¹H-NMR (400 MHz, DMSO-d₆): δ = 8.58 (s, 1H), 7.52 (d, 2H), 7.42-7.36 (m, 5H), 7.01 (d, 2H), 4.38 (dd, 1H), 4.18 (dd, 1H), 3.81 (s, 3H), 3.52 (s, 3H), 2.97-2.89 (m, 2H), 2.69-2.61 (m, 1H), 2.41-2.30 (m, 3H), 2.12 (q, 1H), 1.75-1.35 (m, 4H). |
| **50** | 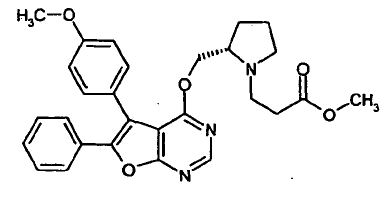 | LC-MS (Methode 6): Rₜ = 1.80 min.; m/z = 488 (M+H)⁺ ¹H-NMR (400 MHz, DMSO-d₆): 8 = 8.58 (s, 1H), 7.52 (d, 2H), 7.42-7.36 (m, 5H), 7.01 (d, 2H), 4.39 (dd, 1H), 4.18 (dd, 1H), 3.81 (s, 3H), 3.52 (s, 3H), 2.97-2.89 (m, 2H), 2.69-2.61 (m, 1H), 2.41-2.30 (m, 3H), 2.12 (q, 1H), 1.75-1.35 (m, 4H). |

### Beispiel 51

### (+/-)-(3-{[5-(4-Methoxyphenyl)-6-phenylfuro[2,3-d]pyrimidin-4-yl]amino}piperidin-1-yl)-propansäuremethylester

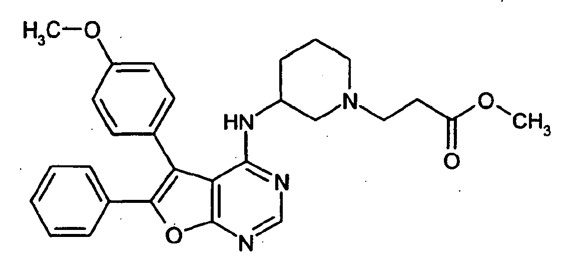

Eine Lösung von 150 mg (0.375 mmol) (+/-)-5-(4-Methoxyphenyl)-6-phenyl-*N*-piperidin-3-ylfuro-[2,3-d]pyrimidin-4-amin (Beispiel 39A) in 0.75 ml THF wird mit 82 µl (0.749 mmol) 3-Brompropansäuremethylester sowie 104 µl (0.749 mmol) Triethylamin versetzt und über Nacht bei 20-40°C gerührt. Die Mischung wird mit Dichlormethan verdünnt und mit gesättigter Natriumhydrogencarbonat-Lösung gewaschen. Nach dem Einengen im Vakuum wird der Rückstand mit Methanol verrührt, das ausgefallene Produkt abgesaugt und im Hochvakuum getrocknet. Aus dem Filtrat wird nach Einengen durch präparative RP-HPLC (Eluent: Acetonitril/Wasser-Gradient) eine zweite Produktcharge isoliert. Insgesamt werden 124 mg (67.1% d. Th.) des Zielprodukts erhalten.
LC-MS (Methode 5): Rₜ = 1.83 min.; m/z = 487 (M+H)⁺
¹H-NMR (400 MHz, DMSO-d₆): δ = 8.31 (s, 1H), 7.49-7.44 (m, 4H), 7.39-7.30 (m, 3H), 7.14 (d, 2H), 5.59 (d, 1H), 4.24 (m, 1H), 3.83 (s, 3H), 3.59 (s, 3H), 2.42-2.35 (m, 2H), 2.28-2.18 (m, 2H), 2.05-1.97 (m, 1H), 1.62-1.55 (m, 1H), 1.40 (br. s, 2H).

### Beispiel 52

### (+/-)-4-[2-({[5-(4-Methoxyphenyl)-6-phenylfuro[2,3-d]pyrimidin-4-yl]oxy}methyl)pyrrolidin-1-yl]-butansäuremethylester

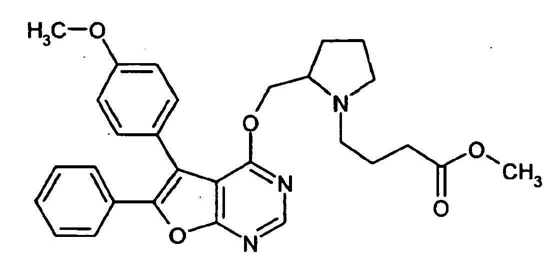

100 mg (0.25 mmol) (+/-)-5-(4-Methoxyphenyl)-6-phenyl-4-(pyrrolidin-2-ylmethoxy)furo[2,3-d]-pyrimidin (Beispiel 38A) werden in 2 ml THF) gelöst und nacheinander mit 65 µl (0.374 mmol) DIEA, 4.1 mg (0.025 mmol) Kaliumiodid und 45 mg (0.25 mmol) 4-Brombuttersäuremethylester versetzt. Die Mischung wird 1 h unter Rückfluss erhitzt und dann nach Abkühlen auf Wasser gegeben. Man extrahiert dreimal mit Ethylacetat und wäscht die vereinigten organischen Phasen zweimal mit Pufferlösung (pH 7) und mit gesättigter Natriumchlorid-Lösung. Die Lösung wird über Magnesiumsulfat getrocknet und im Vakuum eingeengt. Das Rohprodukt wird durch präparative RP-HPLC (Eluent: Acetonitril/Wasser-Gradient) gereinigt. Erhalten werden 47 mg (37.6% d. Th.) des Zielprodukts.
LC-MS (Methode 3): Rₜ =1.73 min.; m/z = 502 (M+H)⁺
¹H-NMR (400 MHz, DMSO-d₆): δ = 8.60 (s, 1H), 7.55-7.52 (m, 2H), 7.42-7.37 (m, 5H), 7.01 (d, 2H), 4.32 (dd, 1H), 4.19 (dd, 1H), 3.80 (s, 3H), 3.49 (s, 3H), 2.93 (t, 1H), 2.62-2.50 (m, 2H), 2.20-2.02 (m, 4H), 1.74-1.67 (m, 1H), 1.60-1.37 (m, 5H).

Die folgenden enantiomerenreinen Verbindungen werden auf analoge Weise ausgehend von den enantiomerenreinen Pyrrolidin-Derivaten Beispiel 40A bzw. 41A hergestellt:

| **Beispiel** | **Struktur** | **Analytische Daten** |
|---|---|---|
| **53** | 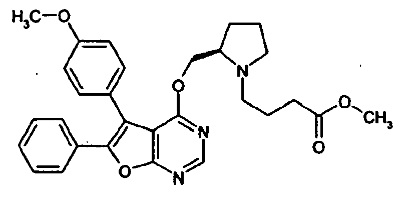 | LC-MS (Methode 3): Rₜ = 1.72 min.; m/z = 502 (M+H)⁺ ¹H-NMR (400 MHz, DMSO-d₆); δ = 8.60 (s, 1H), 7.55-7.52 (m, 2H), 7.42-7.37 (m, 5H), 7.01 (d, 2H), 4.32 (dd, 1H), 4.19 (dd, 1H), 3.80 (s, 3H), 3.49 (s, 3H), 2.93 (t, 1H), 2.62-2.50 (m, 2H), 2.20-2.02 (m, 4H), 1.74-1.67 (m, 1H), 1.60-1.37 (m, 5H). |
| **54** | 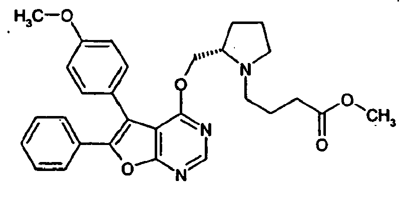 | LC-MS (Methode 3): Rₜ = 1.70 min.; m/z = 502 (M+H)⁺ ¹H-NMR (400 MHz, DMSO-d₆): δ = 8.60 (s, 1H), 7.55-7.52 (m, 2H), 7.42-7.37 (m, 5H), 7.01 (d, 2H), 4.32 (dd, 1H), 4.19 (dd, 1H), 3.80 (s, 3H), 3.49 (s, 3H), 2.93 (t, 1H), 2.62-2.50 (m, 2H), 2.20-2.02 (m, 4H), 1.74-1.67 (m, 1H), 1.60-1.37 (m, 5H). |

### Beispiel 55

### (+/-)-4-(3-{[5-(4-Methoxyphenyl)-6-phenylfuro[2,3-d]pyrimidin-4-yl]amino}piperidin-1-yl)-butansäuremethylester

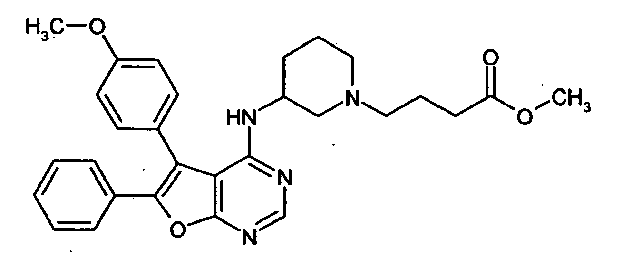

Eine Mischung aus 100 mg (0.25 mmol) (+/-)-5-(4-Methoxyphenyl)-6-phenyl-*N*-piperidin-3-yl-furo[2,3-d]pyrimidin-4-amin (Beispiel 39A), 65 µl (0.375 mmol) DIEA, 4.1 mg (0.025 mmol) Kaliumiodid und 45 mg (0.25 mmol) 4-Brombuttersäuremethylester in 2 ml THF wird 1 h lang unter Rückfluss erhitzt. Nach dem Abkühlen wird die Reaktionsmischung auf Wasser gegeben und dreimal mit Ethylacetat extrahiert. Die vereinigten organischen Phasen werden zweimal mit Pufferlösung (pH 7) und gesättigter Natriumchlorid-Lösung gewaschen, über Magnesiumsulfat getrocknet und im Vakuum konzentriert. Das Rohprodukt wird durch präparative RP-HPLC (Eluent: Acetonitril/Wasser-Gradient), gefolgt von Chromatographie an Silicagel (Laufmittel: Dichlormethan → Dichlormethan/Methanol 50:1) gereinigt. Nach Verrühren des erhaltenen Produkts mit Methanol wird der Niederschlag abgesaugt, mit wenig Methanol nachgewaschen und im Hochvakuum getrocknet. Isoliert werden 58 mg (46.4% d. Th.) des Zielprodukts.
LC-MS (Methode 6): Rₜ = 1.85 min.; m/z = 501 (M+H)⁺
¹H-NMR (400 MHz, DMSO-d₆): δ = 8.31 (s, 1H), 7.48-7.42 (m, 4H), 7.39-7.30 (m, 3H), 7.13 (d, 2H), 5.58 (br. d, 1H), 4.28 (br. s, 1H), 3.83 (s, 3H), 3.59 (s, 3H), 2.50-2.42 (m, 1H), 2.38-2.31 (m, 1H), 2.22-2.15 (m, 3H), 2.12 (t, 1H), 2.02 (br. s, 1H), 1.58-1.40 (m, 6H).

### Beispiel 56

### (+/-)-trans-[(3-{[5-(4-Methoxyphenyl)-6-phenylfuro[2,3-d]pyrimidin-4-yl]amino}cyclohexyl)-oxy]-essigsäure-tert.-butylester

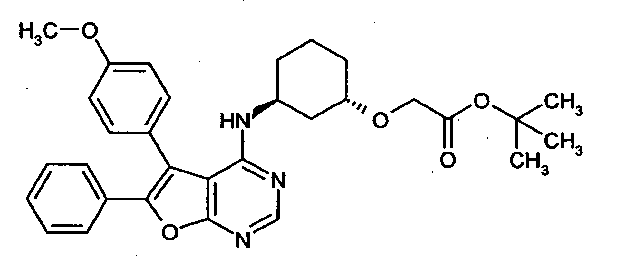

Eine Mischung aus 549 mg (1.63 mmol) 4-Chlor-5-(4-methoxyphenyl)-6-phenylfuro[2,3-d]pyrimidin, 0.43 ml (2.45 mmol) DIEA und 456 mg (+/-)-*trans*-{[3-Aminocyclohexyl]oxy}-essigsäure-*tert*.-butylester (Beispiel 43A / Rohprodukt, ca. 1.63 mmol) in 1.5 ml DMF wird 2 h lang bei 120°C gerührt. Nach dem Abkühlen wird die Mischung auf Wasser gegeben und dreimal mit Ethylacetat extrahiert. Die vereinigten organischen Phasen werden mit gesättigter Natriumchlorid-Lösung gewaschen und im Vakuum eingeengt. Das Rohprodukt wird durch präparative RP-HPLC (Eluent: Acetonitril/Wasser-Gradient) gereinigt. Erhalten werden 434 mg (50.3% d. Th.) des Zielprodukts.
LC-MS (Methode 5): Rₜ = 3.29 min.; m/z = 530 (M+H)⁺
¹H-NMR (400 MHz, DMSO-d₆): δ = 8.35 (s, 1H), 7.50-7.45 (m, 4H), 7.40-7.31 (m, 3H), 7.18 (d, 2H), 4.71 (d, 1H), 4.31 (m, 1H), 3.92 (s, 2H), 3.87 (s, 3H), 3.38-3.30 (m, 1H), 1.77-1.67 (m, 2H), 1.55-1.42 (m, 4H), 1.38 (s, 9H), 1.18-1.10 (m, 2H).

### Trennung der Enantiomere:

0.39 g (+/-)*trans*-[(3-{[5-(4-Methoxyphenyl)-6-phenylfuro[2,3-d]pyrimidin-4-yl]amino}cyclohexyl)oxy]-essigsäure-*tert*.-butylester werden in 4 ml 2-Propanol und 13 ml iso-Hexan gelöst. Das Racemat wird durch präparative HPLC an chiraler Phase in die Enantiomere getrennt (siehe Beispiel 57 und 58) [Säule: Daicel Chiralpak AD-H, 5 µm, 250 mm x 20 mm; Fluss: 15 ml/min; Detektion: 215 nm; Injektionsvolumen 1.0 ml; Temperatur: 30°C; Eluent: t = 0 min 80% iso-Hexan / 20% 2-Propanol → t = 9.5 min 80% iso-Hexan / 20% 2-Propanol].

### Beispiel 57

### (+)-trans-[(3-{[5-(4-Methoxyphenyl)-6-phenylfuro[2,3-d]pyrimidin-4-yl]amino}cyclohexyl)oxy]-essigsäure-tert.-butylester (Enantiomer 1)

[α]_{D}²⁰ = +43.3°, c = 0.51, CHCl₃
¹H-NMR (400 MHz, OMSO-d₆): δ = 8.34 (s, 1H), 7.50-7.45 (m, 4H), 7.40-7.31 (m, 3H), 7.18 (d, 2H), 4.72 (d, 1H), 4.36-4.28 (m, 1H), 3.93 (s, 2H), 3.87 (s, 3H), 3.38-3.30 (m, 1H), 1.77-1.67 (m, 2H), 1.55-1.42 (m, 4H), 1.40 (s, 9H), 1.18-1.10 (m, 2H).

### Beispiel 58

### (-)-trans-[(3-{[5-(4-Methoxyphenyl)-6-phenylfuro[2,3-d]pyrimidin-4-yl]amino}cyclohexyl)oxy]-essigsäure-tert.-butylester (Enantiomer 2)

[α]_{D}²⁰ = -49.1°, c = 0.49, CHCl₃
¹H-NMR (400 MHz, DMSO-d₆): δ = 8.35 (s, 1H), 7.50-7.45 (m, 4H), 7.40-7.31 (m, 3H), 7.18 (d, 2H), 4.72 (d, 1H), 4.36-4.28 (m, 1H), 3.93 (s, 2H), 3.87 (s, 3H), 3.38-3.30 (m, 1H), 1.77-1.67 (m, 2H), 1.55-1.42 (m, 4H), 1.40 (s, 9H), 1.19-1.10 (m, 2H).

### Beispiel 59

### (+/-)-cis-[(-3-{[5-(4-Methoxyphenyl)-6-phenylfuro[2,3-d]pyrimidin-4-yl]amino}cyclohexyl)oxy]-essigsäure-tert.-butylester

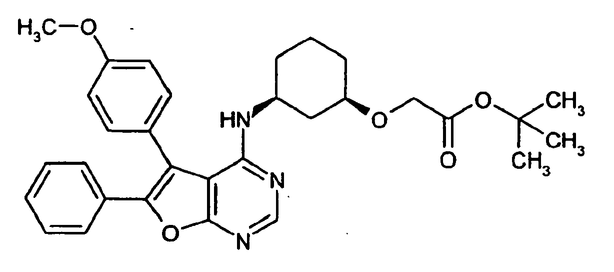

Zu einer Mischung aus 2.02 g 50%-iger Natronlauge (25.3 mmol), 2.5 ml Toluol und 85.8 mg (0.25 mmol) Tetrabutylammoniumhydrogensulfat werden bei 40°C 1.05 g (2.53 mmol) (+/-)-*cis*/*trans*-3-{[5-(4-Methoxyphenyl)-6-phenylfuro[2,3-d]pyrimidin-4-yl]amino}cyclohexanol (Beispiel 44A) als Lösung in 2.5 ml Toluol sowie 0.75 ml (5.05 mmol) Bromessigsäure-*tert*.-butylester gegeben. Die heterogene Mischung wird bei 70°C für 2 h sehr kräftig gerührt. Nach dem Abkühlen wird die Mischung auf Wasser gegeben und dreimal mit Dichlormethan extrahiert. Die vereinigten organischen Phasen werden mit gesättigter Ammoniumchlorid-Lösung gewaschen, über Magnesiumsulfat getrocknet und im Vakuum konzentriert. Aus dem Rohprodukt werden durch Chromatographie an Silicagel (Biotage, Laufmittel: Dichlormethan/Methanol 500:1 → 100:1) 671 mg (50.2% d. Th.) der Zielverbindung gewonnen.
LC-MS (Methode 5): Rₜ = 3.33 min.; m/z = 530 (M+H)⁺
¹H-NMR (400 MHz, DMSO-d₆): δ = 8.34 (s, 1H), 7.48-7.41 (m, 4H), 7.39-7.30 (m, 3H), 7.14 (d, 2H), 5.13 (br. d, 1H), 4.14-4.08 (m, 1H), 3.85 (s, 3H), 3.82 (d, 2H), 3.43-3.35 (m, 1H), 2.09 (br. d, 1H), 1.81-1.60 (m, 3H), 1.41 (s, 9H), 1.30-1.04 (m, 4H).

### Trennung der Enantiomere:

(+/-)-*cis*-[(3-{[5g-(4-Methoxyphenyl)-6-phenylfuro[2,3-d]pyrimidin-4-yl]amino}cyclohexyl)oxy]-essigsäure-*tert*.-butylester wird in gleichen Mengen Ethanol und iso-Hexan gelöst. Das Racemat wird durch präparative HPLC an chiraler Phase in die Enantiomere getrennt (siehe Beispiel 60 und 61) [Säule: Daicel Chiralcel OJ-H, 5 µm, 250 mm x 20 mm; Fluss: 15 ml/min; Detektion: 220 nm; Injektionsvolumen 0.5 ml; Temperatur: 29°C; Eluent: t = 0 min 80% iso-Hexan / 20% Ethanol → t = 12 min 80% iso-Hexan / 20% Ethanol].

### Beispiel 60

### (+)-cis-[(-3-([5-(4-Methoxyphenyl)-6-phenylfuro[2,3-d]pyrimidin-4-yl]amino)cyclohexyl)oxy]-essigsäure-tert.-butylester (Enantiomer 1)

[α]_{D}²⁰ = +77.4°, c = 0.53, CHCl₃
LC-MS (Methode 3): Rₜ = 3.10 min.; m/z = 530 (M+H)⁺
¹H-NMR (400 MHz, DMSO-d₆): δ = 8.34 (s, 1H), 7.48-7.41 (m, 4H), 7.39-7.30 (m, 3H), 7.14 (d, 2H), 5.13 (br. d, 1H), 4.14-4.08 (m, 1H), 3.85 (s, 3H), 3.82 (d, 2H), 3.43-3.35 (m, 1H), 2.09 (br. d, 1H), 1.81-1.71 (m, 2H), 1.68-1.60 (m, 1H), 1.41 (s, 9H), 1.30-1.04 (m, 4H).

### Beispiel 61

### (-)-trans-[(-3-{[5-(4-Methoxyphenyl)-6-phenylfuro[2,3-d]pyrimidin-4-yl]amino}cyclohexyl)oxy]-essigsäure-tert.-butylester (Enantiomer 2)

[a]_{D}²⁰= -71.4°, c = 0.54, CHCl₃
LC-MS (Methode 3): Rₜ = 3.09 min.; m/z = 530 (M+H)^{+ 1}H-NMR (400 MHz, DMSO-d₆): δ = 8.34 (s, 1H), 7.48-7.41 (m, 4H), 7.39-7.30 (m, 3H), 7.14 (d, 2H), 5.13 (br. d, 1H), 4.14-4.08 (m, 1H), 3.85 (s, 3H), 3.82 (d, 2H), 3.43-3.35 (m, 1H), 2.09 (br. d, 1H), 1.81-1.71 (m, 2H), 1.68-1.60 (m, 1H), 1.41 (s, 9H), 1.30-1.04 (m, 4H).

### Beispiel 62

### (+/-)-cis-({-[(5,6-Diphenylfuro[2,3-d]pyrimidin-4-yl)amino]cyclohexyl}oxy)-essigsäure-tert.-butylester

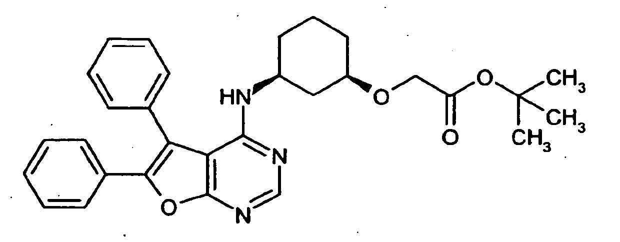

50 mg (0.10 mmol) (+/-)-*cis*-({[(5-Brom-6-phenylfuro[2,3-d]pyrimidin-4-yl)amino]cyclohexyl}-oxy)-essigsäure-*tert*.-butylester (Beispiel 47A) werden in 0.33 ml DMSO gelöst und mit 3.5 mg Bis(triphenylphosphin)palladium(II)chlorid versetzt. Unter Argon werden bei RT nacheinander 0.1 ml 2 N Natriumcarbonat-Lösung und 15.2 mg (0.124 mmol) Phenylboronsäure hinzugegeben. Die heterogene Mischung wird 4 h lang bei 80°C kräftig gerührt. Nach dem Abkühlen wird das Produkt direkt durch präparative RP-HPLC (Eluent: Acetonitril/Wasser-Gradient) isoliert. Erhalten werden 43.9 mg (88.3% d. Th.) der Zielverbindung.
LC-MS (Methode 5): Rₜ = 3.38 min.; m/z = 500 (M+H)⁺
¹H-NMR (400 MHz, DMSO-d₆): δ = 8.35 (s, 1H), 7.64-7.53 (m, 5H), 7.46-7.40 (m, 2H), 7.38-7.31 (m, 3H), 5.03 (d, 1H), 4.07 (br. d, 1H), 3.71 (s, 2H), 3.41-3.35 (m, 1H), 2.08 (d, 1H), 1.70-1.60 (m, 2H), 1.65-1.55 (m, 1H), 1.41 (s, 9H), 1.30-1.12 (m, 2H), 1.08-0.95 (m, 2H).

### Beispiel 63

### (+/-)-cis-({3-[(5,6-Diphenylfuro[2,3-d]pyrimidin-4-yl)oxy]cyclohexyl}oxy)-essigsäure-tert.-butylester

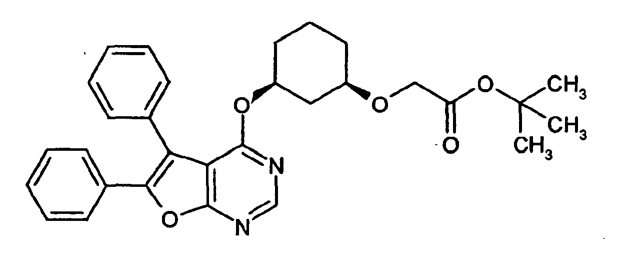

36 mg (0.072 mmol) (+/-)-*cis*-({[(6-Brom-5-phenylfuro[2,3-d]pyrimidin-4-yl)oxy]cyclohexyl}-oxy)-essigsäure-*tert*.-butylester (Beispiel 49A) werden in 0.15 ml DMSO gelöst und mit 2.5 mg Bis(triphenylphosphin)palladium(II)chlorid versetzt. Unter Argon werden bei RT nacheinander 0.07 ml 2 N Natriumcarbonat-Lösung und 10.9 mg (0.089 mmol) Phenylboronsäure hinzugegeben. Die heterogene Mischung wird 4 h lang bei 80°C kräftig gerührt. Nach dem Abkühlen wird das Produkt direkt durch präparative RP-HPLC (Eluent: Acetonitril/Wasser-Gradient) isoliert. Erhalten werden 19.8 mg (55.3% d. Th.) der Zielverbindung.
LC-MS (Methode 6): Rₜ = 3.41 min.; m/z = 501 (M+H)⁺
¹H-NMR (400 MHz, DMSO-d₆): δ = 8.59 (s, 1H), 7.56-7.39 (m, 10H), 5.11 (m, 1H), 3.98 (s, 2H), 3.40 (m, 1H), 2.39 (br. d, 1H), 2.03-1.92 (m, 2H), 1.75-1.67 (m, 1H), 1.40 (s, 9H), 1.28-1.02 (m, 4H).

### Allgemeine Vorschrift D: Hydrolyse von Methyl- oder Ethylestern zu den entsprechenden Carbonsäure-Derivaten

Zu einer Lösung des Methyl- bzw. Ethylesters in THF oder THF/Methanol (1:1) (Konzentration ca. 0.05 bis 0.5 mol/l) werden bei RT 1.5 bis 10 eq. Natriumhydroxid als 1 N wässrige Lösung gegeben. Die Mischung wird für einen Zeitraum von 0.5-18 h bei RT gerührt und dann mit 1 N Salzsäure neutralisiert oder schwach angesäuert. Falls dabei ein Feststoff ausfällt, kann das Produkt durch Filtration, Waschen mit Wasser und Trocknen im Hochvakuum isoliert werden. Alternativ wird die Zielverbindung direkt aus dem Rohprodukt, gegebenenfalls nach extraktiver Aufarbeitung mit Dichlormethan, durch präparative RP-HPLC isoliert (Eluent: Wasser/Acetonitril-Gradient).

### Allgemeine Vorschrift E: Spaltung von tert.-Butylestern zu den entsprechenden Carbonsäure-Derivaten

Zu einer Lösung des *tert*.-Butylesters in Dichlormethan (Konzentration 0.05 bis 1.0 mol/l; zusätzlich ein Tropfen Wasser) wird bei 0°C bis RT tropfenweise TFA hinzugefügt, bis ein Verhältnis Dichlormethan/TFA von ca. 2:1 bis 1:1 erreicht ist. Die Mischung wird 1-18 h bei RT gerührt und dann im Vakuum eingeengt. Der Rückstand wird durch präparative RP-HPLC gereinigt (Eluent: Acetonitril/Wasser-Gradient).

Die folgenden Beispiele werden gemäß der Allgemeinen Vorschrift D oder E aus den zuvor beschriebenen Verbindungen hergestellt:

| **Beispiel** | **Ausgangsmaterial (Methode)** | **Struktur** | **Analytische Daten** |
|---|---|---|---|
| **64** | Beispiel 36 (D) | 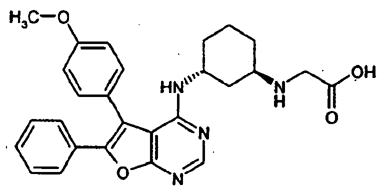 | LC-MS (Methode 6): Rₜ = 1.90 min.; m/z = 473 (M+H)⁺ |
| | | (+/-)-*trans* | |
| **65** | Beispiel 35 (D) | 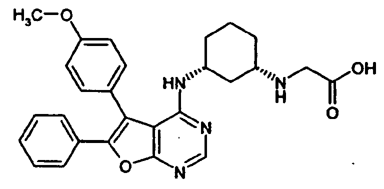 | LC-MS (Methode 6): Rₜ = 1.94 min.; m/z = 473 (M+H)⁺ |
| | | (+/-)-*cis* | |
| **66** | Beispiel 37 (E) | 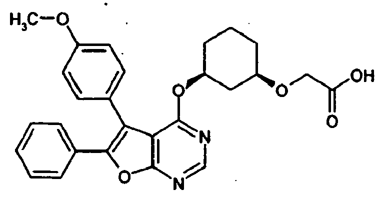 | LC-MS (Methode 3): Rₜ = 2.57 min.; m/z = 475 (M+H)⁺ ¹H-NMR (400 MHz, DMSO-d₆): δ = 8.59 (s, 1H), 7.58-7.53 (m, 2H), 7.44-7.39 (m, 5H), 7.01 (d, 2H), 5.13 (m, 1H), 4.03 (s, 2H), 3.83 (s, 3H), 3.49-3.35(m,2H), 2.46-2.40 (m, 1H), 2.06-1.93 (m, 2H), 1.78-1.70 (m, 1H), 1.37-1.05 (m, 4H). |
| | | *(+l-)-cis* | |
| **67** | Beispiel 38 (E) | 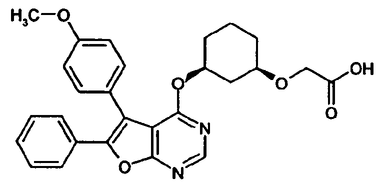 | LC-MS (Methode 5): Rₜ = 2.84 min.; m/z = 475 (M+H)⁺ [α]_{D}²⁰ = -64.2°, c = 0.55, CHCl₃ ¹H-NMR (400 MHz, DMSO-d₆): δ = 8.59 (s, 1H), 7.58-7.53 (m, 2H), 7.44-7.39 (m, 5H), 7.01 (d, 2H), 5.15-5.08 (m, 1H), 4.03 (s, 2H), 3.83 (s, 3H), 3.49-3.35 (m, 1H), 2.46-2.40 (m, 1H), 2.06-1.93 (m, 2H), 1.78-1.70 (m, 1H), 1.37-1.05 (m, 4H). |
| | | (-)-Enantiomer | |
| **68** | Beispiel 39 (E) | 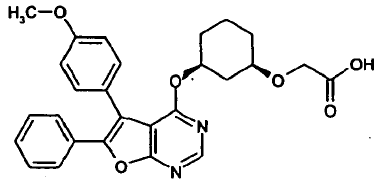 | LC-MS (Methode 5): Rₜ = 2.84 min.; m/z = 475 (M+H)⁺ [α]_{D}²⁰ = +62.8°, c = 0.55, CHCl₃ ¹H-NMR (400 MHz, DMSO-d₆): δ = 8.59 (s, 1H), 7.58-7.53 (m, 2H), 7.44-7.39 (m, 5H), 7.01 (d, 2H), 5.17-5.08 (m, 1H), 4.03 (s, 2H), 3.83 (s, 3H), 3.49-3.35 (m, 1H), 2.46-2.40 (m, 1H), 2.06-1.93 (m, 2H), 1.78-1.70 (m, 1H), 1.37-1.05 (m, 4H). |
| | | (+)-Enantiomer | |
| **69** | Beispiel 40 (E) | 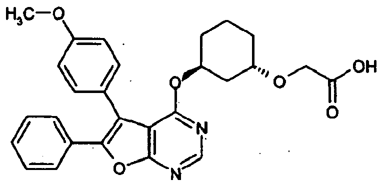 | LC-MS (Methode 3): Rₜ = 2.53 min.; m/z = 475 (M+H)⁺ ¹H-NMR (400 MHz, DMSO-d₆): δ = 12.54 (br. s, 1H), 8.56 (s, 1H), 7.54 (d, 2H), 7.43-7.35 (m, 5H), 7.05 (d, 2H), 5.63-5.59 (m, 1H), 3.90 (d, 2H), 3.81 (s, 3H), 3.30-3.20 (m, 1H), 1.96-1.90 (m, 1H), 1.79-1.43 (m, 5 H),1.30-1.13 (m, 2H). |
| | | (+/-)-*trans* | |
| **70** | Beispiel 41 (E) | 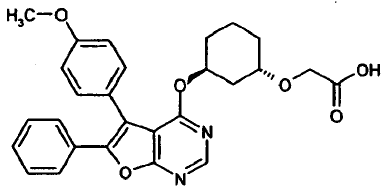 | LC-MS (Methode 3): Rₜ = 2.53 min.; m/z = 475 (M+H)⁺ ¹H-NMR (400 MHz, DMSO-d₆): δ = 12.55 (br. s, 1H), 8.56 (s, 1H), 7.54 (d, 2H), 7.43-7.35 (m, 5H), 7.05 (d, 2H), 5.63-5.59 (m, 1H), 3.90 (d, 2H), 3.81 (s, 3H), 3.30-3.20 (m, 1H), 1.96-1.90 (m, 1H), 1.76-1.69 (m, 1H), 1.65-1.54 (m, 3H), 1.51-1.43 (m, 1H), 1.30-1.13 (m, 2H). [α]_{D}²⁰ = +62.4°, c = 0.48, CHCl₃ |
| | | (+)-Enantiomer | |
| **71** | Beispiel 42 (E) | 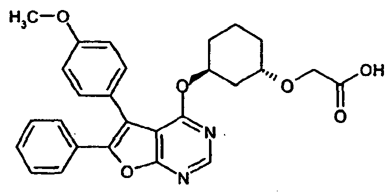 | LC-MS (Methode 5): Rₜ = 2.80 min.; m/z = 475 (M+H)⁺ ¹H-NMR (400 MHz, DMSO-d₆): δ = 12.54 (br. s, 1H), 8.56 (s, 1H), 7.54 (d, 2H), 7.43-7.35 (m, 5H), 7.05 (d, 2H), 5.63-5.59 (m, 1H), 3.90 (d, 2H), 3.81 (s, 3H), 3.30-3.20 (m, 1H), 1.96-1.90 (m, 1H), 1.76-1.69 (m, 1H), 1.65-1.54 (m, 3H), 1.51-1.43 (m, 1H), 1.30-1.13 (m, 2H). [α]_{D}²⁰ = -74.0°, c = 0.50, CHCl₃ |
| | | (-)-Enantiomer | |
| **72** | Beispiel 43 (E) | 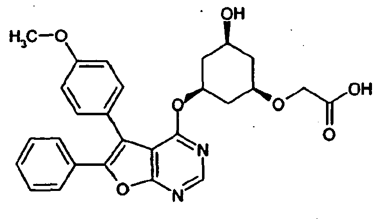 | LC-MS (Methode 5): Rₜ = 2.42 min.; m/z = 491 (M+H)⁺ ¹H-NMR (400 MHz, DMSO-d₆): δ = 8.60 (s, 1H), 7.57 (d, 2H), 7.45-7.38 (m, 5H), 7.02 (d, 2H), 5.15 (m, 1H), 4.80 (br. s, 1H), 4.02 (s, 2H), 3.82 (s, 3H), 3.58-3.35 (m, 2H), 2.45-2.38 (m, 1H), 2.20-2.10 (m, 2H), 1.18-1.02 (m, 3H). |
| | | (+/-)-*cis* | |
| **73** | Beispiel 44 (D) | 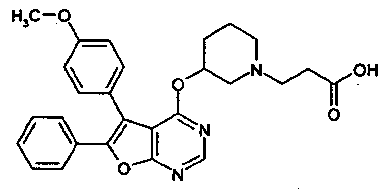 | LC-MS (Methode 3): Rₜ = 1.68 min.; m/z = 474 (M+H)⁺ ¹H-NMR (400 MHz, DMSO-d₆): δ = 8.59 (s, 1H), 7.55 (d, 2H), 7.45-7.40 (m, 5H), 7.01 (d, 2H), 5.26 (m, 1H), 3.82 (s, 3H), 3.45-3.25 (m, 2H), 2.83 (d, 1H), 2.60-2.45 (m, 2H), 2.39-2.27 (m, 4H), 1.90-1.82 (m, 1H), 1.65-1.55 (m, 1H), 1.48-1.31 (m, 2H). |
| | | (rac.) | |
| **74** | Beispiel 48 (D) | 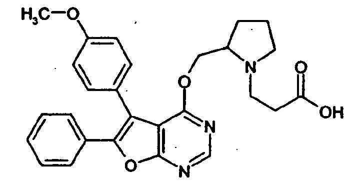 | LC-MS (Methode 6): Rₜ = 1.76 min.; m/z = 464 (M+H)⁺ ¹H-NMR (400 MHz, DMSO-d₆): δ = 8.59 (s, 1H), 7.54 (dd, 2H), 7.43-7.35 (m, 5H), 7.02 (d, 2H), 4.41 (dd, 1H), 4.22 (dd, 1H), 3.82 (s, 3H), 2.99-2.88 (m, 2H), 2.72-2.65 (m, 1H), 2.42-2.38 (m, 1H), 2.30-2.22 (m, 2H), 2.22-2.12 (m, 1H), 1.76-1.68 (m, 1H), 1.65-1.55 (m, 1H), 1.50-1.40 (m, 2H). |
| | | (rac.) | |
| **75** | Beispiel 49 (D) | 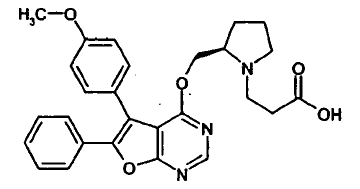 | LC-MS (Methode 5): Rₜ = 1.85 min.; m/z = 474 (M+H)⁺ ¹H-NMR (400 MHz, DMSO-d₆): δ = 8.56 (s, 1H), 7.53 (d, 2H), 7.43-7.36 (m, 5H), 7.01 (d, 2H), 4.42 (dd, 1H), 4.15 (dd, 1H), 3.81 (s, 3H), 2.96-2.88 (m, 2H), 2.67-2.60 (m, 1H), 2.42-2.35 (m, 1H), 2.21-2.10 (m, 3H), 1.73-1.35 (m, 4H). |
| | | (*R*-Enantiomer) | |
| **76** | Beispiel 50 (D) | 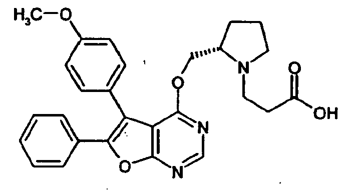 | LC-MS (Methode 5): Rₜ = 1.83 min.; m/z = 474 (M+H)⁺ ¹H-NMR (400 MHz, DMSO-d₆): δ = 8.56 (s, 1H), 7.53 (d, 2H), 7.43-7.36 (m, 5H), 7.01 (d, 2H), 4.42 (dd, 1H), 4.15 (dd, 1H), 3.81 (s, 3H), 2.96-2.88 (m, 2H), 2.67-2.60 (m, 1H), 2.42-2.35 (m, 1H), 2.21-2.10 (m, 3H), 1.73-1.35 (m, 4H). |
| | | (*S*-Enantiomer) | |
| **77** | Beispiel 51 (D) | 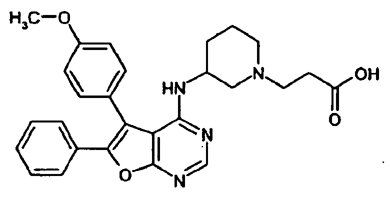 | LC-MS (Methode 6): Rₜ = 1.80 min.; m/z = 473 (M+H)⁺ ¹H-NMR (400 MHz, DMSO-d₆): δ = 8.32 (s, 1H), 7.50-7.45 (m, 4H), 7.40-7.30 (m, 3H), 7.14 (d, 2H), 5.59 (br. d, 1H), 4.25 (br. s, 1H), 3.84 (s, 2H), 2.48-2.35 (m, 2H), 2.27-1.98 (m, 3H), 1.55 (br. s, 1H), 1.48 (br. s, 2H). |
| | | (rac.) | |
| **78** | Beispiel 55 (D) | 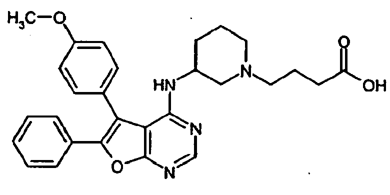 | LC-MS (Methode 5): Rₜ = 1.87 min.; m/z= 487 (M+H)⁺ ¹H-NMR (400 MHz, DMSO-d₆): δ = 8.32 (s, 1H), 7.49-7.45 (m, 4H), 7.39-7.30 (m, 3H), 7.14 (d, 2H), 5.58 (br. d, 1H), 4.27 (br. s, 1H), 3.85 (s, 3H), 2.50-2.42 (m, 1H), 2.39-2.32 (m, 1H), 2.27-2.19 (m, 1H), 2.16-2.02 (m, 4H), 1.55-1.26 (m, 5H). |
| | | (rac.) | |
| **79** | Beispiel 52 (D) | 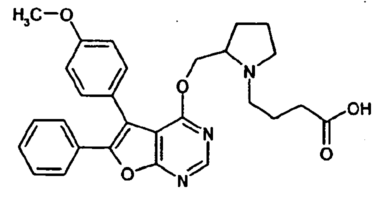 | LC-MS (Methode 3): Rₜ = 1.61 min.; m/z = 488 (M+H)⁺ ¹H-NMR (400 MHz, DMSO-d₆): δ = 8.58 (s, 1H), 7.53 (dd, 2H), 7.42-7.37 (m, 5H), 7.02 (d, 2H), 4.38 (dd, 1H), 4.16 (dd, 1H), 3.81 (s, 3H), 2.95 (br. t, 1H), 2.62-2.50 (m, 1H), 2.18-1.92 (m, 4H), 1.74-1.65 (m, 1H), 1.60-1.37 (m, 6H). |
| | | (rac.) | |
| **80** | Beispiel 53 (D) | 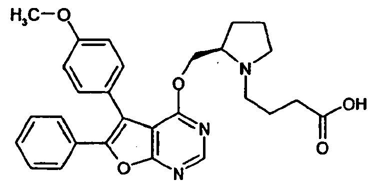 | LC-MS (Methode 3): Rₜ = 1.59 min.; m/z = 488 (M+H)⁺ ¹H-NMR (400 MHz, DMSO-d₆): δ = 8.58 (s, 1H), 7.53 (dd, 2H), 7.42-7.37 (m, 5H), 7.02 (d, 2H), 4.3 8 (dd, 1H), 4.16 (dd, 1H), 3.81 (s, 3H), 2.95 (br. t, 1H), 2.62-2.50 (m, 1H), 2.18-1.92 (m, 4H), 1.74-1.65 (m, 1H), 1.60-1.37 (m, 6H). [α]_{D}²⁰ = -124.4°, c = 0.50, CHCl₃ |
| | | (R-Enantiomer) | |
| **81** | Beispiel 54 (D) | 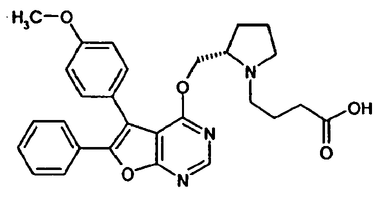 | LC-MS (Methode 5): Rₜ = 1.86 min.; m/z = 488 (M+H)⁺ ¹H-NMR (400 MHz, DMSO-d₆): δ = 8.58 (s, 1H), 7.53 (dd, 2H), 7.42-7.37 (m, 5H), 7.02 (d, 2H), 4.38 (dd, 1H), 4.16 (dd, 1H), 3.81 (s, 3H), 2.95 (br. t, 1H), 2.62-2.50 (m, 1H), 2.18-1.92 (m, 4H), 1.74-1.65 (m, 1H), 1.60-1.37 (m, 6H). [α]_{D}²⁰ = +81.0°, c = 0.50, CHCl₃ |
| | | (*S*-Enantiomer) | |
| **82** | Beispiel 45 (D) | 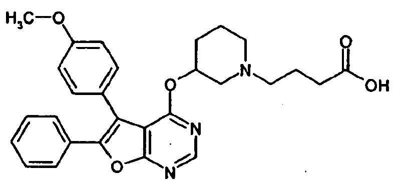 | LC-MS (Methode 5): Rₜ = 1.88 min.; m/z = 488 (M+H)⁺ ¹H-NMR (400 MHz, DMSO-d₆): δ = 8.57 (s, 1H), 7.54 (dd, 2H), 7.45-7.38 (m, 5H), 7.00 (d, 2H), 5.24 (m, 1H), 3.82 (s, 3H), 2.84 (br. d, 1H), 2.53-2.45 (m, 1H), 2.29-2.22 (m, 2H), 2.18-1.99 (m, 4H), 1.93-1.86 (m, 1H), 1.64-1.52 (m, 2H), 1.49-1.36 (m, 1H), 1.34-1.25 (m, 1H). |
| | | (rac.) | |
| **83** | Beispiel 48 (D) | 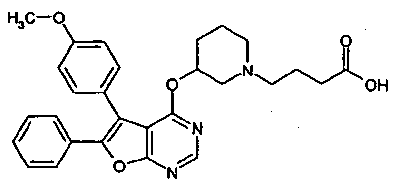 | LC-MS (Methode 5): Rₜ = 1.87 min.; m/z = 488 (M+H)⁺ [α]_{D}²⁰ = +84.0°, c = 0.50, CHCl₃ |
| | | (+)-Enantiomer | |
| **84** | Beispiel 47 (D) | 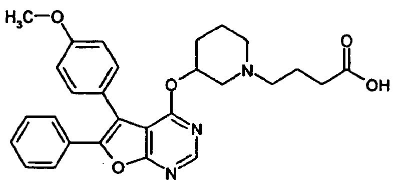 | LC-MS (Methode 5): Rₜ = 1.88 min.; m/z = 488 (M+H)⁺ [α]_{D}²⁰ = -85.7°, c = 0.50, CHCl₃ |
| | | (-)-Enantiomer | |
| **85** | Beispiel 56 (E) | 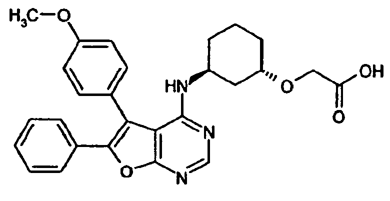 | LC-MS (Methode 3): Rₜ = 2.39 min.; m/z = 474 (M+H)⁺ ¹H-NMR (400 MHz, DMSO-d₆): δ = 8.35 (s, 1H), 7.52-7.47 (m, 2H), 7.39-7.30 (m, 3H), 7.18 (d, 2H), 4.73 (d, 1H), 4.34 (br. s, 1H), 3.95 (br. s, 2H), 3.87 (s, 3H), 3.40-3.25 (m, 1H), 1.80-1.63 (m, 2H), 1.60-1.40 (m, 4H), 1.20-1.10 (m, 2H). |
| | | (+/-)-*trans* | |
| **86** | Beispiel 58 (E) | 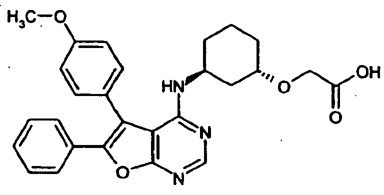 | LC-MS (Methode 6): Rₜ = 2.55 min.; m/z = 474 (M+H)⁺ [α]_{D}²⁰ = -72.2°, c = 0.50, CHCl₃ ¹H-NMR (400 MHz, DMSO-d₆): δ = 12.66 (br. s, 1H), 8.35 (s,1H), 7.52-7.45 (m, 5H), 7.39-7.30 (m, 3H), 7.18 (d, 2H), 4.74 (d, 1H), 4.40-4.3 0 (m, 1H), 3.96 (s, 2H), 3.88 (s, 3H), 3.40-3.25 (m, 1H), 1.80-1.62 (m, 2H), 1.60-1.40 (m, 4H), 1.20-1.08 (m, 2H). |
| | | (-)-Enantiomer | |
| **87** | Beispiel 57 (E) | 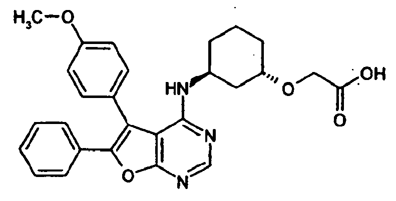 | LC-MS (Methode 6): Rₜ = 2.55 min.; m/z = 474 (M+H)⁺ [α]_{D}²⁰ = +63°, c = 0.50, CHCl₃ ¹H-NMR (400 MHz, DMSO-d₆): δ = 12.66 (br. s, 1H), 8.35 (s, 1H), 7.52-7.45 (m, 5H), 7.39-7.30 (m, 3H), 7.18 (d, 2H), 4.74 (d, 1H), 4.40-4.30 (m, 1H), 3.96 (s, 2H), 3.88 (s, 3H), 3.40-3.25 (m, 1H), 1.80-1.62 (m, 2H), 1.60-1.40 (m, 4H), 1.20-1.08 (m, 2H). |
| | | (+)-Enantiomer | |
| **88** | Beispiel 59 (E) | 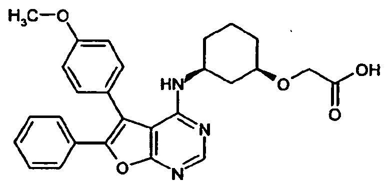 | LC-MS (Methode 3): Rₜ = 2.39 min.; m/z = 474 (M+H)⁺ ¹H-NMR (400 MHz, DMSO-d₆) : δ = 12.40 (br. s, 1H), 8.35 (s, 1H), 7.48-7.42 (m, 4H), 7.39-7.30 (m, 3H), 7.14 (d, 2H), 5.15 (br. d, 1H), 4.15-4.07 (m, 1H), 3.35 (2 s, 5H), 3.45-3.38 (m, 1H), 2.10 (br. d, 1H), 1.82-1.72 (m, 2H), 1.66-1.55 (m, 1H), 1.28-1.18 (m, 2H), 1.12-1.02 (m, 2H). |
| | | *(+*/*-)-cis* | |
| **89** | Beispiel 60 (E) | 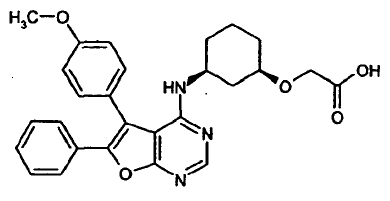 | LC-MS (Methode 6): Rₜ = 2.54 min.; m/z = 474 (M+H)⁺ [α]_{D}²⁰ = +69.5°, c = 0.5, CHCl₃ ¹H-NMR (400 MHz, DMSO-d₆): δ = 12.57 (br. s, 1H), 8.35 (s, 1H), 7.48-7.42 (m, 4H), 7.39-7.30 (m, 3H), 7.14 (d, 2H), 5.18-5.11 (m, 1H), 4.15-4.07 (m, 1H), 3.35 (2 s, 5H), 3.45-3.36 (m, 1H), 2.10 (br. d, 1H), 1.82-1.72 (m, 2H), 1.66-1.55 (m, 1H), 1.28-1.18 (m, 2H), 1.12-1.02 (m, 2H). |
| | | (+)-Enantiomer | |
| **90** | Beispiel 61 (E) | 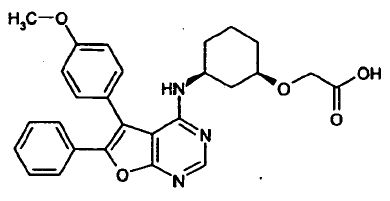 | LC-MS (Methode 6): Rₜ = 2.54 min.; m/z = 474 (M+H)⁺ [α]_{D}²⁰ = -85.4°, c = 0.54, CHCl₃ ¹H-NMR (400 MHz, DMSO-d₆): δ = 12.57 (br. s, 1H), 8.35 (s, 1H), 7.48-7.42 (m, 4H), 7.39-7.30 (m, 3H), 7.14 (d, 2H), 5.18-5.11 (m, 1H), 4.15-4.07 (m, 1H), 3.35 (2 s, 5H), 3.45-3.36 (m, 1H), 2.10 (br. d, 1H), 1.82-1.72 (m, 2H),1.66-1.55 (m, 1H), 1.28-1.18 (m, 2H), 1.12-1.02 (m, 2H). |
| | | (-)-Enantiomer | |
| **91** | Beispiel 63 (E) | 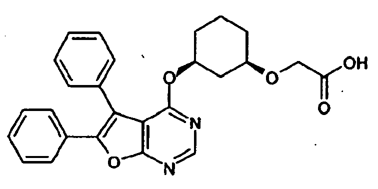 | LC-MS (Methode 3): Rₜ = 2.59 min.; m/z = 445 (M+H)⁺ ¹H-NMR (400 MHz, DMSO-d₆₄): δ = 12.35 (br. s, 1H), 8.61 (s, 1H), 7.56-7.37 (m, 10H), 5.15-5.06 (m,1H), 4.00 (s, 2H), 3.46-3.25 (m, 1H), 2.41 (br. d, 1H), 2.05-1.95 (m, 2H), 1.71 (br. d, 1H), 1.30-1.02 (m, 4H). |
| | | *(+*/*-)-cis* | |
| **92** | Beispiel 62 (E) | 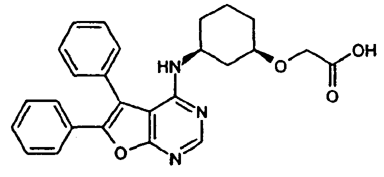 | LC-MS (Methode 5): Rₜ = 2.70 min.; m/z = 444 (M+H)⁺. |
| | | (*+*/*-*)*-cis* | |

### Beispiel 93

### (+/-)-(5-cis,3-trans)-[(3-Fluor-5-{[5-(4-methoxyphenyl)-6-phenylfuro[2,3-d]pyrimidin-4-yl]oxy}-cyclohexyl)oxy]-essigsäure

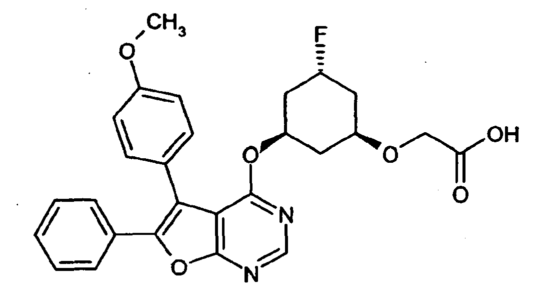

150 mg (0.27 mmol) (+/-)-*all*-*cis*-[(3-Hydroxy-5-{[5-(4-methoxyphenyl)-6-phenylfuro[2,3-d]-pyrimidin-4-yl]oxy}cyclohexyl)oxy]-essigsäure-*tert*.-butylester werden in 2.5 ml Dichlormethan vorgelegt und auf 0°C abgekühlt. Man gibt 53 mg (0.33 mmol) Diethylaminoschwefeltrifluorid (DAST) hinzu und läßt dann auf RT kommen. Man verdünnt danach mit Wasser und Dichlormethan und trennt die Phasen. Man extrahiert die wässrige Phase zweimal mit Dichlormethan, wäscht die vereinigten organischen Phasen einmal mit ges. Natriumchlorid-Lösung, trocknet über Magnesiumsulfat und engt ein. Man löst den Rückstand in 5 ml Dichlormethan, gibt 1 ml Trifluoressigsäure hinzu und rührt 30 min bei RT. Man gibt dann ges. Natriumhydrogencarbonat-Lösung hinzu, trennt die wässige Phase ab und wäscht die wässrige Phase einmal mit Diethylether. Anschließend wird mit 1 N Salzsäure angesäuert und die wässrige Phase zweimal mit Ethylacetat extrahiert. Die vereinigten organischen Phasen werden einmal mit ges. Natriumchlorid-Lösung gewaschen, über Magnesiumsulfat getrocknet und eingeengt. Der Rückstand wird chromatographisch über eine Silicagel-Dickschichtplatte gereinigt (Laufmittel: Dichlormethan/Methanol 9:1). Die Produkt-Zone wird mit Dichlormethan/Methanol 9:1 extrahiert. Man reinigt anschließend nochmals durch präparative RP-HPLC (Eluent: Acetonitril/Wasser-Gradient) und erhält 46 mg (34.0% d. Th.) der Zielverbindung.
LC-MS (Methode 8): Rₜ = 2.76 min.; m/z = 493 (M+H)⁺
¹H-NMR (400 MHz, DMSO-d₆): δ = 12.55 (s, 1H), 8.61 (s, 1H), 7.55 (d, 2H), 7.44-7.35 (m, 5H), 7.01 (d, 2H), 5.45-5.35 (m, 1H), 5.15-4.96 (d, 1H), 4.05 (s, 2H), 3.81 (s, 3H), 3.78-3.69 (m, 1H), 2.52-2.42 (m, 1H), 2.42-2.21 (m, 2H), 1.65-1.33 (m, 3H).

### Allgemeine Vorschrift F: Umsetzung von Nitrilen mit Trimethylsilylazid zu den entsprechenden Tetrazol-Derivaten

Zu einer Lösung des Nitrils in Toluol (Konzentration ca. 100 mg/ml) werden bei RT ca. 15 eq. Trimethylsilylazid und ca. 1.5 eq. Di-n-butylzinnoxid gegeben. Die Mischung wird in einem Temperaturbereich von 70°C bis zum Rückfluss mehrere Stunden, bevorzugt über Nacht, gerührt. Nach Ende der Reaktion wird ein größerer Überschuss Ethylenglycol zugesetzt und die Mischung ca. 1h bei Rückfluss gerührt. Nach Abkühlen wird mit Ethylacetat verdünnt, mit ges. Natriumhydrogencarbonat-Lösung, 1 N Salzsäure und ges. Natriumchlorid-Lösung gewaschen und im Vakuum eingeengt. Das Produkt wird nach Reinigung durch präparative RP-HPLC (Eluent: Wasser/Acetonitril-Gradient) oder durch Chromatographie an Silicagel erhalten.

Die folgenden Beispiele werden gemäß der Allgemeinen Vorschrift F erhalten:

| **Beispiel** | **Struktur** | **Analytische Daten** |
|---|---|---|
| **94** | 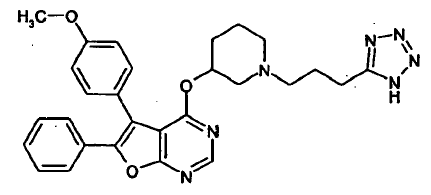 | LC-MS (Methode 5): Rₜ = 1.88 min.; m/z = 512 (M+H)⁺ ¹H-NMR (400 MHz, DMSO-d₆): δ = 8.59 (s, 1H), 7.53 (d, 2H), 7.46-7.35 (m, 5H), 7.00 (d, 2H), 5.78 (s, 0.3H), 5.79-5.71 (m, 1H), 3.81 (s, 3H), 2.90-2.85 (m, 1H), 2.61-2.56 (m, 1H), 2.30 (t, 2H), 2.19-2.04 (m, 2H), 1.95-1.86 (m, 1H), 1.79-1.65 (m, 2H), 1.69-1.57 (m, 1H), 1.50-1.40 (m, 1H), 1.32-1.21 (m, 1H). |
| | (*+*/*-*)*-trans* | |
| **95** | 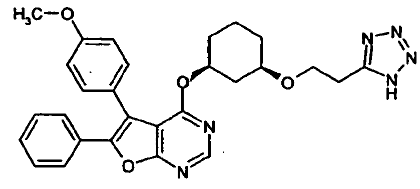 | LC-MS (Methode 6): Rₜ = 2.69 min.; m/z = 513 (M+1-1)⁺ ¹H-NMR (400 MHz, DMSO-d₆): δ = 8.60 (s, 1H), 8.00-7.71 (m, 1H), 7.59-7.50 (m, 2H), 7.44-7.38 (m, 5H), 7.01 (d, 2H), 5.19-5.09 (m, 1H), 3.81 (s, 3H), 3.71-3.62 (m, 2H), 2.82 (t, 2H), 2.47-2.39 (m, 1H), 2.07-1.90 (m, 2H), 1.79-1.68 (m, 1H), 1.32-1.00 (m, 5H). |
| | (*+*/*-*)*-cis* | |
| **96** | 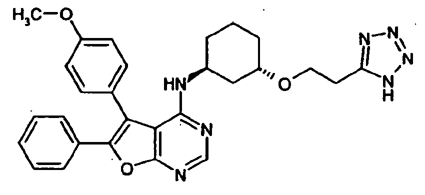 | LC-MS (Methode 3): Rₜ = 2.42 min.; m/z = 512 (M+H)⁺ ¹H-NMR (400 MHz, DMSO-d₆): δ = 8.60 (s, 1H), 7.60-7.52 (m, 2H), 7.41 (d, 2H), 7.31-7.22 (m, 4H), 7.09 (d, 2H), 4.71 (d, 1H), 4.35-4.23 (m, 1H), 3.90 (s, 3H), 3.82-3.78 (m, 1H), 3.70 (s, 1H), 3.40-3.29 (m, 2H), 2.50-2.41 (m, 1H), 1.85-0. 81 (m, 9H). |
| | (*+*/*-*)*-trans* | |
| **97** | 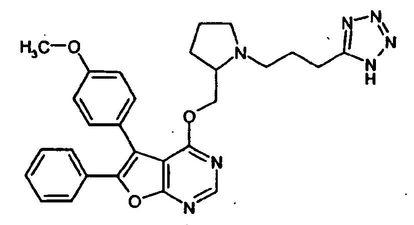 | LC-MS (Methode 8): Rₜ = 1.82 min.; m/z = 512 (M+H)⁺ ¹H-NMR (400 MHz, DMSO-d₆): δ = 8.60 (s, 1H), 7.52 (d, 2H), 7.44-7.32 (m, 5H), 7.00 (d, 2H), 4.43-4.35 (m, 1H), 4.23-4.15 (m, 1H), 3.81 (s, 3H), 3.01-2.94 (m, 1H), 2.72-2.45 (m, 7H), 2.28-2.19 (m, 1H), 2.18-2.07 (m, 1H), 1.79-1.39 (m, 4H). |
| | (+/-) | |
| **98** | 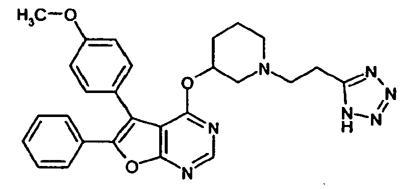 | LC-MS (Methode 3): Rₜ = 1.70 min.; m/z = 498 (M+H)⁺ ¹H-NMR (400 MHz, DMSO-d₆): δ= 8.59 (s, 1H), 7.54 (d, 2H), 7.45-7.32 (m, 5H), 6.98 (d, 2H), 5.76 (s, 0.3H), 5.30-5.21 (m, 1H), 3.80 (s, 3H), 3.01 (t, 2H), 2.88 (d, 1H), 2.73 (t, 2H), 2.42-2.28 (m, 2H), 1.90-1.82 (m, 1H), 1.64-1.53 (m, 1H), 1.50-1.20 (m, 3H). |
| | (+/-) | |
| **99** | 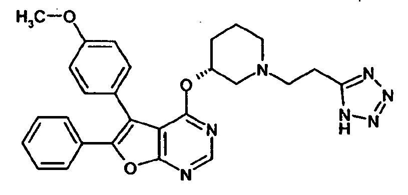 | LC-MS (Methode 3): Rₜ = 1.71 min.; m/z = 498 (M+H)⁺ ¹H-NMR (400 MHz, DMSO-d₆): δ = 8.59 (s, 1H), 7.53 (d, 2H), 7.45-7.32 (m, 5H), 6.98 (d, 2H), 5.31-5.22 (m, 1H), 3.81 (s, 3H), 3.08 (s, 2H), 2.93 (d, 1H), 2.80 (t, 2H), 2.65-2.36 (m, 4H), 1.92-1.82 (m, 1H), 1.68-1.55 (m, 1H), 1.51-1.30 (m, 2H). |
| | (-)-Enantiomer | |
| **100** | 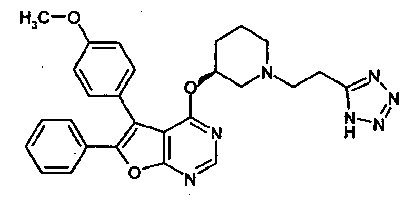 | LC-MS (Methode 6): Rₜ = 1.81 min.; m/z = 498 (M+H)⁺ ¹H-NMR (400 MHz, DMSO-d₆): δ = 8.59 (s,1H), 7.53 (d, 2H), 7.45-7.32 (m, 5H), 6.98 (d, 2H), 5.31-5.22 (m, 1H), 3.81 (s, 3H), 3.08 (s, 2H), 2.93 (d, 1H), 2.80 (t, 2H), 2.65-2.36 (m, 4H), 1.92-1.82 (m, 1H), 1.68-1.55 (m, 1H), 1.51-1.30 (m, 2H). |
| | (+)-Enantiomer | |

### Beispiel 101

### (+)-3-[(3S)-3-{[5-(4-Methoxyphenyl)-6-phenylfuro[2,3-d]pyrimidin-4-yl]oxy}piperidin-1-yl]-propansäure

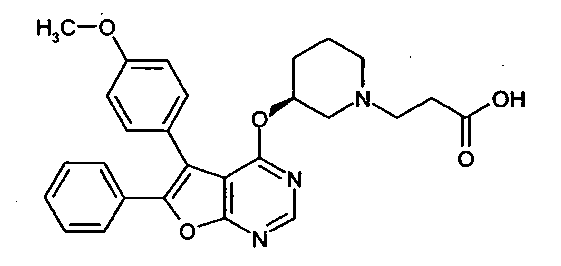

50 mg (0.10 mmol) (+)-3-(3-{[5-(4-Methoxyphenyl)-6-phenylfuro[2,3-d]pyrimidin-4-yl]oxy}-piperidin-1-yl)propannitril werden in 0.5 ml konz. Salzsäure suspendiert und 30 min unter Rückfluss erhitzt. Nach dem Abkühlen wird im Hochvakuum eingeengt und der Rückstand mit 1 N Natronlauge auf pH 7 eingestellt. Die Mischung wird durch präparative RP-HPLC (Eluent: Acetonitril/Wasser-Gradient) gereinigt. Erhalten werden 29.8 mg (57.2% d. Th.) der Zielverbindung.
[α]_{D}²⁰ = +76.1°, c = 0.49, CHCl₃
LC-MS (Methode 8): Rₜ = 1.94 min.; m/z = 474 (M+H)⁺
¹H-NMR (400 MHz, DMSO-d₆): δ = 8.59 (s, 1H), 7.55 (d, 2H), 7.45-7.34 (m, 5H), 7.00 (d, 2H), 5.28-5.19 (m, 1H), 3.80 (s, 3H), 3.60-3.00 (br, 4H), 2.84 (d, 1H), 2.28-2.09 (m, 4H), 1.92-1.81 (m, 1H), 1.64-1.51 (m, 1H), 1.49-1.37 (m, 1H), 1.36-1.21 (m, 1H).

### Beispiel 102

### (-)-3-[(3R)-3-{[5-(4-Methoxyphenyl)-6-phenylfuro[2,3-d]pyrimidin-4-yl]oxy}piperidin-1-yl]-propansäure

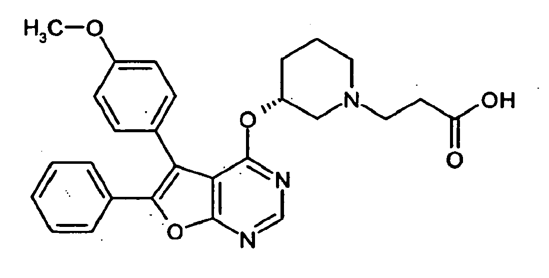

55 mg (0.121 mmol) (-)-3-(3-{[5-(4-Methoxyphenyl)-6-phenylfuro[2,3-d]pyrimidin-4-yl]oxy}-piperidin-1-yl)propannitril werden in 0.55 ml konz. Salzsäure suspendiert und 30 min unter Rückfluss erhitzt. Nach dem Abkühlen wird im Hochvakuum eingeengt und der Rückstand mit 1 N Natronlauge auf pH 7 eingestellt. Die Mischung wird durch präparative RP-HPLC (Eluent: Acetonitril/Wasser-Gradient) gereinigt. Erhalten werden 38.4 mg (67.0% d. Th.) der Zielverbindung.
[α]_{D}²⁰ = -87.9°, c = 0.565, CHCl₃
LC-MS (Methode 3): Rₜ = 1.68 min.; m/z = 474 (M+H)⁺
¹H-NMR (400 MHz, DMSO-d₆): δ = 12.60 (br, 1H), 8.60 (s, 1H), 7.55 (d, 2H), 7.46-7.35 (m, 5H), 7.00 (d, 2H), 5.29-5.20 (m, 1H), 3.80 (s, 3H), 2.82 (d, 1H), 2.61-2.54 (m, 1H), 2.40-2.21 (m, 4H), 2.19 (s, 2H), 1.91-1.81 (m, 1H), 1.65-1.52 (m, 1H), 1.49-1.29 (m, 2H).

### Beispiel 103

### 3-[(2R,4R)-4-{[5-(4-Methoxyphenyl)-6-phenylfuro[2,3-d]pyrimidin-4-yl]oxy}piperidin-2-yl]-propansäure

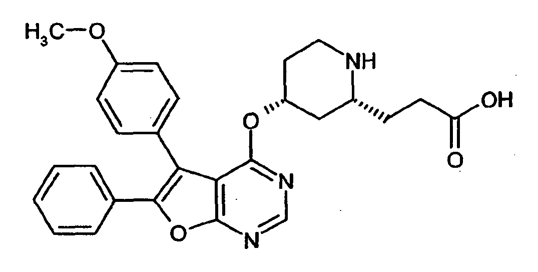

34 mg 3-[(2*R*,4*R*)-1-(*tert*.-Butoxycarbonyl)-4-{[5-(4-methoxyphenyl)-6-phenylro[2,3-d]pyrimidin-4-yl]oxy}piperidin-2-yl]propansäure werden in ca. 0.1 ml einer 3:2-Mischung von Trifluoressigsäure und Dichlormethan 30 min lang bei RT gerührt. Die flüchtigen Komponenten werden danach im Vakuum entfernt und der Rückstand im Hochvakuum getrocknet. Der Rückstand wird in Acetonitril/Wasser aufgenommen und mit 1 N Natronlauge neutral gestellt (pH ca. 7). Der ausgefallene farblose Feststoff wird abgesaugt, zweimal mit Wasser und zweimal mit Acetonitril gewaschen und im Hochvakuum getrocknet. Erhalten werden 20 mg (71.3% d. Th.) der Zielverbindung.

LC-MS (Methode 4): Rₜ = 3.12 min.; m/z = 474 (M+H)⁺
¹H-NMR (400 MHz, DMSO-d₆): δ = 8.59 (s, 1H), 7.55 (d, 2H), 7.45-7.38 (m, 5H), 7.00 (d, 2H), 5.30-5.20 (m, 1H), 3.81 (s, 3H), 3.05-2.99 (m, 1H), 2.31-2.23 (m, 1H), 2.10 (s, 7H), 2.02-1.95 (m, 1H).

### Beispiel 104

### 3-[(2R,4R)-4-{[5-(4-Methoxyphenyl)-6-phenylfuro[2,3-d]pyrimidin-4-yl]oxy}-1-methylpiperidin. 2-yl]propansäure

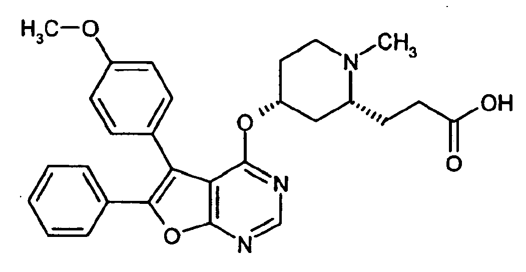

8 mg (17 µmol) 3-[(2*R*,4*R*)-4-{[5-(4-Methoxyphenyl)-6-phenylfuro[2,3-d]pyrimidin-4-yl]oxy}-piperidin-2-yl]propansäure werden in 50 µl Essigsäure gelöst und nacheinander mit 13 µl konz. (ca. 37%) Formalinlösung und 53.7 µg (253 µmol) Natriumtriacetoxyborhydrid versetzt. Die Mischung wird 4 h bei RT gerührt. Danach werden erneut 13 µl konz. Formalinlösung sowie 53.7 µg (253 µmol) Natriumtriacetoxyborhydrid zugegeben und das Gemisch weiter über Nacht gerührt. Die Mischung wird dann direkt durch präparative RP-HPLC (Eluent: AcetonitriWasser-Gradient) aufgereinigt. Erhalten werden 5 mg des Zielprodukts (60.7% d. Th.).
LC-MS (Methode 8): Rₜ = 1.69 min.; m/z = 488 (M+H)⁺
¹H-NMR (400 MHz, DMSO-d₆): δ = 8.59 (s, 1H), 7.55 (d, 2H), 7.43-7.36 (m, 5H), 7.01 (d, 2H), 5.19-5.10 (m, 1H), 3.81 (s, 3H), 2.84-2.79 (m, 1H), 2.66-2.57 (m, 6H), 2.13-1.93 (m, 2H), 1.72-1.58 (m, 2H), 1.48-1.38 (m, 1H), 1.21 (s, 3H).

### Allgemeine Vorschrift G: Palladium-katalysierte Arylierung von 5-Brom-6-phenylfuro[2,3-d]pyrimidin-Derivaten

Zu einer Lösung von 1.0 eq. 5-Brom-6-phenylfuro[2,3-d]pyrimidin-Derivat in DMSO (ca. 0.1 bis 0.5 mol/l) werden bei RT nacheinander 1.2 bis 1.5 eq. der entsprechenden Arylboronsäure und als Base entweder ca. 2.0 eq. Natriumcarbonat (als 2 M wässrige Lösung) oder ca. 1.5 bis 2.5 eq. festes Kaliumcarbonat sowie Methanol (ca. 10 Vol.-%) gegeben. Danach werden unter Argon ca. 5 mol-% Bis(triphenylphosphin)palladium(II)chlorid hinzugefiigt. Die Mischung wird für eine Zeitraum von 3-18 h bei Temperaturen von 70-100°C gerührt. Nach dem Abkühlen wird das Zielprodukt direkt aus der Reaktionslösung durch RP-HPLC (Eluent: Acetonitril/Wasser-Gradient) isoliert. Falls erforderlich, kann eine weitere Aufreinigung durch Chromatographie an Silicagel erfolgen (Laufmittel: Dichlormethan/Methanol- oder Cyclohexan/Ethylacetat-Gemische).

Die folgenden Beispiele werden gemäß der Allgemeinen Vorschrift G erhalten:

| **Beispiel** | **Struktur** | **Analytische Daten** |
|---|---|---|
| **105** | 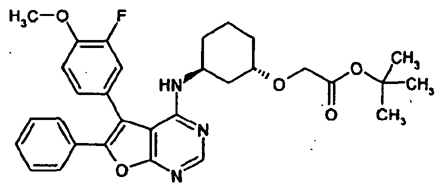 | LC-MS (Methode 6): Rₜ = 3.16 min.; m/z = 548 (M+H)⁺ ¹H-NMR (400 MHz, CDCl₃): δ = 8.41 (s, 1H), 7.58-7.51 (m, 2H), 7.32-7.19 (m, 5H), 7.18-7.10 (m, 1H), 4.59 (d, 1H), 4.50-4.40 (m, 1H), 4.01 (s, 3H), 3.98 (d, 1H), 3.50 (br. s, 1H), 2.01-1.91 (m, 1H), 1.89-1.60 (m, 3H), 1.59 (s, 2H), 1.47 (s, 9H), 1.30-1.17 (m, 3H). |
| | (+/-)-*trans* | |
| **106** | 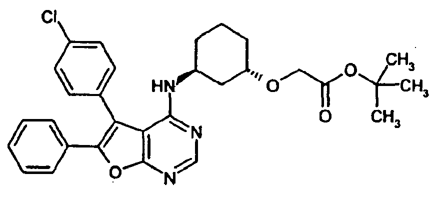 | LC-MS (Methode 8): Rₜ = 3.40 min.; m/z = 534 (M+H)⁺ ¹H-NMR (400 MHz, CDCl₃): δ = 8.40 (s, 1H), 7.58-7.49 (m, 4H), 7.44 (d, 2H), 7.31-7.24 (m, 3H), 4.50-4.40 (m, 2H), 3.99 (d, 2H), 3.48 (br. s, 1H), 2.01-1.91 (m, 1H), 1.88-1.60 (m, 2H), 1.49 (s, 9H), 1.26 (s, 4H), 0.91-0.85 (m, 1H). |
| | (+/-)-*trans* | |
| **107** | 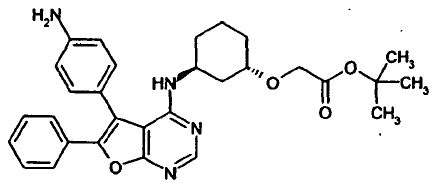 | LC-MS (Methode 8): Rₜ = 3.01 min.; m/z = 515 (M+H)⁺ ¹H-NMR (400 MHz, CDCl₃): δ = 8.39 (s, 1H), 7.60 (d, 2H), 7.30-7.20 (m, 5H), 6.85 (d, 2H), 4.78 (d, 1H), 4.50-4.40 (m, 1H), 4.05-3.88 (m, 3H), 3.40 (br. s, 1H), 1.93-1.82 (m, 1H), 1.80-1.67 (m, 2H), 1.65-1.50 (m, 3H), 1.48 (s, 9H), 1.31-1.19 (m, 3H). |
| | (+/-)-*trans* | |
| **108** | 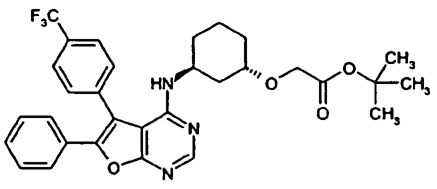 | LC-MS (Methode 8): Rₜ = 3.38 min.; m/z = 568 (M+H)⁺ ¹H-NMR (400 MHz, CDCl₃): δ = 8.43 (s, 1H), 7.72 (d, 2H), 7.68 (d, 2H), 7.53-6.97 (m, 2H), 7.37-7.29 (m, 3H), 4.50-4.40 (m, 1H), 4.38 (d, 1H), 3.97 (s, 2H), 3.46 (br. s, 1H), 2.00-1.90 (m, 1H), 1.88-1.69 (m, 3H), 1.68-1.51 (m, 2H), 1.48 (s, 9H), 1.20-1.09 (m, 2H). |
| | (+/-)-*trans* | |
| **109** | 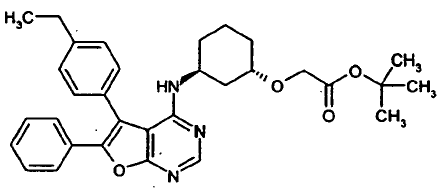 | LC-MS (Methode 3): Rₜ = 3.27 min.; m/z = 528 (M+H)⁺ ¹H-NMR (400 MHz, CDCl₃): δ = 8.39 (s, 1H), 7.59 (d, 2H), 7.42-7.35 (m, 3H), 7.30-7.21 (m, 4H), 4.61 (d, 1H), 4.50-4.40 (m, 1H), 3.95 (d, 2H), 3.42-3.34 (m, 1H), 2.79 (q, 2H), 1.88-1.79 (m, 1H), 1.78-1.62 (m, 2H), 1.60-1.51 (m, 2H), 1.48 (s, 9H), 1.32 (t, 3H), 1.22-1.03 (m, 3H). |
| | (+/-)-*trans* | |
| **110** | 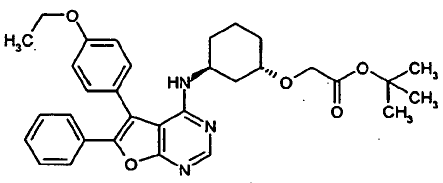 | LC-MS (Methode 6): Rₜ = 3.31 min.; m/z = 544 (M+H)⁺ ¹H-NMR (400 MHz, CDCl₃): δ = 8.40 (s, 1H), 7.58 (d, 2H), 7.39 (d, 2H), 7.30-7.22 (m, 3H); 7.05 (d, 2H), 4.65 (d, 1H), 4.49-4.39 (m, 1H), 4.13 (q, 2H), 3.97 (d, 2H), 3.40 (br. s, 1H), 2.19 (s, 1H), 1.94-1.87 (m, 1H), 1.82-1.66 (m, 2H), 1.62-1.53 (m, 4H), 1.48 (s, 9H), 1.29-1.13 (m, 3H). |
| | (+/-)-*trans* | |
| **111** | 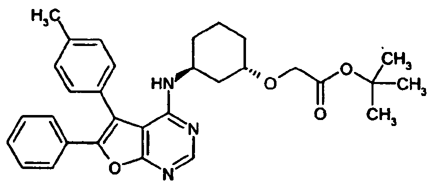 | LC-MS (Methode 3): Rₜ = 3.19 min.; m/z = 514 (M+H)⁺ ¹H-NMR (400 MHz, CDCl₃): δ = 8.40 (s, 1H), 7.60-7.52 (m, 3H), 7.40-7.32 (m, 4H), 7.30-7.21 (m, 2H), 4.61 (d, 1H), 4.48-4.39 (m, 1H), 3.94 (d, 2H), 3.39 (br. s, 1H), 2.50 (s, 3H), 1.90-1.81 (m, 1H), 1.80-1.64 (m, 2H), 1.59 (s, 1H), 1.48 (s, 9H), 1.30-1.11 (m, 4H). |
| | (+/-)-*trans* | |
| **112** | 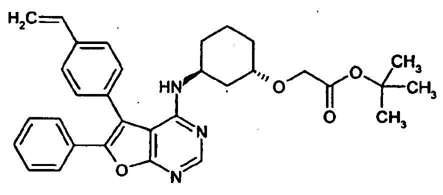 | LC-MS (Methode 6): Rₜ = 3.35 min.; m/z = 526 (M+H)⁺ ¹H-NMR (400 MHz, CDCl₃): δ = 8.43 (s, 1H), 7.88-7.78 (m, 2H), 7.50-7.40 (m, 3H), 7.40-7.30 (m, 1H), 6.97-6.86 (m, 3H), 5.70 (br. s, 1H), 5.52 (d, 1H), 4.64-4.53 (m, 1H), 3.90 (s, 2H), 3.60 (br. s, 1H), 2.12-2.03 (m, 1H), 1.98-1.88 (m, 1H), 1.82-1.71 (m, 1H), 1.64 (s, 4H), 1.50 (s, 9H), 1.26 (s, 2H), 0.91-0.80 (m, 1H). |
| | (+/-)-*trans* | |
| **113** | 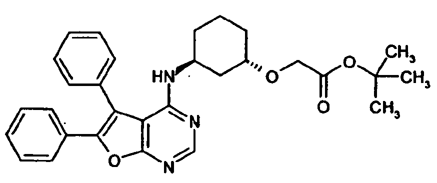 | LC-MS (Methode 3): Rₜ = 3.11 min.; m/z = 500 (M+H)⁺ ¹H-NMR (400 MHz, CDCl₃): δ = 8.40 (s, 1H), 7.60-7.48 (m, 7H), 7.31-7.22 (m, 3H), 4.54 (d, 1H), 4.48-4.39 (m, 1H), 3.96 (d, 2H), 3.40 (br. s, 1H), 1.92-1.83 (m, 1H), 1.80-1.64 (m, 2H), 1.58 (s, 3H), 1.47 (s, 9H), 1.23-1.10 (m, 2H). |
| | (+/-)-*trans* | |
| **114** | 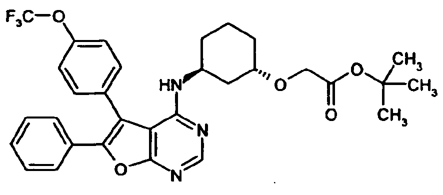 | LC-MS (Methode 6): Rₜ = 3.35 min.; m/z = 584 (M+H)⁺ ¹H-NMR (400 MHz, CDCl₃): δ = 8.41 (s, 1H), 7.58-7.49 (m, 4H), 7.42 (d, 2H), 7.31-7.29 (m, 3H), 4.50-4.38 (m, 2H), 3.96 (d, 2H), 3.49 (br. s, 1H), 1.98-1.90 (m, 1H), 1.86-1.69 (m, 2H), 1.68-1.60 (m, 1H), 1.55 (s, 2H), 1.48 (s, 9H), 1.21-1.09 (m, 2H). |
| | (+/-)-*trans* | |
| **115** | 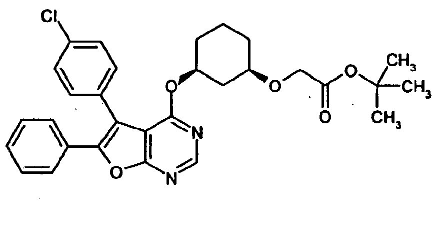 | LC-MS (Methode 3): Rₜ = 3.35 min.; m/z = 535 (M+H)⁺ ¹H-NMR (400 MHz, DMSO-d₆): δ = 8.61 (s, 1H), 7.55-7.49 (m, 6H), 7.48-7.40 (m, 3H), 5.18-5.08 (m, 1H), 3.99 (s, 2H), 3.48-3.38 (m, 1H), 2.45-2.3 (m, 1H), 2.04-1.90 (m, 2H), 1.78-1.69 (m, 1H), 1.40 (s, 9H), 1.29-1.04 (m, 4H). |
| **116** | 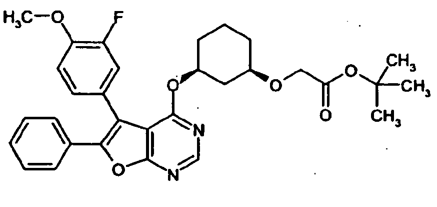 | LC-MS (Methode 3): Rₜ = 3.40 min.; m/z = 547 (M+H)⁺ ¹H-NMR (400 MHz, DMSO-d₆): δ = 8.61 (s, 1H), 7.59-7.51 (m, 2H), 7.48-7.40 (m, 3H), 7.39-7.29 (m, 2H), 7.22-7.19 (m, 1H), 5.18-5.07 (m, 1H), 3.98 (s, 2H), 3.47-3.38 (m, 1H), 2.71 (q, 2H), 2.45-2.37 (m, 1H), 2.06-1.89 (m, 2H), 1.78-1.69 (m, 1H), 1.39 (s, 9H), 1.29-1.01 (m, 7H). |
| **117** | 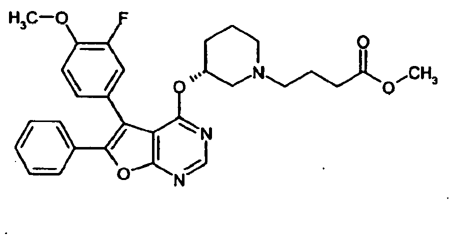 | LC-MS (Methode 8): Rₜ = 1.86 min.; m/z = 520 (M+H)⁺ ¹H-NMR (400 MHz, DMSO-d₆): δ = 8.60 (s, 1H), 7.59-7.51 (m, 2H), 7.47-7.39 (m, 5H), 7.24-7.18 (m, 2H), 7.30-7.20 (m, 1H), 3.90 (s, 3H), 3.52 (s, 2H), 2.80-2.70 (m, 1H), 2.31-2.20 (m, 5H), 2.19-2.11 (m, 1H), 1.93-1.81 (m, 1H), 1.68-1.53 (m, 2H), 1.50-1.30 (m, 2H). |
| **118** | 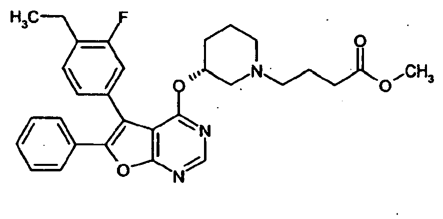 | LC-MS (Methode 8): Rₜ = 2.01 min.; m/z = 518 (M+H)⁺ ¹H-NMR (400 MHz, DMSO-d₆): δ = 8.60 (s, 1H), 7.59-7.51 (m, 2H), 7.48-7.39 (m, 3H), 7.38-7.30 (m, 2H), 7.20 (d, 1H), 5.29-5.20 (m, 1H), 3.50 (s, 3H), 2.75-2.61 (m, 3H), 2.42-2.35 (m, 1H), 2.35-2.12 (m, 5H), 1.90-1.80 (m, 1H), 1.66-1.49 (m, 3H), 1.48-1.30 (m, 2H), 1.29-1.15 (m, 4H). |
| **119** | 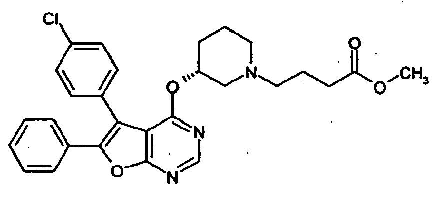 | LC-MS (Methode 8): Rₜ = 1.88 min.; m/z = 506 (M+H)⁺ ¹H-NMR (400 MHz, DMSO-d₆): δ = 8.60 (s, 1H), 7.56-7.47 (m, 6H), 7.48-7.39 (m, 3H), 5.30-5.20 (m, 1H), 3.52 (s, 3H), 2.78-2.69 (m, 1H), 2.48-2.39 (m, 1H), 2.30-2.20 (m, 5H), 2.20-2.11 (m, 1H), 1.90-1.81 (m, 1H), 1.66-1.50 (m, 3H), 1.49-1.29 (m, 2H). |

### Beispiel 120

### (-)-{[(3-{[5-(4-Ethylphenyl)-6-phenylfuro[2,3-d]pyrimidin-4-yl]oxy}cyclohexyl]oxy}essigsäure-tert.-butylester

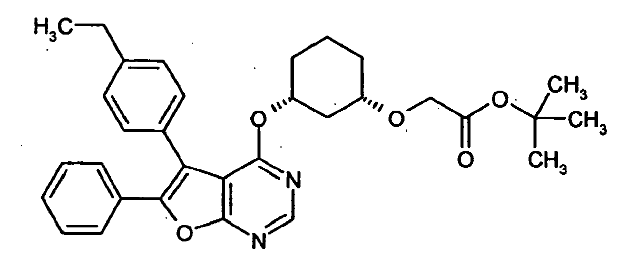

Zu einer Mischung von 40 mg (0.174 mmol) (-)-*cis*-{[3-Hydroxycyclohexyl]oxy}-essigsäure-*tert*.-butylester und 58.15 mg (0.174 mmol) 4-Chlor-5-(4-ethylphenyl)-6-phenylfuro[2,3-d]pyrimidin in 0.3 ml DMF werden unter Kühlung 0.104 ml (0.208 mmol) Phosphazen-Base P2-t-Bu (2 M Lösung in THF) gegeben. Die Mischung wird eine Stunde bei RT gerührt. Danach wird Wasser zugegeben und mit Dichlormethan extrahiert. Die organische Phase wird mit pH 7-Pufferlösung und mit ges. Natriumchlorid-Lösung gewaschen, über Natriumsulfat getrocknet und im Vakuum eingeengt. Aus dem Rückstand werden 45.6 mg (49.7% d. Th.) der Zielverbindung durch präparative RP-HPLC (Eluent: Acetonitril/Wasser) isoliert.
[α]_{D}²⁰ = -56.7°, c = 0.485, CHCl₃
LC-MS (Methode 3): Rₜ = 3.41 min.; m/z = 529 (M+H)⁺
¹H-NMR (400 MHz, DMSO-d₆): δ = 8.60 (s, 1H), 7.58-7.50 (m, 2H), 7.43-7.35 (m, 5H), 7.29. (d, 2H), 5.18-5.07 (m, 1H), 3.90 (s, 2H), 3.46-3.36 (m, 1H), 2.70 (q, 2H), 2.44-2.36 (m, 1H), 2.05-1.90 (m, 2H), 1.78-1.69 (m, 1H), 1.41 (s, 9H), 1.25 (t, 3H), 1.20-1.01 (m, 4H).

### Beispiel 121

### (+)-{[(3-{[5-(4-Ethylphenyl)-6-phenylfuro[2,3-d]pyrimidin-4-yl]oxy}cyclohexyl]oxy}essigsäure-tert.-butylester

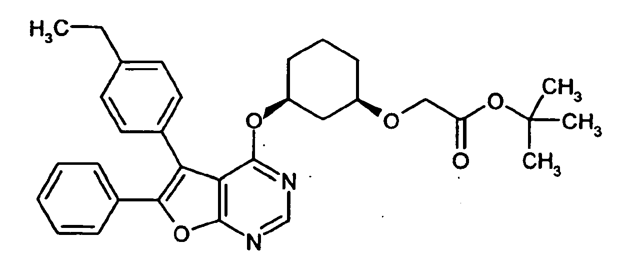

Die Titelverbindung wird analog zu Beispiel 120 durch Umsetzung von (+)-*cis*-{[3-Hydroxycyclohexyl]oxy}-essigsäure-*tert*.-butylester mit 4-Chlor-5-(4-ethylphenyl)-6-phenylfuro[2,3-d]pyrimidin erhalten.
[α]_{D}²⁰ = +54.7°, c = 0.505, CHCl₃
LC-MS (Methode 3): Rₜ = 3.41 min.; m/z = 529 (M+H)⁺
¹H-NMR (400 MHz, DMSO-d₆): δ = 8.60 (s, 1H), 7.58-7.50 (m, 2H), 7.43-7.35 (m, 5H), 7.29 (d, 2H), 5.18-5.07 (m, 1H), 3.90 (s, 2H), 3.46-3.36 (m, 1H), 2.70 (q, 2H), 2.44-2.36 (m, 1H), 2.05-1.90 (m, 2H), 1.78-1.69 (m, 1H), 1.41 (s, 9H), 1.25 (t, 3H), 1.20-1.01 (m, 4H).

### Beispiel 122

### (-)-4-[(3R)-3-{[5-(4-Ethylphenyl)-6-phenylfuro[2,3-d]pyrimidin-4-yl]oxy}piperidin-1-yl]butansäuremethylester

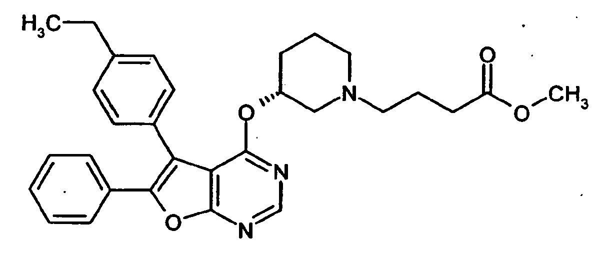

Zu einer Mischung von 798 mg (2.39 mmol) 4-Chlor-5-(4-ethylphenyl)-6-phenylfuro[2,3-d]pyrimidin und 600 mg (2.981 mmol) (-)-4-[(3*R*)-3-Hydroxypiperidin-1-yl]butansäuremethylester in 2 ml DMF werden unter Eiskühlung 1.55 ml (3.10 mmol) Phosphazen-Base P4-t-Bu (1 M Lösung in Hexan) gegeben. Nach 2 h bei RT werden weitere 220 mg (-)-4-[(3*R*)-3-Hydroxypiperidin- 1 -yl]-butansäuremethylester sowie 0.57 ml Phosphazen-Base P4-t-Bu (1 M Lösung in Hexan) hinzugefügt und die Mischung für weitere 2 h bei RT gerührt. Zur Aufarbeitung wird das Gemisch mit Dichlormethan verdünnt, mit Wasser gewaschen, über Natriumsulfat getrocknet und im Vakuum konzentriert. Nach Reinigung des Rückstands durch präparative RP-HPLC (Eluent: Acetonitril/Wasser-Gradient) werden 548.4 mg des Zielprodukts erhalten (46.0% d. Th.).
[α]_{D}²⁰ = -40.6°, c = 0.505, CHCl₃
LC-MS (Methode 8): Rₜ = 1.95 min.; m/z = 500 (M+H)⁺
¹H-NMR (400 MHz, DMSO-d₆): δ = 8.59 (s, 1H), 7.59-7.52 (m, 2H), 7.46-7.38 (m, 5H), 7.30-7.21 (m, 2H), 5-.25-5.19 (m, 1H), 3.52 (s, 3H), 2.73-2.65 (m, 3H), 2.44-2.38 (m, 1H), 2.30-2.20 (m, 5H), 2.19-2.05 (m, 1H), 1.90-1.80 (m, 1H), 1.64-1.50 (m, 3H), 1.44-1.39 (m, 2H), 1.23 (t, 3H).

### Beispiel 123

### 4-[(3S)-3-{[5-(4-Ethylphenyl)-6-phenylfuro[2,3-d]pyrimidin-4-yl]oxy}piperidin-1-yl]butansäuremethylester

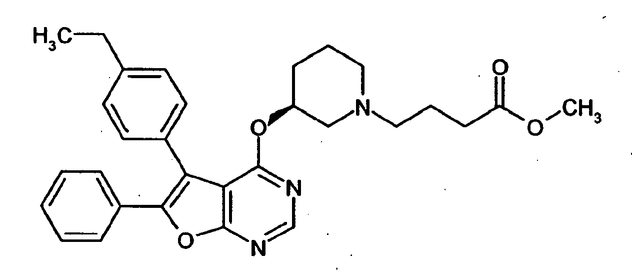

Zu einer Lösung von 70 mg (0.148 mmol) (+)-4-{(3*S*)-3-[(5-Brom-6-phenylfuro[2,3-d]pyrimidin-4-yl)oxy]piperidin-1-yl}butansäuremethylester in 0.4 ml DMSO werden unter Argon nacheinander 5.2 mg (0.007 mmol) Bis(triphenylphosphin)palladium(II)chlorid, 30.6 mg (0.221 mmol) Kaliumcarbonat, 0.04 ml Methanol sowie 31 mg (0.207 mmol) 4-Ethylbenzolboronsäure gegeben. Die Mischung wird insgesamt 3.5 h bei 80°C gerührt. Nach dem Abkühlen wird das Reaktionsgemisch direkt durch präparative RP-HPLC (Eluent: Acetonitril/Wasser-Gradient) aufgereinigt. Isoliert werden 37.3 mg (50.6% d. Th.) der Zielverbindung.
LC-MS (Methode 8): Rₜ = 1.86 min.; m/z = 500 (M+H)⁺
¹H-NMR (400 MHz, DMSO-d₆): δ = 8.59 (s, 1H), 7.59-7.52 (m, 2H), 7.46-7.38 (m, 5H), 7.30-7.21 (m, 2H), 5.25-5.19 (m, 1H), 3.52 (s, 3H), 2.73-2.65 (m, 3H), 2.44-2.38 (m, 1H), 2.30-2.20 (m, 5B), 2.19-2.05 (m, 1H), 1.90-1.80 (m, 1H), 1.64-1.50 (m, 3H), 1.44-1.39 (m, 2H), 1.23 (t, 3H).

### Beispiel 124

### rac-(cis/trans)-{[3-{[5-(4-Methoxyphenyl)-6-phenylfuro[2,3-d]pyrimidin-4-yl]oxy}cyclopentyl]-oxy}essigsäure-tert.-butylester

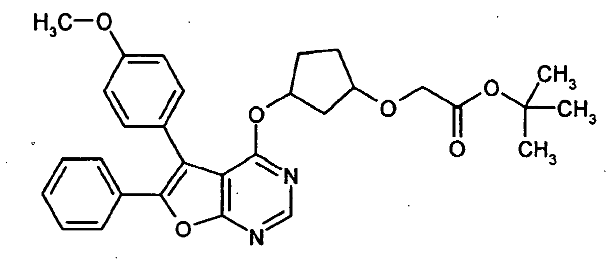

4.97 g (62.1 mmol) 50%-ige Natronlauge und 10 ml Toluol werden bei 40°C nacheinander mit einer Lösung von 2.5 g (6.2 mmol) *rac*-(*cis*/*trans*)-3-{[5-(4-Methoxyphenyl)-6-phenylfuro[2,3-d]-pyrimidin-4-yl]oxy}cyclopentanol in 10 ml Toluol und 10 ml 1,2-Dimethoxyethan, 210.9 mg (0.62 mmol) Tetra-n-butylammoniumhydrogensulfat sowie 1.8 ml (12:4 mmol) Bromessigsäure-*tert*.-butylester versetzt. Die zweiphasige Reaktionsmischung wird bei 60°C insgesamt 3 h lang kräftig gerührt. Nach dem Abkühlen wird die Reaktionsmischung auf Wasser gegeben und mit konz. Salzsäure neutralisiert. Man extrahiert dreimal mit Ethylacetat, vereinigt die organischen Phasen, trocknet über Magnesiumsulfat und engt im Vakuum ein. Aus dem Rückstand werden durch Chromatographie an Silicagel (Laufmittel: Cyclohexan/Ethylacetat 10:1 → 1:1) 300 mg (9.4% d. Th.) der Zielverbindung isoliert.
LC-MS (Methode 3): Rₜ = 3.17 min.; m/z = 517 (M+H)⁺
¹H-NMR (400 MHz, DMSO-d₆): δ = 8.60 (s, 1H), 7.59-7.52 (m, 2H), 7.45-7.36 (m, 5H), 7.08-6.99 (m, 2H), 5.62-5.40 (m, 1H), 3.89 (d, 1H), 3.81 (s, 3H), 2.10-1.60 (m, 6H), 1.40 (d, 9H), 1.10-1.00 (m, 1H), 0.90-0.79 (m, 1H).

### Trennung der cis/trans-Isomere und Enantiomere:

300 mg (0.581 mmol) rac-{cis/trans}-{[3-{[5-(4-Methoxyphenyl)-6-phenylfuro[2,3-d]pyrimidin-4-yl]oxy}cyclopentyl]oxy}essigsäure-tert.-butyl-ester werden durch Chromatographie an chiraler Phase in die Isomere/Enantiomere getrennt (siehe Beispiele 125-128) [Säule: Daicel Chiralpak AD-H 5 µm, 250 mm x 20 mm; Fluss: 15 ml/niin; Detektion: 220 nm; Temperatur: 25°C; Eluent: iso-Hexan/2-Propanol 90:10].

### Beispiel 125

### (-)-cis-{[3-{[5-(4-Methoxyphenyl)-6-phenyfuro[2,3-d]pyrimidin-4-yl]oxy}cyclopentyl]oxy}essigsäure-tert: butylester

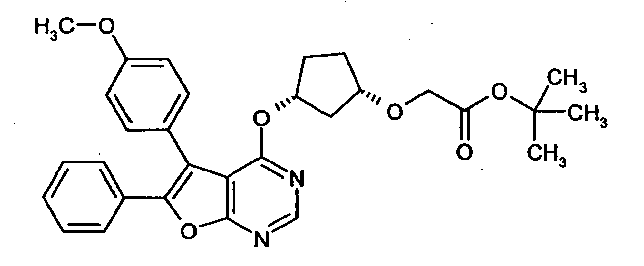

Ausbeute: 75 mg (25.0% d. Th.)
[α]_{D}²⁰ = -24.7°, c = 0.455, CHCl₃
LC-MS (Methode 3): Rₜ = 3.17 min.; m/z = 517 (M+H)⁺
¹H-NMR (400 MHz, DMSO-d₆): δ = 8.59 (s, 1H), 7.56 (d, 2H), 7.47-7.35 (m, 5H), 7.00 (d, 2H), 5.47-5.40 (m, 1H), 4.00-3.92 (m, 1H), 3.88 (d, 2H), 3.80 (s, 3H), 2.37-2.26 (m, 1H), 1.96-1.61 (m, 5H), 1.40 (s, 9H).

### Beispiel 126

### (+)-cis-{{[3-{[5-(4-Methoxyphenyl)-6-phenylfuro[2,3-d]pyrimidin-4-yl]oxy}cyclopentyl]oxy}essigsäure-tert.-butylester

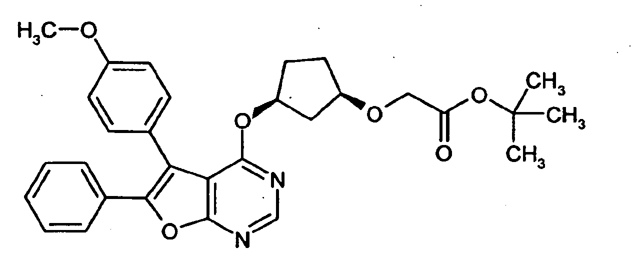

Ausbeute: 57 mg (19.0% d. Th.)
[α]_{D}²⁰ = +24.2°, c = 0.48, CHCl₃.

### Beispiel 127

### (+)-trans-{[3-{[5-(4-Methoxyphenyl)-6-phenylfuro[2,3-d]pyrimidin-4-yl]oxy}cyclopentyl]oxy}-essigsäure-tert:-butylester

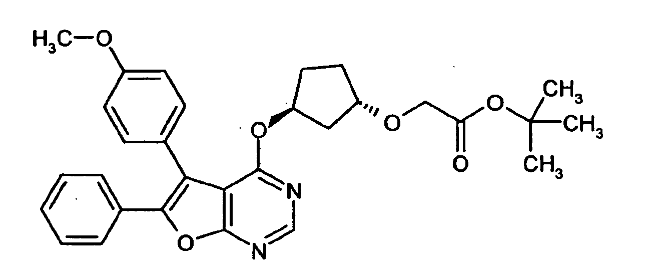

Ausbeute: 23 mg (7.7% d. Th.)
[α]_{D}²⁰ = +32.6°, c = 0.48, CHCl₃
LC-MS (Methode 6): Rₜ = 3.31 min.; m/z = 517 (M+H)⁺
¹H-NMR (400 MHz, DMSO-d₆): δ = 8.59 (s, 1H), 7.56 (d, 2H), 7.45-7.36 (m, 5H), 7.01 (d, 2H), 5.63-5.58 (m, 1H), 3.97-3.90 (m, 1H), 3.89 (s, 2H), 3.82 (s, 3H), 2.10-1.84 (m, 3H), 1.76-1.57 (m, 3H), 1.42 (s, 9H).

### Beispiel 128

### (-)-trans-{[3-{[5-(4-Methoxyphenyl)-6-phenylfuro[2,3-d]pyrimidin-4-yl]oxy}cyclopentyl]oxy}-essigsäure-tert.-butylester

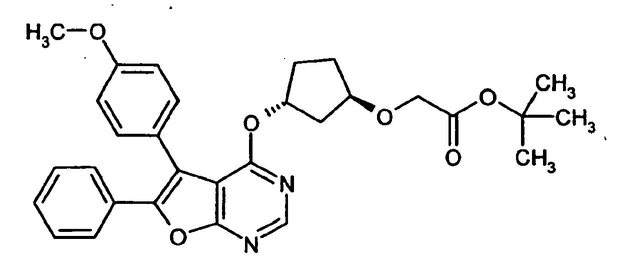

Ausbeute: 39 mg (13.0% d. Th.)
[α]_{D}²⁰ = -30.1°, c = 0.54, CHCl₃.

### Beispiel 129

### (+)-cis-{[(1R,3S)-3-{[5-(4-Methoxyphenyl)-6-phenylfuro[2,3-d]pyrimidin-4-yl]oxy}cyclopentyl]-oxy}essigsäure-tert.-butylester

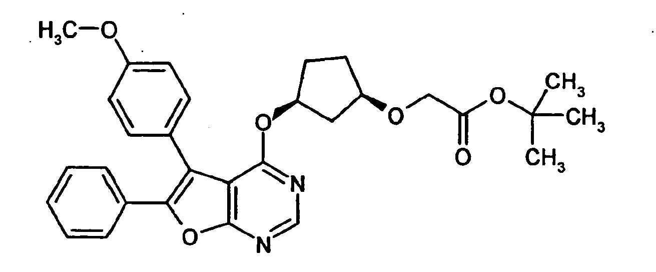

233.6 mg (0.694 mmol) 4-Chlor-5-(4-methoxyphenyl)-6-phenylfuro[2,3-d]pyrimidin und 150 mg (0.694 mmol) *cis*-(-)-{[(1*R*,3*S*)-3-Hydroxycyclopentyl]oxy}essigsäure-*tert*.-butylester werden in 0.35 ml DMF gelöst, auf 0°C gekühlt und mit 0.69 ml (0.69 mmol) Phosphazen-Base P4-t-Bu (1 M Lösung in Hexan) versetzt. Nach 1h Rühren bei RT wird die Reaktionsmischung auf Wasser gegeben, mit 1 N Salzsäure auf pH 7 eingestellt und dreimal mit Dichlormethan extrahiert. Die organischen Phasen werden vereinigt, mit ges. Natriumchlorid-Lösung gewaschen, über Magnesiumsulfat getrocknet und im Vakuum eingeengt. Nach Reinigung durch präparative RP-HPLC (Eluent: Acetonitril/Wasser-Gradient) werden 27.2 mg (7.6% d. Th.) der Zielverbindung erhalten.
[α]_{D}²⁰ = +28.4°, c = 0.48, CHCl₃
LC-MS (Methode 3): Rₜ = 3.18 min.; m/z = 517 (M+H)⁺
¹H-NMR (400 MHz, DMSO-d₆): δ = 8.59 (s, 1H), 7.56 (d, 2H), 7.47-7.35 (m, 5H), 7.00 (d, 2H), 5.47-5.40 (m, 1H), 4.00-3.92 (m, 1H), 3.88 (d, 2H), 3.80 (s, 3H), 2.37-2.26 (m, 1H), 1.96-1.61 (m, 5H), 1.40 (s, 9H).

### Beispiel 130 und Beispiel 131

### rac-trans-{[3-{[5-(4-Methoxyphenyl)-6-phenylfuro[2,3-d]pyrimidin-4-yl]amino}cyclopentyl]-oxy}essigsäure-tert.-butylester und rac-cis-{[3-{[5-(4-Methoxyphenyl)-6-phenylfuro[2,3-d]pyrimidin-4-yl]amino}cyclopentyl]oxy}-essigsäure-tert.-butylester

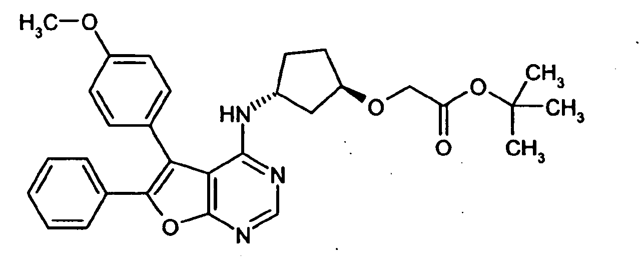

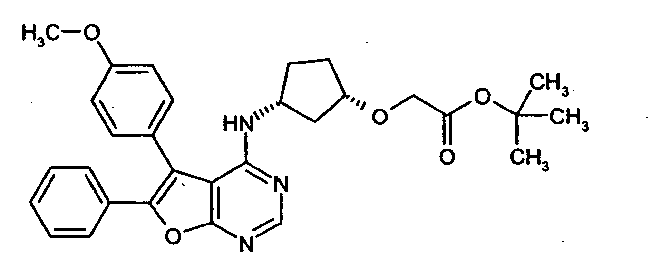

Zu einer Mischung von 560.4 mg (+/-)-*cis*/*trans*-[(3-Aminocyclopentyl)oxy]essigsäure-*tert*.-butylester (Rohprodukt, ca. 2.60 mmol) und 964.3 mg (2.86 mmol) 4-Chlor-5-(4-methoxyphenyl)-6-phenylfuro[2,3-d]pyrimidin in 2.0 ml DMF werden 0.86 ml (5.2 mmol) Diisopropylethylamin gegeben. Man erhitzt das Reaktionsgemisch für 6 h auf 100°C. Nach dem Abkühlen wird Wasser hinzugefügt und mit Dichlormethan extrahiert. Die organische Phase wird mit ges. Natriumhydrogencarbonat-Lösung und ges. Natriumchlorid-Lösung gewaschen, über Natriumsulfat getrocknet und im Vakuum eingeengt. Nach Trocknen im Hochvakuum wird das Produktgemisch durch präparative RP-HPLC (Eluent: Acetonitril/Wasser-Gradient) aufgereinigt und in die *cis*/*trans-*Isomere getrennt.

### rac-trans-Isomer Beispiel 130):

Ausbeute: 153.7 mg (11.5% d. Th.)
LC-MS (Methode 3): Rₜ = 3.02 min.; m/z = 516 (M+H)⁺
¹H-NMR (400 MHz, DMSO-d₆): δ = 8.32 (s, 1H), 7.47-7.39 (m, 4H), 7.38-7.29 (m, 3H), 7.10 (d, 2H), 5.31 (d, 1H), 4.61-4.52 (m, 1H), 3.92 (br. s, 1H), 3.84 (s, 3H), 3.65 (s, 2H), 2.00-1.90 (m, 2H), 1.70-1.60 (m, 2H), 1.52-1.43 (m, 2H), 1.40 (s, 9H).

### rac-cis-Isomer (Beispiel 131):

Ausbeute: 404.1 mg (30.1% d. Th.)
LC-MS (Methode 3): Rₜ = 3.05 min.; m/z = 516 (M+H)⁺
¹H-NMR (400 MHz, DMSO-d₆): δ = 8.35 (s, 1H), 7.51-7.45 (m, 4H), 7.40-7.30 (m, 3H), 7.15 (d, 2H), 4.81 (d, 1H), 4.51-4.40 (m, 1H), 3.90 (br. s, 3H), 3.86 (s, 3H), 2.10-1.99 (m, 2H), 1.81-1.53 (m, 2H), 1.49-1.35 (m, 2H), 1.42 (s, 9H).

### Trennung der racemischen Gemische in die Enantiomere:

350 mg (0.679 mmol) *rac*-*cis*-{[3-{[5-(4-Methoxyphenyl)-6-phenylfuro[2,3-d]pyrimidin-4-yl-amino}cyclopentyl]oxy}essigsäure-*tert*.-butylester bzw. 119 mg (0.231 mmol) *rac-trans-*{[3-{[5-(4-Methoxyphenyl)-6-phenylfuro[2,3-d]pyrimidin-4-yl]amino}cyclopentyl]oxy}essigsäure-*tert*.-butylester werden durch Chromatographie an chiraler Phase jeweils in die Enantiomere getrennt (siehe Beispiele 132-135) [Säule: Sepapak-2 5 µm, 250 mm x 20 mm; Fluss: 15 ml/min; Detektion: 220 nm; Temperatur: 40°C; Eluent: iso-Hexan/2-Propanol 50:50].

### Beispiel 132

### cis-(-)-{[3-{[5-(4-Methoxyphenyl)-6-phenylfuro[2,3-d]pyrimidin-4-yl]amino}cyclopentyl]oxy}-essigsäure-tert.-butykster

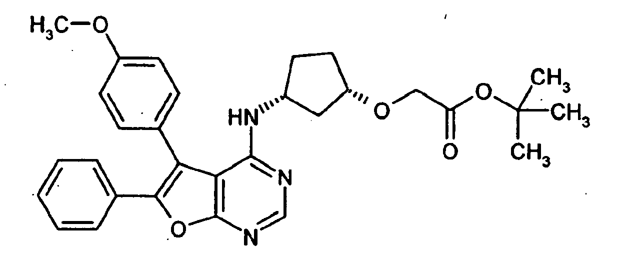

Ausbeute: 165 mg (47. 1 % d. Th.)
[α]_{D}²⁰ = -12.2°, c = 0.455, CHCl₃
LC-MS (Methode 6): Rₜ = 3.20 min.; m/z = 516 (M+H)⁺
¹H-NMR (400 MHz, DMSO-d₆): δ = 8.35 (s, 1H), 7.48-7.40 (m, 4H), 7.39-7.29 (m, 3H), 7.11 (d, 2H), 5.32 (d, 1H), 4.62-4.52 (m, 1H), 3.97-3.90 (m, 1H), 3.82 (s, 3H), 3.68 (s, 2H), 2.00-1.90 (m, 2H), 1.70-1.60 (m, 2H), 1.53-1.44 (m, 2H), 1.40 (s, 9H).

### Beispiel 133

### cis-(+)-{[3-{[5-(4-Methoxyphenyl)-6-phenylfuro[2,3-d]pyrimidin-4-yl]amino}cyclopentyl]oxy}-essigsäure-tert.-butylester

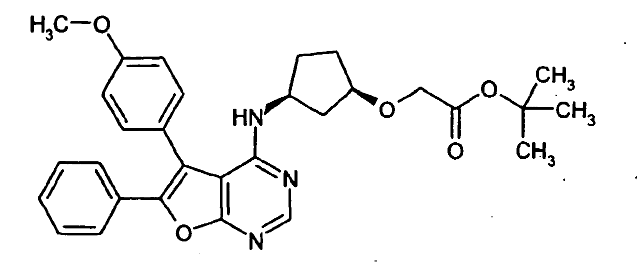

Ausbeute: 163 mg (46.6% d. Th.)
[α]_{D}²⁰ " +8.4°, c = 0.51, CHCl₃
LC-MS (Methode 6): Rₜ = 3.20 min.; m/z = 516 (M+H)⁺
¹H-NMR (400 MHz, DMSO-d₆): δ = 8.34 (s, 1H), 7.47-7.39 (m, 4H), 7.38-7.29 (m, 3H), 7.10 (d, 2H), 5.32 (d, 1H), 4.61-4.51 (m, 1H), 3.97-3.90 (m, 1H), 3.83 (s, 3H), 3.66 (s, 2H), 2.00-1.89 (m, 2H), 1.70-1.60 (m, 2H), 1.53-1.47 (m, 1H), 1.40 (s, 9H), 0.90-0.79 (m, 1H).

### Beispiel 134

### trans-(+)-{[3-{[5-(4-Methoxyphenyl)-6-phenylfuro[2,3-d]pyrimidin-4-yl]amino}cyclopentyl]oxy}-essigsäure-tert.-butylester

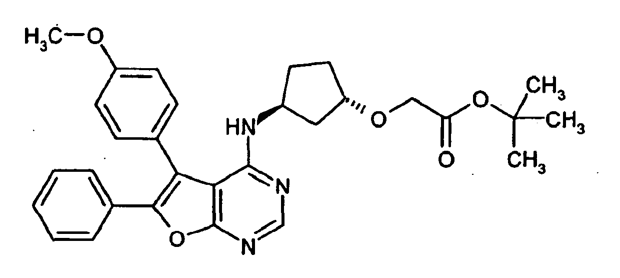

Ausbeute: 54 mg (45.4% d. Th.)
[α]_{D}²⁰= +29.5°, c = 0.46, CHCl₃
¹H-NMR (400 MHz, DMSO-d₆): δ = 8.35 (s, 1H), 7.50-7.45 (m, 4H), 7.40-7.30 (m, 3H), 7.16 (d, 2H), 4.80 (d, 1H), 4.50-4.40 (m, 1H), 3.95-3.39 (m, 3H), 3.86 (s, 3H), 2.08-1.98 (m, 2H), 1.81-1.71 (m, 1H), 1.63-1.54 (m, 1H), 1.42 (s, 9H), 1.40-1.32 (m, 1H), 1.21-1.10-(m, 1H).

### Beispiel 135

### trans-(-)-{[3-{[5-(4-Methoxyphenyl)-6-phenylfuro[2,3-d]pyrimidin-4-yl]amino}cyclopentyl]oxy}-essigsäure-tert.-butylester

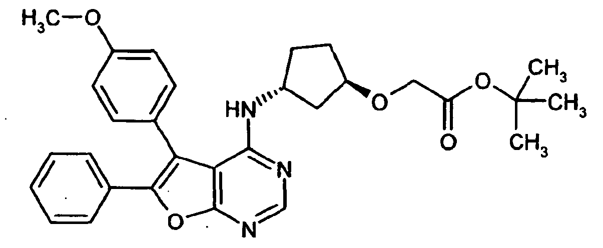

Ausbeute: 50 mg (42.0% d. Th.)
[α]_{D}²⁰ = -30.3°, c = 0.52, CHCl₃
¹H-NMR (400 MHz, DMSO-d₆): δ = 8.36 (s, 1H), 7.51-7.44 (m, 4H), 7.40-7.30 (m, 3H), 7.17 (d, 2H), 4.81 (d, 1H), 4.51-4.40 (m, 1H), 3.95-3.89 (m, 3H), 3.85 (s, 3H), 2.10-1.99 (m, 2H), 1.81-1.71 (m, 1H), 1.65-1.54 (m, 1H), 1.42 (s, 9H), 1.40-1.35 (m, 1H), 1.21-1.11 (m, 1H).

### Beispiel 136

### cis-(+/-)-{[4-{[5-(4-Ethylphenyl)-6-phenylfuro[2,3-d]pyrimidin-4-yl]oxy}cyclopent-2-en-1-yl]-oxy}essigsäure-tert.-butylester

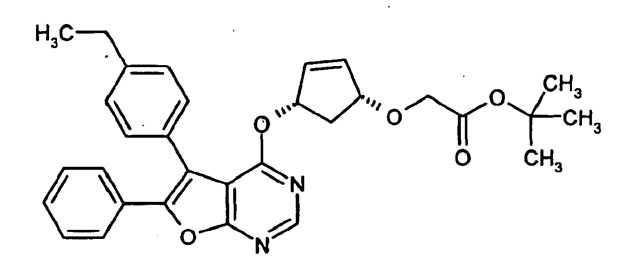

390.7 mg (1.17 mmol) 4-Chlor-5-(4-ethylphenyl)-6-phenylfuro[2,3-d]pyrimidin und 250 mg (1.17 mmol) *cis*(+/-)-{[(4-Hydroxycyclopent-2-en-1-yl]oxy}essigsäure-*tert*.-butylester werden in 0.95 ml DMF gelöst, auf 0°C gekühlt und mit 0.58 ml (1.17 mmol) Phosphazen-Base P2-t-Bu (2 M Lösung in THF) versetzt. Nach Ende der Zugabe wird die Mischung auf RT erwärmt und 1 h nachgerührt. Die Reaktionsmischung wird dann auf Wasser gegeben, mit 1 N Salzsäure auf pH 7 eingestellt und dreimal mit Dichlormethan extrahiert. Man vereinigt die organischen Phasen, wäscht mit ges. Natriumchlorid-Lösung, trocknet über Magnesiumsulfat und konzentriert im Vakuum. Das Rohprodukt wird durch Chromatographie an Silicagel (Laufmittel: Cyclohexan/Ethylacetat 20:1 → 5:1) isoliert. Erhalten werden 510 mg (85.3% d. Th.) der Zielverbindung.
LC-MS (Methode 8): Rₜ = 3.47 min.; m/z = 513 (M+H)⁺
¹H-NMR (400 MHz, DMSO-d₆): δ = 8.61 (s, 1H), 7.49-7.51 (m, 2H), 7.44-7.35 (m, 5H), 7.28 (d, 2H), 6.13 (dd, 2H), 5.84-5.79 (m, 1H), 4.53-4.48 (m, 1H), 3.93 (s, 2H), 2.88-2.79 (m, 1H), 2.68 (q, 2H), 1.52 (td, 1H), 1.40 (s, 9H), 1.23 (t, 3H).

### Beispiel 137

### cis-(-)-{[4-{[5-(4-Ethylphenyl)-6-phenylfuro[2,3-d]pyrimidin-4-yl]oxy}cyclopent-2-en-1-yl]oxy}-essigsäure-tert.-butylester

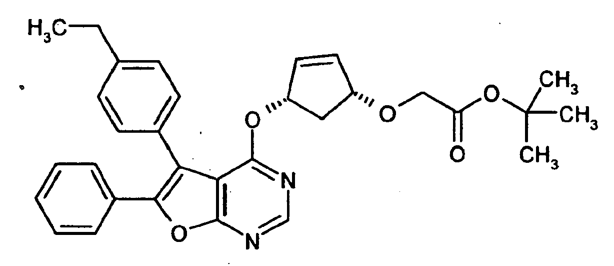

125 mg (0.373 mmol) 4-Chlor-5-(4-ethylphenyl)-6-phenylfuro[2,3-d]pyrimidin und 80 mg (0.373 mmol) *cis-*(+)-{[(4-Hydroxycyclopent-2-en-1-yl]oxy}essigsäure-*tert*.-butylester werden in 0.19 ml DMF gelöst, auf 0°C gekühlt und mit 0.19 ml (0.373 mmol) Phosphazen-Base P2-t-Bu (2 M Lösung in THF) versetzt. Nach Ende der Zugabe wird die Mischung auf RT erwärmt und 1 h nachgerührt. Die Reaktionsmischung wird dann auf Wasser gegeben, mit 1 N Salzsäure auf pH 7 eingestellt und dreimal mit Dichlormethan extrahiert. Man vereinigt die organischen Phasen, wäscht mit ges. Natriumchlorid-Lösung, trocknet über Magnesiumsulfat und konzentriert im Vakuum. Das Rohprodukt wird durch präparative RP-HPLC (Eluent: Acetonitril/Wasser-Gradient) gereinigt. Erhalten werden 140.5 mg (73.4% d. Th.) der Zielverbindung.
[α]_{D}²⁰ = -92.2°, c = 0.515, CHCl₃
LC-MS (Methode 12): Rₜ = 3.37 min.; m/z = 513 (M+H)⁺
¹H-NMR (400 MHz, DMSO-d₆): δ = 8.61 (s, 1H), 7.58-7.52 (m, 2H), 7.42-7.37 (m, 5H), 7.28 (d, 2H), 6.12 (dd, 2H), 5.85-5.79 (m, 1H), 4.53-4.49 (m, 1H), 3.92 (s, 2H), 2.88-2.79 (m, 1H), 2.69 (q, 2H), 1.53 (td, 1H),1.40 (s, 9H), 1.23 (t, 3H).

### Beispiel 138

### cis-(-)-{[4-{[5-(4-Ethylphenyl)-6-phenylfuro[2,3-d]pyrimidin-4-yl]oxy}cyclopent-2-en-1-yl]oxy}-essigsäure-tert.-butylester

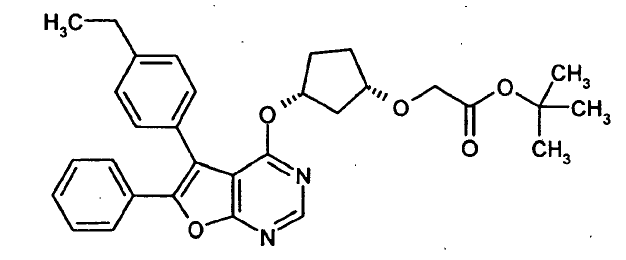

218 mg (0.652 mmol) 4-Chlor-5-(4-ethylphenyl)-6-phenylfuro[2,3-d]pyrimidin und 141 mg (0.652 mmol) *cis-*(+)-{[(1*S*,3*R*)-3-Hydroxycyclopentyl]oxy}essigsäure-*tert*.-butylester werden in 0.19 ml DMF gelöst, auf 0°C gekühlt und mit 0.65 ml (0.65 mmol) Phosphazen-Base P4-t-Bu (1M Lösung in Hexan) versetzt. Nach Ende der Zugabe wird die Mischung auf RT erwärmt und 1h nachgerührt. Die Reaktionsmischung wird dann auf Wasser gegeben, mit 1 N Salzsäure auf pH 7 eingestellt und dreimal mit Dichlormethan extrahiert. Man vereinigt die organischen Phasen, wäscht mit ges. Natriumchlorid-Lösung, trocknet über Magnesiumsulfat und konzentriert im Vakuum. Das Rohprodukt wird durch präparative RP-HPLC (Eluent: Acetonitril/Wasser-Gradient) gereinigt. Erhalten werden 92.1 mg (27.5% d. Th.) der Zielverbindung.
[α]_{D}²⁰.= -36.2°, c = 0.490, CHCl₃
LC-MS (Methode 12): Rₜ = 3.40 min.; m/z = 515 (M+H)⁺
¹H-NMR (400 MHz, DMSO-d₆): δ = 8.60 (s, 1H), 7.54 (d, 2H), 7.45-7.37 (m, 5H), 7.29 (d, 2H), 5.45-5.39 (m, 1H), 4.00-3.94 (m, 1H), 3.81 (d, 2H), 2.69 (q, 2H), 2.34-2.22 (m, 1H), 1.94-1.83 (m, 1H), 1.81-1.71 (m, 1H), 1.70-1.56 (m, 3H), 1.40 (s, 9H), 1.22 (t, 3H).

### Beispiel 139

### trans-(-)-{[4-{[5-(4-Ethylphenyl)-6-phenylfuro[2,3-d]pyrirnidin-4-yl]oxy}cyclopent-2-en-1-yl]-oxy}essigsäure-tert.-butylester

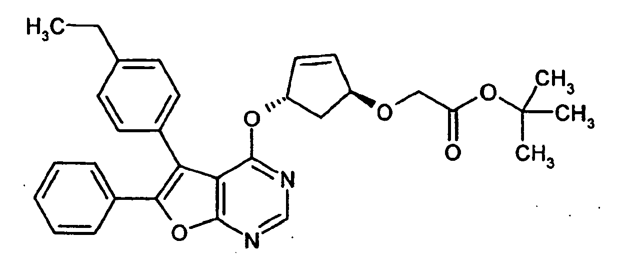

393.9 mg (1.18 mmol) 4-Chlor-5-(4-ethylphenyl)-6-phenylfuro[2,3-d]pyrimidin und 274 mg (80% Reinheit, ca. 1.02 mmol) *trans*-(-)-{[(4-Hydroxycyclopent-2-en-1-yl]oxy}essigsäure-*tert*.-butylester werden in 0.59 ml THF gelöst, auf 0°C gekühlt und langsam mit 1.02 ml (1.02 mmol) Phosphazen-Base P4-t-Bu (1 M Lösung in Hexan) versetzt. Nach 1 h Rühren bei 0°C wird die Reaktionsmischung auf Wasser gegeben. Es wird mit 1 N Salzsäure auf pH 7 eingestellt und dreimal mit Dichlormethan extrahiert. Man vereinigt die organischen Phasen, wäscht mit ges. Natriumchlorid-Lösung, trocknet über Magnesiumsulfat und konzentriert im Vakuum. Das Rohprodukt wird durch präparative RP-HPLC (Eluent: Acetonitril/Wasser-Gradient) gereinigt. Erhalten werden 258.3 mg (42.8% d. Th.) der Zielverbindung.
[α]_{D}²⁰ = -102.7°, c = 0.58, CHCl₃
LC-MS (Methode 8): Rₜ = 3.49 min.; m/z = 513 (M+H)⁺
¹H-NMR (400 MHz, DMSO-d₆): δ = 8.61 (s, 1H), 7.59-7.51 (m, 2H), 7.47-7.31 (m, 5H), 7.30-7.21 (m, 2H), 6.28-6.22 (m, 1H), 6.19-6.09 (m, 2H), 4.67-4.60 (m, 1H), 4.00 (s, 2H), 2.69 (q, 2H), 2.65-2.57 (m, 1H), 2.20-2.10 (m, 1H), 2.05-1.95 (m, 1H), 1.46 (s, 9H), 1.29-1.20 (m, 2H).

### Beispiel 140

### trans-(-)-{[4-{[5-(4-Ethylphenyl)-6-phenylfuro[2,3-d]pyrimidin-4-yl]amino}cyclopent-2-en-1-yl]-oxy}essigsäure-tert.-butylester

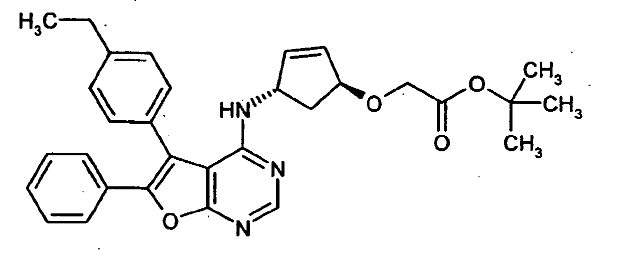

Zu einer Mischung von 128.6 mg (0.384 mmol) 4-Chlor-5-(4-ethylphenyl)-6-phenylfuro[2,3-d]-pyrimidin und 74.5 mg *trans*-{[(4-Aminocyclopent-2-en-1-yl]oxy}essigsäure-*tert*.-butylester (Rohprodukt) in 0.5 ml DMF werden 87 µl (0.524 mmol) Diisopropylethylamin gegeben. Man erhitzt das Reaktionsgemisch für 4.5 h auf 100°C. Nach dem Abkühlen wird Wasser hinzugefügt und mit Dichlormethan extrahiert. Die organische Phase wird mit ges. Natriumhydrogencarbonat-Lösung und ges. Natriumchlorid-Lösung gewaschen, über Natriumsulfat getrocknet und im Vakuum eingeengt. Das Rohprodukt wird durch präparative RP-HPLC (Eluent: Acetonitril/Wasser-Gradient) gereinigt. Erhalten werden 70.5 mg (39.5% d. Th.) der Zielverbindung.
[α]_{D}²⁰ =-195.3°, c = 0.50, CHCl₃
LC-MS (Methode 3): Rₜ = 3.20 min.; m/z = 512 (M+H)⁺
¹H-NMR (400 MHz, DMSO-d₆): δ = 8.39 (s, 1H), 7.52-7.48 (m, 2H), 7.47-7.30 (m, 7H), 6.07-6.0.1 (m, 1H), 5.90 (d, 1H), 5.24-5.16 (m, 1H), 4.65 (d, 1H), 4.59-4.51 (m, 1H), 3.98 (s, 2H), 2.72 (q, 2H), 2.20-2.10 (m, 1H), 1.67-1.58 (m, 1H), 1.42 (s, 9H), 1.27 (t, 3H).

### Beispiel 141

### cis-(+)-{[4-{[5-(4-Ethylphenyl)-6-phenylfuro[2,3-d]pyrimidin-4-yl]oxy}cyclopent-2-en-1-yl]oxy}-essigsäure-tert.-butylester

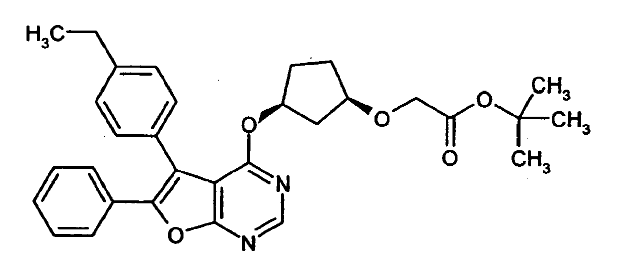

233.6 mg (0.698 mmol) 4-Chlor-5-(4-ethylphenyl)-6-phenylfuro[2,3-d]pyrimidin und 150.9 mg (0.698 mmol) *cis*-(-)-{[(1*R*,3*S*)-3-Hydroxycyclopentyl]oxy}essigssäure-*tert*.-butylester werden in 0.35 ml DMF gelöst, auf 0°C gekühlt und mit 0.7 ml (0.7 mmol) Phosphazen-Base P4-t-Bu (1 M Lösung in Hexan) versetzt. Nach 2 h Rühren bei 0°C wird die Reaktionsmischung auf Wasser gegeben. Es wird mit 1 N Salzsäure auf pH 7 eingestellt und dreimal mit Dichlormethan extrahiert. Man vereinigt die organischen Phasen, wäscht mit ges. Natriumchlorid-Lösung, trocknet über Magnesiumsulfat und konzentriert im Vakuum. Das Rohprodukt wird durch präparative RP-HPLC (Eluent. Acetonitril/Wasser-Gradient) gereinigt. Erhalten werden 60.9 mg (17.0% d. Th.) der Zielverbindung.
[α]_{D}²⁰ = +26.7°, c = 0.475, CHCl₃
LC-MS (Methode 12): Rₜ = 3.39 min.; m/z = 515 (M+H)⁺
¹H-NMR (400 MHz, DMSO-d₆): δ = 8.60 (s, 1H), 7.56 (d, 2H), 7.44-7.38 (m, 5H), 7.29 (d, 2H), 5.45-5.40 (m, 1H), 4.00-3.92 (m, 1H), 3.82 (d, 2H), 2.69 (q, 2H), 2.32-2.25 (m, 1H), 1.92-1.85 (m, 1H), 1.81-1.74 (m, 1H), 1.70-1.58 (m, 3H), 1.40 (s, 9H), 1.22 (t, 3H).

Die folgenden Beispiele werden gemäß der Allgemeinen Vorschrift D oder E (siehe oben) aus den zuvor beschriebenen Verbindungen hergestellt:

| **Beispiel** | **Struktur** | **Analytische Daten** |
|---|---|---|
| **142** | 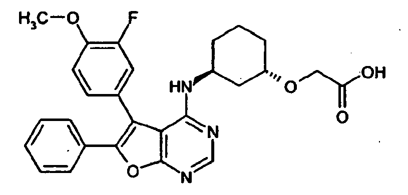 | LC-MS (Methode 6): Rₜ = 2.50 min.; m/z = 492 (M+H)⁺ ¹H-NMR (400 MHz, CDCl₃): δ = 8.40 (s, 1H), 7.59-7.50 (m, 3H), 7.34-7.10 (m, 5H), 4.60-4.53 (m, 1H), 4.49-4.39 (m, 1H), 4.18-4.05 (m, 1H), 4.00 (s, 3H), 3.64-3.50 (m, 1H), 2.11-2.00 (m, 2H), 1.88-1.53 (m, 2H), 1.28 (s, 4H), 0.93-0.80 (m, 2H). |
| | (*+*/*-*)-*trans* | |
| **143** | 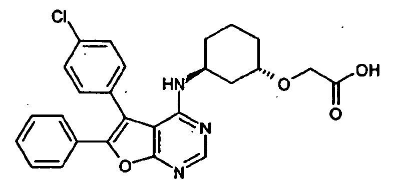 | LC-MS (Methode 6): Rₜ = 2.69 min.; m/z = 478 (M+H)⁺ ¹H-NMR (400 MHz, CDCl₃): δ = 8.40 (s, 1H), 7.59-7.49 (m, 4H), 7.46 (d, 2H), 7.33-7.29 (m, 3H), 4.50-4.38 (m, 2H), 4.20-4.05 (m, 2H), 3.55 (br. s, 1H), 2.10-2.00 (m, 1H), 1.88-1.79 (m, 1H), 1.78-1.62 (m, 2H), 1.61-1.50 (m, 1H), 1.49-1.11 (m, 4H). |
| | (+/-)-*trans* | |
| **144** | 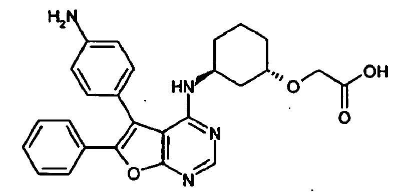 | LC-MS (Methode 6): Rₜ = 2.19 min.; m/z = 459 (M+H)⁺ ¹H-NMR (400 MHz, CDCl₃): δ = 8.40 (s, 1H), 7.57 (d, 2H), 7.33-7.21 (m, 5H), 6.90 (d, 2H), 4.80 (d, 1H), 4.49 (br. s, 1H), 4.10 (dd, 2H), 3.20 (br. s, 1H), 1.88-1.73 (m, 2H), 1.72-1.53 (m, 5H), 1.49-1.38 (m, 1H), 1.36-1.20 (m, 3H). |
| | (*+*/*-*)*-trans* | |
| **145** | 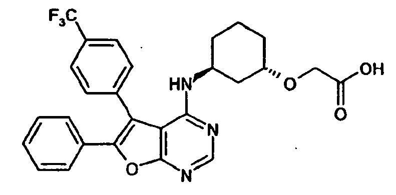 | LC-MS (Methode 6): Rₜ = 2.74 min.; m/z = 512 (M+H)⁺ ¹H-NMR (400 MHz, CDCl₃): δ = 8:43 (s, 1H), 7.83 (d, 2H), 7.65 (d, 2H), 7.51-7.44 (m, 2H), 7.34-7.29 (m, 3H), 4.48-4.38 (m, 1H), 7.34 (d, 1H), 4.01 (dd, 2H), 3.58 (br. s, 1H), 2.11-2.00 (m, 1H), 1.88-1.79 (m, 1H), 1.77-1.63 (m, 2H), 1.58-1.47 (m, 1H), 1.45-1.35 (m, 1H), 1.28 (s, 2H), 1.18-1.04 (m, 1H). |
| | (*+*/*-*)*-trans* | |
| **146** | 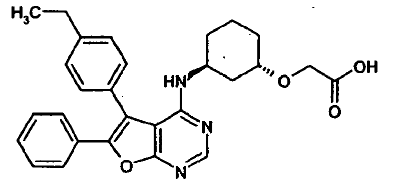 | LC-MS (Methode 6): Rₜ = 2.81 min.; m/z = 472 (M+H)⁺ ¹H-NMR (400 MHz, CDCl₃): δ = 8.40 (s, 1H), 7.60-7.53 (m, 2H), 7.43-7.35 (m, 4H), 7.30-7.22 (m, 3H), 4.60 (d, 1H), 4.48-4.38 (m, 1H), 4.10 (dd, 2H), 3.50 (br. s, 1H), 2.80 (q, 2H), 2.00-1.90 (m, 1H), 1.80-1.45 (m, 5H), 1.32 (t, 3H), 1.29-1.10 (m, 3H). |
| | (*+*/*-*)*-trans* | |
| **147** | 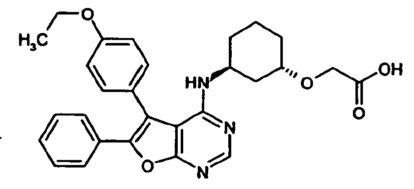 | LC-MS (Methode 8): Rₜ = 2.87 min.; m/z = 488 (M+H)⁺ ¹H-NMR (400 MHz, CDCl₃): δ = 8.40 (s, 1H), 7.57 (d, 2H), 7.40 (d, 2H), 7.32-7.22 (m, 3H), 7.07 (d, 2H), 4.64 (d, 1H), 4.48-4.37 (m, 1H), 4.19-4.02 (m, 4H), 3.49 (br. s, 1H), 2.03-1.94 (m, 1H), 1.80-1.68 (m, 2H), 1.60 (br. s, 2H), 1.51 (t, 3H), 1.39-1.20 (m, 4H). |
| | *(+*/*-)-trans* | |
| **148** | 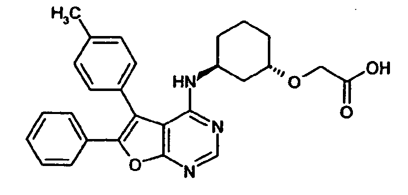 | LC-MS (Methode 6): Rₜ = 2.68 min.; m/z = 458 (M+H)⁺ ¹H-NMR (400 MHz, CDCl₃): δ = 8.40 (s, 1H), 7.59-7.50 (m, 2H), 7.40-7.34 (m, 5H), 7.31-7.21 (m, 2H), 4.60 (d, 1H), 4.48-4.36 (br. m, 1H), 4.19-4.00 (br. m, 2H), 3.50 (br. s, 1H), 2.50 (s, 3H), 2.05-1.92 (m, 1H), 1.81-1.40 (m, 5H), 1.35-1.12 (m, 3H). |
| | (*+*/*-*)*-trans* | |
| **149** | 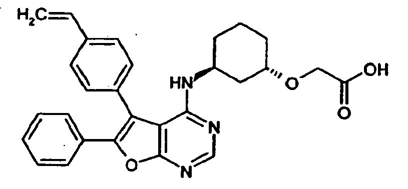 | LC-MS (Methode 3): Rₜ = 2.57 min.; m/z = 470 (M+H)⁺ ¹H-NMR (400 MHz, CDCl₃): δ = 8.49 (s, 1H), 7.62 (d, 2H), 7.55 (s, 2H), 7.49 (d, 2H), 7.29 (d, 2H), 6.82 (dd, 1H), 5.91 (d, 1H), 5.42 (d, 1H), 4.90-4.78 (br. m, 1H), 4.51-4.40 (br. m, 1H), 4.09 (dt, 2H), 3.50 (s, 1H), 2.09-1.90 (m, 1H), 1.89-1.78 (m, 1H), 1.77-1.61 (m, 2H), 1.54-1.42 (m, 1H), 1.41-1.30 (m, 2H), 1.20-1.10 (m, 1H), 0.92-0.80 (m, 1H). |
| | (*+*/*-*)*-trans* | |
| **150** | 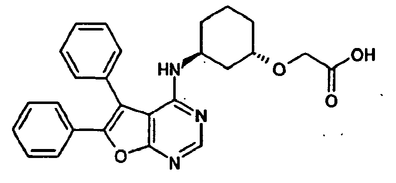 | LC-MS (Methode 6): Rₜ = 2.55 min.; m/z = 444 (M+H)⁺ ¹H-NMR (400 MHz, CDCl₃): δ = 8.42 (s, 1H), 7.60-7.49 (m, 7H), 7.29 (s, 1H), 5.31 (s, 2H), 4.55 (d, 1H), 4.48-4.3 6 (br. m, 1H), 4.10 (dd, 2H), 3.48 (br. s, 1H), 2.02-1.92 (m, 1H), 1.81-1.50 (m, 3H), 1.49-1.39 (m, 1H), 1.38-1.12 (m, 3H). |
| | (*+*/*-*)*-trans* | |
| **151** | 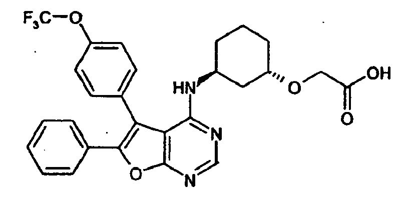 | LC-MS (Methode 3): Rₜ = 2.67 min.; m/z = 528 (M+H)⁺ ¹H-NMR (400 MHz, CDCl₃): δ = 8.42 (s, 1H), 7.56 (d, 2H), 7.52-7.49 (m, 2H), 7.42 (d, 2H), 7.33-7.29 (m, 3H), 4.50-4.38 (m, 2H), 4.11 (dd, 2H), 3.60 (br. s, 1H), 2.11-2.01 (m, 1H), 1.91-1.80 (m, 1H), 1.79-1.62 (m, 2H), 1.58-1.48 (m, 1H), 1.47-1.38 (m, 1H), 1.37-1.22 (m, 1H), 1.17-1.04 (m, 1H). |
| | (*+*/*-*)*-trans* | |
| **152** | 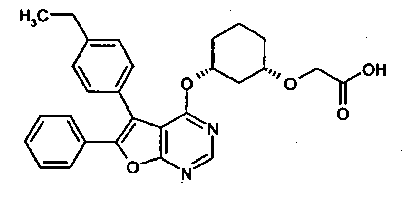 | LC-MS (Methode 3): Pₜ = 2.90 min.; m/z = 473 (M+H)⁺ ¹H-NMR (400 MHz, DMSO-d₆): δ = 12.51 (s, 1H), 8.60 (s, 1H), 7.58-7.51 (m, 2H), 7.43-7.35 (m, 5H), 7.29 (d, 2H), 5.18-5.09 (m, 1H), 4.01 (s, 2H), 3.15-3.08 (m, 1H), 2.74-2.65 (m, 3H), 2.05-1.92 (m; 2H), 1.79-1.69 (m, 1H), 1.24 (t, 3H), 1.20-1.00 (m, 4H). |
| **153** | 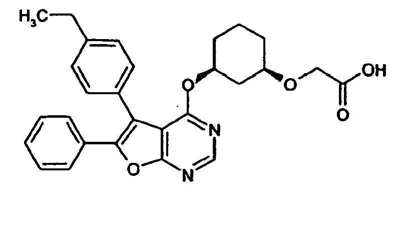 | LC-MS (Methode 3): Rₜ = 2.89 min.; m/z = 473 (M+H)⁺ ¹H-NMR (400 MHz, DMSO-d₆): δ = 12.51 (s, 1H), 8.60 (s, 1H), 7.59-7.51 (m, 2H), 7.43-7.36 (m, 5H), 7.29 (d, 2H), 5.18-5.08 (m, 1H), 4.01 (s, 2H), 3.49-3.39 (m, 1H), 2.70 (q, 2H), 2.05-1.91 (m, 2H), 1.79-1.69 (m, 1H), 1.24 (t, 3H), 1.20-1.00 (m, 5H). |
| **154** | 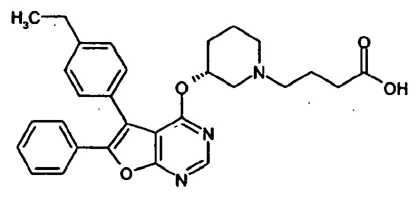 | LC-MS (Methode 6): Rₜ = 1.99 min.; m/z = 486 (M+H)⁺ ¹H-NMR (400 MHz, DMSO-d₆): δ = 8.60 (s, 1H), 7.59-7.51 (m, 2H), 7.43-7.38 (m, 5H), 7.28 (d, 2H), 5.25-5.18 (m, 1H), 3.63-3.59 (m, 1H), 2.80 (d, 1H), 2.70 (q, 2H), 2.25-2.18 (m, 2H), 2.14-2.02 (m, 2H), 1.92-1.72 (m, 4H), 1.61-1.49 (m, 3H), 1.48-1.35 (m, 2H), 1.25 (t, 3H). |
| | (-)-Enantiomer | |
| **155** | 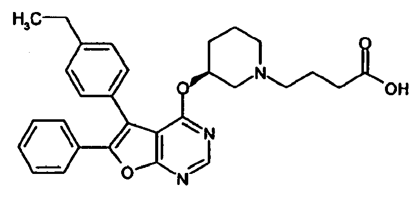 | LC-MS (Methode 8): Rₜ = 1.87 min.; m/z = 486 (M+H)⁺ ¹H-NMR (400 MHz, DMSO-d₆): δ = 8.60 (s, 1H), 7.59-7.51 (m, 2H), 7.43-7.38 (m, 5H), 7.28 (d, 2H), 5.25-5.18 (m, 1H), 3.63-3.59 (m, 1H), 2.80 (d, 1H), 2.70 (q, 2H), 2.25-2.18 (m, 2H), 2.14-2.02 (m, 2H), 1.92-1.72 (m, 4H), 1.61-1.49 (m, 3H), 1.48-1.35 (m, 2H), 1.25 (t, 3H). |
| | (+)-Enantiomer | |
| **156** | 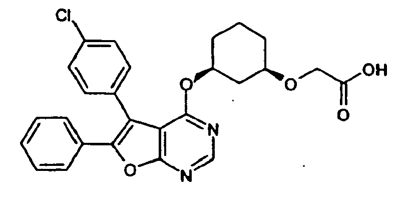 | LC-MS (Methode 3): Rₜ = 2.78 min.; m/z = 479 (M+H)⁺ ¹H-NMR (400 MHz, DMSO-d₆): δ = 8.60 (s, 1H), 7.55-7.49 (m, 6H), 7.56-7.39 (m, 3H), 5.15-5.04 (m, 1H), 3.55 (s, 2H), 2.14 (d, 1H), 2.02-1.90 (m, 2H), 1.75-1.67 (m, 1H), 1.30-1.15 (m, 4H), 1.14-0.95 (m, 2H). |
| **157** | 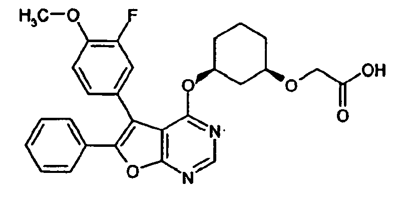 | LC-MS (Methode 8): Rₜ = 3.19 min.; m/z = 491 (M+H)⁺. |
| **158** | 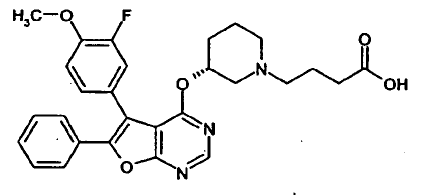 | LC-MS (Methode 3): Rₜ = 1.68 min.; m/z = 506 (M+H)⁺ ¹H-NMR (400 MHz, DMSO-d₆): δ = 8.60 (s, 1H), 7.71-7.68 (m, 1H), 7.66-7.60 (m, 1H), 7.59-7.51 (m, 1H), 7.50-7.35 (m, 4H), 7.23-7.17, (m, 1H), 5.95-5.86 (m, 1H), 5.32-5.21 (m, 1H), 4.70-4.67 (m, 1H), 3.90 (s, 2H), 2.86-2.78 (m, 1H), 2.30-2.07 (m, 3H), 2.00 (s, 1H), 1.95-1.86 (m, 1H), 1.65-1.51 (m, 2H), 1.50-1.40 (m, 1H), 1.40-1.29 (m, 2H). |
| | Enantiomer 1 | |
| **159** | 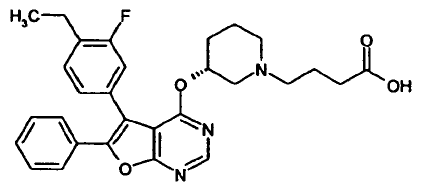 | LC-MS (Methode 8): Rₜ = 1.88 min.; m/z = 504 (M+H)⁺ ¹H-NMR (400 MHz, CDCl₃): δ = 8.51 (s, 1H), 7.65-7.59 (m, 2H), 7.36-7.31 (m, 3H), 7.30-7.18 (m, 3H), 5.40-5.31 (m, 1H), 3.12 (br. s, 1H), 2.86-2.73 (m, 1H), 2.77 (q, 2H), 2.68-2.53 (m, 3H), 2.47 (br. s, 1H), 2.09 (br. s, 1H), 1.83-1.68 (m, 4H), 1.51-1.40 (m, 2H), 1.30 (t, 3H), 1.25 (s, 1H), 0.90-0.81 (m, 1H). |
| | Enantiomer 1 | |
| **160** | 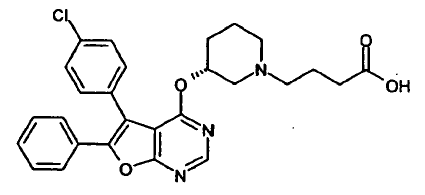 | LC-MS (Methode 8): Rₜ = 1.81 min.; m/z = 492 (M+H)⁺ ¹H-NMR (400 MHz, CDCl₃): δ = 8.54 (s, 1H), 7.61-7.53 (m, 2H), 7.48 (d, 2H), 7.40 (d, 2H), 7.37-7.30 (m, 3H), 5.41-5.32 (m, 1H), 3.08 (br. s, 1H), 2.76 (br. s, 1H), 2.70-2.48 (m, 6H), 2.01 (br. s, 1H), 1.85-1.68 (m, 4H), 1.46 (br. s, 1H), 1.26 (s, 1H). |
| | Enantiomer 1 | |
| **161** | 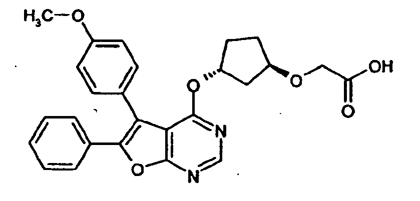 | LC-MS (Methode 8): Rₜ = 2.74 min.; m/z = 461 (M+H)⁺ ¹H-NMR (400 MHz, DMSO-d₆): δ = 8.59 (s, 1H), 7.59-7.51 (m, 2H), 7.44-7.32 (m, 5H), 7.05-6.98 (m, 2H), 5.62-5.55 (m, 1H), 4.11 (s, 1H), 3.71 (d, 2H), 2.14 (d, 1H), 2.05-1.55 (m, 2H). |
| **162** | 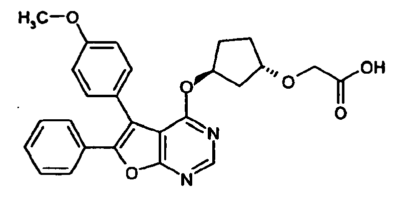 | LC-MS (Methode 8): Rₜ = 2.71 min.; m/z = 461 (M+H)⁺ ¹H-NMR (400 MHz, CDCl₃): δ = 8.50 (s, 1H), 7.67-7.60 (m, 2H), 7.40-7.33 (m, 2H), 7.32-7.28 (m, 3H), 6.99-6.81 (m, 2H), 5.72-5.65 (m, 2H), 5.31 (s, 2H), 4.16 (br. s, 1H), 3.90 (s, 3H), 2.40-2.21 (m, 2H), 2.21-2.10 (m, 1H), 2.06-1.98 (m, 1H), 1.88-1.63 (m, 2H). |
| **163** | 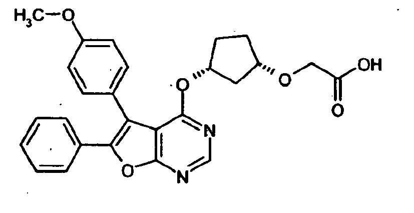 | LC-MS (Methode 8): Rₜ = 2.72 min.; m/z = 461 (M+H)⁺ ¹H-NMR (400 MHz, DMSO-d₆): δ = 8.58 (s, 1H), 7.59-7.51 (m, 2H), 7.45-7.33 (m, 5H), 7.04-6.95 (m, 2H), 5.45-5.38 (m, 1H), 4.06-3.96 (m, 2H), 3.82 (s, 3H), 3.62 (s, 1H), 2.15 (d, 1H), 1.79-1.60 (m, 2H), 1.28 (s, 4H). |
| **164** | 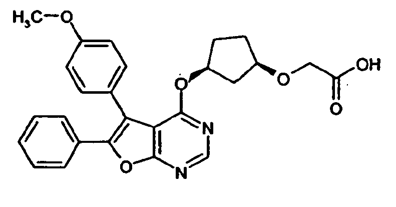 | LC-MS (Methode 3): Rₜ = 2.45 min.; m/z = 461 (M+H)⁺ ¹H-NMR (400 MHz, DMSO-d₆): δ = 8.59 (s, 1H), 7.59-7.52 (m, 2H), 7.46-7.35 (m, 5H), 7.00 (d, 2H), 5.48-5.40 (m, 1H), 4.04-3.97 (m, 1H), 3.91 (s, 2H), 3.82 (s, 2H), 2.39-2.28 (m, 2H), 1.98-1.89 (m, 2H), 1.84-1.76 (m, 1H), 1.75-1.62 (m, 3H). |
| **165** | 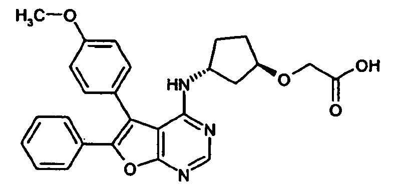 | LC-MS (Methode 3): Rₜ = 2.27 min.; m/z = 460 (M+H)⁺ ¹H-NMR (400 MHz, CDCl₃): δ = 8.42 (s, 1H), 7.55 (d, 2H), 7.40 (d, 2H), 7.31-7.26 (m, 3H), 7.08 (d, 2H), 4.66 (d, 1H), 4.61-4.53 (m, 1H), 4.10-4.00 (m, 3H), 3.91 (s, 3H), 2.29-2.17 (m, 2H), 1.95-1.85 (m, 1H), 1.81-1.71 (m, 1H), 1.50-1.41 (m, 1H), 1.30-1.18 (m, 2H). |
| | (*+*/*-*)*-trans* | |
| **166** | 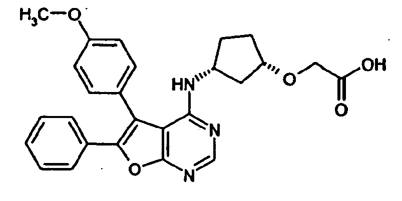 | LC-MS (Methode 3): Ph = 2.25 min.; m/z = 460 (M+H)⁺ ¹H-NMR (400 MHz, DMSO-d₆): δ = 8.41 (s, 1H), 7.52 (d, 2H), 7.39 (d, 2H), 7.30-7.24 (m, 3H), 7.05 (d, 2H), 5.07 (d, 1H), 4.69-4.60 (m, 1H), 4.09-4.03 (m, 1H), 3.90 (s, 3H), 3.88 (d, 2H), 2.20-2.04 (m, 2H), 1.81-1.74 (m, 2H), 1.62-1.48 (m, 3H). |
| | (+/-)-*cis* | |
| **167** | 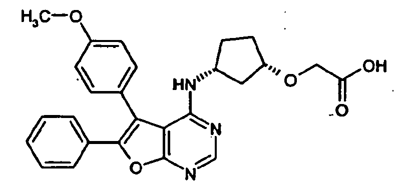 | LC-MS (Methode 6): Rₜ = 2.42 min.; m/z = 460 (M+H)⁺ ¹H-NMR (400 MHz, DMSO-d₆): δ = 12.62 (br. s, 1H), 8.32 (s, 1H), 7.48-7.40 (m, 4H), 7.39-7.29 (m, 3H), 7.11 (d, 2H), 5.31 (d, 1H), 4.61-4.52 (m, 1H), 4.00-3.94 (m, 1H), 3.83 (s, 3H), 3.69 (s, 2H), 2.00-1.88 (m, 2H), 1.70-1.61 (m, 2H), 1.54-1.35 (m, 2H). |
| | (-)-Enantiomer | |
| **168** | 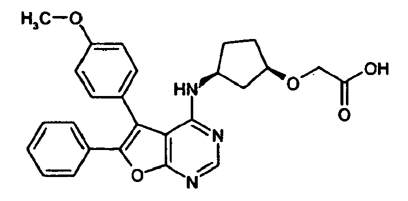 | LC-MS (Methode 6): Rₜ = 2.42 min.; m/z = 460 (M+H)⁺ ¹H-NMR (400 MHz, DMSO-d₆): δ = 12.62 (br. s, 1H), 8.32 (s, 1H), 7.48-7.40 (m, 4H), 7.39-7.29 (m, 3H), 7.11 (d, 2H), 5.31 (d, 1H), 4.61-4.52 (m, 1H), 4.00-3.94 (m, 1H), 3.83 (s, 3H), 3.69 (s, 2H), 2.00-1.88 (m, 2H), 1.70-1.61 (m, 2H), 1.54-1.35 (m, 2H). |
| | (+)-Enantiomer | |
| **169** | 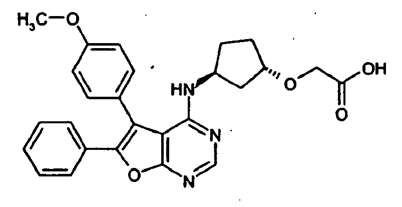 | LC-MS (Methode 8): Rₜ = 2.55 min.; m/z = 460 (M+H)⁺ ¹H-NMR (400 MHz, DMSO-d₆): δ = 12.60 (br. s, 1H), 8.38 (s, 1H), 7.51-7.45 (m, 4H), 7.40-7.30 (m, 3H), 7.27 (d, 2H), 4.81 (d, 1H), 4.52-3.90 (m, 1H), 3.98-3.90 (m, 3H), 3.88 (s, 3H), 2.09-1.99 (m, 2H), 1.82-1.71 (m, 1H), 1.67-1.55 (m, 1H), 1.45-1.3 (m, 1H), 1.21-1.11 (m, 1H). |
| | (+)-Enantiomer | |
| **170** | 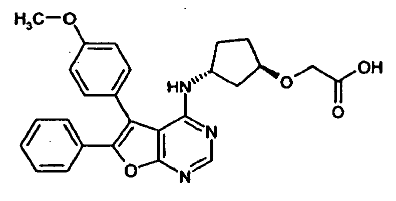 | LC-MS (Methode 8): Rₜ = 2.55 min.; m/z = 460 (M+H)⁺ ¹H-NMR (400 MHz, DMSO-d₆): δ = 12.60 (br. s, 1H), 8.38 (s, 1H), 7.51-7.45 (m, 4H), 7.40-7.30 (m, 3H), 7.27 (d, 2H), 4.81 (d, 1H), 4.52-3.90 (m, 1H), 3.98-3.90 (m, 3H), 3.88 (s, 3H), 2.09-1.99 (m, 2H), 1.82-1.71 (m, 1H), 1.67-1.55 (m, 1H), 1.45-1.35 (m, 1H), 1.21-1.11 (m, 1H). |
| | (-)-Enantiomer | |
| **171** | 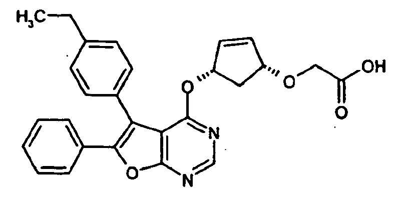 | LC-MS (Methode 3): Rₜ=2.66 min.; m/z= 457 (M+H)⁺ ¹H-NMR (400 MHz, DMSO-d₆): δ = 12.65 (s, 1H), 8.62 (s, 1H), 7.56 (d, 2H), 7.48-7.31 (m, 5H), 7.28 (d, 2H), 6.14 (dd, 2H), 5.89-5.80 (m, 1H), 4.55-4.50 (m, 1H), 3.97 (s, 2H), 2.83 (q, 2H), 1.96-1.88 (m, 1H), 1.53 (td, 1H), 1.22 (t, 3H). |
| | (*+*/*-*)*-cis* | |
| **172** | 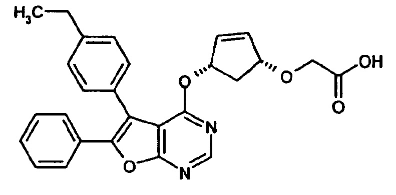 | LC-MS (Methode 8): Rₜ = 2.92 min.; m/z = 457 (M+H)⁺ ¹H-NMR (400 MHz, DMSO-d₆): δ = 8.60 (s, 1H), 7.54 (d, 2H), 7.43-7.38 (m, 5H), 7.28 (d, 2H), 6.20 (d, 1H), 6.00 (d, 1H), 5.82-5.78 (m, 1H), 4.59-4.51 (m, 1H), 3.51 (d, 2H), 2.82-2.71 (m, 1H), 2.69 (q, 2H), 1.52-1.44 (m, 1H), 1.22 (t, 3H), 1.06 (t, 1H). |
| | (-)-Enantiomer | |
| **173** | 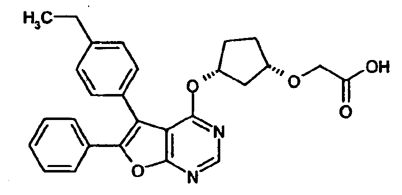 | LC-MS (Methode 3): Rₜ = 2.72 min.; m/z = 459 (M+H)⁺ ¹H-NMR (400 MHz, DMSO-d₆): δ = 8.59 (s, 1H), 7.55 (d, 2H), 7.45-7.36 (m, 5H), 7.29 (d, 2H), 5.46-5.39 (m, 1H), 4.03-3.96 (m, 1H), 3.50 (d, 2H), 2.70 (q, 2H), 2.38-2.25 (m, 1H), 2.22-2.12 (m, 1H), 1.95-1.74 (m, 2H), 1.71-1.58 (m, 3H), 1.25 (t, 3H). |
| | (-)-Enantiomer | |
| **174** | 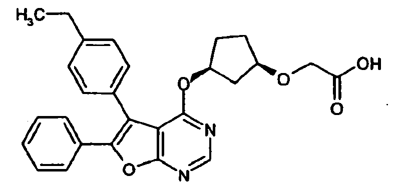 | ¹H-NMR (400 MHz, DMSO-d₆): δ = 12.55 (s, 1H), 8.59 (s, 1H), 7.55 (d, 2H), 7.45-7.36 (m, 5H), 7.29 (d, 2H), 5.46-5.39 (m, 1H), 4.03-3.96 (m, 1H), 3.88 (d, 2H), 2.70 (q, 2H), 2.38-2.25 (m, 1H), 1.95-1.74 (m, 2H), 1.71-1.58 (m, 3H), 1.25 (t, 3H). |
| | (+)-Enantiomer | |
| **175** | 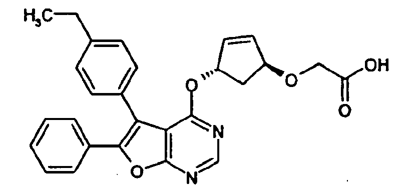 | LC-MS (Methode 8): Rₜ = 3.13 min.; m/z = 457 (M+H)⁺ ¹H-NMR (400 MHz, DMSO-d₆): δ = 12.65 (s, 1H), 8.61 (s, 1H), 7.60-7.52 (m, 2H), 7.47-7.30 (m, 5H), 7.27 (d, 2H), 6.28 (d, 1H), 6.15 (d, 1H), 6.11 (d, 1H), 4.70-4.63 (m, 1H), 4.02 (s, 2H), 2.70 (q, 2H), 2.20-2.10 (m, 1H), 2.05-1.98 (m, 1H), 1.24 (t, 3H). |
| | (-)-Enantiomer | |
| **176** | 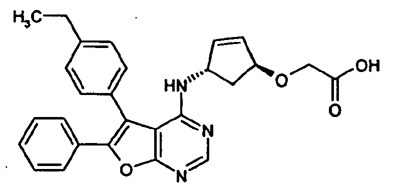 | LC-MS (Methode 12): Rₜ = 2.66 min.; m/z = 456 (M+H)⁺ ¹H-NMR (400 MHz, DMSO-d₆): δ = 12.60 (s, 1H), 8.39 (s, 1H), 7.50 (d, 2H), 7.47-7.30 (m, 7H), 6.06 (d, 1H), 5.90 (d, 1H), 5.22-5.16 (m, 1H), 4.65 (d, 1H), 4.60-4.54 (m, 1H), 4.00 (s, 2H), 2.71 (q, 2H), 2.20-2.11 (m, 1H), 1.67-1.58 (m, 1H), 1.23. (t, 3H). |
| | (-)-Enantiomer | |

### Beispiel 177

### [(3-{[5-(4-Ethylphenyl)-6-(2-fluorphenyl)furo[2,3-d]pyrimidin-4-yl]oxy}cyclohexyl)oxy]essigsäure-tert.-butylester (rac. Diastereomerengemisch)

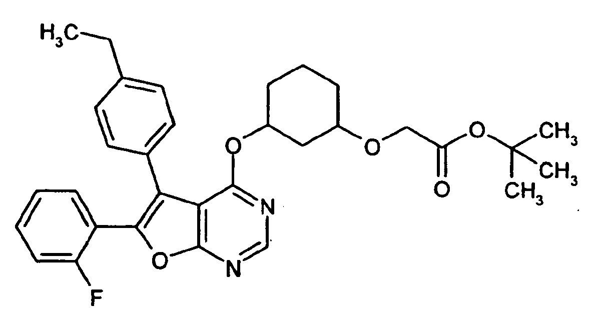

Eine Lösung von 700 mg (1.62 mmol) 3-{[5-(4-Ethylphenyl)-6-(2-fluorphenyl)furo[2,3-d]pyrimidin-4-yl]oxy}cyclohexanol in 15 ml Toluol wird bei 70°C mit 1.4 ml einer 11.25 N Natronlauge versetzt. Nach Zugabe von 55 mg (0.16 mmol) Tetra-n-butylammoniumhydrogensulfat und 631 mg (3.24 mmol) Bromessigsäure-*tert*.-butylester wird das Reaktionsgemisch 30 Stunden bei 70°C gerührt. Das Reaktionsgemisch wird anschließend mit weiteren 330 mg (1.69 mmol) Bromessigsäure-*tert*.-butylester versetzt und erneut 14 Stunden bei 70°C gerührt. Nach Abkühlen auf Raumtemperatur wird mit konz. Salzsäure auf pH 7 eingestellt. Es wird mit Dichlormethan extrahiert. Die organische Phase wird mit ges. Natriumchlorid-Lösung gewaschen, über Natriumsulfat getrocknet, filtriert und im Vakuum eingeengt. Der Rückstand wird mittels präparativer RP-HPLC (Eluent: Wasser/Acetonitril-Gradient) gereinigt. Es werden 632 mg (69% d. Th.) des gewünschten Produkts als racemisches Diastereomerengemisch erhalten.
LC-MS (Methode 8): Rₜ = 3.47 min.; m/z = 547 (M+H)⁺
¹H-NMR (400 MHz, DMSO-d₆): [Minder-Stereoisomer in Klammern] δ = 8.62 (s, 1H), 7.57-7.50 (m, 2H), 7.34-7.28 (m, 4H), 7.21-7.19 (m, 2H), [5.69-5.64, m, 1H], 5.23-5.16 (m, 1H), 3.99 (d, 2H), [3.89, d, 2H], 3.47-3.40 (m, 1H), 2.64 (q, 2H), 2.46-2.42 (m, 1H), 2.09-2.05 (m, 1H), 1.99-1.93 (m, 1H), 1.78-1.73 (m, 1H), 1.41 (s, 9H), 1.30-1.12 (m, 4H), 1.20 (t, 3H).

### Beispiel 178

### [(3-{[5-(4-Ethylphenyl)-6-(2-fluorphenyl)furo[2,3-d]pyrimidin-4-yl]oxy}cyclohexyl)oxy]essigsäure-tert.-butylester (cis-Enantiomer 1)

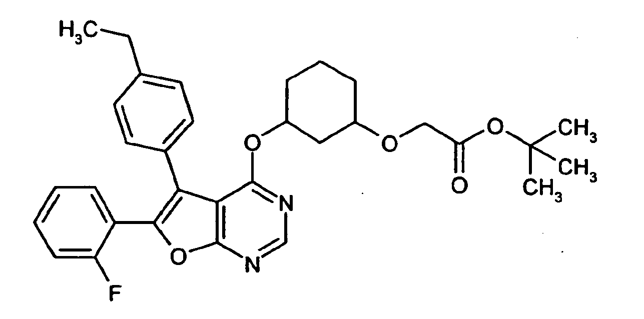

Ausgehend von 600 mg (1.10 mmol) [(3-{[5-(4-Ethylphenyl)-6-(2-fluorphenyl)furo[2,3-d]pyrimidin-4-yl]oxy}cyclohexyl)oxy]essigsäure-*tert*.-butylester (rac. Diastereomerengemisch) werden nach chromatographischer Enantiomerentrennung an chiraler Phase 236 mg (39% d. Th.) des reinen *cis*-Enantiomer 1 erhalten [Säule: Daicel Chiralpak AD-H, 5 µm, 250 mm x 20 mm; Fluss: 15 ml/min; Detektion: 220 nm; Temperatur: 30°C; Eluent: 93% iso-Hexan / 7% Ethanol].
HPLC [Säule: Daicel Chiralpak AD-H, 5 µm, 250 mm x 4.6 mm; Fluss: 1 ml/min; Detektion: 215 nm; Temperatur: 35°C; Eluent: 93% iso-Hexan / 7% Ethanol]: Rₜ = 6.64 min.
¹H-NMR (400 MHz, DMSO-d₄): δ = 8.62 (s, 1H), 7.56-7.52 (m, 2H), 7.34-7.28 (m, 4H), 7.21-7.19 (m, 2H), 5.23-5.16 (m, 1H), 3.98 (d, 2H), 3.47-3.40 (m, 1H), 2.64 (q, 2H), 2.48-2.44 (m, 1H), 2.09-2.05 (m, 1H), 1.98-1.94 (m, 1H), 1.78-1.73 (m, 1H), 1.41 (s, 9H), 1.30-1.10 (m, 4H), 1.20 (t, 3H).

### Beispiel 179

### [(3-{[5-(4-Ethylphenyl)-6-(2-fluorphenyl)furo[2,3-d]pyrimidin-4-yl]oxy}cyclohexyl)oxy]essigsäure-tert.-butylester (cis-Enantiomer 2)

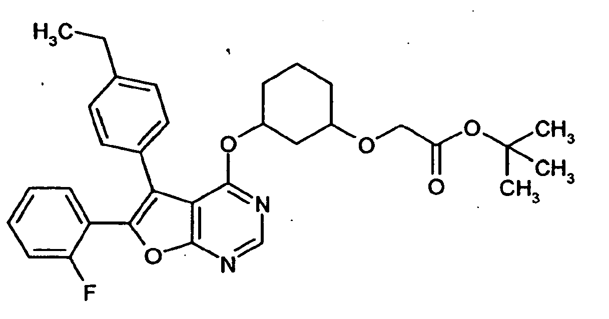

Ausgehend von 600 mg (1.10 mmol) [(3-{[5-(4-Ethylphenyl)-6-(2-fluorphenyl)furo[2,3-d]pyrimidin-4-yl]oxy}cyclohexyl)oxy]essigsäure-*tert*.-butylester (rac. Diastereomerengemisch) werden nach chromatographischer Enantiomerentrennung an chiraler Phase 263 mg (43% d. Th.) des reinen *cis*-Enantiomer 2 erhalten [Säule: Daicel Chiralpak AD-H, 5 µm, 250 mm x 20 mm; Fluss: 15 ml/min; Detektion: 220 nm; Temperatur: 30°C; Eluent: 93% iso-Hexan / 7% Ethanol].
HPLC [Säule: Daicel Chiralpak AD-H, 5 µm, 250 mm x 4.6 mm; Fluss: 1 ml/min; Detektion: 215 nm; Temperatur: 35°C; Eluent: 93% iso-Hexan / 7% Ethanol]: Rₜ = 8.06 min.
¹H-NMR (400 MHz, DMSO-d₆): δ = 8.62 (s, 1H), 7.56-7.52 (m, 2H), 7.34-7.28 (m, 4H), 7.21-7.19 (m, 2H), 5.23-5.16 (m, 1H), 3.98 (d, 2H), 3.47-3.40 (m, 1H), 2.64 (q, 2H), 2.48-2.44 (m, 1H), 2.09-2.05 (m, 1H), 1.98-1.94 (m, 1H), 1.78-1.73 (m, 1H), 1.41 (s, 9H), 1.30-1.10 (m, 4H), 1.20 (t, 3H).

### Beispiel 180

### 4-[(3R)-3-{[5-(4-Ethylphenyl)-6-(2-fluorphenyl)furo[2,3-d]pyrimidin-4-yl]oxy}piperidin-1-yl]-buttersäuremethylester

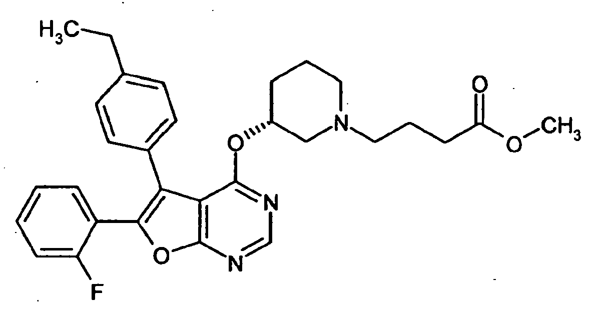

Eine Lösung von 2250 mg (4.9 mmol) 5-(4-Ethylphenyl)-6-(2-fluorphenyl)-4-[(3*R*)-piperidin-3-yl-oxy]furo[2,3-d]pyrimidin in 100 ml THF und 10 ml Acetonitril wird mit 1677 mg (12.1 mmol) Kaliumcarbonat versetzt. Anschließend werden 0.74 ml (1054 mg, 5.8 mmol) 4-Brombuttersäüremethylester und 72 mg (0.19 mmol) Tetra-n-butylammoniumiodid zugegeben. Das Reaktionsgemisch wird 13 Stunden bei 80°C gerührt. Nach Abkühlen auf Raumtemperatur wird der Rückstand abfiltriert, mit THF nachgewaschen, das Filtrat im Vakuum eingeengt und der Rückstand säulenchromatographisch an Kieselgel aufgereinigt (Laufmittel: Cyclohexan/Essigsäureethylester 1:2). Es werden 2005 mg (75% d. Th.) der Zielverbindung erhalten.
LC-MS (Methode 12): Rₜ = 1.82 min.; m/z = 518 (M+H)⁺
¹H-NMR (300 MHz, DMSO-d₆): δ = 8.63 (s, 1H), 7.56-7.53 (m, 2H), 7.35-7.28 (m, 4H), 7.18 (d, 2H), 5.33-5.31 (m, 1H), 3.52 (s, 3H), 2.78-2.75 (m, 1H), 2.64 (q, 2H), 2.44-2.40 (m, 1H), 2.36-2.23 (m, 6H), 1.93-1.89 (m, 1H), 1.67-1.59 (m, 3H), 1.44-1.42 (m, 2H), 1.19 (t, 3H).

### Beispiel 181

### 4-[(3R)-3-{[5-(4-Ethylphenyl)-6-(2-fluorphenyl)furo[2,3-d]pyrimidin-4-yl]oxy}piperidin-1-yl]-buttersäure

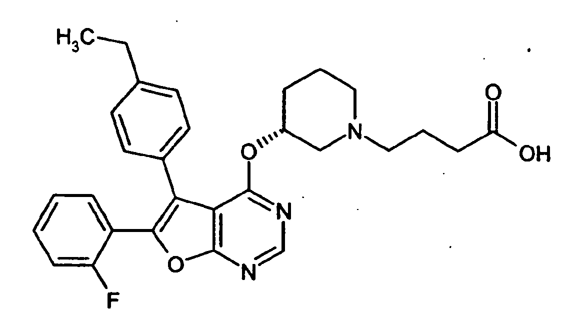

500 mg (0.97 mmol) 4-[(3*R*)-3-{[5-(4-Ethylphenyl)-6-phenylfuro[2,3-d]pyrimidin-4-yl]oxy}piperidin-1-yl]buttersäuremethylester werden in 10 ml Dioxan gelöst und mit 2.9 ml einer 1 N Natronlauge versetzt. Es wird 16 Stunden bei Raumtemperatur gerührt. Danach werden 2.9 ml 1 N Salzsäure zugegeben, und das Gemisch wird mit 20 ml Essigsäureethylester extrahiert. Die organische Phase wird abgetrennt, über Natriumsulfat getrocknet, filtriert und eingeengt. Der Rückstand wird durch präparative RP-HPLC (Eluent: Wasser/Acetonitril-Gradient mit 0.1% Ameisensäure) gereinigt. Das erhaltene Produkt wird in 10 ml Essigsäureethylester aufgenommen und zweimal mit je 10 ml einer 1 M wässrigen Natriumhydrogencarbonat-Lösung gewaschen. Die organische Phase wird abgetrennt, über Natriumsulfat getrocknet, filtriert und eingeengt. Es werden 309 mg (62% d. Th.) der Zielverbindung erhalten.
LC-MS (Methode 3): Rₜ = 1.74 min.; m/z = 504 (M+H)⁺.
¹H-NMR (400 MHz, DMSO-d₆): δ = 12.12 (s, 1H), 8.62 (s, 1H), 7.56-7.51 (m, 2H), 7.36-7.27 (m, 4H), 7.18 (d, 2H), 5.32 (t, 1H), 2.83-2.80 (m, 1H), 2.63 (q, 2H), 2.49-2.47 (m, 1H), 2.33-2.25 (m, 4H), 2.19 (t, 2H), 1.94-1.91 (m, 1H), 1.65-1.57 (m, 3H), 1.43-1.39 (m, 2H), 1.19 (t, 3H).

### Beispiel 182

### 4-[(3R)-3-{[5-(4-Ethylphenyl)-6-(2-fluorphenyl)furo[2,3-d]pyrimidin-4-yl]oxy}piperidin-1-yl]-buttersäure-Formiat

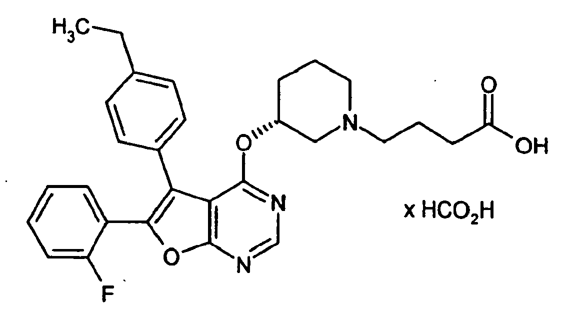

500 mg (0.97 mmol) 4-[(3*R*)-3-{[5-(4-Ethylphenyl)-6-phenylfuro[2,3-d]pyrimidin-4-yl]oxy}piperidin-1-yl]buttersäuremethylester werden in 10 ml Dioxan gelöst und mit 2.9 ml einer 1 N Natronlauge versetzt. Es wird 16 Stunden bei Raumtemperatur gerührt. Danach werden 2.9 ml 1N Salzsäure zugegeben, und das Gemisch wird mit 20 ml Essigsäureethylester extrahiert. Die organische Phase wird abgetrennt, über Natriumsulfat getrocknet, filtriert und eingeengt. Der Rückstand wird durch präparative RP-HPLC (Eluent: Wasser/Acetonitril-Gradient mit 0.1% Ameisensäure) gereinigt. Es werden 411 mg (77% d. Th.) der Zielverbindung erhalten.
LC-MS (Methode 8): Rₜ =1.88 min.; m/z = 504 (M-HCO₂H+H)⁺
¹H-NMR (400 MHz, DMSO-d₆): δ = 8.62 (s, 1H), 8.14 (s, 1H), 7.56-7.51 (m, 2H), 7.36-7.29 (m, 4H), 7.19 (d, 2H), 5.33 (t, 1H), 2.83-2.80 (m, 1H), 2.63 (q, 2H), 2.49-2.47 (m, 1H), 2.33-2.25 (m, 4H), 2.19 (t, 2H), 1.94-1.91 (m, 1H), 1.66-1.58 (m, 3H), 1.48-1.34 (m, 2H), 1.19 (t, 3H).

### Beispiel 183

### 4-[(3R)-3-{[6-(2-Fluorphenyl)-5-(4-methoxyphenyl)furo[2,3-d]pyrimidin-4-yl]oxy}piperidin-1-yl]-buttersäuremethylester

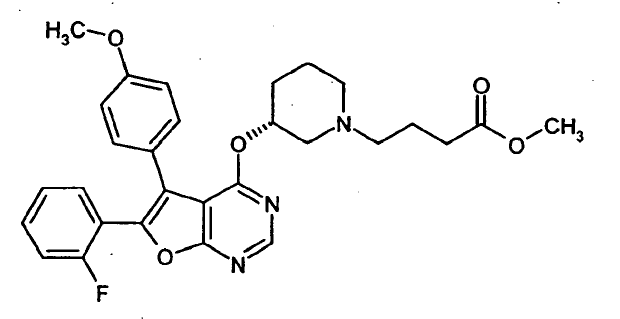

Eine Suspension von 500 mg (1.2 mmol) 6-(2-Fluorphenyl)-5-(4-methoxyphenyl)-4-[(3*R*)-piperidin-3-yloxy]furo[2,3-d]pyrimidin in 10 ml THF wird mit 411 mg (3.0 mmol) Kaliumcarbonat versetzt. Anschließend werden 0.18 ml (259 mg, 1.4 mmol) 4-Brombuttersäuremethylester und 17 mg (0.05 mmol) Tetra-n-butylammoniumiodid zugegeben. Das Reaktionsgemisch wird 13 Stunden bei 80°C gerührt. Danach werden 10 ml DMF zugesetzt und das Gemisch erneut für 13 Stunden bei 70°C gerührt. Nach Zugabe von je 10 ml Wasser, 1N Salzsäure sowie Essigsäureethylester wird die organische Phase abgetrennt, im Vakuum eingeengt und der Rückstand mittels präparativer RP-HPLC (Eluent: Wasser/Acetonitril-Gradient) gereinigt. Es werden 146 mg (22% d. Th.) der Zielverbindung erhalten.
LC-MS (Methode 13): Rₜ = 2.77 min.; m/z = 520 (M+H)⁺
¹H-NMR (400 MHz, DMSO-d₆): δ = 8.61 (s, 1H), 7.56-7.52 (m, 2H), 7.35 (d, 2H), 7.32-7.28 (m, 2H), 6.90 (d, 2H), 5.34-5.31 (m, 1H), 3.76 (s, 1H), 3.53 (s, 3H), 2.81-2.79 (m, 1H), 2.48-2.42 (m, 1H), 2.32-2.18 (m, 6H), 2.00-1.92 (m, 1H), 1.67-1.60 (m, 3H), 1.45-1.41 (m, 2H).

### Beispiel 184

### 4-[(3R)-3-{[6-(2-Fluorphenyl)-5-(4-methoxyphenyl)furo[2,3-d]pyrimidin-4-yl]oxy}piperidin-1-yl]-buttersäuremethylester-Formiat

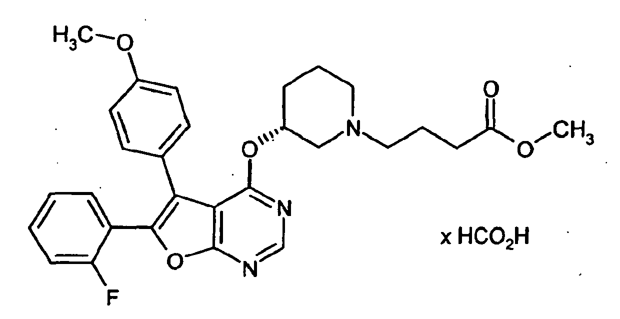

Eine Lösung von 70 mg (0.15mmol) 6-(2-Fluorphenyl)-5-(4-methoxyphenyl)-4-[(3*R*)-piperidin-3-yloxy]furo[2,3-d]pyrimidin-Formiat in 1 ml THF wird mit 52 mg (0.38 mmol) Kaliumcarbonat versetzt. Anschließend werden 0.02 ml (33 mg, 0.18 mmol) 4-Brombuttersäuremethylester und 2 mg (0.01 mmol) Tetra-n-butylammoniumiodid zugegeben. Das Reaktionsgemisch wird 13 Stunden bei 80°C gerührt. Nach Abkühlen auf Raumtemperatur wird im Vakuum eingeengt und der Rückstand mittels präparativer RP-HPLC (Eluent: Wasser/Acetonitril-Gradient mit 0.1% Ameisensäure) gereinigt. Es werden 40 mg (42% d. Th.) der Zielverbindung erhalten.
LC-MS (Methode 3): Rₜ = 1.75 min.; m/z = 520 (M+H)⁺
¹H-NMR (300 MHz, DMSO-d₆): δ = 8.62 (s, 1H), 8.15 (s, 1H), 7.53-7.51 (m, 2H), 7.35 (d, 2H), 7.32-7.28 (m, 2H), 6.90 (d, 2H), 5.5-5.30 (m, 1H), 3.76 (s, 3H), 3.52 (s, 3H), 2.82-2.79 (m, 1H), 2.36-2.24 (m, 5H), 1.94-1.88 (m, 1H), 1.67-1.60 (m, 2H), 1.45-1.41 (m, 2H).

### Beispiel 185

### 4-[(3R)-3-{[6-(2-Fluorphenyl)-5-(4-methoxyphenyl)furo[2,3-d]pyrimidin-4-yl]oxy}piperidin-1-yl]-buttersäure

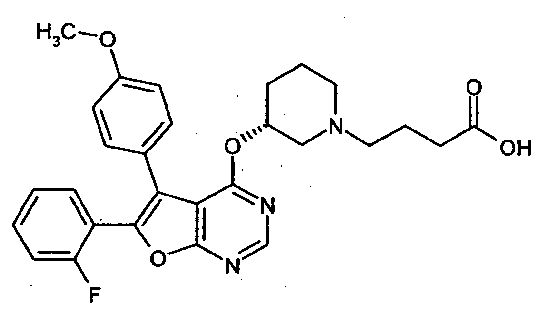

113 mg (0.20 mmol) 4-[(3*R*)-3-{[6-(2-Fluorphenyl)-5-(4-methoxyphenyl)furo[2,3-d]pyrimidin-4-yl]oxy}piperidin-1-yl]buttersäuremethylester werden in 3 ml Dioxan gelöst und mit 0.8 ml einer 1 N Natronlauge versetzt. Es wird 16 Stunden bei Raumtemperatur gerührt, dann 0.8 ml 1N Salzsäure zugegeben und mit 10 ml Essigsäureethylester versetzt. Die organische Phase wird abgetrennt, über Natriumsulfat getrocknet, filtriert und eingeengt. Es werden 95 mg (90% d. Th.) der Zielverbindung erhalten.
LC-MS (Methode 8): Rₜ = 1.70 min.; m/z = 504 (M+H)⁺
¹H-NMR (400 MHz, DMSO-d₆): δ = 12.20 (br. s, 1H), 8.63 (s, 1H), 7.56-7.51 (m, 2H), 7.37-7.29 (m, 4H), 6.91 (d, 2H), 535 (t, 1H), 3.76 (s, 1H), 2.99-2.94 (m, 1H), 2.64-2.62 (m, 1H), 2.40-2.32 (m, 4H), 2.20 (t, 2H), 2.02-1.98 (m, 1H), 1.67-1.63 (m, 3H), 1.43-1.39 (m, 2H).

### Beispiel 186

### {[(1S,3R)-3-{[5-(4-Ethylphenyl)-6-(2-fluorphenyl)furo[2,3-d]pyrimidin-4-yl]oxy}cyclohexyl]-oxy}essigsäure

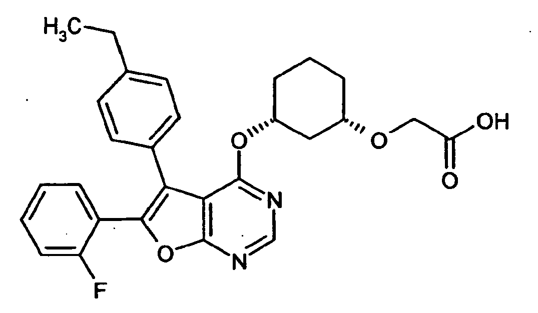

237 mg (0.43 mmol) {[(1*S*,3*R*)-3-{[5-(4-Ethylphenyl)-6-(2-fluorphenyl)furo[2,3-d]pyrimidin-4-yl]-oxy}cyclohexyl]oxy}essigsäure-*tert*.-butylester werden mit 10 ml 4 N Chlorwasserstoff in Dioxan versetzt und 16 Stunden bei Raumtemperatur gerührt. Nach Entfernen des Lösungsmittels im Vakuum wird der Rückstand mittels präparativer RP-HPLC (Eluent: Wasser/Acetonitril-Gradient) gereinigt. Es werden 132 mg (62% d. Th.) der Zielverbindung erhalten.
LC-MS (Methode 8): Rₜ = 3.10 min.; m/z = 491 (M+H)⁺
¹H-NMR (400 MHz, DMSO-d₆): δ = 12.54 (s, 1H), 8.63 (s, 1H), 7.56-7.52 (m, 2H), 7.34-7.28 (m, 4H), 7.20 (d, 2H), 5.22-5.16 (m, 1H), 4.03 (s, 2H), 3.48-3.43 (m, 1H), 2.63 (q, 2H), 2.12-2.06 (m, 1H), 2.00-1.96 (m, 1H), 1.77-1.73 (m, 1H), 1.29-1.08 (m, 4H), 1.19 (t, 3H).
[α]_{D}²⁰ = +62°, c = 0.525, CHCl₃.

### Beispiel 187

### {[(1R,3S)-3-{[5-(4-Ethylphenyl)-6-(2-fluorphenyl)furo[2,3-d]pyrimidin-4-yl]oxy}cyclohexyl]-oxy} Essigsäure

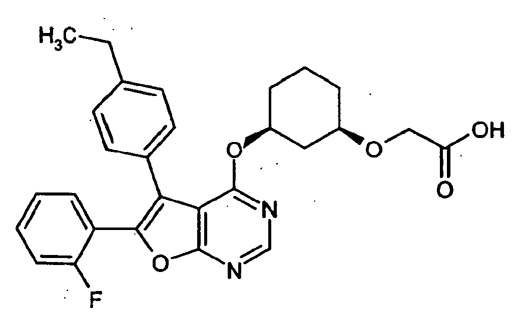

215 mg (0.39 mmol) {[(1*R*,3*S*)-3-{[5-(4-Ethylphenyl)-6-(2-fluorphenyl)furo[2,3-d]pyrimidin-4-yl]-oxy}cyclohexyl]oxy}essigsäure-*tert*.-butylester werden mit 10 ml 4N Chlorwasserstoff in Dioxan versetzt und 16 Stunden bei Raumtemperatur gerührt. Nach Entfernen des Lösungsmittels im Vakuum wird der Rückstand mittels präparativer RP-HPLC (Eluent: Wasser/Acetonitril-Gradient) gereinigt. Es werden 128 mg (66% d. Th.) der Zielverbindung erhalten.
LC-MS (Methode 8): Rₜ = 3.11 min.; m/z = 491 (M+H)⁺
¹H-NMR (300 MHz, DMSO-d₆): δ = 12.54 (s, 1H), 8.63 (s, 1H), 7.56-7.52 (m, 2H), 7.34-7.28 (m, 4H), 7.20 (d, 2H), 5.22-5.16 (m, 1H), 4.03 (s, 2H), 3.48-3.43 (m, 1H), 2.63 (q, 2H), 2.12-2.06 (m, 1H), 2.00-1.96 (m, 1H), 1.77-1.74 (m, 1H), 1.29-1.08 (m, 4H), 1.18 (t, 3H).
[α]_{D}²⁰ = -57°, c = 0.660, CHCl₃.

### Beispiel 188

### {[1-({[5-(4-Methoxyphenyl)-6-phenylfuro[2,3-d]pyrimidin-4-yl]oxy}methyl)cyclobutyl]methoxy}-essigsäure-tert.-butylester

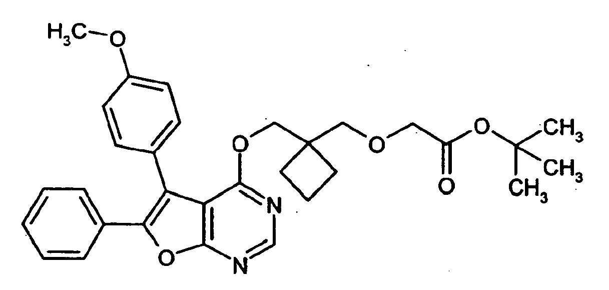

Eine Lösung von 285 mg (0.68 mmol) [1-({[5-(4-Methoxyphenyl)-6-phenylfuro[2,3-d]pyrimidin-4-yl]oxy}methyl)cyclobutyl]methanol in 5 ml Toluol wird mit 0.6 ml einer 11.25 N Natronlauge versetzt. Nach Zugabe von 23 mg (0.07 mmol) Tetra-*n*-butylammoniumhydrogensulfat und 267 mg (1.37 mmol) Bromessigsäure-*tert*.-butylester wird das Reaktionsgemisch 20 h bei 70°C gerührt. Nach Abkühlen auf Raumtemperatur wird mit konz. Salzsäure auf pH 7 eingestellt. Es wird dreimal mit je 20 ml Dichlormethan extrahiert. Die vereinigten organischen Extrakte werden mit ges. wässriger Natriumchlorid-Lösung gewaschen, über Natriumsulfat getrocknet und filtriert. Das Filtrat wird im Vakuum eingeengt. Das Rohprodukt wird mittels präparativer RP-HPLC (Gradient: Wasser/Acetonitril) gereinigt. Es werden 260 mg (72% d. Th.) des gewünschten Produkts erhalten.
LC-MS (Methode 8): Rₜ = 3.38 min.; m/z = 531 (M+H)⁺
¹H-NMR (400 MHz, DMSO-d₆): δ = 8.58 (s, 1H), 7.56-7.54 (m, 2H), 7.42-7.37 (m, 5H), 7.04-7.00 (m, 2H), 4.34 (s, 2H), 3.85 (s, 2H), 3.81 (s, 3H), 3.22 (s, 2H), 1.78-1.65 (m, 6H), 1.38 (s, 9H).

### Beispiel 189

### {[1-({[5-(4-Methoxyphenyl)-6-phenylfuro[2,3-d]pyrimidin-4-yl]oxy}methyl)cyclobutyl]methoxy}-essigsäure

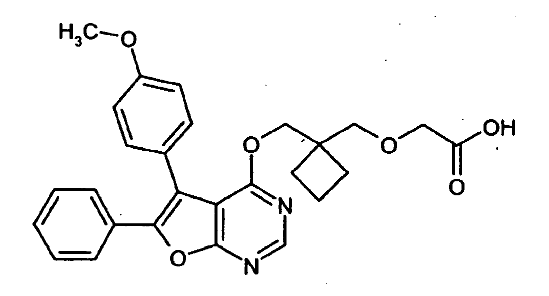

237 mg (0.45 mmol) {[1-({[5-(4-Methoxyphenyl)-6-phenylfuro[2,3-d]pyrimidin-4-yl]oxy}methyl)-cyclobutyl]methoxy}essigsäure-*tert*.-butylester werden in 1 ml Dioxan gelöst, mit 2 ml 4 N Chlorwasserstoff in Dioxan versetzt und 16h bei RT gerührt. Nach Einengen der Reaktionslösung im Vakuum wird der Rückstand mittels präparativer RP-HPLC (Gradient: Wasser/Acetonitril) aufgereinigt. Es werden 180 mg (85% d. Th.) des gewünschten Produkts erhalten.
LC-MS (Methode 8): Rₜ = 2.84 min.; m/z = 475 (M+H)⁺
¹H-NMR (400 MHz, DMSO-d₆): δ = 12.49 (br. s, 1H), 8.57 (s, 1H), 7.56-7.54 (m, 2H), 7.42-7.36 (m, 5H), 7.04-7.00 (in, 2H), 4.34 (s, 2H), 3.87 (s, 2H), 3.81.(s, 3H), 3.23 (s, 2H), 1.80-1.67 (m, 6H).

### B. Bewertung der pharmakologischen Wirksamkeit

Die pharmakologische Wirkung der erfindungsgemäßen Verbindungen kann in folgenden Assays gezeigt werden:

### B-1. Bindungsstudien mit Prostacyclin-Rezeptoren (IP-Rezeptoren) von humanen Thrombozytenmembranen

Zur Gewinnung von Thrombozytenmembranen werden 50 ml Humanblut (Buffy coats mit CDP-Stabilizer, Fa. Maco Pharma, Langen) für 20 min bei 160 x g zentrifugiert. Der Überstand (plättchenreiches Plasma, PRP) wird abgenommen und anschließend nochmals bei 2000 x g für 10 min bei Raumtemperatur zentrifugiert. Das Sediment wird in 50 mM Tris-(hydroxymethyl)-aminomethan, welches mit 1 N Salzsäure auf einen pH-Wert von 7.4 eingestellt ist, re-suspendiert und bei -20°C über Nacht aufbewahrt. Am folgenden Tag wird die Suspension bei 80000 x g und 4°C 30 min lang zentrifugiert. Der Überstand wird verworfen. Das Sediment wird in 50 mM Tris-(hydroxymethyl)-aminomethan/Salzsäure, 0.25 mM Ethylendiamintetraessigsäure (EDTA), pH 7.4 re-suspendiert und danach nochmals bei 80000 x g und 4°C für 30 min zentrifugiert. Das Membransediment wird in Bindungspuffer (50 mM Tris-(hydroxymethyl)-aminomethan/Salzsäure, 5 mM Magnesiumchlorid, pH 7.4) aufgenommen und bis zum Bindungsversuch bei -70°C gelagert.

Für den Bindungsversuch werden 3 nM ³H-Iloprost (592 GBq/mmol, Fa. AmershamBioscience) 60 min lang mit 300-1000 µg/ml humanen Thrombozytenmembranen pro Ansatz (max. 0.2 ml) in Gegenwart der Testsubstanzen bei Raumtemperatur inkubiert. Nach dem Abstoppen werden die Membranen mit kaltem Bindungspuffer versetzt und mit 0.1% Rinderserumalbumin gewaschen. Nach Zugabe von Ultima Gold-Szintillator wird die an den Membranen gebundene Radioaktivität mittels eines Szintillationszählers quantifiziert. Die nicht-spezifische Bindung wird als Radioaktivität in Gegenwart von 1 µM Iloprost (Fa. Cayman Chemical, Ann Arbor) definiert und beträgt in der Regel < 25% der gebundenen Gesamt-Radioaktivität. Die Bindungsdaten (IC₅₀-Werte) werden mittels des Programmes GraphPad Prism Version 3.02 bestimmt.

Repräsentative Ergebnisse zu den erfmdungsgemäßen Verbindungen sind in Tabelle 1 aufgeführt:

**Tabelle 1**

| **Beispiel Nr.** | **IC₅₀ [nM]** |
|---|---|
| 6 | 237 |
| 9 | 43 |
| 10 | 18 |
| 21 | 11 |
| 24 | 15 |
| 67 | 388 |
| 68 | 34 |
| 69 | 8 |
| 84 | 152 |
| 87 | 17 |
| 99 | 34 |
| 146 | 20 |
| 154 | 28 |
| 156 | 293 |
| 159 | 83 |
| 162 | 37 |
| 168 | 71 |
| 170 | 6 |
| 175 | 13 |
| 176 | 24 |
| 181 | 27 |
| 183 | 218 |

### B-2. IP-Rezeptor-Stimulierung auf Ganzzellen

Die IP-agonistische Wirkung von Testsubstanzen wird mit Hilfe der humanen Erythroleukämie-Zelllinie (HEL), die den IP-Rezeptor endogen exprimiert, bestimmt [Murray, R, FEBS Letters 1989, 1: 172-174]. Dazu werden die Suspensionszellen (4 x 10⁷ Zellen/ml) in Puffer [10 mM HEPES (4-(2-Hydroxyethyl)-1-piperazinethansulfonsäure) / PBS (Phosphat-gepufferte Salzlösung, Fa. Oxoid, UK)], 1 mM Calciumchlorid, 1 mM Magnesiumchlorid, 1 mM IBMX (3-Isobutyl-1-methylxanthin), pH 7.4, mit der jeweiligen Testsubstanz 5 Minuten lang bei 30°C inkubiert. Anschließend wird die Reaktion durch Zugabe von 4°C kaltem Ethanol gestoppt und die Ansätze weitere 30 Minuten bei 4°C gelagert. Danach werden die Proben bei 10000 x g und 4°C zentrifugiert. Der resultierende Überstand wird verworfen und das Sediment zur Bestimmung der Konzentration an cyclischem Adenosinmonophosphat (cAMP) in einem kommerziell erhältlichen cAMP-Radioimmunoassay (Fa. IBL, Hamburg) eingesetzt. IP-Agonisten führen in diesem Test zu einem Anstieg der cAMP-Konzentration, IP-Antagonisten sind wirkunglos. Die effektive Konzentration (EC₅₀-Werte) wird mittels des Programmes GraphPad Prism Version 3.02 bestimmt.

### B-3. Thrombozytenaggregationshemmung in vitro

Zur Bestimmung der Thrombozytenaggregationshemmung wird Blut von gesunden Probanden beiderlei Geschlechts verwendet. Einem Teil 3.8%-iger Natriumcitrat-Lösung als Koagulans werden 9 Teile Blut zugemischt. Das Blut wird mit 900 U/min für 20 min zentrifugiert. Der pH-Wert des gewonnenen plättchenreichen Plasmas wird mit ACD-Lösung (Natriumcitrat/Citronensäure/Gluccose) auf pH 6.5 eingestellt. Die Thrombozyten werden anschließend abzentrifugiert, in Puffer aufgenommen und erneut abzentrifugiert. Der Thrombozyten-Niederschlag wird in Puffer aufgenommen und zusätzlich mit 2 mmol/l Calciumchlorid re-suspendiert.

Für die Aggregationsmessungen werden Aliquots der Thrombozytensuspension mit der Prüfsubstanz 10 min lang bei 37°C inkubiert. Anschließend wird die Aggregation durch Zugabe von ADP induziert und mittels der turbidometrischen Methode nach Born im Aggregometer bei 37°C bestimmt [Born G.V.R., J. Physiol. (London) 168, 178-179 (1963)].

### B-4. Blutdruckmessung an narkotisierten Ratten

Männliche Wistar-Ratten mit einem Körpergewicht von 300-350 g werden mit Thiopental (100 mg/kg i.p.) anästhesiert. Nach der Tracheotomie wird die Arteria femoralis zur Blutdruckmessung katheterisiert. Die zu prüfenden Substanzen werden als Lösung oral mittels Schlundsonde oder über die Femoralvene intravenös in einem geeigneten Vehikel verabreicht.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000, mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfmdungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 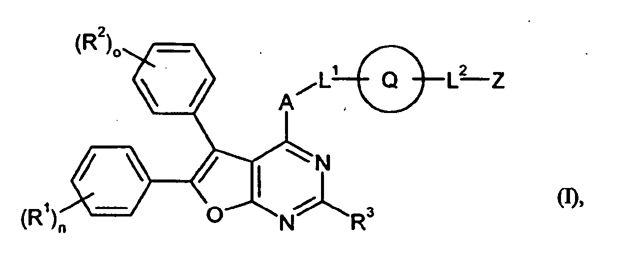 in welcher
A für O, S oder N-R⁴ steht, worin
R⁴ Wasserstoff, (C₁-C₆)-Alkyl, (C₃-C₇)-Cycloalkyl oder (C₄-C₇)-Cycloalkenyl bedeutet,
L¹ für eine Bindung oder für (C₁-C₄)-Alkandiyl steht, '
der Ring Q für (C₃-C₇)-Cycloalkyl, (C₄-C₇)-Cycloalkenyl, einen 5- bis 7-gliedrigen Hetero- cyclus, Phenyl oder 5- oder 6-gliedriges Heteroaryl steht, welche jeweils bis zu zweifach, gleich oder verschieden, mit Fluor, Chlor, (C₁-C₄)-Alkyl, Trifluormethyl, Hydroxy, (C₁-C₄)-Alkoxy, Trifluormethoxy, Amino, Mono-(C₁-C₄)-alkylamino und/oder Di-(C₁-C₄)-alkylamino substituiert sein können,
wobei (C₁-C₄)-Alkyl seinerseits mit Hydroxy, (C₁-C₄)-Alkoxy, Amino, Mono- oder Di-(C₁-C₄)-alkylamino substituiert sein kann,
L² für (C₁-C₄)-Alkandiyl, welches ein- oder zweifach mit Fluor substituiert und in welchem eine Methylengruppe gegen O oder N-R⁵, worin
R⁵ Wasserstoff, (C₁-C₆)-Alkyl oder (C₃-C₇)-Cycloalkyl bedeutet,
ausgetauscht sein kann,
oder für (C₂-C₄)-Alkendiyl steht,
Z für eine Gruppe der Formel 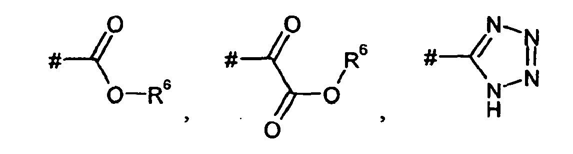 oder 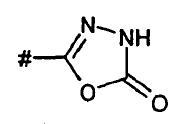 steht, worin
# die Verknüpfungsstelle mit der Gruppe L²
und
R⁶ Wasserstoff oder (C₁-C₄)-Alkyl bedeutet,
R¹ und R² unabhängig voneinander für einen Substituenten ausgewählt aus der Reihe Halogen, Cyano, Nitro, (C₁-C₆)-Alkyl, (C₂-C₆)-Alkenyl, (C₁-C₄)-Alkinyl, (C₃₋C₇)- Cycloalkyl, (C₄-C₇)-Cycloalkenyl, (C₁-C₆)-Alkoxy, Trifluormethyl, Trifluormeth- oxy, (C₁-C₆)-Alkylthio, (C₁-C₆)-Acyl, Amino, Mono-(C₁-C₆)-alkylanaino, Di-(C₁- C₆)-alkylamino und (C₁-C₆)-Acylamino stehen,
wobei (C₁-C₆)-Alkyl und (C₁-C₆)-Alkoxy ihrerseits jeweils mit Cyano, Hydroxy, (C₁-C₄)-Alkoxy, (C₁-C₄)-Alkylthio, Amino, Mono- oder Di-(C₁-C₄)-alkylamino substituiert sein können,
oder
zwei an benachbarte Kohlenstoffatome des jeweiligen Phenylrings gebundene Reste R¹ und/oder R² zusammen eine Gruppe der Formel -O-CH₂-O-, -O-CHF-O-, -O-CF₂-O-, -O-CH₂-CH₂-O- oder -O-CF₂-CF₂-O- bilden,
n und o unabhängig voneinander für die Zahl 0, 1, 2 oder 3 stehen,
wobei für den Fall, dass R¹ oder R² mehrfach auftreten, ihre Bedeutungen jeweils gleich oder verschieden sein können,
und
R³ für Wasserstoff, (C₁-C₄)-Alkyl oder Cyclopropyl steht,
sowie ihre Salze, Solvate und Solvate der Salze.

2. Verbindung der Formel (I) nach Anspruch 1, in welcher
A für O, S oder N-R⁴ steht, worin
R⁴ Wasserstoff, (C₁-C₆)-Alkyl, (C₃-C₇)-Cycloalkyl oder (C₄-C₇)-Cycloalkenyl bedeutet,
L¹ für eine Bindung oder für (C₁-C₄)-Alkandiyl steht,
der Ring Q für (C₃-C₇)-Cycloalkyl, (C₄-C₇)-Cycloalkenyl, einen 5- bis 7-gliedrigen Hetero- cyclus, Phenyl oder 5- oder 6-gliedriges Heteroaryl steht, welche jeweils bis zu zweifach, gleich oder verschieden, mit Fluor, Chlor, (C₁-C₄)-Alkyl, Trifluormethyl, Hydroxy, (C₁-C₄)Alkoxy, Trifluormethoxy, Amino, Mono-(C₁-C₄)-alkylamino und/oder Di-(C₁-C₄)-alkylamino substituiert sein können,
wobei (C₁-C₄)-Alkyl seinerseits mit Hydroxy, (C₁-C₄)-Alkoxy, Amino, Mono- oder Di-(C₁-C₄)-alkylamino substituiert sein kann,
L² für (C₁-C₄)-Akmdiyl, welches ein- oder zweifach mit Fluor substituiert und in welchem eine Methylengruppe gegen O oder N-R⁵, worin
R⁵ Wasserstoff, (C₁-C₆)-Alkyl oder (C₃-C₇)-Cycloalkyl bedeutet,
ausgetauscht sein kann,
oder für (C₂-C₄)-Alkendiyl steht,
Z für eine Gruppe der Formel 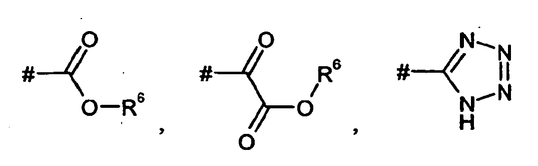 oder 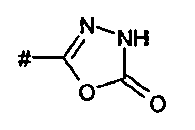 steht, worin
# die Verknüpfungsstelle mit der Gruppe L²
und
R⁶ Wasserstoff oder (C₁-C₄)-Alkyl bedeutet,
R¹ und R² unabhängig voneinander für einen Substituenten ausgewählt aus der Reihe Halogen, Cyano, Nitro, (C₁-C₆)-Alkyl, (C₂-C₆)-Alkenyl, (C₂-C₄)-Alkinyl, (C₃-C₇)- Cycloalkyl, (C₄-C₇)-Cycloalkenyl, (C₁-C₆)-Alkoxy, Trifluormethyl, Trifluormeth- oxy, (C₁-C₆)-Alkylthio, (C₁-C₆)-Acyl, Amino, Mono-(C₁-C₆)-alkylamino, Di-(C₁- C₆)-alkylamino und (C₁-C₆)-Acylamino stehen,
wobei (C₁-C₆)-Alkyl und (C₁-C₆)-Alkoxy ihrerseits jeweils mit Hydroxy, (C₁-C₄)- Alkoxy, Amino, Mono- oder Di-(C₁-C₄)-alkylamino substituiert sein können,
oder
zwei an benachbarte Kohlenstoffatome des jeweiligen Phenylrings gebundene Reste R¹ und/oder R² zusammen eine Gruppe der Formel -O-CH₂-O-, -O-CHF-O-, -O-CF₂-O-, -O-CH₂-CH₂-O- oder -O-CF₂-CF₂-O- bilden,
n und o unabhängig voneinander für die Zahl 0, 1, 2 oder 3 stehen,
wobei für den Fall, dass R¹ oder R² mehrfach auftreten, ihre Bedeutungen jeweils gleich oder verschieden sein können,
und
R³, für Wasserstoff, (C₁-C₄)-Alkyl oder Cyclopropyl steht,
sowie ihre Salze, Solvate und Solvate der Salze.

3. Verbindung der Formel (I) nach Anspruch 1 oder 2, in welcher
A für O oder N-R⁴ steht, worin
R⁴ Wasserstoff, (C₁-C₄)-Alkyl oder (C₃-C₆)-Cycloalkyl bedeutet,
L¹ für eine Bindung oder für (C₁-C₃)-Alkandiyl steht,
der Ring Q für (C₃-C₆)-Cycloalkyl, (C₄-C₆)-Cycloalkenyl, einen 5- oder 6-gliedrigen Heterocyclus, Phenyl oder 5- oder 6-gliedriges Heteroaryl steht, welche jeweils bis zu zweifach, gleich oder verschieden, mit Fluor, Chlor, (C₁-C₃)-Alkyl, Trifluor- methyl, Hydroxy, Methoxy, Ethoxy, Trifluormethoxy, Amino, Methylamino, Ethylamino, Dimethylamino und/oder Diethylamino substituiert sein können,
wobei (C₁-C₃)-Alkyl seinerseits mit Hydroxy, Methoxy, Ethoxy, Amino, Methyl- amino, Ethylamino, Dimethylamino oder Diethylamino substituiert sein kann,
L² für (C₁-C₃)-Alkandiyl, welches ein- oder zweifach mit Fluor substituiert sein kann, für (C₂-C₃)-Alkendiyl oder für eine Gruppe der Formel *-M-CR⁷R⁸-, *-M-CH₂- CR⁷R⁸- oder *-CH₂-M-CR⁷R⁸- steht, worin
* die Verknüpfungsstelle mit dem Ring Q,
M O oder N-R⁵, worin
R⁵ Wasserstoff, (C₁-C₃)-Alkyl oder Cyclopropyl darstellt,
und
R⁷ und R⁸ unabhängig voneinander Wasserstoff oder Fluor
bedeuten,
Z für eine Gruppe der Formel 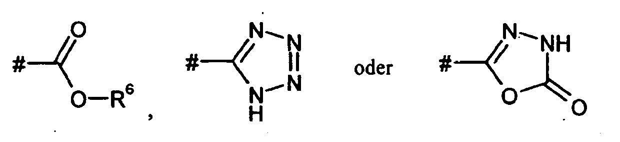 steht, worin
# die Verknüpfungsstelle mit der Gruppe L²
und
R⁶ Wasserstoff, Methyl oder Ethyl bedeutet,
R¹ und R² unabhängig voneinander für einen Substituenten ausgewählt aus der Reihe Fluor, Chlor, Cyano, (C₁-C₅)-Alkyl, (C₂-C₅)-Alkenyl, (C₃-C₆)-Cycloalkyl, (C₄-C₆)- Cycloalkenyl, (C₁-C₄)-Alkoxy, Trifluormethyl, Trifluormethoxy, (C₁-C₄)-Alkyl- thio, (C₁-C₅)-Acyl, Amino, Mono-(C₁-C₄)-alkylamino, Di-(C₁-C₄)-alkylamino und (C₁-C₄)-Acylamino stehen,
oder
zwei an benachbarte Kohlenstoffatome des jeweiligen Phenylrings gebundene Reste R¹ und/oder R² zusammen eine Gruppe der Formel -O-CH₂-O-, -O-CHF-O- oder -O-CF₂-O- bilden,
n und o unabhängig voneinander für die Zahl 0, 1, 2 oder 3 stehen, wobei für den Fall, dass R¹ oder R² mehrfach auftreten, ihre Bedeutungen jeweils gleich oder verschieden sein können,
und
R³ für Wasserstoff oder (C₁-C₃)-Alkyl steht,
sowie ihre Salze, Solvate und Solvate der Salze.

4. Verbindung der Formel (I) nach Anspruch 1, 2 oder 3, in welcher
A für O oder N-R⁴ steht, worin
R⁴ Wasserstoff oder (C₁-C₄)-Alkyl bedeutet,
L¹ für eine Bindung oder für (C₁-C₃)-Alkandiyl steht,
der Ring Q für (C₄-C₆)-Cycloalkyl, (C₅-C₆)-Cycloalkenyl, einen 5- oder 6-gliedrigen Heterocyclus oder Phenyl steht, welche jeweils bis zu zweifach, gleich oder ver- schieden, mit Fluor, Chlor, (C₁-C₃)-Alkyl, Trifluormethyl, Hydroxy, Methoxy, Ethoxy, Trifluormethoxy, Amino, Methylamino, Ethylamino, Dimethylamino und/ oder Diethylamino substituiert sein können,
L² für (C₁-C₃)-Alkandiyl, welches ein- oder zweifach mit Fluor substituiert sein kann, für (C₂-C₃)-Alkendiyl oder für eine Gruppe der Formel *-M-CR⁷R⁸-, *-M-CH₂- CR⁷R⁸- oder *-CH₂-M-CR⁷R⁸- steht, worin
* die Verknüpfungsstelle mit dem Ring Q,
M O oder N-R⁵, worin
R⁵ Wasserstoff oder (C₁-C₃)-Alkyl darstellt,
und
R⁷ und R⁸ unabhängig voneinander Wasserstoff oder Fluor
bedeuten,
Z für eine Gruppe der Formel 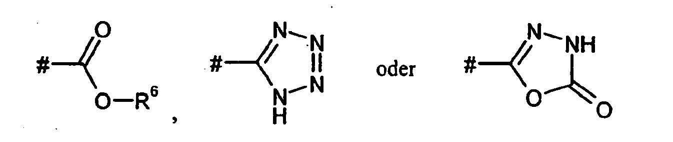 steht, worin
# die Verknüpfungsstelle mit der Gruppe L²
und
R⁶ Wasserstoff, Methyl oder Ethyl bedeutet,
R¹ und R² unabhängig voneinander für einen Substituenten ausgewählt aus der Reihe Fluor, Chlor, Cyano, (C₁-C₃)-Ancyl, (C₂-C₅)-Alkenyl, (C₃-C₆)-Cycloalkyl, (C₄-C₆)- Cycloalkenyl, (C₁-C₄)-Alkoxy, Trifluormethyl, Trifluormethoxy, (C₁-C₄)-Alkyl- thio, (C₁-C₅)-Acyl, Amino, Mono-(C₁-C₄)-alkylamino, Di-(C₁-C₄)-alkylamino und (C₁-C₄)-Acylamino stehen,
oder
zwei an benachbarte Kohlenstoffatome des jeweiligen Phenylrings gebundene Reste R¹ und/oder R² zusammen eine Gruppe der Formel -O-CH₂-O-, -O-CHF-O- oder -O-CF₂-O- bilden,
n und o unabhängig voneinander für die Zahl 0, 1 oder 2 stehen,
wobei für den Fall, dass R¹ oder R² zweifach auftreten, ihre Bedeutungen jeweils gleich oder verschieden sein können,
und
R³ für Wasserstoff oder (C₁-C₃)-Alkyl steht,
sowie ihre Salze, Solvate und Solvate der Salze.

5. Verbindung der Formel (I) nach einem der Ansprüche 1 bis 4, in welcher
A für O oder NH steht,
L¹ für eine Bindung, Methylen, Ethan-1,1-diyl oder Ethan-1,2-diyl steht,
der Ring Q für Cyclobutyl, Cyclopentyl, Cyclopentenyl, Cyclohexyl, Pyrrolidinyl, Piperidinyl, Tetrahydrofuranyl, Tetrahydropyranyl, Morpholinyl oder Phenyl steht, welche jeweils bis zu zweifach, gleich oder verschieden, mit Fluor, Methyl, Ethyl, Trifluormethyl, Hydroxy, Methoxy, Ethoxy, Amino, Methylamino und/oder Di- methylamino substituiert sein können,
L² für (C₁-C₃)-Alkandiyl, (C₂-C₃)-Alkendiyl oder eine Gruppe der Formel *-M-CH₂- oder *-M-CH₂-CH₂- steht, worin
* die Verknüpfungsstelle mit dem Ring Q
und
M O oder N-R⁵, worin
R⁵ Wasserstoff oder (C₁-C₃)-Alkyl darstellt,
bedeutet,
bedeutet,
Z für eine Gruppe der Formel 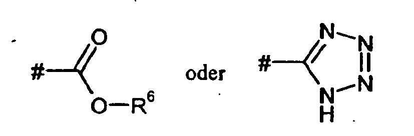 steht, worin
# die Verknüpfungsstelle mit der Gruppe L²
und
R⁶ Wasserstoff, Methyl oder Ethyl bedeutet,
R¹ und R² unabhängig voneinander für einen Substituenten ausgewählt aus der Reihe Fluor, Chlor, Cyano, (C₁-C₅)-Alkyl, (C₂-C₅)-Alkenyl, (C₃-C₆)-Cycloalkyl, (C₄-C₆)- Cycloalkenyl, (C₁-C₄)-Alkoxy, Trifluormethyl, Trifluormethoxy, (C₁-C₄)-Alkyl- thio, (C₁-C₅)-Acyl, Amino, Mono-(C₁-C₄)-alkylamino, Di-(C₁-C₄)-alkylamino und (C₁-C₄)-Acylamino stehen,
oder
zwei an benachbarte Kohlenstoffatome des jeweiligen Phenylrings gebundene Reste R¹ und/oder R² zusammen eine Gruppe der Formel -O-CH₂-O-, -O-CHF-O- oder -O-CF₂-O- bilden,
n und o unabhängig voneinander für die Zahl 0, 1 oder 2 stehen,
wobei für den Fall, dass R¹ oder R² zweifach auftreten, ihre Bedeutungen jeweils gleich oder verschieden sein können,
und
R³ für Wasserstoff steht,
sowie ihre Salze, Solvate und Solvate der Salze.

6. Verbindung der Formel (I) nach einem der Ansprüche 1 bis 5, in welcher
A für O oder NH steht,
L¹ für eine Bindung, Methylen oder Ethan-1,1-diyl steht,
der Ring Q für Cyclobutyl, Cyclopentyl, Cyclopentenyl, Cyclohexyl, Pyrrolidinyl, Piperidinyl oder Phenyl steht, welche jeweils bis zu zweifach, gleich oder ver- schieden, mit Fluor, Methyl, Hydroxy und/oder Methoxy substituiert sein können,
L² für (C₁-C₃)-Alkandiyl, (C₂-C₃)-Alkendiyl oder eine Gruppe der Formel *-M-CH₂- oder *-M-CH₂-CH₂- steht, worin
* die Verknüpfungsstelle mit dem Ring Q
und
M O oder NH bedeutet,
Z für eine Gruppe der Formel 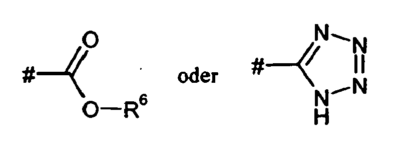 steht, worin
# die Verknüpfungsstelle mit der Gruppe L²
und
R⁶ Wasserstoff, Methyl oder Ethyl bedeutet,
R¹ für einen Substituenten ausgewählt aus der Reihe Fluor, Chlor, Methyl, Ethyl, Vinyl, Trifluormethyl und Methoxy steht,
R² für einen Substituenten ausgewählt aus der Reihe Fluor, Chlor, Cyano, Methyl, Ethyl, n-Propyl, Vinyl, Trifluormethyl, Methoxy, Ethoxy, Trifluormethoxy; Methylthio, Ethylthio, Amino, Methylamino und Ethylamino steht,
n und o unabhängig voneinander für die Zahl 0, 1 oder 2 stehen, wobei für den Fall, dass R¹ oder R² zweifach auftreten, ihre Bedeutungen jeweils gleich oder verschieden sein können,
und
R³ für Wasserstoff steht,
sowie ihre Salze, Solvate und Solvate der Salze.

7. Verfahren zur Herstellung von Verbindungen der Formel (I), wie in den Ansprüchen 1 bis 6 definiert, in welcher Z für -COOH oder -C(=O)-COOH steht, **dadurch gekennzeichnet, dass** man entweder
[A] Verbindungen der Formel (II) 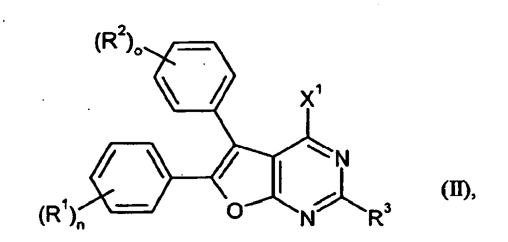 in welcher R¹, R², R³, n und o jeweils die in den Ansprüchen 1 bis 6 angegebenen Bedeutungen haben
und
X¹ für eine Abgangsgruppe wie beispielsweise Halogen, insbesondere für Chlor steht,
in Gegenwart einer Base gegebenenfalls in einem inerten Lösungsmittel mit einer Verbindung der Formel (III) 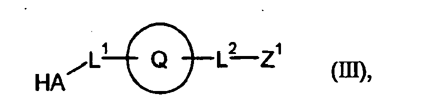 in welcher A, L¹, L² und Q jeweils die in den Ansprüchen 1 bis 6 angegebenen Bedeutungen haben
und
Z¹ für Cyano oder eine Gruppe der Formel -[C(O)]_{y}-COOR^{6A} steht, worin
y die Zahl 0 oder 1
und
R^{6A} (C₁-C₄)Alkyl bedeutet,
zu Verbindungen der Formel (IV) 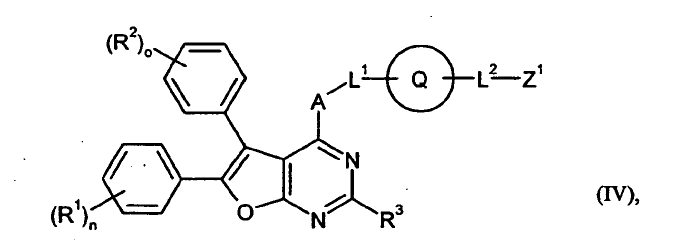 in welcher A, L¹, L², Q, Z¹, R¹, R², R³, n und o jeweils die oben angegebenen Bedeutungen haben,
umsetzt
oder
[B] Verbindungen der Formel (V-1) 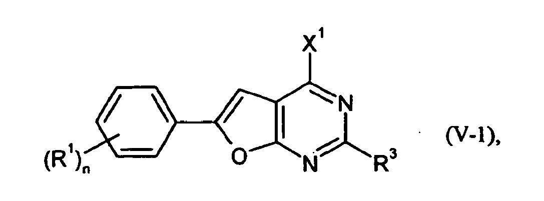 in welcher R¹, R³, X¹ und n jeweils die in den Ansprüchen 1 bis 6 angegebenen Bedeutungen haben,
in Gegenwart einer Base gegebenenfalls in einem inerten Lösungsmittel mit einer Verbindung der Formel (III) zu Verbindungen der Formel (VI-1) 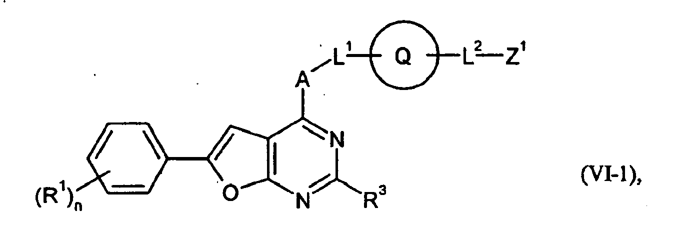 in welcher A, L¹, L², Q, Z¹, R¹, R³ und n jeweils die oben angegebenen Bedeutungen haben,
umsetzt, dann in einem inerten Lösungsmittel zu Verbindungen der Formel (VII-1) 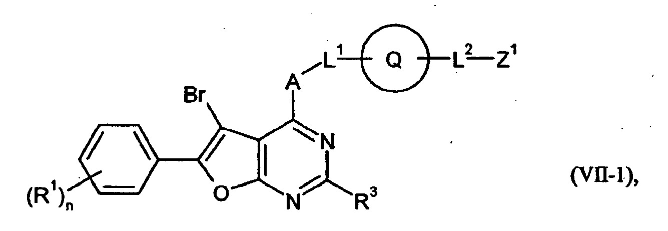 in welcher A, L¹, L², Q, Z¹, R¹, R³ und n jeweils die oben angegebenen Bedeutungen haben,
bromiert und diese anschließend in einem inerten Lösungsmittel in Gegenwart einer Base und eines geeigneten Palladium-Katalysators mit einer Phenylboronsäure der Formel (VIII-1) 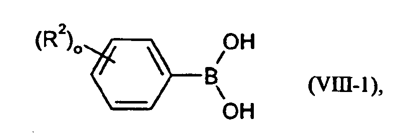 in welcher R² und o die in den Ansprüchen 1 bis 6 angegebenen Bedeutungen haben,
zu Verbindungen der Formel (IV) kuppelt
oder
[C] Verbindungen der Formel (V-2) 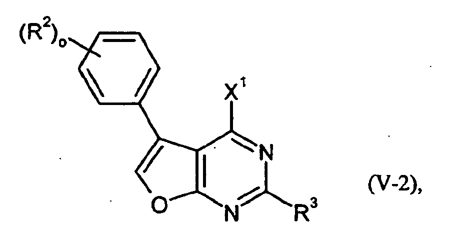 in welcher R², R³, X¹ und o jeweils die in den Ansprüchen 1 bis 6 angegebenen Bedeutungen haben,
in Gegenwart einer Base gegebenenfalls in einem inerten Lösungsmittel mit einer Verbindung der Formel (III) zu Verbindungen der Formel (VI-2) 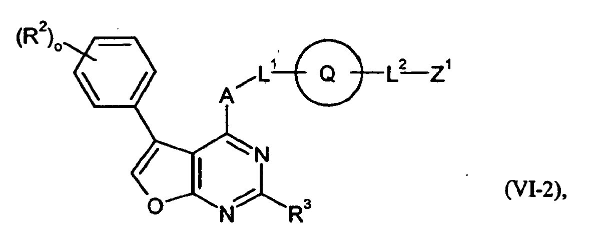 in welcher A, L¹, L², Q, Z¹, R², R³ und o jeweils die oben angegebenen Bedeutungen haben,
umsetzt, dann in einem inerten Lösungsmittel zu Verbindungen der Formel (VII-2) 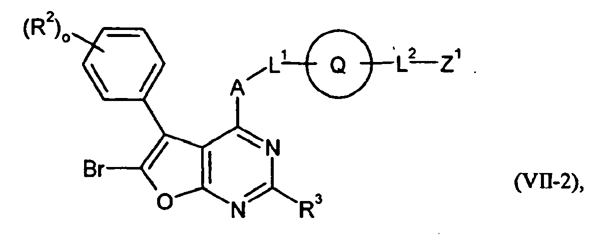 in welcher A, L¹, L², Q, Z¹, R², R³ und o jeweils die oben angegebenen Bedeutungen haben,
bromiert und diese anschließend in einem inerten Lösungsmittel in Gegenwart einer Base und eines geeigneten Palladium-Katalysators mit einer Phenylboronsäure der Formel (VIII-2) 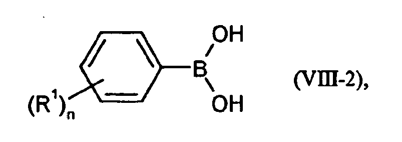 in welcher R¹ und n die in den Ansprüchen 1 bis 6 angegebenen Bedeutungen haben,
zu Verbindungen der Formel (IV) kuppelt,
und die jeweils resultierenden Verbindungen der Formel (IV) dann durch Hydrolyse der Ester- bzw. Cyano-Gruppe Z¹ in die Carbonsäuren der Formel (I-A) 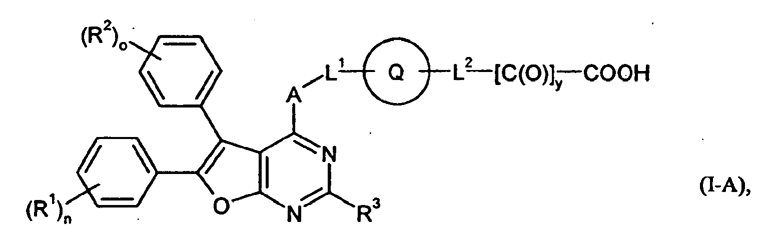 in welcher A, L¹, L², Q, R¹, R², R³, n, o und y jeweils die oben angegebenen Bedeutungen haben,
überführt und diese gegebenenfalls mit den entsprechenden (i) Lösungsmitteln und/oder (ii) Basen oder Säuren zu ihren Solvaten, Salzen und/oder Solvaten der Salze umsetzt.

8. Verbindung der Formel (I), wie in einem der Ansprüche 1 bis 6 definiert, zur Behandlung und/oder Prophylaxe von Krankheiten.

9. Verwendung einer Verbindung der Formel (I), wie in einem der Ansprüche 1 bis 6 definiert, zur Herstellung eines Arzneimittels zur Behandlung und/oder Prophylaxe von kardiovaskulären Erkrankungen.

10. Arzneimittel enthaltend eine Verbindung der Formel (I), wie in einem der Ansprüche 1 bis 6 definiert, in Kombination mit einem inerten, nicht-toxischen, pharmazeutisch geeigneten Hilfsstoff.

11. Arzneimittel enthaltend eine Verbindung der Formel (I), wie in einem der Ansprüche 1 bis 6 definiert, in Kombination mit einem weiteren Wirkstoff.

12. Arzneimittel nach Anspruch 10 oder 11 zur Behandlung und/oder Prophylaxe von kardiovaskulären Erkrankungen.

## Claims

1. Compound of formula (I) 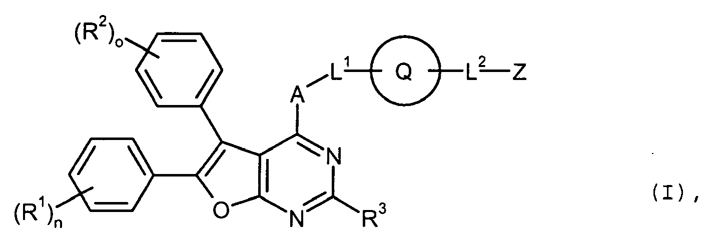 in which
A stands for O, S or N-R⁴ where
R⁴ denotes hydrogen, (C₁-C₆) alkyl, (C₃-C₇) cycloalkyl or (C₄-C₇) cycloalkenyl,
L¹ stands for a bond or for (C₁-C₄) alkanediyl,
the Q ring stands for (C₃-C₇) cycloalkyl, (C₄-C₇) cycloalkenyl, a 5- to 7-membered heterocycle, phenyl or 5- or 6-membered heteroaryl, each of which may be up to disubstituted, identically or differently, by fluorine, chlorine, (C₁-C₄) alkyl, trifluoromethyl, hydroxyl, (C₁-C₄) alkoxy, trifluoromethoxy, amino, mono-(C₁-C₄) alkylamino and/or di-(C₁-C₄) alkylamino,
where (C₁-C₄) alkyl may in turn be substituted by hydroxyl, (C₁-C₄) alkoxy, amino, mono- or di-(C₁- C₄) alkylamino,
L² stands for (C₁-C₄) alkanediyl, which is mono- or disubstituted by fluorine and in which one methylene group may be exchanged for O or N-R⁵ in which
R⁵ denotes hydrogen, (C₁-C6) alkyl or (C₃-C₇) cycloalkyl,
or stands for (C₂-C₄) alkenediyl,
Z stands for a group of formula 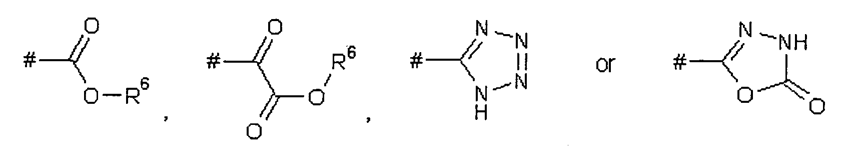 where
# denotes the point of linkage with group L²
and
R⁶ denotes hydrogen or (C₁-C₄) alkyl,
R¹ and R², independently of one another, stand for a substituent selected from the group comprising halogen, cyano, nitro, (C₁-C₆) alkyl, (C₂-C₆) alkenyl, (C₂-C₄) alkinyl, (C₃-C₇) cycloalkyl, (C₄- C₇) cycloalkenyl, (C₁-C₆) alkoxy, trifluoromethyl, trifluoromethoxy, (C₁-C₆) alkylthio, (C₁-C₆) acyl, amino, mono- (C₁-C₆) alkylamino, di-(C₁-C₆) alkylamino and (C₁-C₆) acylamino,
in which (C₁-C₆) alkyl and (C₁-C₆) alkoxy can in their turn each be substituted with cyano, hydroxy, (C₁-C₄) alkoxy, (C₁-C₄) alkylthio, amino, mono- or di-(C₁-C₄) alkylamino,
or
two residues R¹ and/or R² bound to adjacent carbon atoms of the respective phenyl ring together form a group of formule -O-CH₂-O-, -O-CHF-O-, -O-CF₂-O-, -O-CH₂-CH₂-O- or -O-CF₂-CF₂-O-,
n and o, independently of one another, stand for the number 0, 1, 2 or 3,
and for the case when R¹ or R² occurs more than once, their meanings can in each case be identical or different,
and
R³ stands for hydrogen, (C₁-C₄) alkyl or cyclopropyl,
and their salts, solvates and solvates of the salts.

2. Compound of formula (I) according to Claim 1, in which
A stands for O, S or N-R⁴, where
R⁴ denotes hydrogen, (C₁-C₆) alkyl, (C₃-C₇) cycloalkyl or (C₄-C₇) cycloalkenyl,
L¹ stands for a bond or for (C₁-C₄) alkanediyl,
the Q ring stands for (C₃-C₇) cycloalkyl, (C₁-C₇) cycloalkenyl, a 5- to 7-membered heterocycle, phenyl or 5- or 6-membered heteroaryl, each of which may be up to disubstituted, identically or differently, by fluorine, chlorine, (C₁-C₄) alkyl, trifluoromethyl, hydroxyl, (C₁-C₄) alkoxy, trifluoromethoxy, amino, mono-(C₁-C₄) alkylamino and/or di-(C₁-C₄) alkylamino,
where (C₁-C₄) alkyl may in turn be substituted by hydroxyl, (C₁-C₄) alkoxy, amino, mono- or di-(C₁- C₄) alkylamino,
L² stands for (C₁-C₄) alkanediyl, which is mono- or disubstituted by fluorine and in which one methylene group may be exchanged for O or N-R⁵ in which
R⁵ denotes hydrogen, (C₁-C₆) alkyl or (C₃-C₇) cycloalkyl,
or stands for (C₂-C₄) alkenediyl,
Z stands for a group of formula 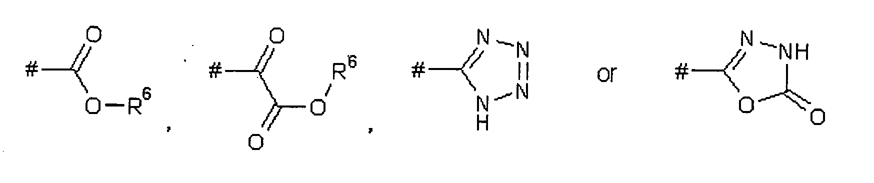 where
# denotes the point of linkage with group L²
and
R² denotes hydrogen or (C₁-C₄) alkyl,
R¹ and R², independently of one another, stand for a substituent selected from the group comprising halogen, cyano, nitro, (C₁-C₆) alkyl, (C₂-C₆) alkenyl, (C₂-C₄) alkinyl, (C₃-C₇) cycloalkyl, (C₄- C₇) cycloalkenyl, (C₁-C₆) alkoxy, trifluoromethyl, trifluoromethoxy, (C₁-C₆) alkylthio, (C₁-C₆) acyl, amino, mono-(C₁-C₆) alkylamino, di-(C₁-C₆) alkylamino and (C₁-C₆) acylamino,
and (C₁-C₆) alkyl and (C₁-C₆) alkoxy in their turn can each be substituted with hydroxy, (C₁-C₄) alkoxy, amino, mono- or di-(C₁-C₄) alkylamino,
or
two residues R¹ and/or R² bound to adjacent carbon atoms of the respective phenyl ring together form a group of formula -O-CH₂-O-, -O-CHF-O-, -O-CF₂-O-, -O-CH₂-CH₂-O- or -O-CF₂-CF₂-O-,
n and o, independently of one another, stand for the number 0, 1, 2 or 3, and for the case when R¹ or R²occurs more than once, their meanings can in each case be identical or different,
and
R³ stands for hydrogen, (C₁-C₄) alkyl or cyclopropyl,
and their salts, solvates and solvates of the salts.

3. Compound of formula (I) according to Claim 1 or 2, in which
A stands for O or N-R⁴, where
R⁴ denotes hydrogen, (C₁-C₄) alkyl or (C₃-C₆) cycloalkyl,
L¹ stands for a bond or (C₁-C₃) alkanediyl,
the Q ring stands for (C₃-C₆) cycloalkyl, (C₄-C₆) cycloalkenyl, a 5- or 6-membered heterocycle, phenyl or 5- or 6-membered heteroaryl, each of which may be up to disubstituted, identically or differently, by fluorine, chlorine, (C₁-C₃) alkyl, trifluoromethyl, hydroxyl, methoxy, ethoxy, trifluoromethoxy, amino, methylamino, ethylamino, dimethylamino and/or diethylamino,
where (C₁-C₃) alkyl may in turn be substituted by hydroxyl, methoxy, ethoxy, amino, methylamino, ethylamino, dimethylamino or diethylamino,
L² stands for (C₁-C₃) alkanediyl which may be mono- or disubstituted by fluorine, (C₂-C₃) alkenediyl or a group of the formula *-M-CR⁷R⁸-, *-M-CH₂- CR⁷R^{B}- or *-CH₂-M-CR⁷R⁸-,
in which
* denotes the point of linkage with the Q ring,
M is O or N-R⁵ in which
R⁵ is hydrogen, (C₁-C₃) alkyl or cyclopropyl,
and
R⁷ and R⁸, independently of one another, are hydrogen or fluorine,
Z stands for a group of formula 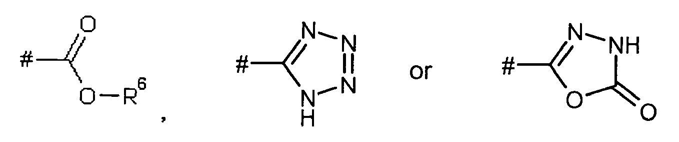 where
# denotes the point of linkage with group L²
and
R⁶ denotes hydrogen, methyl or ethyl,
R¹ and R², independently of one another, stand for a substituent selected from the group comprising fluorine, chlorine, cyano, (C₁-C₅) alkyl, (C₂-C₅) alkenyl, (C₃-C₆) cycloalkyl, (C₄-C₆) cycloalkenyl, (C₁-C₄) alkoxy, trifluoromethyl, trifluoromethoxy, (C₁-C₄) alkylthio, (C₁-C₅) acyl, amino, mono-(C₁-C₄) alkylamino, di-(C₁-C₄) alkylamino and (C₁-C₄) acyl- amino
or
two residues R¹ and/or R² bound to adjacent carbon atoms of the respective phenyl ring together form a group of formula -O-CH₂-O-, -O-CHF-O- or -O- CF₂-O-,
n and o, independently of one another, stand for the number 0, 1, 2 or 3, and for the case when R¹ or R² occurs more than once, their meanings can in each case be identical or different,
and
R³ stands for hydrogen or (C₁-C₃) alkyl,
and their salts, solvates and solvates of the salts.

4. Compound of formula (I) according to Claim 1, 2 or 3, in which
A stands for O or N-R⁴ in which
R⁴ is hydrogen or (C₁-C₄) alkyl,
L¹ stands for a bond or (C₁-C₃) alkanediyl,
the Q ring stands for (C₄-C₆) cycloalkyl, (C₅-C₆) cycloalkenyl, a 5- or 6-membered heterocycle or phenyl, each of which may be up to disubstituted, identially or differently, by fluorine, chlorine, (C₁-C₃) alkyl, trifluoromethyl, hydroxyl, methoxy, ethoxy, trifluoromethoxy, amino, methylamino, ethylamino, dimethylamino and/or diethylamino,
L² stands for (C₁-C₃) alkanediyl which may be mono- or disubstituted by fluorine, (C₂-C₃) alkenediyl or a group of the formula *-M-CR⁷R⁸-, *-M-CH₂- CR⁷R⁸- or *-CH₂-M-CR⁷R⁸-, in which
* denotes the point of linkage with the Q ring,
M is O or N-R⁵ in which
R⁵ is hydrogen or (C₁-C₃) alkyl,
and
R⁷ and R⁸, independently of one another, denote hydrogen or fluorine,
Z stands for a group of the formula 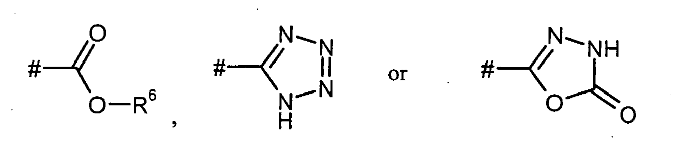 where
# denotes the point of linkage with group L²
and
R⁶ denotes hydrogen, methyl or ethyl,
R¹ and R², independently of one another, stand for a substituent selected from the group comprising fluorine, chlorine, cyano, (C₁-C₅) alkyl, (C₂-C₅) alkenyl, (C₃-C₆) cycloalkyl, (C₄-C₆) cycloalkenyl, (C₁-C₄) alkoxy, trifluoromethyl, trifluoromethoxy, (C₁-C₄) alkylthio, (C₁-C₅) acyl, amino, mono-(C₁-C₄) alkylamino, di-(C₁-C₄) alkylamino and (C₁-C₄) acyl- amino
or
two residues R¹ and/or R² bound to adjacent carbon atoms of the respective phenyl ring together form a group of formula -O-CH₂-O-, -O-CHF-O- or -O- CF₂-O-, independently of one another, stand for the number 0, 1 or 2,
and for the case when R¹ or R² occurs twice, their meanings can in each case be identical or different,
and
R³ stands for hydrogen or (C₁-C₃) alkyl,
and their salts, solvates and solvates of the salts.

5. Compound of formula (I) according to one of Claims 1 to 4, in which
A stands for O or NH,
L¹ stands for a bond, methylene, ethane-1,1-diyl or ethane-1,2-diyl,
the Q ring stands for cyclobutyl, cyclopentyl, cyclopentenyl, cyclohexyl, pyrrolidinyl, piperidinyl, tetrahydrofuranyl, tetrahydropyranyl, morpholinyl or phenyl, each of which may be up to disubstituted, identically or differently, by fluorine, methyl, ethyl, trifluoromethyl, hydroxyl, methoxy, ethoxy, amino, methylamino and/or dimethylamino,
L² stands for (C₁-C₃) alkanediyl, (C₂-C₃) alkenediyl or a group of the formula *-M-CH₂- or *-M-CH₂-CH₂-, in which
* denotes the point of linkage to the Q ring
and
M denotes 0 or N-R⁵, in which
R⁵ is hydrogen or (C₁-C₃) alkyl,
Z stands for a group of formula 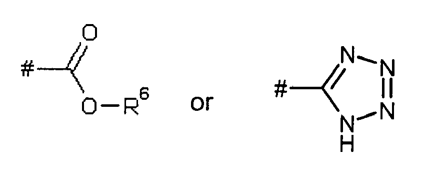 where
# denotes the point of linkage with group L²
and
R⁶ denotes hydrogen, methyl or ethyl,
R¹ and R², independently of one another, stand for a substituent selected from the group comprising fluorine, chlorine, cyano, (C₁-C₅) alkyl, (C₂-C₅) alkenyl, (C₃-C₆) cycloalkyl, (C₄-C₆) cycloalkenyl, (C₁-C₄) alkoxy, trifluoromethyl, trifluoromethoxy, (C₁-C₄) alkylthio, (C₁-C₅) acyl, amino, mono-(C₁-C₄) alkylamino, di-(C₁-C₄) alkylamino and (C₁-C₄) acyl- amino
or
two residues R¹ and/or R² bound to adjacent carbon atoms of the respective phenyl ring together form a group of formula -O-CH₂-O-, -O-CHF-O- or -O- CF₂-O-,
n and o, independently of one another, stand for the number 0, 1 or 2,
and for the case when R¹ or R² occurs twice, their meanings can in each case be identical or different,
and
R³ stands for hydrogen,
and their salts, solvates and solvates of the salts.

6. Compound of formula (I) according to one of the Claims 1 to 5, in which
A stands for O or NH,
L¹ stands for a bond, methylene or ethane-1,1-diyl,
the Q ring stands for cyclobutyl, cyclopentyl, cyclopentenyl, cyclohexyl, pyrrolidinyl, piperidinyl or phenyl, each of which may be up to disubstituted, identically or differently, by fluorine, methyl, hydroxyl and/or methoxy,
L² is (C₁-C₃) alkanediyl, (C₂-C₃) alkenediyl or a group of the formula *-M-CH₂- or *-M-CH₂-CH₂-, in which
* denotes the point of linkage with the Q ring,
and
M denotes O or NH,
Z stands for a group of formula 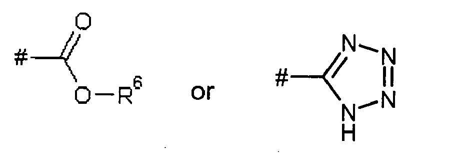 where
# denotes the point of linkage with group L²
and
R⁶ denotes hydrogen, methyl or ethyl,
stands for a substituent selected from the group comprising fluorine, chlorine, methyl, ethyl, vinyl, trifluoromethyl and methoxy,
R² stands for a substituent selected from the group comprising fluorine, chlorine, cyano, methyl, ethyl, n-propyl, vinyl, trifluoromethyl, methoxy, ethoxy, trifluoromethoxy, methylthio, ethylthio, amino, methylamino and ethylamino,
n and o, independently of one another, stand for the number 0, 1 or 2,
and for the case when R¹ or R² occurs twice, their meanings can in each case be identical or different, and
R³ stands for hydrogen,
and their salts, solvates and solvates of the salts.

7. Method of production of compounds of formula (I), as defined in Claims 1 to 6, in which Z stands for -COOH or -C(=O)-COOH, **characterized in that** either
[A] Compounds of formula (II) 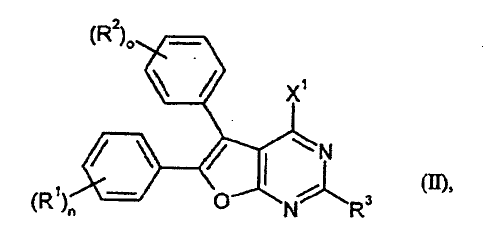 in which R¹, R², R³, n and o have the respective meanings given in Claims 1 to 6
and
X¹ stands for a leaving group such as halogen, especially for chlorine,
in the presence of a base if necessary in an inert solvent with a compound of formula (III) 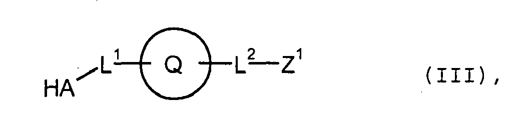 in which A, L¹, L² and Q have the respective meanings given in Claims 1 to 6
and
Z¹ stands for cyano or a group of formula - [C(O)]_{y}-COOR^{6A}, where
y denotes the number 0 or 1
and
R^{6A} denotes (C₁-C₄) alkyl,
are converted to compounds of formula (IV) 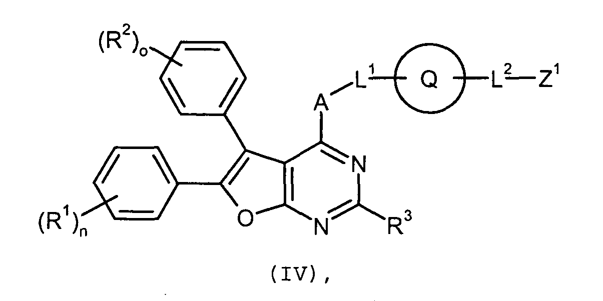 in which A, L¹, L², Q, Z¹, R¹, R², R³, n and o have the respective meanings given above,
or
[B] Compounds of formula (V-1) 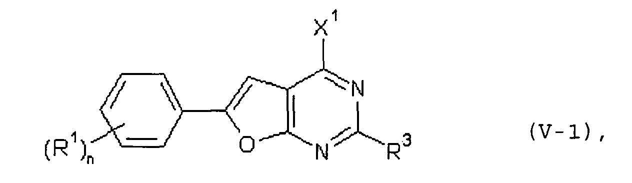 in which R¹, R³, X¹ and n have the respective meanings given in Claims 1 to 6,
are reacted in the presence of a base if necessary in an inert solvent with a compound of formula (III) to compounds of formula (VI-1) 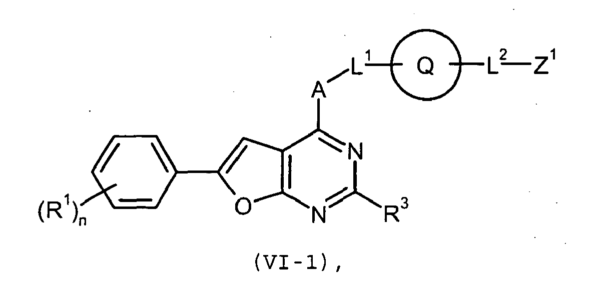 in which A, L¹, L², Q, Z¹, R¹, R³ and n have the respective meanings given above,
then brominated in an inert solvent to compounds of formula (VII-1) 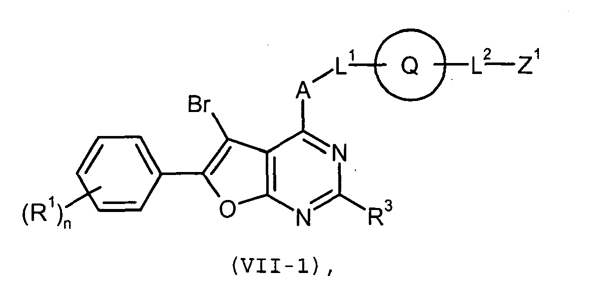 in which A, L¹, L², Q, Z¹, R¹, R³ and n have the respective meanings given above,
and these are then coupled in an inert solvent in the presence of a base and a suitable palladium catalyst with a phenylboronic acid of formula (VIII-1) 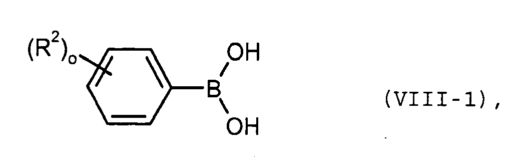 in which R² and o have the meanings given in Claims 1 to 6,
to compounds of formula (IV)
or
[C] Compounds of formula (V-2) 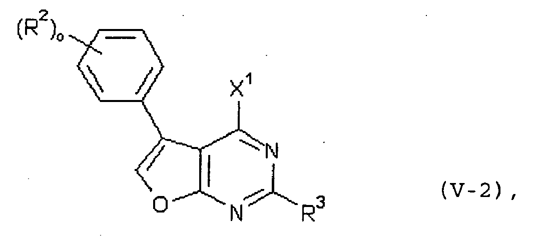 in which R², R³, X¹ and o have the respective meanings given in Claims 1 to 6,
are reacted in the presence of a base if necessary in an inert solvent with a compound of formula (III) to compounds of formula (VI-2) 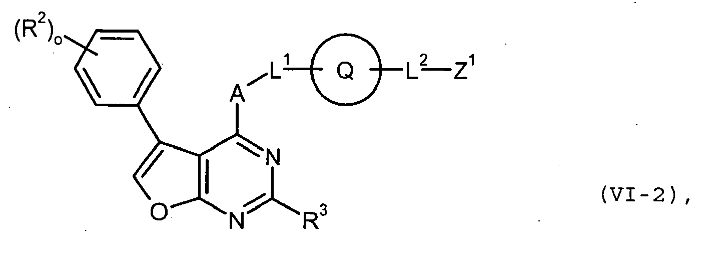 in which A, L¹, L², Q, Z¹, R², R³ and o have the respective meanings given above,
then brominated in an inert solvent to compounds of formula (VII-2) 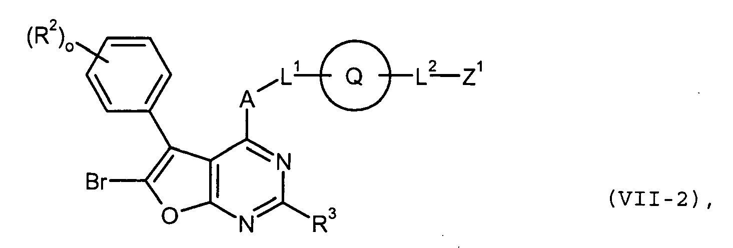 in which A, L¹, L², Q, Z¹, R², R³ and o have the respective meanings given above,
and these are then coupled in an inert solvent in the presence of a base and a suitable palladium catalyst with a phenylboronic acid of formula (VIII-2) 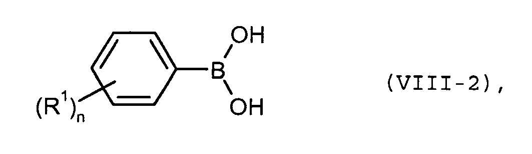 in which R¹ and n have the meanings given in Claims 1 to 6,
to compounds of formula (IV),
and in each case the resultant compounds of formula (IV) are then converted by hydrolysis of the ester or cyano group Z¹ to the carboxylic acids of formula (I-A) 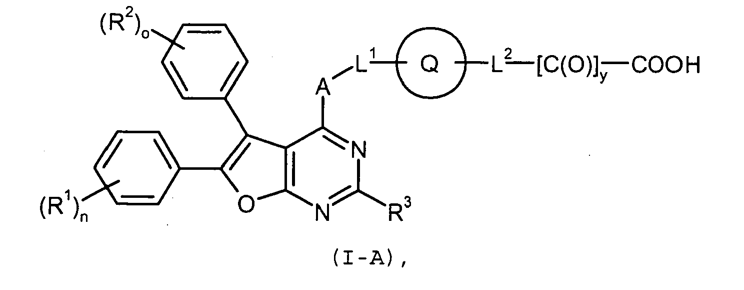 in which A, L¹, L², Q, R¹, R², R³, n, o and y have the respective meanings given above,
and these are converted if necessary with the corresponding (i) solvents and/or (ii) bases or acids to their solvates, salts and/or solvates of the salts.

8. Compound of formula (I), as defined in one of the Claims 1 to 6, for the treatment and/or prophylaxis of diseases.

9. Use of a compound of formula (I) , as defined in one of the Claims 1 to 6, for the production of a medicinal product for the treatment and/or prophylaxis of cardiovascular diseases.

10. Medicinal product containing a compound of formula (I), as defined in one of the Claims 1 to 6, in combination with an inert, nontoxic, pharmaceutically acceptable excipient.

11. Medicinal product containing a compound of formula (I), as defined in one of the Claims 1 to 6, in combination with another active substance.

12. Medicinal product according to Claim 10 or 11 for the treatment and/or prophylaxis of cardiovascular diseases.

## Revendications

1. Composé de formule (I) 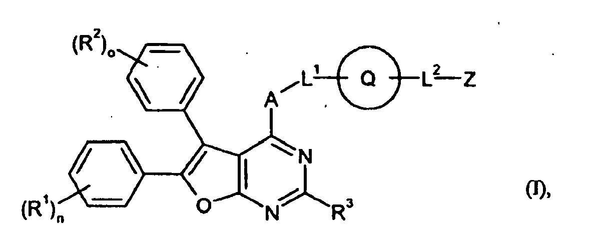 où
A représente O, S ou N-R⁴, où
R⁴ représente hydrogène, alkyle en C₁-C₆, cycloalkyle en C₃-C₇ ou cycloalcényle en C₄-C₇,
L¹ représente une liaison ou (alcane en C₁-C₄)-diyle,
le cycle Q représente cycloalkyle en C₃-C₇, cycloalcényle en C₄-C₇, un hétérocycle à 5 à 7 chaînons, phényle ou un hétéroaryle à 5 ou 6 chaînons, lesquels peuvent être substitués jusqu'à deux fois, de manière identique ou différente, par fluor, chlore, alkyle en C₁-C₄, trifluorométhyle, hydroxy, alcoxy en C₁-C₄, trifluoromé- thoxy, amino, mono-(alkyle en C₁-C₄)amino et/ou di-(alkyle en C₁-C₄)amino,
alkyle en C₁-C₄ pouvant de son côté être substitué par hydroxy, alcoxy en C₁-C₄, amino, mono- ou di-(alkyle en C₁-C₄)amino,
L² représente (alcane en C₁-C₄)diyle, lequel peut être substitué une ou deux fois par fluor et dans lequel un groupe méthylène peut être remplacé par O ou N-R⁵, où
R⁵ représente hydrogène, alkyle en C₁-C₆ ou cycloalkyle en C₃-C₇,
ou (alcène en C₂-C₄)diyle,
Z représente un groupe de formule 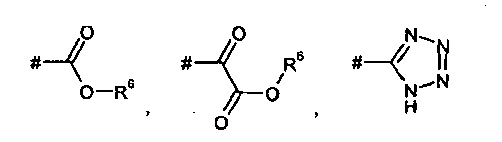 ou 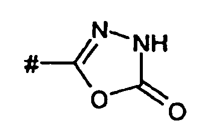 où
# représente le site de liaison avec le groupe L²
et
R⁶ représente hydrogène ou alkyle en C₁-C₄,
R¹ et R² représentent indépendamment l'un de l'autre un substituant de la série halogène, cyano, nitro, alkyle en C₁-C₆, alcényle en C₂- C₆, alcynyle en C₂-C₄, cycloalkyle en C₃-C₇, cycloalcényle en C₄- C₇, alcoxy en C₁-C₆, trifluorométhyle, trifluorométhoxy, (alkyle en C₁-C₆)thio, acyle en C₁-C₆, amino, mono-(alkyle en C₁- C₆)amino, di-(alkyle en C₁-C₆)amino et (acyle en C₁-C₅)amino, alkyle en C₁-C₆ et alcoxy en C₁-C₆ pouvant de leur côté être substitués par cyano, hydroxy, alcoxy en C₁-C₄, (alkyle en C₁-C₄)thio, amino, mono- ou di-(alkyle en C₁-C₄)amino,
ou
deux résidus R¹ et/ou R² liés à des atomes de carbone voisins du cycle phényle considéré forment un groupe de formule -O- CH₂-O-, -O-CHF-O-, -OCF₂-O-, -O-CH₂-CH₂-O- ou -O-CF₂-CF₂-O-,
n et o indépendamment l'un de l'autre représentent le chiffre 0, 1, 2 ou 3, étant entendu que dans le cas où R¹ ou R² sont présents plusieurs fois, leurs significations peuvent être identiques ou différentes,
et
R³ représente hydrogène, alkyle en C₁-C₄ ou cyclopropyle,
ainsi que ses sels, solvates ou solvates des sels.

2. Composé de formule (I) selon la revendication 1, où
A représente O, S ou N-R⁴, où
R⁴ représente hydrogène, alkyle en C₁-C₆, cycloalkyle en C₃-C₇ ou cycloalcényle en C₄-C₇,
L¹ représente une liaison ou (alcane en C₁-C₄)-diyle,
le cycle Q représente cycloalkyle en C₃-C₇, cycloalcényle en C₁-C₇, un hétérocycle à 5 à 7 chaînons, phényle ou un hétéroaryle à 5 ou 6 chaînons, lesquels peuvent être substitués jusqu'à deux fois, de manière identique ou différente, par fluor, chlore, alkyle en C₁-C₄, trifluorométhyle, hydroxy, alcoxy en C₁-C₄, trifluoromé- thoxy, amino, mono-(alkyle en C₁-C₄)amino et/ou di-(alkyle en C₁-C₄)amino,
alkyle en C₁-C₄ pouvant de son côté être substitué par hydroxy, alcoxy en C₁-C₄, amino, mono- ou di-(alkyle en C₁-C₄)amino,
L² représente (alcane en C₁-C₄)diyle, lequel peut être substitué une ou deux fois par fluor et dans lequel un groupe méthylène peut être remplacé par O ou N-R⁵, où
R⁵ représente hydrogène, alkyle en C₁-C₆ ou cycloalkyle en C₃-C₇,
ou (alcène en C₂-C₄)diyle,
Z représente un groupe de formule 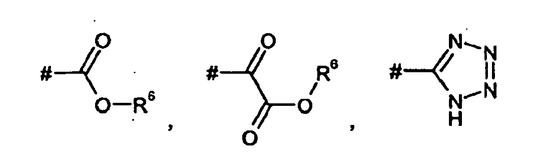 ou 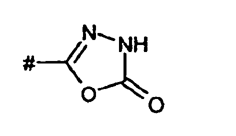 où
# représente le site de liaison avec le groupe L² et
R⁶ représente hydrogène ou alkyle en C₁-C₄,
R¹ et R² représentent indépendamment l'un de l'autre un substituant de la série halogène, cyano, nitro, alkyle en C₁-C₆, alcényle en C₂- C₆, alcynyle en C₂-C₄, cycloalkyle en C₃-C₇, cycloalcényle en C₄- C₇, alcoxy en C₁-C₆, trifluorométhyle, trifluorométhoxy, (alkyle en C₁-C₆)thio, acyle en C₁-C₆, amino, mono-(alkyle en C₁- C₆)amino, di-(alkyle en C₁-C₆)amino et (acyle en C₁-C₆)amino, alkyle en C₁-C₆ et alcoxy en C₁-C₆ pouvant de leur côté être substitués par hydroxy, alcoxy en C₁-C₄, amino, mono- ou di- (alkyle en C₁-C₄)amino,
ou
deux résidus R¹ et/ou R² liés à des atomes de carbone voisins du cycle phényle considéré forment un groupe de formule -O- CH₂-O-, -O-CHF-O-, -OCF₂-O -O-CH₂-CH₂-O- ou -O-CF₂-CF₂-O-,
n et o indépendamment l'un de l'autre représentent le chiffre 0, 1, 2 ou 3, étant entendu que dans le cas où R¹ ou R² sont présents plusieurs fois, leurs significations peuvent être identiques ou différentes,
et
R³ représente hydrogène, alkyle en C₁-C₄ ou cyclopropyle,
ainsi que ses sels, solvates ou solvates des sels.

3. Composé de formule (I) selon la revendication 1 ou 2, où
A représente O ou N-R⁴, où
R⁴ représente hydrogène, alkyle en C₁-C₄ ou cycloalkyle en C₃-C₆,
L¹ représente une liaison ou (alcane en C₁-C₃)-diyle,
le cycle Q représente cycloalkyle en C₃-C₆, cycloalcényle en C₄-C₆, un hétérocycle à 5 à 6 chaînons, phényle ou un hétéroaryle à 5 ou 6 chaînons, lesquels peuvent être substitués jusqu'à deux fois, de manière identique ou différente, par fluor, chlore, alkyle en C₁-C₃, trifluorométhyle, hydroxy, méthoxy, éthoxy, trifluoromé- thoxy, amino, méthylamino, éthylamino, diméthylamino et/ou diéthylamino,
alkyle en C₁-C₃ pouvant de son côté être substitué par hydroxy, méthoxy, éthoxy, amino, méthylamino, éthylamino, diméthyl- amino ou diéthylamino,
L² représente (alcane en C₁-C₃)diyle, lequel peut être substitué une ou deux fois par fluor, (alcène en C₂-C₃)diyle ou un groupe de formule *-M-CR⁷R⁸-, *-M-CH₂-CR⁷R⁸- ou *-CH₂-M- CR⁷R⁸- où
* représente le site de liaison avec le cycle Q, M représente O ou N-R⁵, où
R⁵ représente hydrogène, alkyle en C₁-C₃ ou cyclopropyle,
et
R⁷ et R⁸ représentent indépendamment l'un de l'autre hydrogène ou fluor
Z représente un groupe de formule 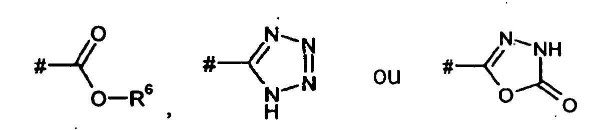 où
# représente le site de liaison avec le groupe L² et
R⁶ représente hydrogène, méthyle ou éthyle,
R¹ et R² représentent indépendamment l'un de l'autre un substituant de la série fluor, chlore, cyano, alkyle en C₁-C₅, alcényle en C₂-C₅, cycloalkyle en C₃-C₆, cycloalcényle en C₄-C₆, alcoxy en C₁-C₄, trifluorométhyle, trifluorométhoxy, (alkyle en C₁-C₄)thio, acyle en C₁-C₅, amino, mono-(alkyle en C₁-C₄)amino, di-(alkyle en C₁- C₄)amino et (acyle en C₁C₄)amino,
ou
deux résidus R¹ et/ou R² liés à des atomes de carbone voisins du cycle phényle considéré forment un groupe de formule -O- CH₂-O-, -O-CHF-O- ou -OCF₂-O-,
n et o indépendamment l'un de l'autre représentent le chiffre 0, 1, 2 ou 3, étant entendu que dans le cas où R¹ ou R² sont présents plusieurs fois, leurs significations peuvent être identiques ou différentes,
et
R³ représente hydrogène ou alkyle en C₁-C₃,
ainsi que ses sels, solvates ou solvates des sels.

4. Composé de formule (I) selon la revendication 1, 2 ou 3, où
A représente O ou N-R⁴, où
R⁴ représente hydrogène ou alkyle en C₁-C₄,
L¹ représente une liaison ou (alcane en C₁-C₃)-diyle,
le cycle Q représente cycloalkyle en C₄-C₆, cycloalcényle en C₅-C₆, un hétérocycle à 5 à 6 chaînons ou phényle, lesquels peuvent être substitués jusqu'à deux fois, de manière identique ou différente, par fluor, chlore, alkyle en C₁-C₃, trifluorométhyle, hydroxy, méthoxy, éthoxy, trifluorométhoxy, amino, méthylamino, éthylamino, diméthylamino et/ou diéthylamino,
L² représente (alcane en C₁-C₃)diyle, lequel peut être substitué une fois ou deux fois par fluor, (alcène en C₂-C₃)diyle ou un groupe de formule *-M-CR⁷R⁸-, *-M-CH₂-CR⁷R⁸- ou *-CH₂-M- CR⁷R⁸-, où
* représente le site de liaison avec le cycle Q,
M représente O ou N-R⁵, où
R⁵ représente hydrogène ou alkyle en C₁-C₃,
et
R⁷ et R⁸ représentent indépendamment l'un de l'autre hydrogène ou fluor,
Z représente un groupe de formule 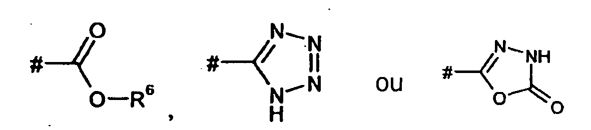 où
# représente le cycle de liaison avec le groupe L² et
R⁶ représente hydrogène, méthyle ou éthyle,
R¹ et R² représentent indépendamment l'un de l'autre un substituant de la série fluor, chlore, cyano, alkyle en C₁-C₅, alcényle en C₂-C₅, cycloalkyle en C₃-C₆, cycloalcényle en C₄-C₆, alcoxy en C₁-C₄, trifluorométhyle, trifluorométhoxy, (alkyle en C₁-C₄)thio, acyle en C₁-C₅, amino, mono-(alkyle en C₁-C₄)amino, di-(alkyle en C₁- C₄)amino et (acyle en C₁-C₄)amino,
ou
deux résidus R¹ et/ou R² liés à des atomes de carbone voisins du cycle phényle considéré forment un groupe de formule -O- CH₂-O-, -O-CHF-O- ou -OCF₂-O-,
n et o indépendamment l'un de l'autre représentent le chiffre 0, 1 ou 2, étant entendu que dans le cas où R¹ ou R² sont présents plusieurs fois, leurs significations peuvent être identiques ou différentes,
et
R³ représente hydrogène ou alkyle en C₁-C₃,
ainsi que ses sels, solvates ou solvates des sels.

5. Composé de formule (I) selon l'une des revendications 1 à 4, où
A représente O ou NH,
L¹ représente une liaison ou méthylène, éthane-1,1-diyle ou éthane-1,2-diyle
le cycle Q représente cyclobutyle, cyclopentyle, cyclopentényle, cyclohexyle, pyrrolidinyle, pipéridinyle, tétrahydrofuranyle, tétrahydropyranyle, morpholinyle ou phényle, lesquels peuvent être substitués jusqu'à deux fois, de manière identique ou différente, par fluor, méthyle, éthyle, trifluorométhyle, hydroxy, méthoxy, éthoxy, amino, méthylamino et/ou diméthylamino,
L² représente (alcane en C₁-C₃)diyle, (alcène en C₂-C₃)diyle ou un groupe de formule *-M-CH₂- ou *-M-CH₂-CH₂-, où
* représente le site de liaison avec le cycle Q et
M représente O ou N-R⁵, où
R⁵ représente hydrogène ou alkyle en C₁-C₃,
Z représente un groupe de formule 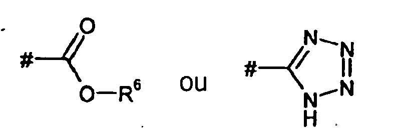 où
# représente le cycle de liaison avec le groupe L² et
R⁶ représente hydrogène, méthyle ou éthyle,
R¹ et R² représentent indépendamment l'un de l'autre un substituant de la série fluor, chlore, cyano, alkyle en C₁-C₅, alcényle en C₂-C₅, cycloalkyle en C₃-C₆, cycloalcényle en C₄-C₆, alcoxy en C₁-C₄, trifluorométhyle, trifluorométhoxy, (alkyle en C₁-C₄)thio, acyle en C₁-C₅, amino, mono-(alkyle en C₁-C₄)amino, di-(alkyle en C₁- C₄)amino et (acyle en C₁-C₄)amino,
ou
deux résidus R¹ et/ou R² liés à des atomes de carbone voisins du cycle phényle considéré forment un groupe de formule -O- CH₂-O -O-CHF-O- ou -OCF₂-O-,
n et o indépendamment l'un de l'autre représentent le chiffre 0, 1 ou 2, étant entendu que dans le cas où R¹ ou R² sont présents plusieurs fois, leurs significations peuvent être identiques ou différentes,
et
R³ représente hydrogène,
ainsi que ses sels, solvates ou solvates des sels.

6. Composé de formule (I) selon l'une des revendications 1 à 5, où
A représente O ou NH,
L¹ représente une liaison ou méthylène ou éthane-1,1-diyle,
le cycle Q représente cyclobutyle, cyclopentyle, cyclopentényle, cyclohexyle, pyrrolidinyle, pipéridinyle ou phényle, lesquels peuvent être substitués jusqu'à deux fois, de manière identique ou différente, par fluor, méthyle, hydroxy, et/ou méthoxy,
L² représente (alcane en C₁-C₃)diyle, (alcène en C₂-C₃)diyle ou un groupe de formule *-M-CH₂- ou *-M-CH₂-CH₂- où
* représente le site de liaison avec le cycle Q
et
M représente O ou NH,
Z représente un groupe de formule 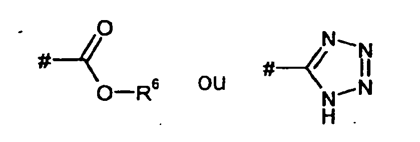 où
# représente le site de liaison avec le groupe L²
et
R⁶ représente hydrogène, méthyle ou éthyle,
R¹ représente un substituant de la série fluor, chlore, méthyle, éthyle, vinyle, trifluorométhyle et méthoxy,
R² représente un substituant de la série fluor, chlore, cyano, méthyle, éthyle, n-propyle, vinyle, trifluorométhyle, méthoxy, éthoxy, trifluorométhoxy, méthylthio, éthylthio, amino, méthylamino et éthylamino,
n et o indépendamment l'un de l'autre représentent le chiffre 0, 1 ou 2, étant entendu que dans le cas où R¹ ou R² sont présents plusieurs fois, leurs significations peuvent être identiques ou différentes,
et
R³ représente hydrogène,
ainsi que ses sels, solvates ou solvates des sels.

7. Procédé de préparation de composés de formule (I) selon les revendications 1 à 6, où Z représente -COOH ou -C(=O)-COOH, **caractérisé en ce qu'**on transforme
[A] des composés de formule (II) 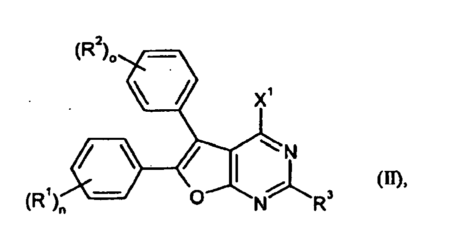 où R¹, R², R³, n et o ont les significations indiquées respectivement dans les revendications 1 à 6
et
X¹ représente un groupe qui part tel que par exemple halogène, notamment chlore,
en présence d'une base le cas échéant dans un solvant inerte au moyen d'un composé de formule (III) 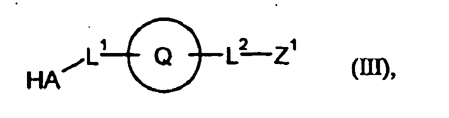 où A, L¹ L² et Q ont respectivement les significations indiquées dans les revendications 1 à 6
et
Z¹ représente cyano ou un groupe de formule -[C(O)]_{y}-COOR^{6A}, où
y représente le chiffre 0 ou 1 et
R^{6A} représente alkyle en C₁-C₄,
en composés de formule (IV) 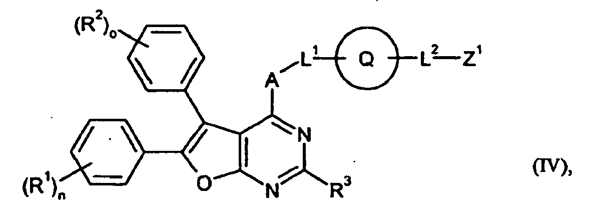 où A, L¹, L², Q, Z¹, R¹, R², R³, n et o ont respectivement les significations indiquées ci-dessus,
ou
qu'on transforme
[B] des composés de formule (V-1) 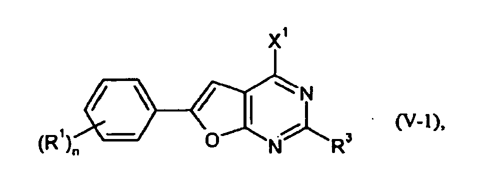 où R¹, R³, X¹ et n ont respectivement les significations indiquées dans les revendications 1 à 6,
en présence d'une base le cas échéant dans un solvant inerte au moyen d'un composé de formule (III) en composés de formule (VI-1) 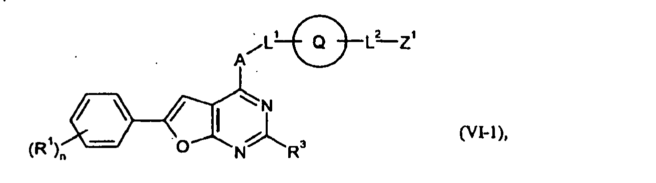 où A, L¹, L², Q, Z¹, R¹, R³ et n ont respectivement les significations indiquées ci-dessus,
qu'on les brome alors dans un solvant inerte pour former des composés de formule (VII-1) 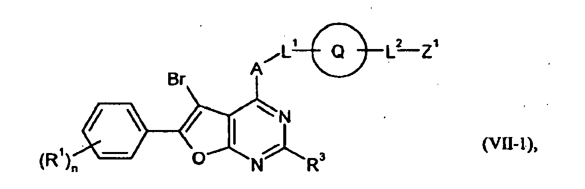 où A, L¹, L², Q, Z¹, R¹, R³ et n ont respectivement les significations indiquées ci-dessus,
et qu'on les couple ensuite dans un solvant inerte en présence d'une base et d'un catalyseur au palladium approprié au moyen d'un acide phénylboronique de formule (VIII-1) 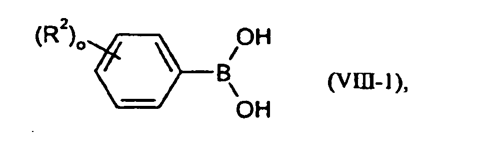 où R² et o ont les significations indiquées dans les revendications 1 à 6,
pour former des composés de formule (IV)
ou
qu'on transforme
[C] des composés de formule (V-2) 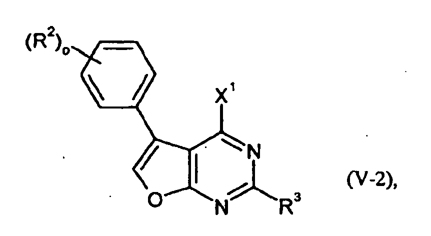 où R², R³, X¹ et o ont respectivement les significations indiquées dans les revendications 1 à 6,
en présence d'une base le cas échéant dans un solvant inerte au moyen d'un composé de formule (III) en composés de formule (VI-2) 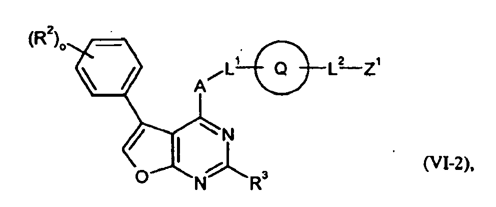 où A, L¹, L², Q, Z¹, R², R³ et o ont respectivement les significations indiquées ci-dessus,
qu'on les brome alors dans un solvant inerte pour former des composés de formule (VII-2) 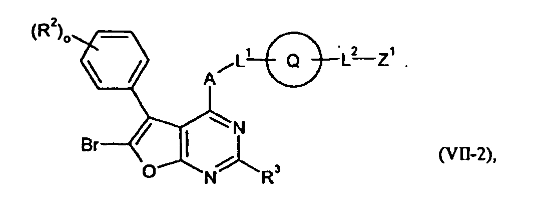 où A, L¹, L², Q, Z¹, R², R³ et o ont respectivement les significations indiquées ci-dessus,
et qu'on les couple ensuite dans un solvant inerte en présence d'une base et d'un catalyseur au palladium approprié au moyen d'un acide phénylboronique de formule (VIII-2) 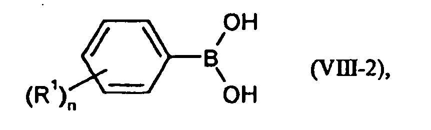 où R¹ et n ont les significations indiquées dans les revendications 1 à 6,
pour former des composés de formule (IV),
et qu'on convertit alors les composés obtenus de formule (IV) par hydrolyse du groupe ester ou cyano Z¹ en acides carboxyliques de formule (I-A) 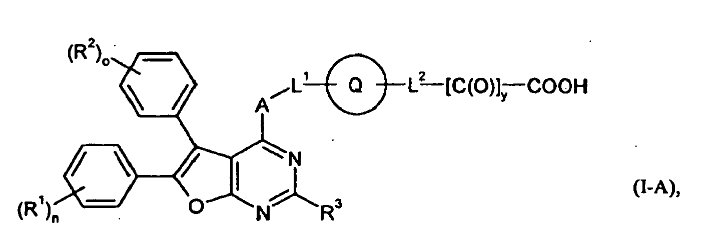 où A, L¹, L², Q, R¹, R², R³, n, o et y ont respectivement les significations indiquées ci-dessus,
et qu'on les transforme le cas échéant au moyen de (i) solvants et/ou (ii) bases ou acides correspondants en leurs solvates, sels et/ou solvates des sels.

8. Composé de formule (I) selon l'une quelconque des revendications 1 à 6, destiné au traitement et/ou à la prévention de maladies.

9. Utilisation d'un composé de formule (I) selon l'une quelconque des revendications 1 à 6 pour la préparation d'un médicament destiné au traitement et/ou à la prévention de maladies cardiovasculaires.

10. Médicament contenant un composé de formule (I) selon l'une quelconque des revendications 1 à 6 en combinaison avec un adjuvant inerte, non toxique, pharmaceutiquement approprié.

11. Médicament contenant un composé de formule (I) selon l'une des revendications 1 à 6 en combinaison avec un autre principe actif.

12. Médicament selon la revendication 10 ou 11 destiné au traitement et/ou à la prévention de maladies cardiovasculaires.
